# EUROPEAN PATENT APPLICATION

(11) **EP 4 834 902 A1**
(43) Date of publication of application: **04.11.2026**
(21) Application number: 24913038.6
(22) Date of filing: 26.12.2024
(51) Int. Cl.: C07D 471/04, A61K 31/437, A61K 31/444, A61K 31/4709, A61K 31/4725, A61K 31/496, A61K 31/5377, A61K 31/551, A61K 31/553, A61P 1/04, A61P 9/10, A61P 19/08, A61P 27/02, A61P 27/04, A61P 27/06, A61P 35/00, A61P 43/00, C07D 519/00

(54) **THIAZOLYL-PYRAZOLO[1,5-A]PYRIDINE COMPOUND AND USE THEREOF AS MYLK4 INHIBITOR**

(30) Priority: 27.12.2023 JP 2023220602
(71) Applicant: Zenyaku Kogyo Co., Ltd., Tokyo 112-8650 (JP)
(72) Inventor: SAITOH Kenichi, Tokyo 112-8650 (JP); HONJO Ayaka, Hachioji-shi, Tokyo 192-0364 (JP); WATANABE Tetsuo, Hachioji-shi, Tokyo 192-0364 (JP); HOTA Kuniyoshi, Hachioji-shi, Tokyo 192-0364 (JP); NAGASAKI Tsubasa, Hachioji-shi, Tokyo 192-0364 (JP); YAMAGUCHI HIGASHI Eika, Hachioji-shi, Tokyo 192-0364 (JP); OHSHITA Ryuichiro, Hachioji-shi, Tokyo 192-0364 (JP); NAKAJIMA Yasutomo, Hachioji-shi, Tokyo 192-0364 (JP); MURATA Yuma, Hachioji-shi, Tokyo 192-0364 (JP)
(74) Representative: J A Kemp LLP
(86) International application number: PCT/JP2024/046238
(87) International publication number: WO 2025/143152

(57) **Abstract**

The present invention provides a novel compound that inhibits MYLK4 activity, and a pharmaceutical use thereof.

Provided are a compound of formula (I) or a pharmaceutically acceptable derivative thereof: 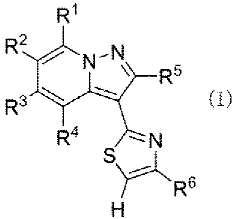 wherein R¹ to R⁵ are each independently hydrogen or a C₁₋₃ chain aliphatic hydrocarbon, and R⁶ is an optionally substituted 5- to 10-membered aromatic hydrocarbon, an optionally substituted 5- to 10-membered heterocycle having 1 to 5 heteroatoms each independently selected from nitrogen, oxygen, and sulfur as a ring-constituting atom(s), or a fused ring in which either the aromatic hydrocarbon or the heterocycle is fused with an optionally substituted 4- to 10-membered heterocyclyl having 1 to 3 heteroatoms each independently selected from nitrogen, oxygen, and sulfur as ring-constituting atoms; and
a pharmaceutical use of the compound or the derivative

## Description

### TECHNICAL FIELD

The present invention relates to a thiazolyl-pyrazolo[1,5-a]pyridine compound and use thereof.

### BACKGROUND ART

Myosin is a motor protein that moves on an actin filament while hydrolyzing ATP, and is a hexamer composed of two heavy chains, two regulatory light chains, and two essential light chains. The ATPase activity of myosin is regulated by phosphorylation and dephosphorylation of the regulatory light chain.

Myosin light chain kinase (MYLK) is involved in the phosphorylation of the regulatory light chain of myosin. It is known that MYLK has four isoforms, MYLK1, MYLK2, MYLK3, and MYLK4, and all of them are considered to be involved in phosphorylation of a regulatory light chain of myosin.

In addition to MYLK, myosin phosphatase (MLCP) is known as a protein that regulates the level of phosphorylation of the regulatory light chain of myosin. MLCP dephosphorylates myosin light chains. The activity of MLCP is regulated by Rho-kinase, which phosphorylates MLCP, thereby reducing the activity of MLCP. That is, Rho-kinase prevents dephosphorylation of the myosin light chain by phosphorylating MLCP, and inhibition of Rho-kinase induces a decrease in the phosphorylated myosin light chain.

Many studies have been conducted on the in vivo roles of MYLK and Rho-kinase, and it has been reported that inhibition of MYLK and Rho-kinase (Rho-kinase inhibitors are also referred to as ROCK inhibitors) is useful for prevention, treatment, or alleviation of various symptoms or diseases (see Non Patent Literatures 1 to 13 and Patent Literature 1, and the tables at the end of the present description).

As MYLK inhibitors, compounds having the following structures referred to as ML-7 or ML-9 have been reported (Non Patent Literatures 2, 3, 7, 10, 11, 12, and 13).

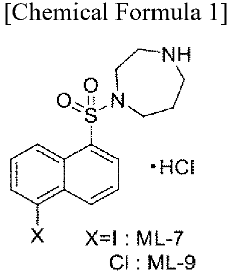

In addition, it has been reported that a compound having the following structure has dual inhibitory activity against MYLK4 and Rho-kinase and reduces intraocular pressure in a rabbit model of high intraocular pressure (Patent Literature 1 and Non Patent Literature 1). 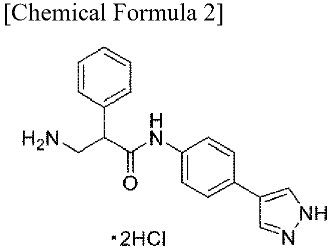 Chemical Formula: C₁₈H₂₀Cl₂N₄O Molecular Weight: 379.2850

### CITATION LIST

### PATENT LITERATURE

PATENT LITERATURE 1: JP 2021-113185 A

### NON PATENT LITERATURE

NON PATENT LITERATURE 1: Invest. Ophthalmol. Vis. Sci. (2021) 62(13), 12.
NON PATENT LITERATURE 2: Exp. Eye Res. (2000) 71(6), 551-566.
NON PATENT LITERATURE 3: Exp. Eye Res. (2005) 80(6), 837-845.
NON PATENT LITERATURE 4: J. Ophthalmol. (2010) 175163.
NON PATENT LITERATURE 5: Invest. Ophthalmol. Vis. Sci. (2015) 56(2), 1335-1348.
NON PATENT LITERATURE 6: Invest. Ophthalmol. Vis. Sci. (2017) 58(12), 5584-5593.
NON PATENT LITERATURE 7: Invest. Ophthalmol. Vis. Sci. (2013) 54(4), 2705-2710.
NON PATENT LITERATURE 8: Curr. Opin. Cell Biol. (2008) 20(2), 242-248.
NON PATENT LITERATURE 9: Int. J. Mol. Sci. (2023) 24(13), 11219.
NON PATENT LITERATURE 10: Dig. Dis. Sci. (2013) 58(1), 107-114.
NON PATENT LITERATURE 11: J. Exp. Clin. Cancer Res. (2021) 40(1), 166.
NON PATENT LITERATURE 12: Mol. Med. Rep. (2015) 12(3), 4109-4116.
NON PATENT LITERATURE 13: J. Proteomics. (2012) 75(17), 5386-5395.

### SUMMARY OF INVENTION

### SOLUTION TO PROBLEM

In the state of the art, the present invention provides a novel compound that inhibits MYLK4 activity, and a pharmaceutical use thereof. Specifically, the present invention provides the following.
[1] A compound of formula (I): 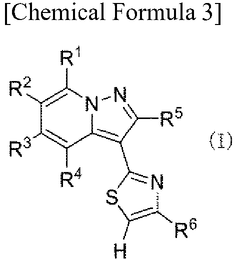
   wherein R¹ to R⁵ are each independently hydrogen or a C₁₋₃ chain aliphatic hydrocarbon; and
   R⁶ is an optionally substituted 5- to 10-membered aromatic hydrocarbon, an optionally substituted 5- to 10-membered heterocycle having 1 to 5 heteroatoms each independently selected from nitrogen, oxygen, and sulfur as ring-constituting atoms, or a fused ring in which either the aromatic hydrocarbon or the heterocycle is fused with an optionally substituted 4- to 10-membered heterocyclyl having 1 to 3 heteroatoms each independently selected from nitrogen, oxygen, and sulfur as ring-constituting atoms,
   or a pharmaceutically acceptable derivative thereof.
[2] The compound or pharmaceutically acceptable derivative thereof according to [1], wherein
   the 5- to 10-membered aromatic hydrocarbon, the 5- to 10-membered heterocycle, and the 4- to 10-membered heterocyclyl forming a fused ring with either the aromatic hydrocarbon or the heterocycle, in R⁶, are each independently unsubstituted, or substituted with a substituent selected from the group consisting of:
      (1) halogen, nitro, cyano, hydroxy, difluoromethyl, trifluoromethyl,
      (2) -NR³¹₂, -N(R³¹)(CH₂)₁₋₅NR³¹₂, -N(R³¹)S(O)₂R³², -N(R³¹)C(O)R³¹, - N(R³¹)C(O)(CH₂)₀₋₃NR³¹₂, -N(R³¹)C(O)(CH₂)₀₋₃R³²,
      (3) C₁₋₆ alkyl, -(CH₂)₁₋₅OR³¹, -(CH₂)₁₋₅O(CH₂)₁₋₅OR³¹, -(CH₂)₁₋₅R³², -(CH₂)₁₋₅R³³,
      (4) -(CH₂)₁₋₅NR³¹₂, -(CH₂)₁₋₅N(R³¹)R³², -(CH₂)₁₋₅N(R³¹)(CH₂)₀₋₅R³⁸, -(CH₂)₁₋₅N(R³¹)C(O)R³¹, -(CH₂)₁₋₅N(R³¹)C(O)(CH₂)₁₋₅OR³¹, -(CH₂)₁₋₅N(C(O)R³¹)(CH₂)₁₋₅C(O)OR³¹, - (CH₂)₁₋₅N(R³¹)C(O)(CH₂)₀₋₅NR³¹₂, -(CH₂)₁₋₅N(R³¹)C(O)R³²,
      (5) -(CH₂)₁₋₅N(R³¹)S(O)₂R³³,
      (6) -C(R³¹)₂C(O)R³²,
      (7) -C(R³¹)₂C(O)NR³¹₂, -C(R³¹)₂C(O)N(R³¹)OR³¹,
      (8) -C(O)R³¹, -C(O)R³²,
      (9) -C(O)OR³¹,
      (10) -C(O)NR³¹₂, -C(O)N(R³¹)(CH₂)₀₋₅R³²,
      (11) -OC(R³¹)₂C(O)N(R³¹)(CH₂)₁₋₅OR³¹, -OC(R³¹)₂C(O)OR³¹, -OC(R³¹)₂C(O)NR³¹₂, - OC(R³¹)₂C(O)R³²,
      (12) -O(CH₂)₁₋₆R³¹, -(O(CH₂)₁₋₅)₁₋₅(CH₂)₀₋₃NR³¹₂, -(O(CH₂)₁₋₅)₁₋₅(CH₂)₀₋₃NR³¹C(O)R³¹, -(O(CH₂)₁₋₅)₁₋₅NR³¹C(NH)NR³¹₂, -O((CH₂)₁₋₅O)₁₋₅R³¹,
      (13) -OC(O)C(R³¹)₂(O(CH₂)₁₋₅)₁₋₅OR³¹, and
      (14) 5- or 6-membered heterocyclyl containing nitrogen as a ring-constituting atom and optionally substituted with C₁₋₃ alkyl;
   R³¹ is each independently hydrogen or a C₁₋₄ chain aliphatic hydrocarbon;
   R³² is an optionally substituted 4- to 10-membered heterocyclyl having 1 to 3 heteroatoms selected from nitrogen, sulfur, and oxygen as ring-constituting atoms, or a fused ring in which the heterocyclyl is fused with an optionally substituted 5- or 6-membered monocyclic aromatic hydrocarbon, where the 4- to 10-membered heterocyclyl forming the fused ring is optionally substituted with a C₁₋₅ chain aliphatic hydrocarbon;
   R³³ is a 3- to 10-membered cycloaliphatic hydrocarbon or a 4- to 10-membered heterocyclyl having 1 to 5 heteroatoms independently selected from nitrogen, oxygen and sulfur as ring-constituting atoms; and
   R³⁸ is cyano, hydroxy, or S(O)₂R³¹.
[3] The compound or pharmaceutically acceptable derivative thereof according to [1], wherein R⁶ is selected from the group consisting of: 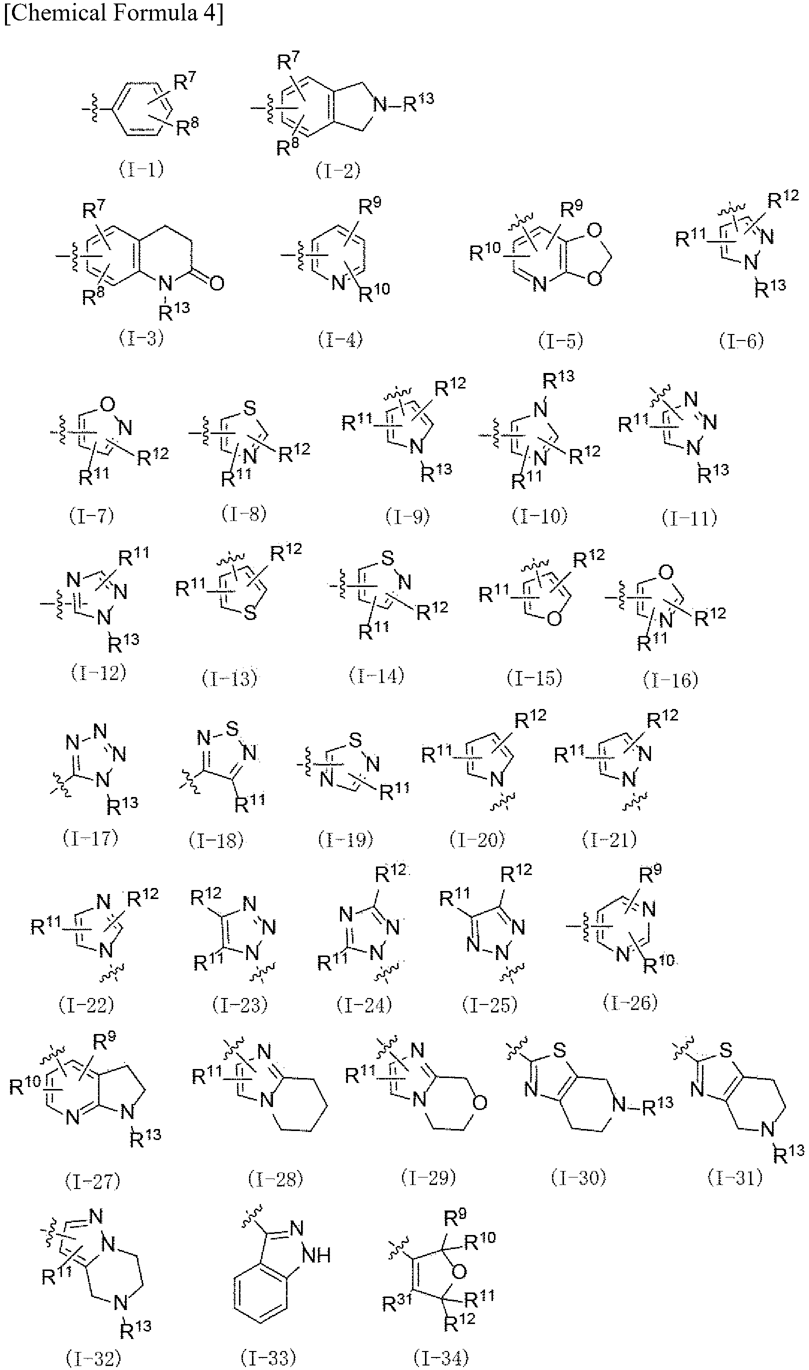 in each formula,
   R⁷ is each independently hydrogen, halogen, nitro, cyano, hydroxy, C₁₋₆ alkyl, C₁₋₆ alkoxy, or -NR³¹₂;
   R⁸ is each independently selected from the group consisting of:
      (1) hydrogen, halogen, nitro, cyano, hydroxy,
      (2) -NR³¹₂, -N(R³¹)S(O)₂R³²⁻¹, where R³²⁻¹ is an optionally substituted 4- to 10-membered heterocyclyl having 1 to 3 heteroatoms selected from nitrogen and oxygen as ring-constituting atoms, -N(R³¹)C(O)R³¹, -N(R³¹)C(O)(CH₂)₀₋₃NR³¹₂, -N(R³¹)C(O)(CH₂)₀₋₃R³²⁻², where R³²⁻² is an optionally substituted 4- to 10-membered heterocyclyl having 1 to 3 heteroatoms selected from nitrogen and oxygen as ring-constituting atoms,
      (3) C₁₋₆ alkyl, -(CH₂)₁₋₅OR³¹, -(CH₂)₁₋₅R³²⁻³, where R³²⁻³ is an optionally substituted 4-to 10-membered heterocyclyl having 1 to 3 heteroatoms selected from nitrogen and oxygen as ring-constituting atoms,
      (4) -(CH₂)₁₋₅NR³¹₂, -(CH₂)₁₋₅N(R³¹)R³²⁻⁴, where R³²⁻⁴ is an optionally substituted 3- to 10-membered cycloaliphatic hydrocarbon or an optionally substituted 4- to 10-membered heterocyclyl having 1 to 3 heteroatoms selected from nitrogen and oxygen as ring-constituting atoms, -(CH₂)₁₋₅N(R³¹)C(O)R³¹, -(CH₂)₁₋₅N(R³¹)C(O)(CH₂)₁₋₅OR³¹, -(CH₂)₁₋₅N(C(O)R³¹)(CH₂)₁₋₅C(O)OR³¹, -(CH₂)₁₋₅N(R³¹)C(O)(CH₂)₀₋₅NR³¹₂, -(CH₂)₁₋₅N(R³¹)C(O)R³²⁻⁵, where R³²⁻⁵ is an optionally substituted 3- to 10-membered cycloaliphatic hydrocarbon or an optionally substituted 4- to 10-membered heterocyclyl having 1 to 3 heteroatoms selected from nitrogen and oxygen as ring-constituting atoms,
      (5) -(CH₂)₁₋₅N(R³¹)S(O)₂R³³⁻¹, where R³³⁻¹ is 3- to 10-membered cycloalkyl,
      (6) -C(R³¹)₂C(O)R³²⁻⁶, where R³²⁻⁶ is an optionally substituted 4- to 10-membered heterocyclyl having 1 to 3 heteroatoms selected from nitrogen and oxygen as ring-constituting atoms,
      (7) -C(R³¹)₂C(O)NR³¹₂, -C(R³¹)₂C(O)N(R³¹)OR³¹,
      (8) -C(O)R³²⁻⁷, where R³²⁻⁷ is an optionally substituted 4- to 10-membered heterocyclyl having 1 to 3 heteroatoms selected from nitrogen and oxygen as ring-constituting atoms, or a fused ring in which the heterocyclyl is fused with an optionally substituted 5- or 6-membered monocyclic aromatic hydrocarbon,
      (9) -C(O)OR³¹,
      (10) -C(O)NR³¹₂, -C(O)N(R³¹)(CH₂)₀₋₅R³²⁻⁸, where R³²⁻⁸ is an optionally substituted 3-to 10-membered cycloaliphatic hydrocarbon or an optionally substituted 4- to 10-membered heterocyclyl having 1 to 3 heteroatoms selected from nitrogen and oxygen as ring-constituting atoms,
      (11) -OC(R³¹)₂C(O)N(R³¹)(CH₂)₁₋₅OR³¹, -OC(R³¹)₂C(O)OR³¹, -OC(R³¹)₂C(O)NR³¹₂, - OC(R³¹)₂C(O)R³²⁻⁹, where R³²⁻⁹ is an optionally substituted 4- to 10-membered heterocyclyl having 1 to 3 heteroatoms selected from nitrogen and sulfur as ring-constituting atoms,
      (12) -O(CH₂)₁₋₆R³¹, -(O(CH₂)₁₋₅)₁₋₅(CH₂)₀₋₃NR³¹₂, -(O(CH₂)₁₋₅)₁₋₅(CH₂)₀₋₃NR³¹C(O)R³¹, -(O(CH₂)₁₋₅)₁₋₅NR³¹C(NH)NR³¹₂, -O((CH₂)₁₋₅O)₁₋₅R³¹,
      (13) -OC(O)C(R³¹)₂(O(CH₂)₁₋₅)₁₋₅OR³¹, and
      (14) 5- or 6-membered nitrogen-containing heterocycle optionally substituted with C₁₋₃ alkyl;
   R⁹, R¹⁰, R¹¹ and R¹² are each independently selected from the group consisting of: hydrogen, halogen, cyano, hydroxy, C₁₋₅ alkyl, 3- to 6-membered cycloalkyl, C₁₋₅ alkoxy, - N(R³¹)C(O)R³¹, -N(R³¹)C(O)(CH₂)₀₋₃NR³¹₂, -N(R³¹)(CH₂)₁₋₅NR³¹₂, -(CH₂)₁₋₅OR³¹, -(CH₂)₁₋₅O(CH₂)₁₋₅OR³¹, -(CH₂)₁₋₅R³⁵, -(CH₂)₁₋₅N(R³¹)C(O)R³¹, -(CH₂)₁₋₅C(O)NR³¹₂, -C(R³¹)₂NR³¹₂, - (CH₂)₁₋₅NR³¹₂, -C(O)R³⁶, -C(O)OR³¹, -C(O)NR³¹₂, -NR³¹₂, -C(H)₂R³⁵, -C(H)R³⁵₂ and -CR³⁵₃;
   R³¹ is each independently hydrogen or a C₁₋₄ chain aliphatic hydrocarbon;
   R³⁵ is each independently halogen, cyano, a 3- to 10-membered cycloaliphatic hydrocarbon optionally substituted with C₁₋₅ alkyl, or -OR³¹;
   R³⁶ is a 4- to 10-membered heterocyclyl having 1 to 3 heteroatoms independently selected from nitrogen, oxygen and sulfur as ring-constituting atoms and optionally substituted with C₁₋₅ alkyl;
   R¹³ is each independently hydrogen, C₁₋₅ alkyl, -(CH₂)₁₋₅OR³¹, -(CH₂)₁₋₅NR³¹₂, -(CH₂)₁₋₅N(R³¹)C(O)R³¹, -(CH₂)₁₋₅N(R³¹)(CH₂)₀₋₅R³⁸, -C(O)R³¹, -C(O)R³⁷ or -(CH₂)₁₋₅R³⁷;
   R³⁷ is a 3- to 10-membered cycloaliphatic hydrocarbon or a 3- to 10-membered heterocyclyl having 1 to 3 heteroatoms selected from nitrogen and oxygen;
   R³⁸ is cyano, hydroxy, or S(O)₂R³¹; and
   the heterocyclic rings in the structures shown in formulas (I-2), (I-3) and (I-27) to (I-32) and the benzene ring in the structure shown in formula (I-33) are each independently optionally substituted with a C₁₋₅ chain aliphatic hydrocarbon.
[4] The compound or pharmaceutically acceptable derivative thereof according to [1], wherein R⁶ is selected from: 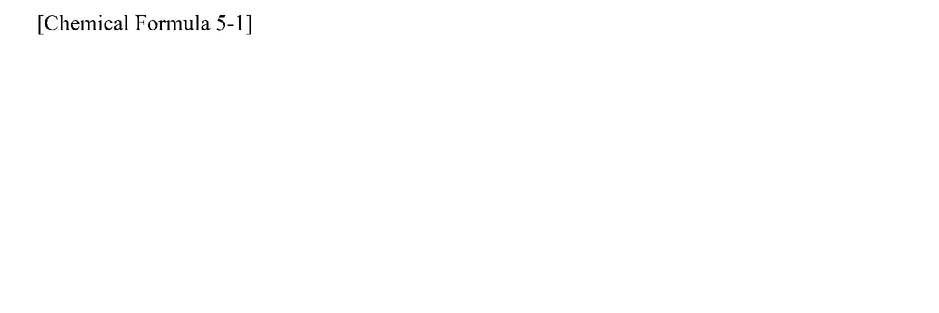 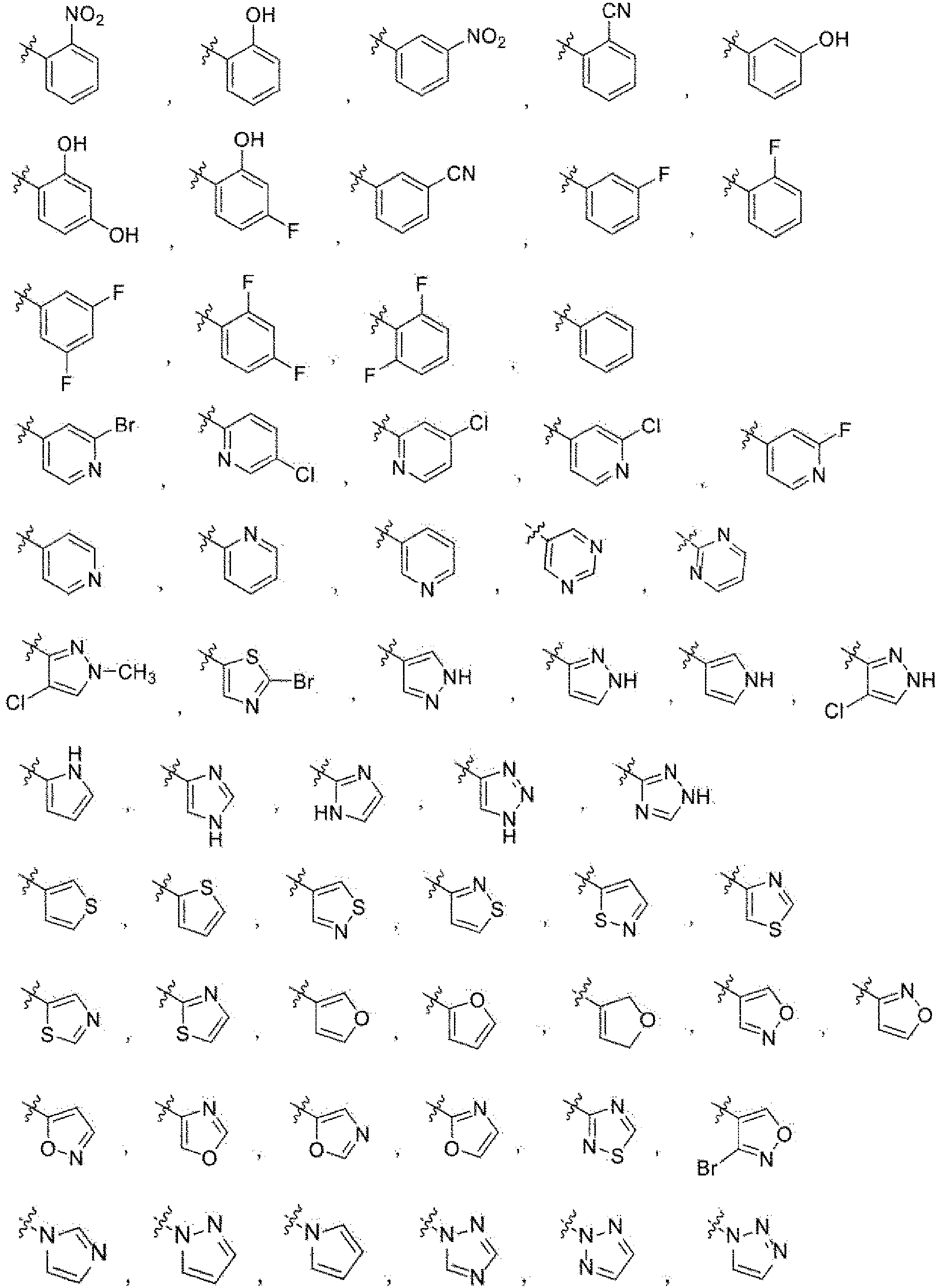 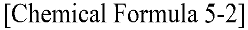 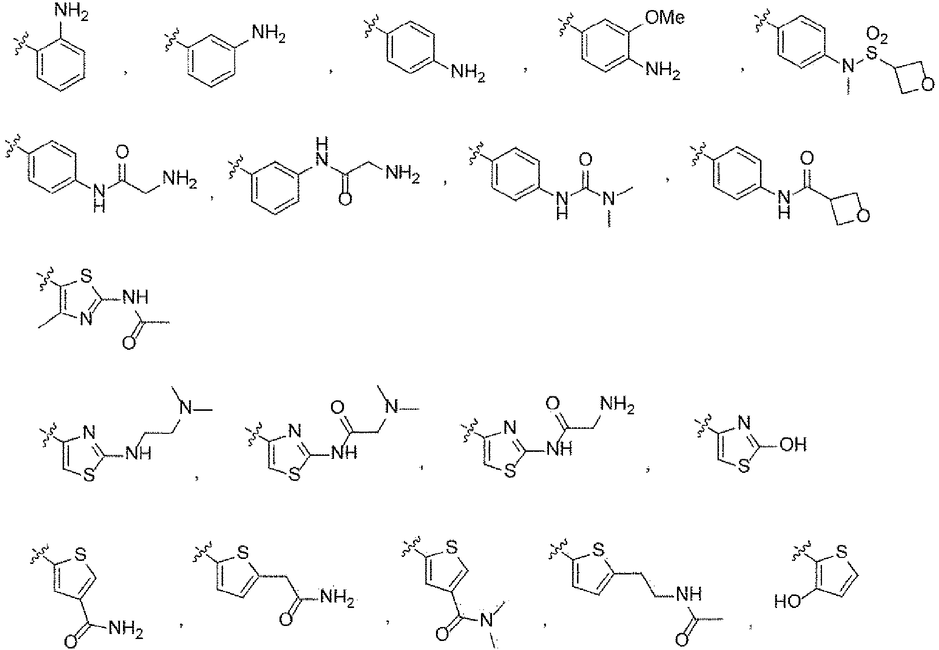 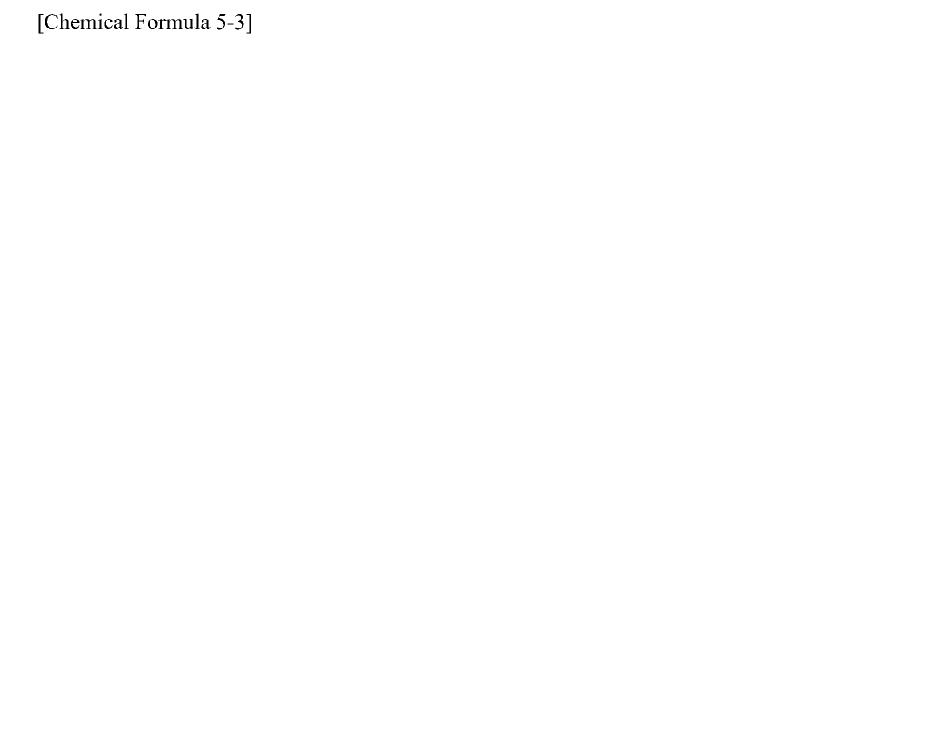 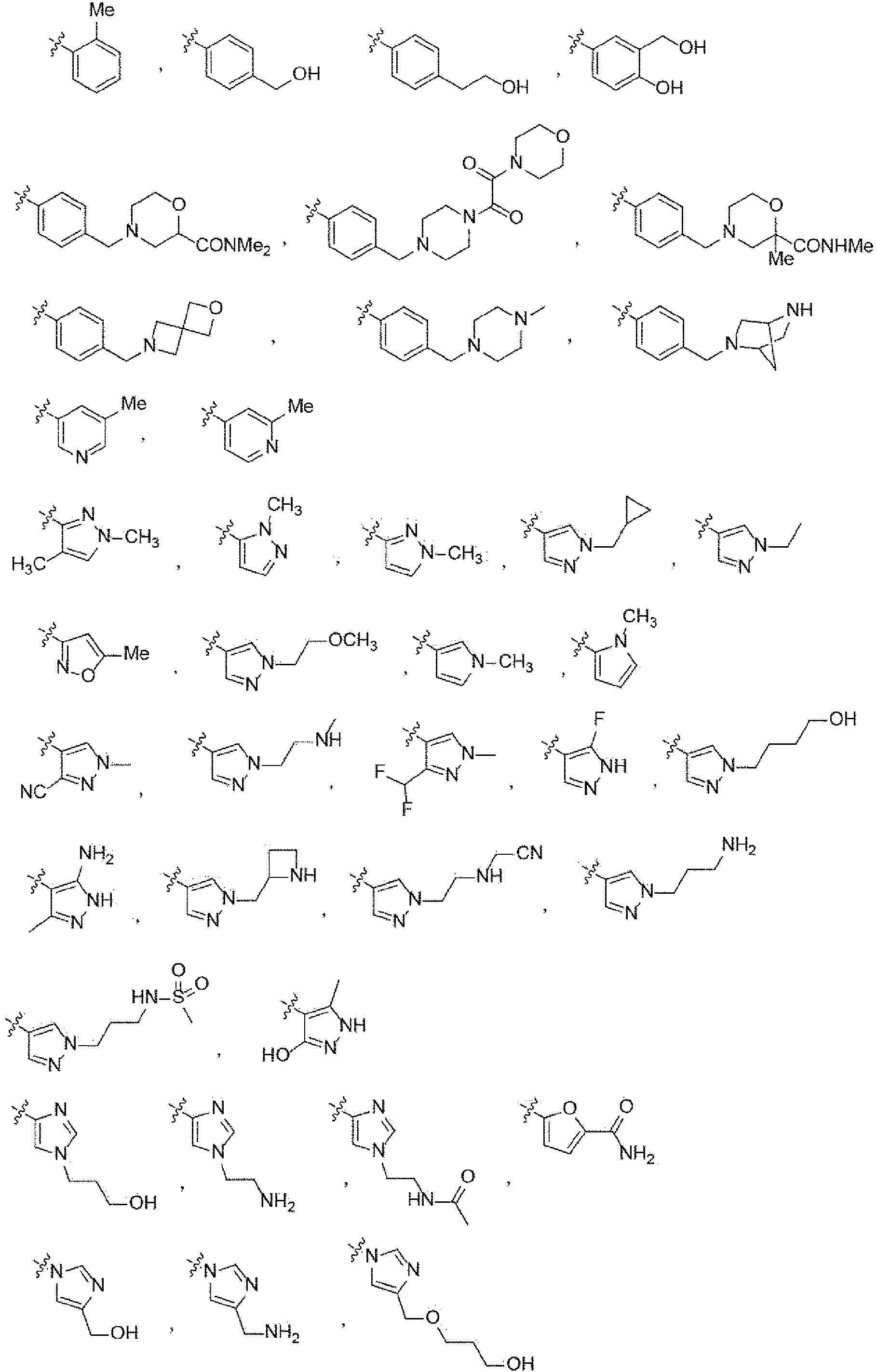 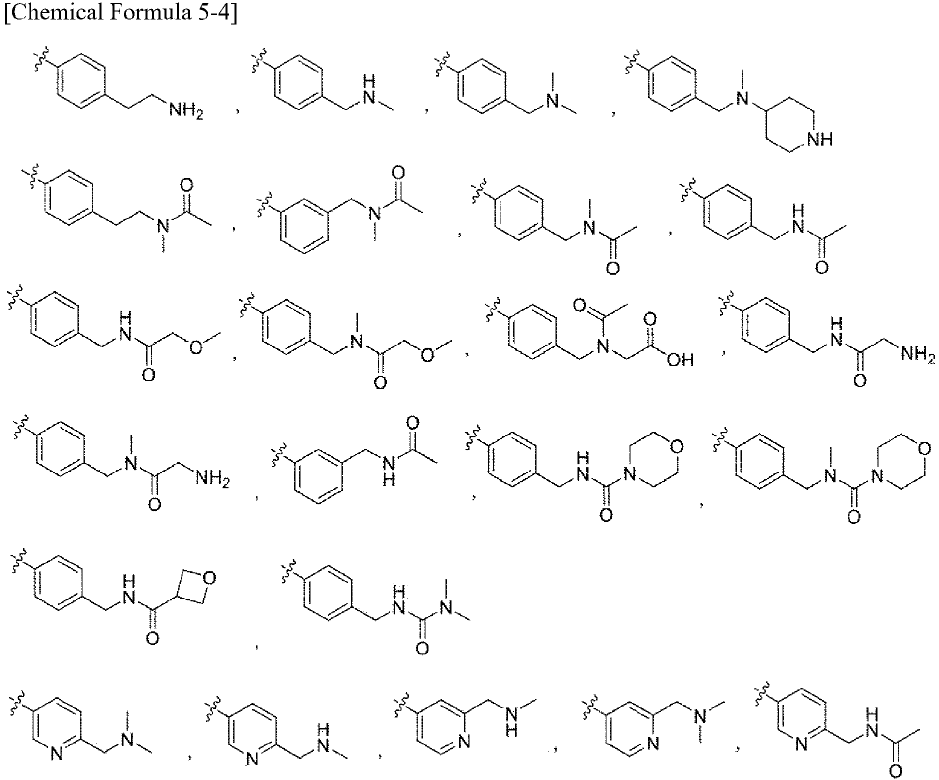 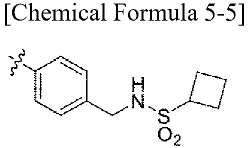 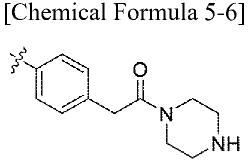 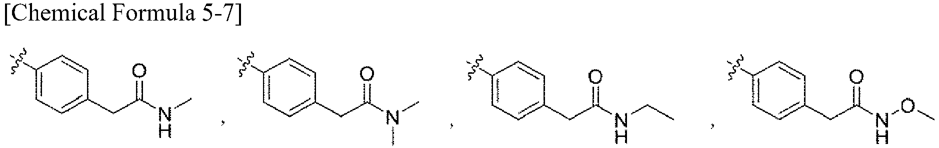 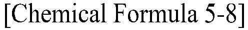 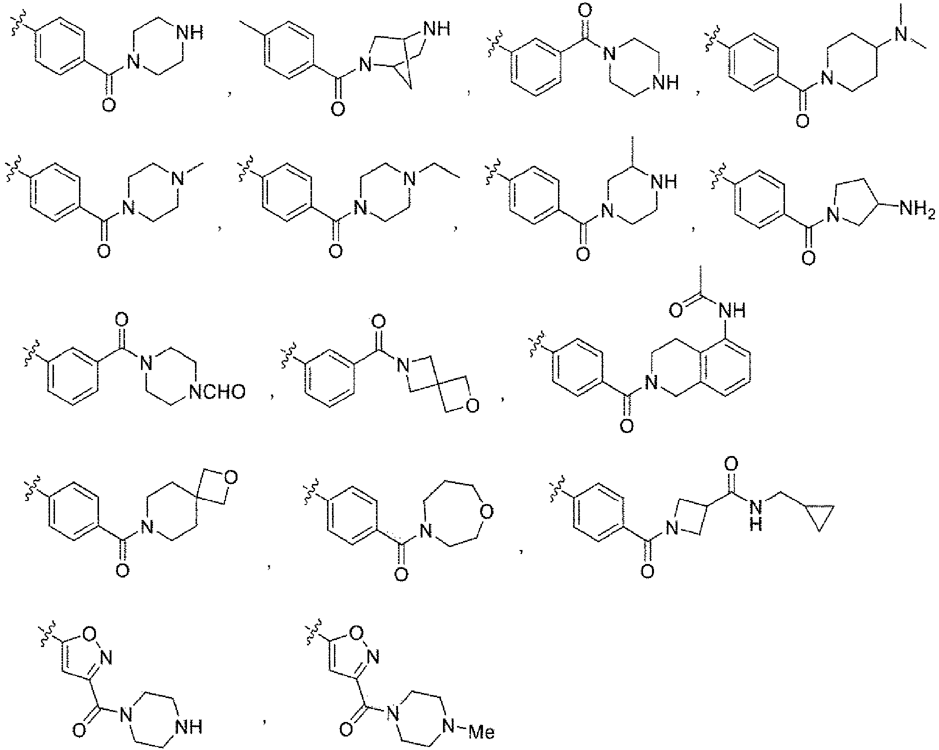 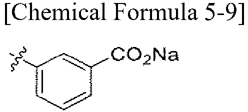 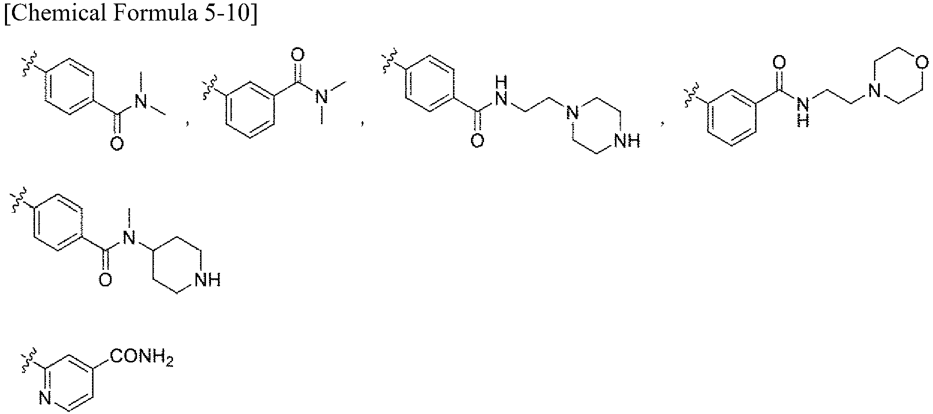 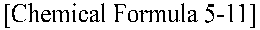 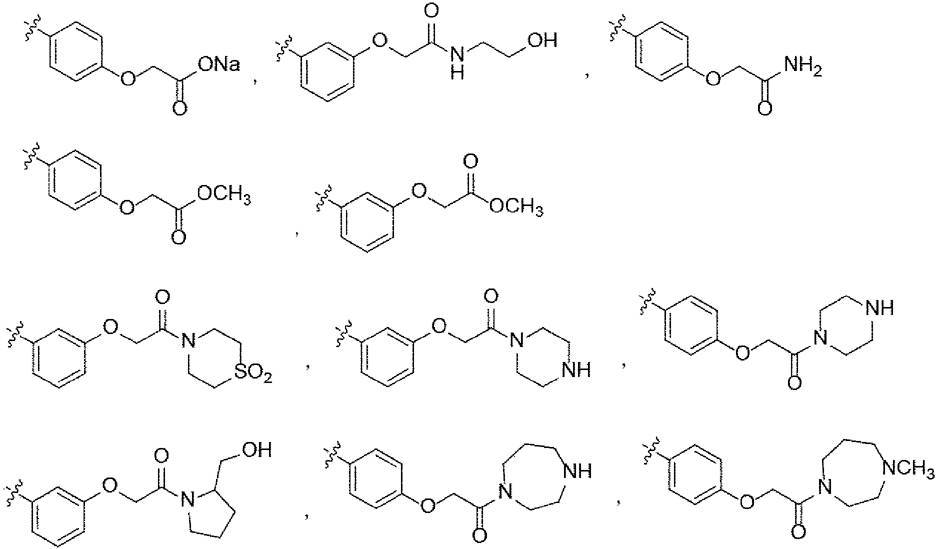 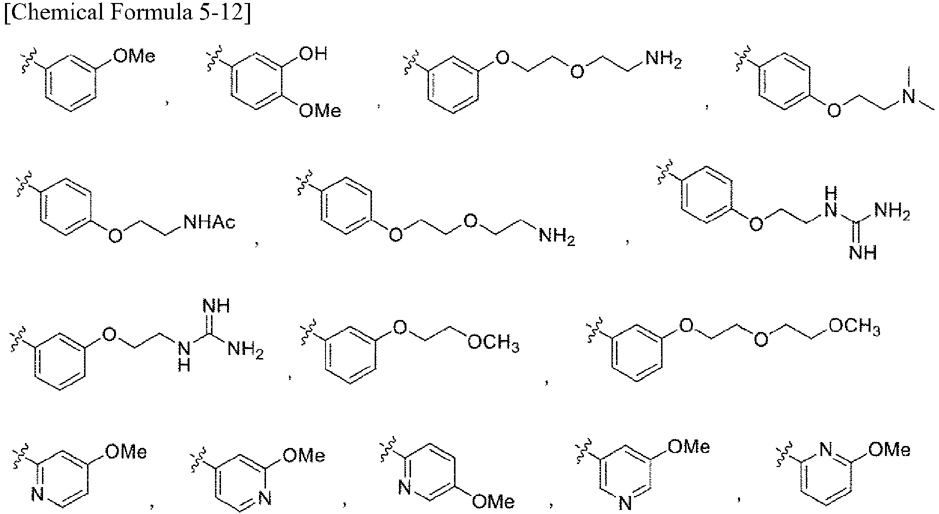 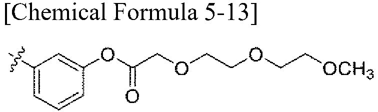 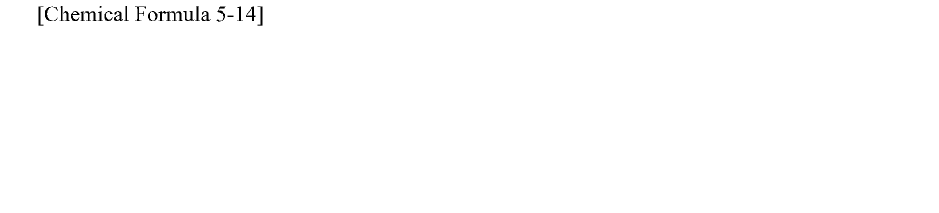 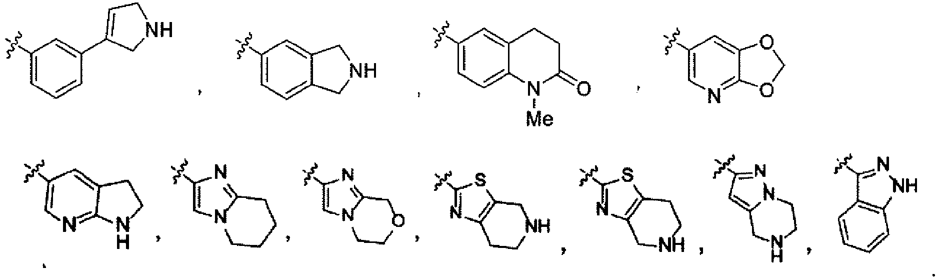
[5] A compound selected from the group consisting of:
   3-(2-(pyrazolo[1,5-a]pyridin-3-yl)thiazol-4-yl)phenol (compound 3),
   4-phenyl-2-(pyrazolo[1,5-a]pyridin-3-yl)thiazole (compound 71),
   2-methoxy-5-(2-(pyrazolo[1,5-a]pyridin-3-yl)thiazol-4-yl)phenol (compound 79),
   4-(3-fluorophenyl)-2-(pyrazolo[1,5-a]pyridin-3-yl)thiazole (compound 112),
   4-(2-fluorophenyl)-2-(pyrazolo[1,5-a]pyridin-3-yl)thiazole (compound 122),
   4-(2-(pyrazolo[1,5-a]pyridin-3-yl)thiazol-4-yl)benzene-1,3-diol (compound 120),
   4-(2,4-difluorophenyl)-2-(pyrazolo[1,5-a]pyridin-3-yl)thiazole (compound 121),
   4-(3-methoxyphenyl)-2-(pyrazolo[1,5-a]pyridin-3-yl)thiazole (compound 83),
   2-(pyrazolo[1,5-a]pyridin-3-yl)-4-(o-tolyl)thiazole (compound 132),
   1-methyl-6-(2-(pyrazolo[1,5-a]pyridin-3-yl)thiazol-4-yl)-3,4-dihydroquinolin-2(1H)-one (compound 131),
   N-(4-(2-(pyrazolo[1,5-a]pyridin-3-yl)thiazol-4-yl)benzyl)acetamide (compound 12),
   4-(4-methoxypyridin-2-yl)-2-(pyrazolo[1,5-a]pyridin-3-yl)thiazole (compound 7),
   2-(pyrazolo[1,5-a]pyridin-3-yl)-4-(pyridin-3-yl)thiazole (compound 15),
   2-(pyrazolo[1,5-a]pyridin-3-yl)-4-(pyridin-4-yl)thiazole (compound 92),
   2-(pyrazolo[1,5-a]pyridin-3-yl)-4-(pyridin-2-yl)thiazole (compound 93),
   4-(2-chloropyridin-4-yl)-2-(pyrazolo[1,5-a]pyridin-3-yl)thiazole (compound 94),
   4-(2-methoxypyridin-4-yl)-2-(pyrazolo[1,5-a]pyridin-3-yl)thiazole (compound 95),
   4-(6-methoxypyridin-2-yl)-2-(pyrazolo[1,5-a]pyridin-3-yl)thiazole (compound 96),
   4-(5-methylpyridin-3-yl)-2-(pyrazolo[1,5-a]pyridin-3-yl)thiazole (compound 97),
   6-(2-(pyrazolo[1,5-a]pyridin-3-yl)thiazol-4-yl)-[1,3]dioxolo[4,5-b]pyridine (compound 98),
   4-(2-methylpyridin-4-yl)-2-(pyrazolo[1,5-a]pyridin-3-yl)thiazole (compound 99),
   4-(2-fluoropyridin-4-yl)-2-(pyrazolo[1,5-a]pyridin-3-yl)thiazole (compound 100),
   4-(2-bromopyridin-4-yl)-2-(pyrazolo[1,5-a]pyridin-3-yl)thiazole (compound 101),
   4-(5-chloropyridin-2-yl)-2-(pyrazolo[1,5-a]pyridin-3-yl)thiazole (compound 125),
   4-(4-chloropyridin-2-yl)-2-(pyrazolo[1,5-a]pyridin-3-yl)thiazole (compound 135),
   2'-bromo-2-(pyrazolo[1,5-a]pyridin-3-yl)-4,5'-bithiazole (compound 37),
   4-(2-(pyrazolo[1,5-a]pyridin-3-yl)thiazol-4-yl)aniline hydrochloride (compound 1),
   4-(3-nitrophenyl)-2-(pyrazolo[1,5-a]pyridin-3-yl)thiazole (compound 4),
   3-(2-(pyrazolo[1,5-a]pyridin-3-yl)thiazol-4-yl)aniline hydrochloride (compound 10),
   4-(2-nitrophenyl)-2-(pyrazolo[1,5-a]pyridin-3-yl)thiazole (compound 133),
   2-(2-(pyrazolo[1,5-a]pyridin-3-yl)thiazol-4-yl)aniline hydrochloride (compound 134),
   2-methoxy-4-(2-(pyrazolo[1,5-a]pyridin-3-yl)thiazol-4-yl)aniline (compound 90),
   2-(2-(pyrazolo[1,5-a]pyridin-3-yl)thiazol-4-yl)phenol (compound 118),
   3-(2-(pyrazolo[1,5-a]pyridin-3-yl)thiazol-4-yl)benzonitrile (compound 115),
   2-(2-(pyrazolo[1,5-a]pyridin-3-yl)thiazol-4-yl)benzonitrile (compound 116),
   4-(3,5-difluorophenyl)-2-(pyrazolo[1,5-a]pyridin-3-yl)thiazole (compound 119),
   4-(2,6-difluorophenyl)-2-(pyrazolo[1,5-a]pyridin-3-yl)thiazole (compound 123),
   5-fluoro-2-(2-(pyrazolo[1,5-a]pyridin-3-yl)thiazol-4-yl)phenol (compound 114),
   2-(2-(pyrazolo[1,5-a]pyridin-3-yl)thiazol-4-yl)isonicotinamide (compound 126),
   4-(5-methoxypyridin-3-yl)-2-(pyrazolo[1,5-a]pyridin-3-yl)thiazole (compound 124),
   4-(5-methoxypyridin-2-yl)-2-(pyrazolo[1,5-a]pyridin-3-yl)thiazole (compound 127),
   4-(1H-pyrazol-4-yl)-2-(pyrazolo[1,5-a]pyridin-3-yl)thiazole (compound 107),
   4-(4-chloro-1-methyl-1H-pyrazol-3-yl)-2-(pyrazolo[1,5-a]pyridin-3-yl)thiazole (compound 53),
   4-(1,4-dimethyl-1H-pyrazol-3-yl)-2-(pyrazolo[1,5-a]pyridin-3-yl)thiazole hydrobromide (compound 54),
   4-(1-methyl-1H-pyrazol-5-yl)-2-(pyrazolo[1,5-a]pyridin-3-yl)thiazole hydrobromide (compound 106),
   4-(1H-pyrazol-3-yl)-2-(pyrazolo[1,5-a]pyridin-3-yl)thiazole (compound 109),
   5-methyl-3-(2-(pyrazolo[1,5-a]pyridin-3-yl)thiazol-4-yl)isoxazole hydrobromide (compound 105),
   N-methyl-N-(4-(2-(pyrazolo[1,5-a]pyridin-3-yl)thiazol-4-yl)phenyl)oxetane-3-sulfonamide (compound 69),
   N-methyl-N-(4-(2-(pyrazolo[1,5-a]pyridin-3-yl)thiazol-4-yl)benzyl)acetamide (compound 16),
   N-(4-(2-(pyrazolo[1,5-a]pyridin-3-yl)thiazol-4-yl)benzyl)morpholine-4-carboxamide (compound 72),
   N-methyl-1-(4-(2-(pyrazolo[1,5-a]pyridin-3-yl)thiazol-4-yl)phenyl)methanamine hydrochloride (compound 74),
   N-methyl-N-(4-(2-(pyrazolo[1,5-a]pyridin-3-yl)thiazol-4-yl)benzyl)morpholine-4-carboxamide (compound 76),
   2-methoxy-N-methyl-N-(4-(2-(pyrazolo[1,5-a]pyridin-3-yl)thiazol-4-yl)benzyl)acetamide (compound 78),
   1,1-dimethyl-3-(4-(2-(pyrazolo[1,5-a]pyridin-3-yl)thiazol-4-yl)phenyl)urea (compound 59),
   1,1-dimethyl-3-(4-(2-(pyrazolo[1,5-a]pyridin-3-yl)thiazol-4-yl)benzyl)urea (compound 75),
   2-methoxy-N-(4-(2-(pyrazolo[1,5-a]pyridin-3-yl)thiazol-4-yl)benzyl)acetamide (compound 77),
   N-(4-(2-(pyrazolo[1,5-a]pyridin-3-yl)thiazol-4-yl)benzyl)cyclobutanesulfonamide (compound 82),
   N-(4-(2-(pyrazolo[1,5-a]pyridin-3-yl)thiazol-4-yl)benzyl)oxetane-3-carboxamide (compound 85),
   2-amino-N-(4-(2-(pyrazolo[1,5-a]pyridin-3-yl)thiazol-4-yl)phenyl)acetamide hydrochloride (compound 64),
   N-(4-(2-(pyrazolo[1,5-a]pyridin-3-yl)thiazol-4-yl)phenyl)oxetane-3-carboxamide (compound 66),
   2-amino-N-(3-(2-(pyrazolo[1,5-a]pyridin-3-yl)thiazol-4-yl)phenyl)acetamide hydrochloride (compound 65),
   2-amino-N-methyl-N-(4-(2-(pyrazolo[1,5-a]pyridin-3-yl)thiazol-4-yl)benzyl)acetamide hydrochloride (compound 81),
   2-amino-N-(4-(2-(pyrazolo[1,5-a]pyridin-3-yl)thiazol-4-yl)benzyl)acetamide hydrochloride (compound 80),
   sodium 2-(4-(2-(pyrazolo[1,5-a]pyridin-3-yl)thiazol-4-yl)phenoxy)acetate (compound 5),
   N-acetyl-N-(4-(2-(pyrazolo[1,5-a]pyridin-3-yl)thiazol-4-yl)benzyl)glycine (compound 73),
   2-(4-(2-(pyrazolo[1,5-a]pyridin-3-yl)thiazol-4-yl)phenoxy)acetamide (compound 26),
   3-(2-(pyrazolo[1,5-a]pyridin-3-yl)thiazol-4-yl)phenyl-2-(2-(2-methoxyethoxy)ethoxy)acetate (compound 13),
   4-(3-(2-methoxyethoxy)phenyl)-2-(pyrazolo[1,5-a]pyridin-3-yl)thiazole (compound 14),
   4-(3-(2-(2-methoxyethoxy)ethoxy)phenyl)-2-(pyrazolo[1,5-a]pyridin-3-yl)thiazole (compound 22),
   4-(1-(cyclopropylmethyl)-1H-pyrazol-4-yl)-2-(pyrazolo[1,5-a]pyridin-3-yl)thiazole (compound 108),
   4-(1-ethyl-1H-pyrazol-4-yl)-2-(pyrazolo[1,5-a]pyridin-3-yl)thiazole (compound 110),
   4-(1-(2-methoxyethyl)-1H-pyrazol-4-yl)-2-(pyrazolo[1,5-a]pyridin-3-yl)thiazole hydrochloride (compound 111),
   2-(2-(4-(2-(pyrazolo[1,5-a]pyridin-3-yl)thiazol-4-yl)phenoxy)ethoxy)ethan-1-amine hydrochloride (compound 32),
   2-(2-(3-(2-(pyrazolo[1,5-a]pyridin-3-yl)thiazol-4-yl)phenoxy)ethoxy)ethan-1-amine hydrochloride (compound 20),
   N,N-dimethyl-2-(4-(2-(pyrazolo[1,5-a]pyridin-3-yl)thiazol-4-yl)phenoxy)ethan-1-amine hydrochloride (compound 47),
   1-(2-(3-(2-(pyrazolo[1,5-a]pyridin-3-yl)thiazol-4-yl)phenoxy)ethyl)guanidine hydrochloride (compound 52),
   1-(2-(4-(2-(pyrazolo[1,5-a]pyridin-3-yl)thiazol-4-yl)phenoxy)ethyl)guanidine hydrochloride (compound 51),
   N-(2-(4-(2-(pyrazolo[1,5-a]pyridin-3-yl)thiazol-4-yl)phenoxy)ethyl)acetamide (compound 45),
   N-(2-hydroxyethyl)-2-(3-(2-(pyrazolo[1,5-a]pyridin-3-yl)thiazol-4-yl)phenoxy)acetamide (compound 19),
   methyl 2-(3-(2-(pyrazolo[1,5-a]pyridin-3-yl)thiazol-4-yl)phenoxy)acetate (compound 18),
   methyl 2-(4-(2-(pyrazolo[1,5-a]pyridin-3-yl)thiazol-4-yl)phenoxy)acetate (compound 25),
   1-(piperazin-1-yl)-2-(4-(2-(pyrazolo[1,5-a]pyridin-3-yl)thiazol-4-yl)phenoxy)ethan-1-one (compound 31),
   1-(1,4-diazepan-1-yl)-2-(4-(2-(pyrazolo[1,5-a]pyridin-3-yl)thiazol-4-yl)phenoxy)ethan-1-one hydrochloride (compound 55),
   1-(4-methyl-1,4-diazepan-1-yl)-2-(4-(2-(pyrazolo[1,5-a]pyridin-3-yl)thiazol-4-yl)phenoxy)ethan-1-one hydrochloride (compound 102),
   1-(2-(hydroxymethyl)pyrrolidin-1-yl)-2-(3-(2-(pyrazolo[1,5-a]pyridin-3-yl)thiazol-4-yl)phenoxy)ethan-1-one (compound 21),
   1-(piperazin-1-yl)-2-(3-(2-(pyrazolo[1,5-a]pyridin-3-yl)thiazol-4-yl)phenoxy)ethan-1-one (compound 23),
   1-(1,1-dioxidothiomorpholino)-2-(3-(2-(pyrazolo[1,5-a]pyridin-3-yl)thiazol-4-yl)phenoxy)ethan-1-one (compound 24),
   (4-methylpiperazin-1-yl)(5-(2-(pyrazolo[1,5-a]pyridin-3-yl)thiazol-4-yl)isoxazol-3-yl)methanone hydrochloride (compound 103),
   piperazin-1-yl(5-(2-(pyrazolo[1,5-a]pyridin-3-yl)thiazol-4-yl)isoxazol-3-yl)methanone hydrochloride (compound 104),
   N-(4'-methyl-2-(pyrazolo[1,5-a]pyridin-3-yl)-[4,5'-bithiazole]-2'-yl)acetamide (compound 46),
   N,N-dimethyl-4-(2-(pyrazolo[1,5-a]pyridin-3-yl)thiazol-4-yl)benzamide (compound 49),
   sodium 3-(2-(pyrazolo[1,5-a]pyridin-3-yl)thiazol-4-yl)benzoate (compound 6),
   (3-(2-(pyrazolo[1,5-a]pyridin-3-yl)thiazol-4-yl)phenyl)(2-oxa-6-azaspiro[3.3]heptan-6-yl)methanone (compound 33),
   N,N-dimethyl-2-(4-(2-(pyrazolo[1,5-a]pyridin-3-yl)thiazol-4-yl)phenyl)acetamide (compound 87),
   N-(cyclopropylmethyl)-1-(4-(2-(pyrazolo[1,5-a]pyridin-3-yl)thiazol-4-yl)benzoyl)azetidine-3-carboxamide (compound 9),
   (4-(2-(pyrazolo[1,5-a]pyridin-3-yl)thiazol-4-yl)phenyl)(2-oxa-7-azaspiro[3.5]nonan-7-yl)methanone (compound 40),
   (1,4-oxazepan-4-yl)(4-(2-(pyrazolo[1,5-a]pyridin-3-yl)thiazol-4-yl)phenyl)methanone (compound 41),
   (4-(dimethylamino)piperidin-1-yl)(4-(2-(pyrazolo[1,5-a]pyridin-3-yl)thiazol-4-yl)phenyl)methanone (compound 48),
   (4-methylpiperazin-1-yl)(4-(2-(pyrazolo[1,5-a]pyridin-3-yl)thiazol-4-yl)phenyl)methanone (compound 50),
   (4-ethylpiperazin-1-yl)(4-(2-(pyrazolo[1,5-a]pyridin-3-yl)thiazol-4-yl)phenyl)methanone (compound 56),
   (3-methylpiperazin-1-yl)(4-(2-(pyrazolo[1,5-a]pyridin-3-yl)thiazol-4-yl)phenyl)methanone (compound 58),
   N,N-dimethyl-3-(2-(pyrazolo[1,5-a]pyridin-3-yl)thiazol-4-yl)benzamide (compound 61),
   4-(3-(2-(pyrazolo[1,5-a]pyridin-3-yl)thiazol-4-yl)benzoyl)piperazine-1-carbaldehyde (compound 68),
   N-(2-morpholinoethyl)-3-(2-(pyrazolo[1,5-a]pyridin-3-yl)thiazol-4-yl)benzamide (compound 30),
   N-methyl-2-(4-(2-(pyrazolo[1,5-a]pyridin-3-yl)thiazol-4-yl)phenyl)acetamide (compound 88),
   N-ethyl-2-(4-(2-(pyrazolo[1,5-a]pyridin-3-yl)thiazol-4-yl)phenyl)acetamide (compound 89),
   N-methoxy-2-(4-(2-(pyrazolo[1,5-a]pyridin-3-yl)thiazol-4-yl)phenyl)acetamide (compound 91),
   (2,5-diazabicyclo[2.2.1]heptan-2-yl)(4-(2-(pyrazolo[1,5-a]pyridin-3-yl)thiazol-4-yl)phenyl)methanone hydrochloride (compound 43),
   N-(2-(piperazin-1-yl)ethyl)-4-(2-(pyrazolo[1,5-a]pyridin-3-yl)thiazol-4-yl)benzamide hydrochloride (compound 11),
   (3-aminopyrrolidin-1-yl)(4-(2-(pyrazolo[1,5-a]pyridin-3-yl)thiazol-4-yl)phenyl)methanone hydrochloride (compound 42),
   N-methyl-N-(piperidin-4-yl)-4-(2-(pyrazolo[1,5-a]pyridin-3-yl)thiazol-4-yl)benzamide hydrochloride (compound 44),
   piperazin-1-yl(4-(2-(pyrazolo[1,5-a]pyridin-3-yl)thiazol-4-yl)phenyl)methanone hydrochloride (compound 57),
   piperazin-1-yl(3-(2-(pyrazolo[1,5-a]pyridin-3-yl)thiazol-4-yl)phenyl)methanone hydrochloride (compound 63),
   1-(piperazin-1-yl)-2-(4-(2-(pyrazolo[1,5-a]pyridin-3-yl)thiazol-4-yl)phenyl)ethan-1-one hydrochloride (compound 86),
   N-(2-(4-(2-(pyrazolo[1,5-a]pyridin-3-yl)thiazol-4-yl)benzoyl)-1,2,3,4-tetrahydroisoquinolin-5-yl)acetamide (compound 60),
   2-(hydroxymethyl)-4-(2-(pyrazolo[1,5-a]pyridin-3-yl)thiazol-4-yl)phenol (compound 8),
   (4-(2-(pyrazolo[1,5-a]pyridin-3-yl)thiazol-4-yl)phenyl)methanol (compound 2),
   2-(4-(2-(pyrazolo[1,5-a]pyridin-3-yl)thiazol-4-yl)phenyl)ethan-1-ol (compound 84),
   4-(3-(2,5-dihydro-1H-pyrrol-3-yl)phenyl)-2-(pyrazolo[1,5-a]pyridin-3-yl)thiazole hydrochloride (compound 17),
   6-(4-(2-(pyrazolo[1,5-a]pyridin-3-yl)thiazol-4-yl)benzyl)-2-oxa-6-azaspiro[3.3]heptane (compound 29),
   N,N-dimethyl-1-(4-(2-(pyrazolo[1,5-a]pyridin-3-yl)thiazol-4-yl)phenyl)methanamine (compound 27),
   4-(4-((4-methylpiperazin-1-yl)methyl)phenyl)-2-(pyrazolo[1,5-a]pyridin-3-yl)thiazole (compound 28),
   N,N-dimethyl-4-(4-(2-(pyrazolo[1,5-a]pyridin-3-yl)thiazol-4-yl)benzyl)morpholine-2-carboxamide (compound 38),
   N,2-dimethyl-4-(4-(2-(pyrazolo[1,5-a]pyridin-3-yl)thiazol-4-yl)benzyl)morpholine-2-carboxamide (compound 39),
   1-morpholino-2-(4-(4-(2-(pyrazolo[1,5-a]pyridin-3-yl)thiazol-4-yl)benzyl)piperazin-1-yl)ethane-1,2-dione hydrochloride (compound 36),
   4-(4-((2,5-diazabicyclo[2.2.1]heptan-2-yl)methyl)phenyl)-2-(pyrazolo[1,5-a]pyridin-3-yl)thiazole hydrochloride (compound 34),
   N-methyl-N-(4-(2-(pyrazolo[1,5-a]pyridin-3-yl)thiazol-4-yl)benzyl)piperidin-4-amine hydrochloride (compound 35),
   4-(isoindolin-5-yl)-2-(pyrazolo[1,5-a]pyridin-3-yl)thiazole hydrochloride (compound 117),
   N-methyl-N-(4-(2-(pyrazolo[1,5-a]pyridin-3-yl)thiazol-4-yl)phenethyl)acetamide (compound 62),
   2-(4-(2-(pyrazolo[1,5-a]pyridin-3-yl)thiazol-4-yl)phenyl)ethan-1-amine hydrochloride (compound 113),
   N-(3-(2-(pyrazolo[1,5-a]pyridin-3-yl)thiazol-4-yl)benzyl)acetamide (compound 67),
   N-methyl-N-(3-(2-(pyrazolo[1,5-a]pyridin-3-yl)thiazol-4-yl)benzyl)acetamide (compound 70),
   N,N-dimethyl-1-(4-(2-(pyrazolo[1,5-a]pyridin-3-yl)thiazol-4-yl)pyridin-2-yl)methanamine (compound 137),
   N,N-dimethyl-1-(5-(2-(pyrazolo[1,5-a]pyridin-3-yl)thiazol-4-yl)pyridin-2-yl)methanamine (compound 128),
   N-methyl-1-(4-(2-(pyrazolo[1,5-a]pyridin-3-yl)thiazol-4-yl)pyridin-2-yl)methanamine (compound 136),
   N-((5-(2-(pyrazolo[1,5-a]pyridin-3-yl)thiazol-4-yl)pyridin-2-yl)methyl)acetamide (compound 129),
   N-methyl-1-(5-(2-(pyrazolo[1,5-a]pyridin-3-yl)thiazol-4-yl)pyridin-2-yl)methanamine (compound 130),
   2-(pyrazolo[1,5-a]pyridin-3-yl)-4-(1H-pyrrol-3-yl)thiazole (compound 138),
   2-(pyrazolo[1,5-a]pyridin-3-yl)-4-(1H-pyrrol-2-yl)thiazole (compound 139),
   4-(1H-imidazol-4-yl)-2-(pyrazolo[1,5-a]pyridin-3-yl)thiazole (compound 140),
   4-(1H-imidazol-2-yl)-2-(pyrazolo[1,5-a]pyridin-3-yl)thiazole (compound 141),
   2-(pyrazolo[1,5-a]pyridin-3-yl)-4-(1H-1,2,3-triazol-4-yl)thiazole (compound 142),
   2-(pyrazolo[1,5-a]pyridin-3-yl)-4-(1H-1,2,4-triazol-3-yl)thiazole (compound 143),
   2-(pyrazolo[1,5-a]pyridin-3-yl)-4-(thiophen-3-yl)thiazole (compound 144),
   2-(pyrazolo[1,5-a]pyridin-3-yl)-4-(thiophen-2-yl)thiazole (compound 145),
   3-(2-(pyrazolo[1,5-a]pyridin-3-yl)thiazol-4-yl)isothiazole (compound 146),
   4-(2-(pyrazolo[1,5-a]pyridin-3-yl)thiazol-4-yl)isothiazole (compound 147),
   5-(2-(pyrazolo[1,5-a]pyridin-3-yl)thiazol-4-yl)isothiazole (compound 148),
   2-(pyrazolo[1,5-a]pyridin-3-yl)-4,4'-bithiazole (compound 149),
   2-(pyrazolo[1,5-a]pyridin-3-yl)-4,5'-bithiazole (compound 150),
   2'-(pyrazolo[1,5-a]pyridin-3-yl)-2,4'-bithiazole (compound 151),
   4-(furan-3-yl)-2-(pyrazolo[1,5-a]pyridin-3-yl)thiazole (compound 152),
   4-(furan-2-yl)-2-(pyrazolo[1,5-a]pyridin-3-yl)thiazole (compound 153),
   4-(2-(pyrazolo[1,5-a]pyridin-3-yl)thiazol-4-yl)isoxazole (compound 154),
   3-(2-(pyrazolo[1,5-a]pyridin-3-yl)thiazol-4-yl)isoxazole (compound 155),
   5-(2-(pyrazolo[1,5-a]pyridin-3-yl)thiazol-4-yl)isoxazole (compound 156),
   4-(2-(pyrazolo[1,5-a]pyridin-3-yl)thiazol-4-yl)oxazole (compound 157),
   5-(2-(pyrazolo[1,5-a]pyridin-3-yl)thiazol-4-yl)oxazole (compound 158),
   2-(2-(pyrazolo[1,5-a]pyridin-3-yl)thiazol-4-yl)oxazole (compound 159),
   4-(1-methyl-1H-pyrazol-3-yl)-2-(pyrazolo[1,5-a]pyridin-3-yl)thiazole (compound 160),
   4-(1-methyl-1H-pyrrol-3-yl)-2-(pyrazolo[1,5-a]pyridin-3-yl)thiazole (compound 161),
   3-(2-(pyrazolo[1,5-a]pyridin-3-yl)thiazol-4-yl)-1,2,4-thiadiazole (compound 162),
   4-(1-methyl-1H-pyrrol-2-yl)-2-(pyrazolo[1,5-a]pyridin-3-yl)thiazole (compound 163),
   3-bromo-4-(2-(pyrazolo[1,5-a]pyridin-3-yl)thiazol-4-yl)isoxazole (compound 164),
   4-(1H-imidazol-1-yl)-2-(pyrazolo[1,5-a]pyridin-3-yl)thiazole (compound 165),
   4-(1H-pyrazol-1-yl)-2-(pyrazolo[1,5-a]pyridin-3-yl)thiazole (compound 166),
   2-(pyrazolo[1,5-a]pyridin-3-yl)-4-(1H-pyrrol-1-yl)thiazole (compound 167),
   2-(pyrazolo[1,5-a]pyridin-3-yl)-4-(1H-1,2,4-triazol-1-yl)thiazole (compound 168),
   2-(pyrazolo[1,5-a]pyridin-3-yl)-4-(2H-1,2,3-triazol-2-yl)thiazole (compound 169),
   2-(pyrazolo[1,5-a]pyridin-3-yl)-4-(1H-1,2,3-triazol-1-yl)thiazole (compound 170),
   5-(2-(pyrazolo[1,5-a]pyridin-3-yl)thiazol-4-yl)thiophene-3-carboxamide (compound 171),
   2-(5-(2-(pyrazolo[1,5-a]pyridin-3-yl)thiazol-4-yl)thiophen-2-yl)acetamide (compound 172),
   N,N-dimethyl-5-(2-(pyrazolo[1,5-a]pyridin-3-yl)thiazol-4-yl)thiophene-3-carboxamide (compound 173),
   N-(2-(5-(2-(pyrazolo[1,5-a]pyridin-3-yl)thiazol-4-yl)thiophen-2-yl)ethyl)acetamide (compound 174),
   4-(3-(difluoromethyl)-1-methyl-1H-pyrazol-4-yl)-2-(pyrazolo[1,5-a]pyridin-3-yl)thiazole (compound 178),
   2-(2-(pyrazolo[1,5-a]pyridin-3-yl)thiazol-4-yl)thiophen-3-ol (compound 175),
   1-methyl-4-(2-(pyrazolo[1,5-a]pyridin-3-yl)thiazol-4-yl)-1H-pyrazole-3-carbonitrile (compound 176),
   N-methyl-2-(4-(2-(pyrazolo[1,5-a]pyridin-3-yl)thiazol-4-yl)-1H-pyrazol-1-yl)ethan-1-amine (compound 177),
   4-(5-fluoro-1H-pyrazol-4-yl)-2-(pyrazolo[1,5-a]pyridin-3-yl)thiazole (compound 179),
   3-methyl-4-(2-(pyrazolo[1,5-a]pyridin-3-yl)thiazol-4-yl)-1H-pyrazol-5-amine (compound 181),
   4-(4-(2-(pyrazolo[1,5-a]pyridin-3-yl)thiazol-4-yl)-1H-pyrazol-1-yl)butan-1-ol (compound 180),
   4-(1-(azetidin-2-ylmethyl)-1H-pyrazol-4-yl)-2-(pyrazolo[1,5-a]pyridin-3-yl)thiazole (compound 182),
   2-((2-(4-(2-(pyrazolo[1,5-a]pyridin-3-yl)thiazol-4-yl)-1H-pyrazol-1-yl)ethyl)amino)acetonitrile (compound 183),
   3-(4-(2-(pyrazolo[1,5-a]pyridin-3-yl)thiazol-4-yl)-1H-pyrazol-1-yl)propan-1-amine (compound 184),
   N-(3-(4-(2-(pyrazolo[1,5-a]pyridin-3-yl)thiazol-4-yl)-1H-pyrazol-1-yl)propyl)methanesulfonamide (compound 185),
   5-methyl-4-(2-(pyrazolo[1,5-a]pyridin-3-yl)thiazol-4-yl)-1H-pyrazol-3-ol (compound 186),
   N¹,N¹-dimethyl-N²-(2'-(pyrazolo[1,5-a]pyridin-3-yl)-[4,4'-bithiazole]-2-yl)ethane-1,2-diamine (compound 187),
   2-(dimethylamino)-N-(2'-(pyrazolo[1,5-a]pyridin-3-yl)-[4,4'-bithiazole]-2-yl)acetamide (compound 188),
   2-amino-N-(2'-(pyrazolo[1,5-a]pyridin-3-yl)-[4,4'-bithiazole]-2-yl)acetamide hydrochloride (compound 189),
   2'-(pyrazolo[1,5-a]pyridin-3-yl)-[4,4'-bithiazole]-2-ol (compound 190),
   5-(2-(pyrazolo[1,5-a]pyridin-3-yl)thiazol-4-yl)furan-2-carboxamide (compound 191),
   4-(2,5-dihydrofuran-3-yl)-2-(pyrazolo[1,5-a]pyridin-3-yl)thiazole (compound 192),
   (1-(2-(pyrazolo[1,5-a]pyridin-3-yl)thiazol-4-yl)-1H-imidazol-4-yl)methanol (compound 193),
   (1-(2-(pyrazolo[1,5-a]pyridin-3-yl)thiazol-4-yl)-1H-imidazol-4-yl)methanamine hydrochloride (compound 194),
   3-((1-(2-(pyrazolo[1,5-a]pyridin-3-yl)thiazol-4-yl)-1H-imidazol-4-yl)methoxy)propan-1-ol (compound 195),
   3-(4-(2-(pyrazolo[1,5-a]pyridin-3-yl)thiazol-4-yl)-1H-imidazol-1-yl)propan-1-ol (compound 196),
   2-(4-(2-(pyrazolo[1,5-a]pyridin-3-yl)thiazol-4-yl)-1H-imidazol-1-yl)ethan-1-amine (compound 197),
   N-(2-(4-(2-(pyrazolo[1,5-a]pyridin-3-yl)thiazol-4-yl)-1H-imidazol-1-yl)ethyl)acetamide (compound 198),
   2-(pyrazolo[1,5-a]pyridin-3-yl)-4-(pyrimidin-5-yl)thiazole (compound 199),
   2-(pyrazolo[1,5-a]pyridin-3-yl)-4-(pyrimidin-2-yl)thiazole (compound 200),
   4-(2,3-dihydro-1H-pyrrolo[2,3-b]pyridin-5-yl)-2-(pyrazolo[1,5-a]pyridin-3-yl)thiazole (compound 201),
   2-(pyrazolo[1,5-a]pyridin-3-yl)-4-(5,6,7,8-tetrahydroimidazo[1,2-a]pyridin-2-yl)thiazole (compound 202),
   2-(2-(pyrazolo[1,5-a]pyridin-3-yl)thiazol-4-yl)-5,6-dihydro-8H-imidazo[2,1-c][1,4]oxazine (compound 203),
   2-(2-(pyrazolo[1,5-a]pyridin-3-yl)thiazol-4-yl)-4,5,6,7-tetrahydrothiazolo[5,4-c]pyridine (compound 204),
   2-(2-(pyrazolo[1,5-a]pyridin-3-yl)thiazol-4-yl)-4,5,6,7-tetrahydrothiazolo[4,5-c]pyridine (compound 205),
   2-(pyrazolo[1,5-a]pyridin-3-yl)-4-(4,5,6,7-tetrahydropyrazolo[1,5-a]pyrazin-2-yl)thiazole (compound 206),
   4-(1H-indazol-3-yl)-2-(pyrazolo[1,5-a]pyridin-3-yl)thiazole (compound 207),
   4-(4-chloro-1H-pyrazol-3-yl)-2-(pyrazolo[1,5-a]pyridin-3-yl)thiazole (compound 208),
   2-(6-methylpyrazolo[1,5-a]pyridin-3-yl)-4-phenylthiazole (compound 209),
   2-(5-methylpyrazolo[1,5-a]pyridin-3-yl)-4-phenylthiazole (compound 210),
   2-(4-methylpyrazolo[1,5-a]pyridin-3-yl)-4-phenylthiazole (compound 211),
   4-(4-chloro-1-methyl-1H-pyrazol-3-yl)-2-(5-methylpyrazolo[1,5-a]pyridin-3-yl)thiazole (compound 212), and
   N-((5-(2-(5-methylpyrazolo[1,5-a]pyridin-3-yl)thiazol-4-yl)pyridin-2-yl)methyl)acetamide (compound 213).
[6] A composition comprising the compound or pharmaceutically acceptable derivative thereof according to any one of [1] to [5], and a pharmaceutically acceptable carrier.
[7] A pharmaceutical composition comprising the compound or pharmaceutically acceptable derivative thereof according to any one of [1] to [5].
[8] The pharmaceutical composition according to [7] for use in inhibiting myosin light chain kinase 4 (MYLK4) activity.
[9] The pharmaceutical composition according to [7] for use in alleviating or treating a disease, disorder or condition involving myosin light chain kinase 4 (MYLK4).
[10] The pharmaceutical composition according to [9], wherein the disease, disorder or condition involving myosin light chain kinase 4 (MYLK4) is arteriosclerosis, inflammatory bowel disease, osteosarcoma, ischemic heart disease, glaucoma, ocular hypertension, impaired aqueous humor outflow, dry eye, uveitis, age-related macular degeneration, or diabetic retinopathy.
[11] The pharmaceutical composition according to [7] for use in alleviating or treating dry eye, uveitis, age-related macular degeneration, or diabetic retinopathy.
[12] The pharmaceutical composition according to [7] for use in alleviating or treating glaucoma, ocular hypertension, or impaired aqueous humor outflow. [0011]
[13] The compound or pharmaceutically acceptable derivative thereof according to any one of [1] to [5], for use as a medicament.
[14] The compound or pharmaceutically acceptable derivative thereof according to [13], for use in inhibiting myosin light chain kinase 4 (MYLK4) activity.
[15] The compound or pharmaceutically acceptable derivative thereof according to [13], for use in alleviating or treating a disease, disorder or condition involving myosin light chain kinase 4 (MYLK4).
[16] The compound or pharmaceutically acceptable derivative thereof according to [15], wherein the disease, disorder or condition involving myosin light chain kinase 4 (MYLK4) is arteriosclerosis, inflammatory bowel disease, osteosarcoma, ischemic heart disease, glaucoma, ocular hypertension, impaired aqueous humor outflow, dry eye, uveitis, age-related macular degeneration, or diabetic retinopathy.
[17] The compound or pharmaceutically acceptable derivative thereof according to [13], for use in alleviating or treating dry eye, uveitis, age-related macular degeneration, or diabetic retinopathy.
[18] The compound or pharmaceutically acceptable derivative thereof according to [13], for use in alleviating or treating glaucoma, ocular hypertension, or impaired aqueous humor outflow.
[19] Use of the compound or pharmaceutically acceptable derivative thereof according to any one of [1] to [5], for use in preparing a medicament.
[20] The use according to [19] for preparing a medicament for use in inhibiting myosin light chain kinase 4 (MYLK4) activity.
[21] The use according to [19] for preparing a medicament for use in alleviating or treating a disease, disorder or condition involving myosin light chain kinase 4 (MYLK4).
[22] The use according to [21], wherein the disease, disorder or condition involving myosin light chain kinase 4 (MYLK4) is arteriosclerosis, inflammatory bowel disease, osteosarcoma, ischemic heart disease, glaucoma, ocular hypertension, impaired aqueous humor outflow, dry eye, uveitis, age-related macular degeneration, or diabetic retinopathy.
[23] The use according to [19] for preparing a medicament for use in alleviating or treating dry eye, uveitis, age-related macular degeneration, or diabetic retinopathy.
[24] The use according to [19] for use in preparing a medicament for alleviating or treating glaucoma, ocular hypertension, or impaired aqueous humor outflow.
[25] A method of inhibiting myosin light chain kinase 4 (MYLK4) activity, the method comprising administering to a patient an MYLK4 activity-inhibiting effective amount of the compound or pharmaceutically acceptable derivative thereof according to any one of [1] to [5].
[26] A method of alleviating or treating a disease, disorder, or condition involving myosin light chain kinase 4 (MYLK4), the method comprising administering to a patient a therapeutically effective amount of the compound or pharmaceutically acceptable derivative thereof according to any one of [1] to [5].
[27] The method according to [26], wherein the disease, disorder or condition involving myosin light chain kinase 4 (MYLK4) is arteriosclerosis, inflammatory bowel disease, osteosarcoma, ischemic heart disease, glaucoma, ocular hypertension, impaired aqueous humor outflow, dry eye, uveitis, age-related macular degeneration, or diabetic retinopathy.
[28] A method of alleviating or treating dry eye, uveitis, age-related macular degeneration, or diabetic retinopathy, the method comprising administering to a patient a therapeutically effective amount of the compound or pharmaceutically acceptable derivative thereof according to any one of [1] to [5].
[29] A method of alleviating or treating glaucoma, ocular hypertension, or impaired aqueous humor outflow, the method comprising administering to a patient a therapeutically effective amount of the compound or pharmaceutically acceptable derivative thereof according to any one of [1] to [5].

Terms related to structures or substituents used herein are defined below. Unless otherwise defined herein, the terms relating to substituents have the following meanings.

The terms "halogen" and "halo" mean fluorine, chlorine, bromine or iodine and are used as terms to represent substituents by themselves or to indicate portions of larger substituents or structures. The terms "halide" and "halogenated compound" refer to compounds containing a halogen(s), and examples thereof include a fluoride, a chloride, a bromide, and an iodide.

The terms "hydroxy" and "hydroxy group" mean a -OH group and are used as terms to represent substituents by themselves or to indicate portions of larger substituents or structures.

The terms "cyano" and "cyano group" mean a -CN group and are used as terms to represent substituents by themselves or to indicate portions of larger substituents or structures.

The terms "difluoromethyl" and "difluoromethyl group" mean a -CHF₂ group and are used as terms to represent substituents by themselves or to indicate portions of larger substituents or structures. The terms "trifluoromethyl" and "trifluoromethyl group" mean a -CF₃ group and are used as terms to represent substituents by themselves or to indicate portions of larger substituents or structures.

The terms "acetyl" and "acetyl group" mean a CH₃-C(O)- group and are used as terms to represent substituents by themselves or to indicate portions of larger substituents or structures.

The terms "amino" and "amino group" mean an -NR₂ group (where R is hydrogen or a substituent, or the two R groups together with nitrogen form a cyclic structure), which corresponds to a functional group derived from ammonia or an amine by removing one hydrogen atom bonded to the nitrogen. The terms are used as terms to represent substituents by themselves or to indicate portions of larger substituents or structures. The term "amine" means a compound having an "amino group".

The terms "nitro" and "nitro group" mean a -NO₂ group and are used as terms to represent substituents by themselves or to indicate portions of larger substituents or structures. The term "nitro compound" means a compound having "nitro".

The terms "aldehyde", "aldehyde group", "formyl", and "formyl group" mean a -CHO group and are used as terms to represent substituents by themselves or to indicate portions of larger substituents or structures. The term "aldehyde compound" means a compound having "aldehyde".

The terms "carbonyl" and "carbonyl group" mean a -C(O)- group that is a divalent group and are used as terms to represent substituents by themselves or to indicate portions of larger substituents or structures.

The term "thiocarbonyl" means a -C(S)- group that is a divalent group and is used as a term to represent a substituent by itself or to indicate a portion of a larger substituent or structure.

The terms "alkyl" and "alkyl group" mean a straight-chain or branched-chain saturated hydrocarbon group, where the monovalent group is represented by the general formula: -CₙH₂ₙ₊₁. The terms represent substituents by themselves or are used as portions of larger substituents or structures. The terms "alkyl" and "alkyl group", when they represent substituents by themselves, represent a monovalent group, but when used as terms to indicate portions of larger substituents or structures, may represent a divalent or higher valent group, in which case the terms are synonymous with "alkylene". As used herein, the term "C_{x-y} alkyl" means an alkyl having x to y carbon atoms.

Examples of the alkyl include methyl, ethyl, propyl (e.g., propan-1-yl, propan-2-yl (or isopropyl)), butyl (e.g., 2-methylpropan-2-yl (or tert-butyl), butan-1-yl, and butan-2-yl), pentyl (e.g., pentan-1-yl, pentan-2-yl, pentan-3-yl), 2-methylbutan-1-yl, 3-methylbutan-1-yl, and hexyl (e.g., hexan-1-yl).

The terms "alkenyl" and "alkenyl group" mean a straight-chain or branched-chain unsaturated hydrocarbon group having at least one double bond, where the monovalent group is represented by the general formula: -CₙH₂ₙ₋₁. The terms represent substituents by themselves or are used as portions of larger substituents or structures. The terms "alkenyl" and "alkenyl group", when they represent substituents by themselves, represent a monovalent group, but when used as terms to indicate portions of larger substituents or structures, may mean a divalent or higher valent group, in which case the terms are synonymous with "alkenylene". As used herein, the term "C_{x-y} alkenyl" means an alkenyl having x to y carbon atoms.

Examples of the alkenyl include vinyl, 1-propenyl, 2-propenyl, isopropenyl, 1,3-butadienyl, 1-butenyl, 2-butenyl, 3-butenyl, 2-methyl-1-propenyl, 1-pentenyl, 2-pentenyl, 3-pentenyl, 4-pentenyl, 3-methyl-2-butenyl, 1-hexenyl, 2-hexenyl, 3-hexenyl, 2,4-hexadienyl, and 5-hexenyl.

The terms "alkynyl" and "alkynyl group" mean a straight-chain or branched-chain unsaturated hydrocarbon group having at least one triple bond, where the monovalent group is represented by the general formula: -CₙH₂ₙ₋₃. The terms represent substituents by themselves or are used as portions of larger substituents or structures. The terms "alkynyl" and "alkynyl group", when they represent substituents by themselves, represent a monovalent group, but when used as terms to indicate portions of larger substituents or structures, may mean a divalent or higher valent group, in which case the terms are synonymous with "alkynylene". As used herein, the term "C_{x-y} alkynyl" means an alkynyl having x to y carbon atoms.

Examples of the alkynyl include 2-propynyl, 2-butynyl, and 1,3-hexadien-5-ynyl.

The terms "chain aliphatic hydrocarbon" and "chain aliphatic hydrocarbon group" mean a straight-chain or branched-chain non-aromatic hydrocarbon group. Thus, "chain aliphatic hydrocarbon" and "chain aliphatic hydrocarbon group" include saturated or unsaturated hydrocarbon groups, and more particularly include straight-chain or branched-chain alkyl, alkenyl, or alkynyl. As used herein, the term "C_{x-y} chain aliphatic hydrocarbon" means a chain aliphatic hydrocarbon having x to y carbon atoms. Examples of the alkyl, the alkenyl, and the alkynyl are as described above.

The terms "cycloaliphatic hydrocarbon" and "cycloaliphatic hydrocarbon group" mean a monocyclic or polycyclic non-aromatic cyclic hydrocarbon group and include monocyclic or polycyclic cycloalkyl or cycloalkenyl. The terms "cycloaliphatic hydrocarbon" and "cycloaliphatic hydrocarbon group" represent substituents by themselves or are used as portions of larger substituents or structures, and when representing substituents by themselves, they represent a monovalent group, but when used as portions of larger substituents or structures, may mean a divalent or higher valent group. The term "x- to y-membered cycloaliphatic hydrocarbon" means a monocyclic or polycyclic non-aromatic cyclic hydrocarbon in which the ring is composed of from x to y carbon atoms, and includes monocyclic or polycyclic cycloalkyl or cycloalkenyl in which the ring is composed of from x to y carbon atoms.

The polycyclic non-aromatic cyclic hydrocarbon group includes bicyclic or tricyclic non-aromatic cyclic hydrocarbon groups. In addition, the polycyclic non-aromatic cyclic hydrocarbon group includes a bridged ring hydrocarbon and a spiro hydrocarbon.

Examples of the cycloaliphatic hydrocarbon include cyclopropyl, cyclopropenyl, cyclobutyl, cyclobutenyl, cyclopentyl, cyclopentenyl, cyclohexyl, cyclohexenyl, cycloheptyl, cycloheptenyl, cyclooctyl, cyclooctenyl, cyclooctadienyl, bicyclo[2.2.1]heptyl, bicyclo[2.2.2]octyl, bicyclo[3.2.1]octyl, and adamantyl.

The terms "cycloalkyl" and "cycloalkyl group" mean monocyclic or polycyclic saturated hydrocarbon groups, and the terms represent substituents by themselves or are used as portions of larger substituents or structures. The terms "cycloalkyl" and "cycloalkyl group", when they represent substituents by themselves, represent a monovalent group, but when used as portions of larger substituents or structures, may represent a divalent or higher valent group, in which case the terms are synonymous with "cycloalkylene". The term "x- to y-membered cycloalkyl" means a monocyclic or polycyclic cycloalkyl in which the ring is composed of from x to y carbon atoms. Examples of the cycloalkyl include cyclopropyl, cyclobutyl, cyclopentyl, cyclohexyl, cycloheptyl, cyclooctyl, bicyclo[2.2.1]heptyl, bicyclo[2.2.2]octyl, and bicyclo[3.2.1]octyl.

The terms "cycloalkenyl" and "cycloalkenyl group" mean monocyclic or polycyclic non-aromatic cyclic hydrocarbon group containing at least one carbon-carbon double bond, and the terms represent substituents by themselves or are used as portions of larger substituents or structures. The terms "cycloalkenyl" and "cycloalkenyl group", when they represent substituents by themselves, represent a monovalent group, but when used as portions of larger substituents or structures, may represent a divalent or higher valent group, in which case the terms are synonymous with "cycloalkenylene". The term "x- to y-membered cycloalkenyl" is a monocyclic or polycyclic cycloalkenyl in which the ring is composed of from x to y carbon atoms.

Examples of the cycloalkenyl include cyclopentenyl, cyclohexenyl, and cycloheptenyl.

The terms "aromatic hydrocarbon" and "aromatic hydrocarbon group" are used interchangeably with the term "aryl" and mean hydrocarbon groups having a monocyclic or polycyclic structure composed of carbon atoms, in which single and double bonds are alternating and electrons are delocalized. The terms represent substituents by themselves or are used as portions of larger substituents or structures. The terms "aromatic hydrocarbon" and "aromatic hydrocarbon group", when they represent substituents by themselves, represent a monovalent group, but when used as portions of larger substituents or structures, may represent a divalent or higher valent group. The term "x- to y-membered aromatic hydrocarbon" means an aromatic hydrocarbon whose ring is composed of from x to y carbons. Examples of the aromatic hydrocarbon include phenyl, 1-naphthyl, 2-naphthyl, 1-anthryl, 2-anthryl, and 9-anthryl.

The terms "heterocycle" and "heterocyclic group" mean monocyclic or polycyclic groups having a ring structure that contains heteroatoms as ring-constituting atoms. The terms represent substituents by themselves or are used as portions of larger substituents or structures. The terms "heterocycle" and "heterocyclic group", when they represent substituents by themselves, represent a monovalent group, but when used as portions of larger substituents or structures, may represent a divalent or higher valent group. The term "x- to y-membered heterocycle" means a heterocycle in which the ring is composed of from x to y atoms, including carbon and heteroatoms. Examples of the heterocycle include aromatic heterocycles, heterocyclyl, and heterocycloalkenyl.

The terms "aromatic heterocycle" and "aromatic heterocyclic group" are used interchangeably with the term "heteroaryl" and mean monocyclic or polycyclic groups having a ring structure in which single and double bonds are alternating and electrons are delocalized, and which contains heteroatoms as ring-constituting atoms. The terms represent substituents by themselves or are used as portions of larger substituents or structures. The terms "aromatic heterocycle" and "aromatic heterocyclic group", when they represent substituents by themselves, represent a monovalent group, but when used as portions of larger substituents or structures, may represent a divalent or higher valent group. The term "x- to y-membered aromatic heterocycle" means an aromatic heterocycle in which the ring is composed of from x to y atoms, including carbon and heteroatoms.

Examples of the aromatic heterocycle include:
5- or 6-membered monocyclic aromatic heterocycles such as thiophene, furan, pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, triazole, tetrazole, triazine; and
8- to 14-membered fused polycyclic (preferably bicyclic or tricyclic) aromatic heterocycle such as benzothiophene, benzofuran, benzimidazole, benzoxazole, benzisoxazole, benzothiazole, benzisothiazole, benzotriazole, imidazopyridine, thienopyridine, furopyridine, pyrrolopyridine, pyrazolopyridine, oxazolopyridine, thiazolopyridine, imidazopyrazine, imidazopyrimidine, thienopyrimidine, furopyrimidine, pyrrolopyrimidine, pyrazolopyrimidine, oxazolopyrimidine, thiazolopyrimidine, pyrazolopyrimidine, pyrazolotriazine, naphtho[2,3-b]thiophene, phenoxathiine, indole, isoindole, 1H-indazole, purine, isoquinoline, quinoline, phthalazine, naphthyridine, quinoxaline, quinazoline, cinnoline, carbazole, β-carboline, phenanthridine, acridine, phenazine, phenothiazine, and phenoxazine.

The terms "heterocyclyl" and "heterocyclyl group" mean monocyclic or polycyclic groups having a saturated ring structure that contains heteroatoms as ring-constituting atoms. The terms represent substituents by themselves or are used as portions of larger substituents or structures. The terms "heterocyclyl" and "heterocyclyl group", when they represent substituents by themselves, represent a monovalent group, but when used as portions of larger substituents or structures, may represent a divalent or higher valent group. The term "x- to y-membered heterocyclyl" means a heterocyclyl in which the ring is composed of from x to y atoms, including carbon and heteroatoms. The polycyclic heterocyclyl includes bicyclic or tricyclic heterocyclyl. The polycyclic heterocyclyl includes a bridged ring and a spiro ring.

Heterocyclyl typically includes saturated heterocycles containing one or more, for example 1 to 3, heteroatoms selected from nitrogen, oxygen, and sulfur atoms.

The terms "heterocycloalkenyl" and "heterocycloalkenyl group" mean monocyclic or polycyclic non-aromatic heterocyclic groups containing at least one carbon-carbon double bond and containing heteroatoms as ring-constituting atoms. The terms represent substituents by themselves or are used as portions of larger substituents or structures. The terms "heterocycloalkenyl" and "heterocycloalkenyl group", when they represent substituents by themselves, represent a monovalent group, but when used as portions of larger substituents or structures, may represent a divalent or higher valent group, in which case the terms are synonymous with "heterocycloalkenylene". The term "x- to y-membered heterocycloalkenyl" is a monocyclic or polycyclic cycloalkenyl in which the ring is composed of from x to y carbon atoms.

Examples of the heterocycloalkenyl include dihydrofuran, pyran, pyrroline, and pyrazoline.

The terms "aralkyl" and "aralkyl group" mean an alkyl substituted with phenyl, and represent substituents by themselves or represent portions of larger substituents or structures. The terms "aralkyl" and "aralkyl group", when they represent substituents by themselves, represent a monovalent group, but when used as portions of larger substituents or structures, may represent a divalent or higher valent group. The term "C_{x-y} aralkyl" means an aralkyl in which the alkyl moiety has x to y carbon atoms. Examples of the aralkyl include benzyl, phenethyl, naphthylmethyl, and phenylpropyl.

The terms "alkoxy" and "alkoxy group" mean a group represented by the formula: -OR, wherein R is alkyl. The terms represent substituents by themselves or represent portions of larger substituents or structures. The terms "alkoxy" and "alkoxy group", when they represent substituents by themselves, represent a monovalent group, but when used as portions of larger substituents or structures, may represent a divalent or higher valent group. The term "C_{x-y} alkoxy" means alkoxy having from x to y carbon atoms.

Examples of the alkoxy include methoxy, ethoxy, propoxy (for example, 1-propoxy, 2-propoxy), butoxy (for example, 1-butoxy, 2-butoxy, 2-methyl-2-propoxy), pentyloxy (for example, 1-pentyloxy, 2-pentyloxy), and hexyloxy (for example, 1-hexyloxy, 3-hexyloxy).

The terms "cycloalkoxy" and "cycloalkoxy group" mean a group represented by the formula: -OR, wherein R is cycloalkyl. The terms represent substituents by themselves or represent portions of larger substituents or structures. The terms "cycloalkoxy" and "cycloalkoxy group", when they represent substituents by themselves, represent a monovalent group, but when used as portions of larger substituents or structures, may represent a divalent or higher valent group. The term "x- to y-membered cycloalkoxy" means a cycloalkoxy in which the ring of the cycloalkyl is composed of from x to y carbon atoms.

Examples of the cycloalkoxy include cyclopropyloxy, cyclobutyloxy, cyclopentyloxy, cyclohexyloxy, cycloheptyloxy, and cyclooctyloxy.

The terms "alkylthio" and "alkylthio group" mean a group represented by the formula: - SR, wherein R is an alkyl group. The terms represent substituents by themselves or represent portions of larger substituents or structures. The terms "alkylthio" and "alkylthio group", when they represent substituents by themselves, represent a monovalent group, but when used as portions of larger substituents or structures, may represent a divalent or higher valent group. The term "C_{x-y} alkylthio" means alkylthio having from x to y carbon atoms. Examples of the alkylthio include methylthio, difluoromethylthio, trifluoromethylthio, ethylthio, propylthio, isopropylthio, butylthio, 4,4,4-trifluorobutylthio, pentylthio, and hexylthio.

The terms "cycloalkylthio" and "cycloalkylthio group" mean a group represented by the formula: -SR, wherein R is cycloalkyl. The terms represent substituents by themselves or represent portions of larger substituents or structures. The terms "cycloalkylthio" and "cycloalkylthio group" when they represent substituents by themselves, represent a monovalent group, but when used as portions of larger substituents or structures, may represent a divalent or higher valent group. The term "x- to y-membered cycloalkylthio" represents a cycloalkylthio in which the ring of the cycloalkyl is composed of from x to y carbon atoms.

The terms "alkylsulfonyl" and "alkylsulfonyl group", "cycloalkylsulfonyl" and "cycloalkylsulfonyl group" and "heterocyclosulfonyl" and "heterocyclosulfonyl group" mean a group represented by the formula: -S(O)₂-R, wherein R is alkyl, cycloalkyl and heterocyclyl, respectively. The terms represent substituents by themselves or represent portions of larger substituents or structures. The terms "alkylsulfonyl" and "alkylsulfonyl group", "cycloalkylsulfonyl" and "cycloalkylsulfonyl group", and "heterocyclosulfonyl" and "heterocyclosulfonyl group", when they represent substituents by themselves, represent a monovalent group, but when used as portions of larger substituents or structures, may represent a divalent or higher valent group. The term "C_{x-y} alkylsulfonyl" means an alkylsulfonyl in which the alkyl has x to y carbon atoms, and the term "x- to y-membered cycloalkylsulfonyl" means a cycloalkylsulfonyl wherein the ring of the cycloalkyl is composed of from x to y carbon atoms, and the term "x- to y-membered heterocyclosulfonyl" means a heterocyclosulfonyl wherein the heterocyclo ring is composed of from x to y atoms, including carbon and heteroatoms.

Examples of the alkylsulfonyl include methylsulfonyl, ethylsulfonyl, propylsulfonyl, isopropylsulfonyl, butylsulfonyl, sec-butylsulfonyl, and tert-butylsulfonyl.

The terms "arylsulfonyl" and "arylsulfonyl group" are also used in a manner similar to that described above, and examples of the arylsulfonyl include phenylsulfonyl, 1-naphthylsulfonyl, and 2-naphthylsulfonyl.

The terms "alkylcarbonyl" and "alkylcarbonyl group", "cycloalkylcarbonyl" and "cycloalkylcarbonyl group", and "heterocyclocarbonyl" and "heterocyclocarbonyl group" mean a group represented by the formula: -C(O)-R, wherein R is alkyl, cycloalkyl and heterocyclyl, respectively. The terms represent substituents by themselves or represent portions of larger substituents or structures. The terms "alkylcarbonyl" and "alkylcarbonyl group", "cycloalkylcarbonyl" and "cycloalkylcarbonyl group", and "heterocyclocarbonyl" and "heterocyclocarbonyl group", when they represent substituents by themselves, represent a monovalent group, but when used as portions of larger substituents or structures, may represent a divalent or higher valent group. The term "C_{x-y} alkylcarbonyl" means an alkylcarbonyl in which the alkyl has x to y carbon atoms, the term "x- to y-membered cycloalkylcarbonyl" means a cycloalkylcarbonyl in which the ring of the cycloalkyl is composed of from x to y carbon atoms, and the term "x- to y-membered heterocyclocarbonyl" means a heterocyclocarbonyl in which the heterocyclo ring is composed of from x to y atoms, including carbon atoms and heteroatoms .

Examples of the alkylcarbonyl include acetyl, propanoyl, butanoyl, 2-methylpropanoyl, pentanoyl, 3-methylbutanoyl, 2-methylbutanoyl, 2,2-dimethylpropanoyl, hexanoyl, and heptanoyl.

The terms "alkoxycarbonyl" and "alkoxycarbonyl group", and "cycloalkoxycarbonyl" and "cycloalkoxycarbonyl group" mean a group represented by the formula: -C(O)-R, wherein R is alkoxy and cycloalkoxy, respectively. The terms represent substituents by themselves or represent portions of larger substituents or structures. The terms "alkoxycarbonyl" and "alkoxycarbonyl group", and "cycloalkoxycarbonyl" and "cycloalkoxycarbonyl group", when they represent substituents by themselves, represent a monovalent group, but when used as portions of larger substituents or structures, may represent a divalent or higher valent group. The term "C_{x-y} alkoxycarbonyl" means an alkoxycarbonyl in which the alkoxy has x to y carbon atoms, and the term "x- to y-membered cycloalkoxycarbonyl" means a cycloalkoxycarbonyl in which the cycloalkyl ring is composed of from x to y carbon atoms.

Examples of the alkoxycarbonyl include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, and hexyloxycarbonyl.

The terms "arylcarbonyl" and "arylcarbonyl group", "aralkylcarbonyl" and "aralkylcarbonyl group", "aromatic heterocyclic carbonyl" and "aromatic heterocyclic carbonyl group", and "heterocyclylcarbonyl" and "heterocyclylcarbonyl group" are also used in a manner similar to that described above. Examples of the arylcarbonyl include benzoyl, 1-naphthoyl, and 2-naphthoyl, examples of the aralkylcarbonyl include phenylacetyl and phenylpropionyl, examples of the aromatic heterocyclic carbonyl include nicotinoyl, isonicotinoyl, thenoyl, and furoyl, and examples of the heterocyclylcarbonyl include morpholinylcarbonyl, piperidinylcarbonyl, and pyrrolidinylcarbonyl.

The terms "amide" and "amide group" and "carboxylic acid amide" and "carboxylic acid amide group" mean a compound having a structure represented by the formula: R-C(O)-NR'R'' (wherein R is any substituent or structure; R, R' and R'' are each independently hydrogen or any substituent or structure; and R' and R" may form a cyclic structure with N). The terms "thioamide" and "thioamide group" and "thioacid amide" and "thioacid amide group" mean a compound having a structure represented by the formula: R-C(S)-NR'R'' (wherein R is any substituent or structure; and R, R' and R" are each independently hydrogen or any substituent or structure). The terms "acetamide" and "acetamide group" and the symbol "-NHAc" mean a monovalent substituent represented by CH₃C(O)NH-.

The terms "alkylcarbamoyl" and "alkylcarbamoyl group", "cycloalkylcarbamoyl" and "cycloalkylcarbamoyl group", and "heterocyclocarbamoyl" and "heterocyclocarbamoyl group" mean a group represented by the formula: -C(O)-NR'R'', wherein R' and R" are each independently hydrogen, or alkyl, cycloalkyl, or heterocyclyl, and at least one of R' and R" is alkyl, cycloalkyl, or heterocyclyl. The terms represent substituents by themselves or represent portions of larger substituents or structures. The terms "alkylcarbamoyl" and "alkylcarbamoyl group", "cycloalkylcarbamoyl" and "cycloalkylcarbamoyl group", and "heterocyclocarbamoyl" and "heterocyclocarbamoyl group", when they represent substituents by themselves, represent a monovalent group, but when used as portions of larger substituents or structures, may represent a divalent or higher valent group. The term "C_{x-y} alkylcarbamoyl" means an alkylcarbamoyl in which the alkyl has x to y carbon atoms, the term "x- to y-membered cycloalkylcarbamoyl" means a cycloalkylcarbamoyl in which the ring of the cycloalkyl is composed of from x to y carbon atoms, and the term "x- to y-membered heterocyclocarbamoyl" means a heterocyclocarbamoyl in which the heterocyclo ring is composed of from x to y atoms, including carbon atoms and heteroatoms.

Examples of the alkylcarbamoyl include methylcarbamoyl, ethylcarbamoyl, dimethylcarbamoyl, diethylcarbamoyl, and N-ethyl-N-methylcarbamoyl.

The terms "aralkylcarbamoyl" and "aralkylcarbamoyl group" are also used in a manner similar to that described above, and examples of "aralkylcarbamoyl" include benzylcarbamoyl and phenethylcarbamoyl.

The term "ester" refers to a structure represented by the formula: R-C(O)O-R' (wherein R is a structure such as C₁₋₃ alkyl or -O-C-; and R' is a substituent such as alkyl and aromatic hydrocarbon), and "ester compound" refers to a compound having an ester.

The term "ether" refers to a structure represented by the formula: R-O-R' (wherein R and R' are each a substituent such as an alkyl or an aromatic hydrocarbon), and "ether compound" refers to a compound having an ether.

### DESCRIPTION OF EMBODIMENTS

Embodiments of the present invention will be described in detail. However, the present invention is not limited to the following embodiments.

### 1. Novel thiazolyl-pyrazolopyridine compound

The novel thiazolyl-pyrazolopyridine compound provided by the present invention (hereinafter, referred to as "the present compound") is a compound of formula (I): 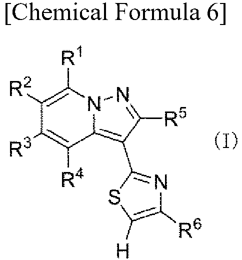 or a pharmaceutically acceptable derivative thereof.

In the formula (I), R¹ to R⁵ are each independently hydrogen or a C₁₋₃ chain aliphatic hydrocarbon; and
R⁶ is an optionally substituted 5- to 10-membered aromatic hydrocarbon, an optionally substituted 5- to 10-membered heterocycle having 1 to 5 heteroatoms each independently selected from nitrogen, oxygen, and sulfur as ring-constituting atoms, or a fused ring in which either the aromatic hydrocarbon or the heterocycle is fused with an optionally substituted 4- to 10-membered heterocyclyl having 1 to 3 heteroatoms each independently selected from nitrogen, oxygen, and sulfur as ring-constituting atoms.

R¹ to R⁵ in the formula (I) are each independently preferably hydrogen or a chain C₁₋₃ alkyl group, preferably hydrogen or a straight-chain C₁₋₃ alkyl group, more preferably hydrogen or a methyl group, and particularly preferably hydrogen. R¹, R², and R⁴ are preferably hydrogen, and R¹, R², R⁴, and R⁵ are more preferably hydrogen.

The "5- to 10-membered aromatic hydrocarbon" in R⁶ of the formula (I) is preferably a 5- or 6-membered monocyclic aromatic hydrocarbon or an 8- to 10-membered bicyclic or tricyclic aromatic hydrocarbon, more preferably phenyl, 1-naphthyl or 2-naphthyl, and particularly preferably phenyl.

The "5- to 10-membered heterocycle" in R⁶ of the formula (I) includes, for example, a 5- to 10-membered aromatic heterocycle and a 5- or 6-membered heterocycloalkenyl, preferably a 5- or 6-membered monocyclic aromatic heterocycle, an 8- to 10-membered bicyclic or tricyclic aromatic heterocycle, or a 5-membered heterocycloalkenyl, more preferably a 5- or 6-membered monocyclic aromatic heterocycle or an 8- to 10-membered bicyclic aromatic heterocycle, and particularly preferably a 5- or 6-membered monocyclic aromatic heterocycle. Among them, pyridine, pyrazole, thiazole, isoxazole, pyrrole, imidazole, triazole, thiophene, furan, and isothiazole are preferable.

Examples of the "4- to 10-membered heterocyclyl" that forms the "fused ring" with the "5- to 10-membered aromatic hydrocarbon" or "5- to 10-membered heterocycle" (preferably "5-to 10-membered aromatic heterocycle") in R⁶ of the formula (I) include a saturated 4- to 10-membered heterocycle containing 1 to 3 heteroatoms selected from nitrogen, oxygen, and sulfur. Examples of such a "4- to 10-membered heterocyclyl" include dioxolane such as 1,3-dioxolane; dihydropyridin-2(1H)-one, oxetanyl, azetidinyl, piperazinyl, piperidinyl, morpholinyl, thiomorpholinyl, pyrrolidinyl, tetrahydropyranyl, tetrahydrofuranyl, oxazolidinyl, thiazolidinyl, imidazolidinyl, pyrazolidinyl, thianyl, oxanyl, thioxanyl, azepinyl, and tropanyl.

Among them, 5- or 6-membered monocyclic heterocyclyl containing one or two oxygen or nitrogen atoms as ring-constituting atoms is preferable, and 5- or 6-membered monocyclic heterocyclyl containing one or two nitrogen atoms as ring-constituting atoms is more preferable. Such monocyclic heterocyclyls include dioxolanes such as 1,3-dioxolane, dihydropyridine-2(1H)-one, and pyrrolidinyl.

Therefore, the "fused ring" for R⁶ of the formula (I) is preferably a fused ring of a 5- or 6-membered monocyclic aromatic hydrocarbon and a 5- or 6-membered monocyclic heterocyclyl, wherein the aromatic hydrocarbon is linked to the thiazole of the formula (I), or a fused ring of a 5- or 6-membered monocyclic nitrogen-containing aromatic heterocycle containing one or two nitrogen atoms as ring-constituting atoms and a 5- or 6-membered monocyclic heterocyclyl, wherein the aromatic heterocycle is linked to the thiazole of the formula (I), more preferably a bicyclic fused ring of phenyl and a 5- or 6-membered monocyclic nitrogen-containing heterocyclyl, wherein the phenyl is linked to the thiazole of formula (I), or a fused ring of pyridine and a 5- or 6-membered monocyclic nitrogen or oxygen-containing heterocyclyl, wherein the pyridine is linked to the thiazole of formula (I), and particularly preferably a bicyclic fused ring of phenyl and a 6-membered monocyclic nitrogen-containing heterocyclyl, wherein the phenyl is linked to the thiazole of the formula (I).

The "5- to 10-membered aromatic hydrocarbon", "5- to 10-membered heterocycle", and "4- to 10-membered heterocyclyl" forming a fused ring with either of these in R⁶ of the formula (I) may be optionally substituted. Here, the term "optionally substituted" means unsubstituted or substituted with a predetermined substituent. Substituents are attached to appropriate positions of these ring structures in chemically possible forms and are selected depending on the attachment positions to these ring structures. The substituent of the "5- to 10-membered aromatic hydrocarbon" or the "5- to 10-membered heterocycle" is, for example, selected from the group consisting of:
(1) halogen, nitro, cyano, hydroxy, difluoromethyl, trifluoromethyl,
(2) -NR³¹₂, -N(R³¹)(CH₂)₁₋₅NR³¹₂, -N(R³¹)S(O)₂R³², -N(R³¹)C(O)R³¹, - N(R³¹)C(O)(CH₂)₀₋₃NR³¹₂, -N(R³¹)C(O)(CH₂)₀₋₃R³²,
(3) C₁₋₆ alkyl, -(CH₂)₁₋₅OR³¹, -(CH₂)₁₋₅O(CH₂)₁₋₅OR³¹, -(CH₂)₁₋₅R³², -(CH₂)₁₋₅R³³,
(4) -(CH₂)₁₋₅NR³¹₂, -(CH₂)₁₋₅N(R³¹)R³², -(CH₂)₁₋₅N(R³¹)(CH₂)₀₋₅R³⁸, -(CH₂)₁₋₅N(R³¹)C(O)R³¹, -(CH₂)₁₋₅N(R³¹)C(O)(CH₂)₁₋₅OR³¹, -(CH₂)₁₋₅N(C(O)R³¹)(CH₂)₁₋₅C(O)OR³¹, - (CH₂)₁₋₅N(R³¹)C(O)(CH₂)₀₋₅NR³¹₂, -(CH₂)₁₋₅N(R³¹)C(O)R³²,
(5) -(CH₂)₁₋₅N(R³¹)S(O)₂R³³,
(6) -C(R³¹)₂C(O)R³²,
(7) -C(R³¹)₂C(O)NR³¹₂, -C(R³¹)₂C(O)N(R³¹)OR³¹,
(8) -C(O)R³¹, -C(O)R³²,
(9) -C(O)OR³¹,
(10) -C(O)NR³¹₂, -C(O)N(R³¹)(CH₂)₀₋₅R³²,
(11) -OC(R³¹)₂C(O)N(R³¹)(CH₂)₁₋₅OR³¹, -OC(R³¹)₂C(O)OR³¹, -OC(R³¹)₂C(O)NR³¹₂, - OC(R³¹)₂C(O)R³²,
(12) -O(CH₂)₁₋₆R³¹, -(O(CH₂)₁₋₅)₁₋₅(CH₂)₀₋₃NR³¹₂, -(O(CH₂)₁₋₅)₁₋₅(CH₂)₀₋₃NR³¹C(O)R³¹, -(O(CH₂)₁₋₅)₁₋₅NR³¹C(NH)NR³¹₂, -O((CH₂)₁₋₅O)₁₋₅R³¹,
(13) -OC(O)C(R³¹)₂(O(CH₂)₁₋₅)₁₋₅OR³¹, and
(14) 5- or 6-membered heterocyclyl containing nitrogen as a ring-constituting atom and optionally substituted with C₁₋₃ alkyl.

R³¹ is each independently hydrogen or a C₁₋₄ chain aliphatic hydrocarbon, preferably hydrogen or a chain C₁₋₄ alkyl, more preferably hydrogen or a straight-chain C₁₋₄ alkyl, further preferably hydrogen, methyl or ethyl, and particularly preferably hydrogen.

R³² is an optionally substituted 4- to 10-membered heterocyclyl having 1 to 3 heteroatoms selected from nitrogen, sulfur and oxygen as ring-constituting atoms, or a fused ring in which the heterocyclyl is fused with an optionally substituted 5- or 6-membered monocyclic aromatic hydrocarbon, and the substituent is preferably selected from the group consisting of a C₁₋₁₂ chain aliphatic hydrocarbon, -(CH₂)₁₋₅OH, halogen, nitro, hydroxy, cyano, -(CH₂)₀₋₅NR³¹₂, - (CH₂)₀₋₅N(R³¹)R³³, -(CH₂)₀₋₅NC(O)R³³, -OR³¹, -SR³¹, -S(O)₂R³¹, -C(O)R³¹, -C(O)OR³¹, - C(O)C(O)R³³, -C(O)NR³¹₂, -C(O)N(R³¹)(CH₂)₁₋₅R³³, -N(R³¹)SO₂R³¹, -N(R³¹)C(O)R³¹, - N(R³¹)C(O)OR³¹, -N(R³¹)C(O)NR³¹₂, and -N(R³¹)S(O)₂NR³¹₂, more preferably selected from the group consisting of C₁₋₃ alkyl, -(CH₂)₁₋₃OH, -NR³¹₂, -CHO, -C(O)C(O)R³³, -C(O)NR³¹₂, - C(O)N(R³¹)(CH₂)₁₋₅ 3- to 10-membered cycloaliphatic hydrocarbon, and acetyl.

When sulfur is contained as a ring-constituting atom, the sulfur element forms -S(O)₂-.

R³³ is a 3- to 10-membered cycloaliphatic hydrocarbon or a 4- to 10-membered heterocyclyl having 1 to 5 heteroatoms independently selected from nitrogen, oxygen and sulfur as ring-constituting atoms.

R³⁸ is cyano, hydroxy, or S(O)₂R³¹.

Note that R³¹, R³², R³³, and R³⁸ as used herein represent similar groups or structures in the following description unless otherwise specified.

Examples of substituents of the "4- to 10-membered heterocyclyl" that forms a fused ring include C₁₋₅ chain aliphatic hydrocarbon, and preferably C₁₋₃ alkyl.

As used herein, substituents on the "cycloaliphatic hydrocarbon", "aromatic hydrocarbon", "aromatic heterocycle", "heterocycloalkenyl", "heterocyclyl", or a fused ring formed from any two thereof may be introduced in a chemically possible number at chemically appropriate positions. In the case of halogen, nitro, hydroxy, cyano, and amino, the number of substituents is usually 4 or less, preferably 3 or less, and more preferably 2 or less. For substituents other than halogen, nitro, hydroxy, cyano, and amino, the number of substituents is usually 2 or less, and preferably 1. When the number of substituents is two or more, the substituents may be the same or different.

In a preferred embodiment, R⁶ is selected from the group consisting of: 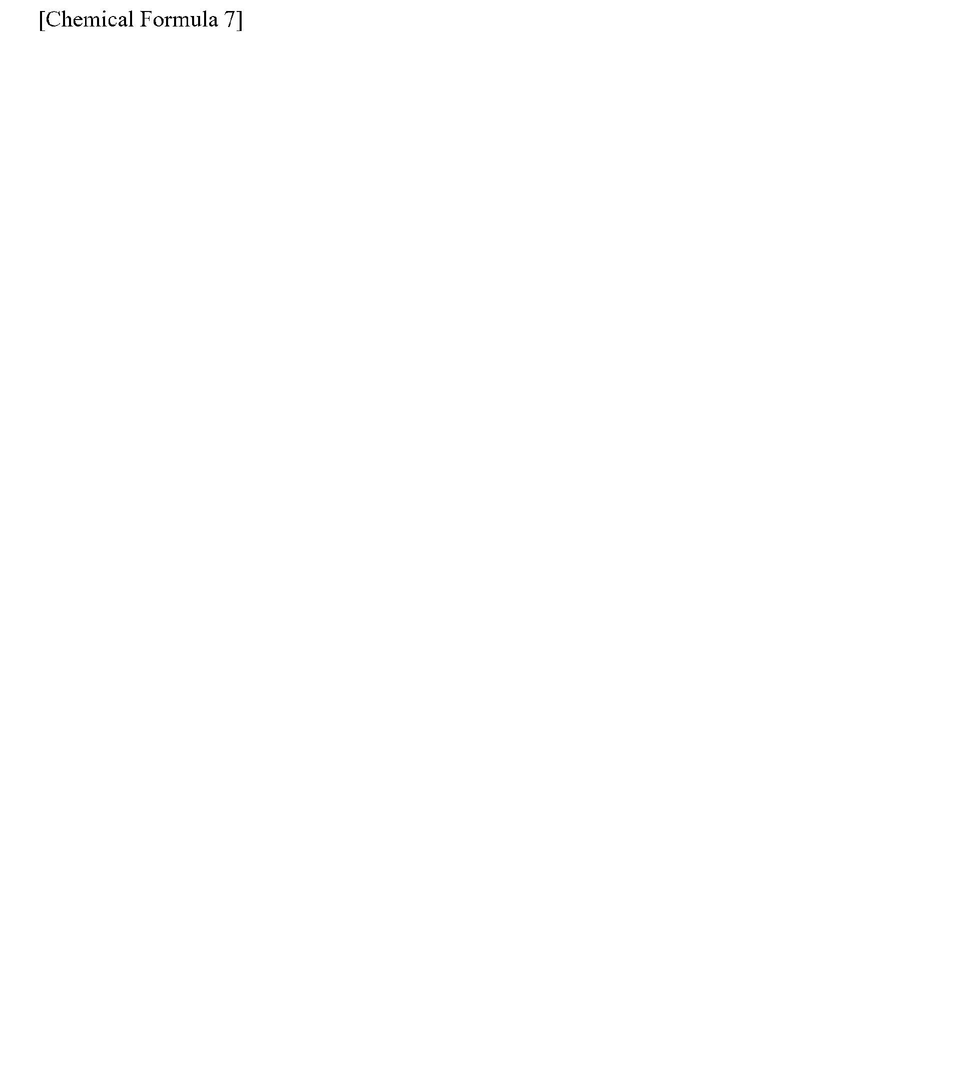 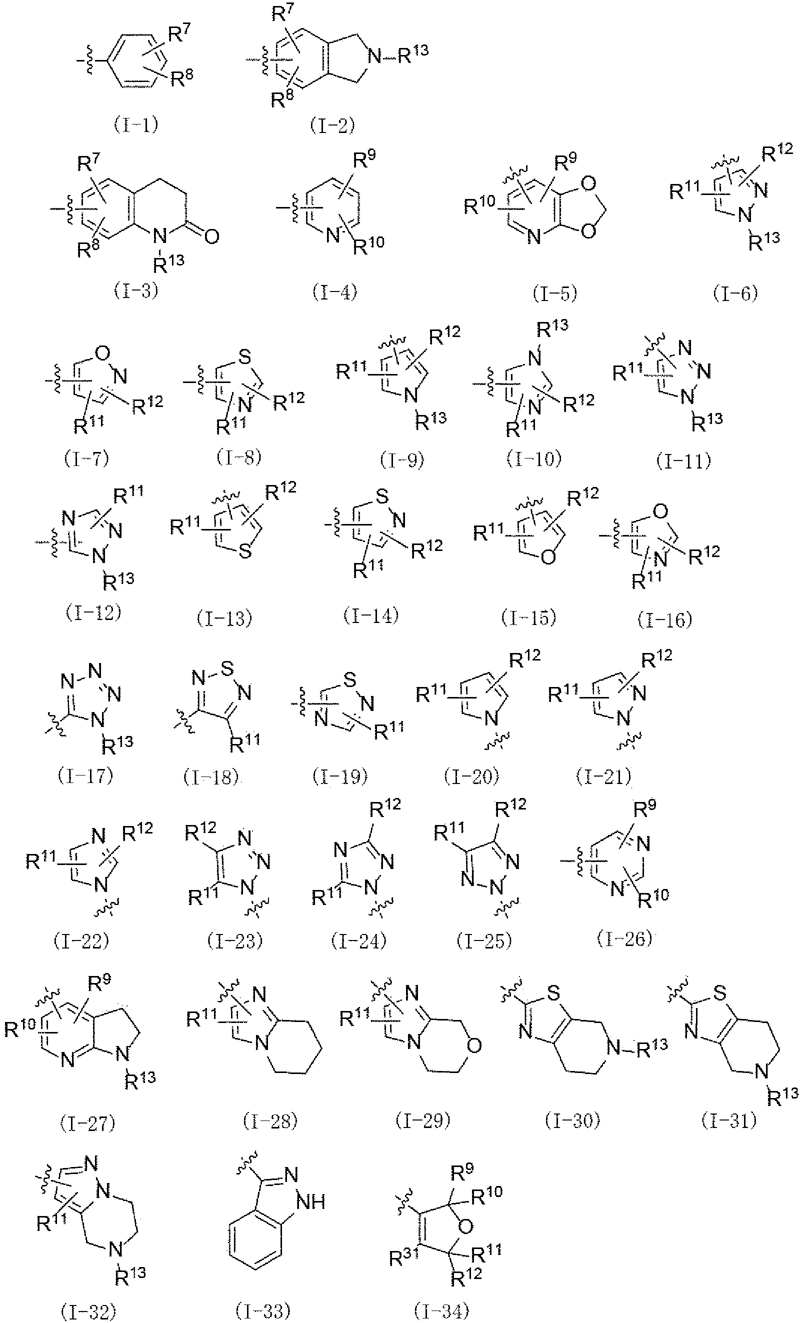 more preferably from the group consisting of:

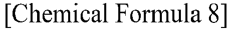

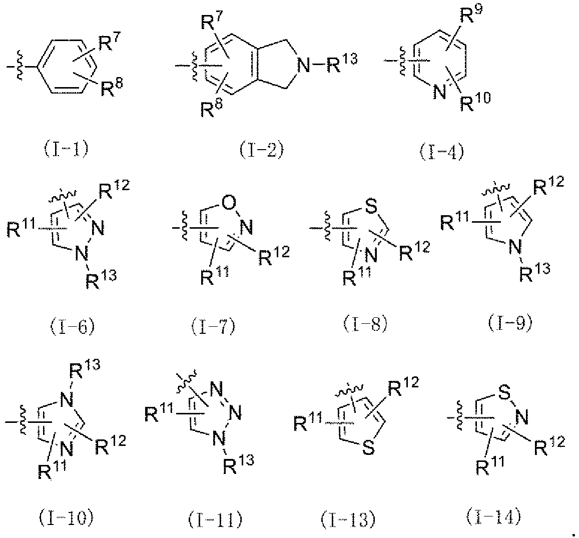

In each formula,
R⁷ is each independently hydrogen, halogen, nitro, cyano, hydroxy, C₁₋₆ alkyl, C₁₋₆ alkoxy, or -NR³¹₂;
R⁸ is each independently selected from the group consisting of:
   (1) hydrogen, halogen, nitro, cyano, hydroxy;
   (2)
      (i) -NR³¹₂,
      (ii) -N(R³¹)S(O)₂ R³²⁻¹, where R³²⁻¹ is an optionally substituted 4- to 10-membered heterocyclyl having 1 to 3 heteroatoms selected from nitrogen and oxygen as ring-constituting atoms, where the heterocyclyl is preferably optionally substituted with C₁₋₃ alkyl, -(CH₂)₁₋₃OH, - NR³¹₂, -CHO, -C(O)C(O)R³³, -C(O)NR³¹₂, -C(O)N(R³¹)(CH₂)₁₋₅ 3- to 10-membered cycloaliphatic hydrocarbon or acetyl, and R³²⁻¹ is preferably a heterocyclyl of formula: 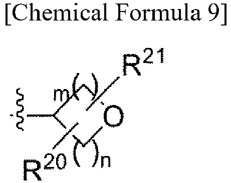 wherein R²⁰ and R²¹ are each independently selected from the group consisting of hydrogen, C₁₋₃ alkyl, -(CH₂)₁₋₃OH, -NR³¹₂, -CHO, -C(O)C(O)R³³, -C(O)NR³¹₂, - C(O)N(R³¹)(CH₂)₁₋₅ 3- to 10-membered cycloaliphatic hydrocarbon and acetyl, preferably hydrogen or methyl; and m and n are each independently an integer of 1 to 4, preferably an integer of 1 to 2,
      (iii) -N(R³¹)C(O)R³¹,
      (iv) -N(R³¹)C(O)(CH₂)₀₋₃NR³¹₂,
      (v) -N(R³¹)C(O)(CH₂)₀₋₃R³²⁻², where R³²⁻² is an optionally substituted 4- to 10-membered heterocyclyl having 1 to 3 heteroatoms selected from nitrogen and oxygen as ring-constituting atoms, where the heterocyclyl is preferably optionally substituted with C₁₋₃ alkyl, - (CH₂)₁₋₃OH, -NR³¹₂, -CHO, -C(O)C(O)R³³, -C(O)NR³¹₂, -C(O)N(R³¹)(CH₂)₁₋₅ 3- to 10-membered cycloaliphatic hydrocarbon or acetyl, R³²⁻² is preferably a heterocyclyl of formula: 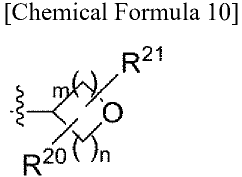 wherein R²⁰ and R²¹ are each independently selected from the group consisting of hydrogen, C₁₋₃ alkyl, -(CH₂)₁₋₃OH, -NR³¹₂, -CHO, -C(O)C(O)R³³, -C(O)NR³¹₂, - C(O)N(R³¹)(CH₂)₁₋₅ 3- to 10-membered cycloaliphatic hydrocarbon and acetyl, preferably hydrogen or methyl; and m and n are each independently an integer of 1 to 4, preferably an integer of 1 to 2;
   (3)
      (i) C₁₋₆ alkyl,
      (ii) -(CH₂)₁₋₅OR³¹,
      (iii) -(CH₂)₁₋₅R³²⁻³, where R³²⁻³ is an optionally substituted 4- to 10-membered heterocyclyl having 1 to 3 heteroatoms selected from nitrogen and oxygen as ring-constituting atoms, where the heterocyclyl is preferably optionally substituted with C₁₋₃ alkyl, -(CH₂)₁₋₃OH, - NR³¹₂, -CHO, -C(O)C(O)R³³, -C(O)NR³¹₂, -C(O)N(R³¹)(CH₂)₁₋₅ 3- to 10-membered cycloaliphatic hydrocarbon or acetyl, and R³²⁻³ is preferably a heterocyclyl of formula: 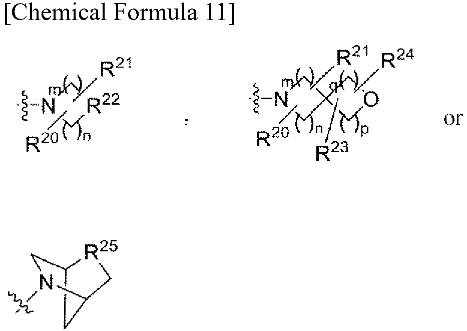
         wherein R²⁰ and R²¹ are each independently selected from the group consisting of hydrogen, C₁₋₃ alkyl, -(CH₂)₁₋₃OH, -NR³¹₂, -CHO, -C(O)C(O)R³³, -C(O)NR³¹₂, - C(O)N(R³¹)(CH₂)₁₋₅ 3- to 10-membered cycloaliphatic hydrocarbon and acetyl, preferably selected from the group consisting of hydrogen, methyl, -(CH₂)₁₋₃OH, -C(O)C(O)R³³ and - C(O)NR³¹₂,
         R²³ and R²⁴ are each independently selected from the group consisting of hydrogen and C₁₋₁₂ chain aliphatic hydrocarbon, preferably hydrogen or methyl,
         R²² is selected from the group consisting of O, SO₂, CR³¹₂, and NR²²⁻¹, where R²²⁻¹ is selected from the group consisting of a C₁₋₁₂ chain aliphatic hydrocarbon, -(CH₂)₁₋₅OH, - C(O)R³¹, -C(O)OR³¹, -C(O)C(O)R³³, -C(O)NR³¹₂, and -C(O)N(R³¹)(CH₂)₁₋₅R³³, more preferably selected from the group consisting of C₁₋₃ alkyl, -(CH₂)₁₋₃OH, -CHO, -C(O)C(O)R³³, - C(O)NR³¹₂, -C(O)N(R³¹)(CH₂)₁₋₅ 3- to 10-membered cycloaliphatic hydrocarbon and acetyl, R²² is preferably O, CR³¹₂, or NR²²⁻², where R²²⁻² is selected from the group consisting of C₁₋₃ alkyl and -C(O)C(O)R³³,
         R²⁵ is selected from the group consisting of O, SO₂, CR³¹₂, and NR³¹, preferably NR³¹,
         R³¹ is preferably hydrogen or methyl, more preferably hydrogen, and
         m, n, p and q are each independently an integer of 1 to 4;
   (4)
      (i) -(CH₂)₁₋₅NR³¹₂,
      (ii) -(CH₂)₁₋₅N(R³¹)R³²⁻⁴, where R³²⁻⁴ is an optionally substituted 3- to 10-membered cycloaliphatic hydrocarbon or an optionally substituted 4- to 10-membered heterocyclyl having 1 to 3 heteroatoms selected from nitrogen and oxygen as ring-constituting atoms, wherein the cycloaliphatic hydrocarbon and the heterocyclyl are preferably each independently optionally substituted with C₁₋₃ alkyl, -(CH₂)₁₋₃OH, -NR³¹₂, -CHO, -C(O)C(O)R³³, -C(O)NR³¹₂, - C(O)N(R³¹)(CH₂)₁₋₅ 3- to 10-membered cycloaliphatic hydrocarbon or acetyl, and R³²⁻⁴ is preferably a cycloaliphatic hydrocarbon or heterocyclyl of formula: 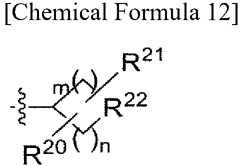
         wherein R²⁰ and R²¹ are each independently selected from the group consisting of hydrogen, C₁₋₃ alkyl, -(CH₂)₁₋₃OH, -NR³¹₂, -CHO, -C(O)C(O)R³³, -C(O)NR³¹₂, - C(O)N(R³¹)(CH₂)₁₋₅ 3- to 10-membered cycloaliphatic hydrocarbon and acetyl, preferably hydrogen or methyl,
         R²² is selected from the group consisting of O, SO₂, CR³¹₂, and NR³¹, preferably NR³¹,
         R³¹ is preferably hydrogen or methyl,
         m and n are each independently an integer of 1 to 4,
      (iii) -(CH₂)₁₋₅N(R³¹)C(O)R³¹,
      (iv) -(CH₂)₁₋₅N(R³¹)C(O)(CH₂)₁₋₅OR³¹,
      (v) -(CH₂)₁₋₅N(C(O)R³¹)(CH₂)₁₋₅C(O)OR³¹,
      (vi) -(CH₂)₁₋₅N(R³¹)C(O)(CH₂)₀₋₅NR³¹₂,
      (vii) -(CH₂)₁₋₅N(R³¹)C(O)R³²⁻⁵, where R³²⁻⁵ is an optionally substituted 3- to 10-membered cycloaliphatic hydrocarbon or an optionally substituted 4- to 10-membered heterocyclyl having 1 to 3 heteroatoms selected from nitrogen and oxygen as ring-constituting atoms, wherein the cycloaliphatic hydrocarbon and the heterocyclyl are preferably each independently optionally substituted with C₁₋₃ alkyl, -(CH₂)₁₋₃OH, -NR³¹₂, -CHO, -C(O)C(O)R³³, -C(O)NR³¹₂, -C(O)N(R³¹)(CH₂)₁₋₅ 3- to 10-membered cycloaliphatic hydrocarbon or acetyl, and R³²⁻⁵ is preferably a cycloaliphatic hydrocarbon or heterocyclyl of formula: 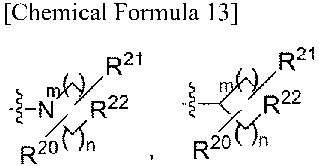
         wherein R²⁰ and R²¹ are each independently selected from the group consisting of hydrogen, C₁₋₃ alkyl, -(CH₂)₁₋₃OH, -NR³¹₂, -CHO, -C(O)C(O)R³³, -C(O)NR³¹₂, - C(O)N(R³¹)(CH₂)₁₋₅ 3- to 10-membered cycloaliphatic hydrocarbon and acetyl, preferably hydrogen or methyl,
         R²² is selected from the group consisting of O, SO₂, CR³¹₂, and NR³¹, preferably O,
         R³¹ is preferably hydrogen or methyl,
         m and n are each independently an integer of 1 to 4;
   (5) -(CH₂)₁₋₅N(R³¹)S(O)₂R³³⁻¹, where R³³⁻¹ is a 3- to 10-membered cycloalkyl;
   (6) -C(R³¹)₂C(O)R³²⁻⁶, where R³²⁻⁶ is an optionally substituted 4- to 10-membered heterocyclyl having 1 to 3 heteroatoms selected from nitrogen and oxygen as ring-constituting atoms, where the heterocyclyl is preferably optionally substituted with C₁₋₃ alkyl, -(CH₂)₁₋₃OH, - NR³¹₂, -CHO, -C(O)C(O)R³³, -C(O)NR³¹₂, -C(O)N(R³¹)(CH₂)₁₋₅ 3- to 10-membered cycloaliphatic hydrocarbon or acetyl, and R³²⁻⁶ is preferably a heterocyclyl of formula: 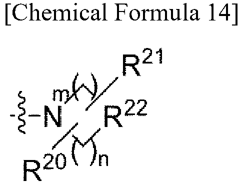
      wherein R²⁰ and R²¹ are each independently selected from the group consisting of hydrogen, C₁₋₃ alkyl, -(CH₂)₁₋₃OH, -NR³¹₂, -CHO, -C(O)C(O)R³³, -C(O)NR³¹₂, - C(O)N(R³¹)(CH₂)₁₋₅ 3- to 10-membered cycloaliphatic hydrocarbon and acetyl, preferably hydrogen or methyl,
      R²² is selected from the group consisting of O, SO₂, CR³¹₂, and NR³¹, preferably NR³¹,
      R³¹ is preferably hydrogen or methyl,
      m and n are each independently an integer of 1 to 4;
   (7)
      (i) -C(R³¹)₂C(O)NR³¹₂,
      (ii) -C(R³¹)₂C(O)N(R³¹)OR³¹,
   (8) -C(O)R³²⁻⁷, where R³²⁻⁷ is a 4- to 10-membered heterocyclyl having 1 to 3 heteroatoms selected from nitrogen and oxygen as ring-constituting atoms and optionally substituted with C₁₋₃ alkyl, -(CH₂)₁₋₃OH, -NR³¹₂, -CHO, -C(O)C(O)R³³, -C(O)NR³¹₂, - C(O)N(R³¹)(CH₂)₁₋₅ 3- to 10-membered cycloaliphatic hydrocarbon or acetyl, or a fused ring in which the heterocyclyl is fused with a 5- or 6-membered monocyclic aromatic hydrocarbon optionally substituted with a C₁₋₁₂ chain aliphatic hydrocarbon, acetamide (-NHAc) or acetyl, and R³²⁻⁷ is preferably a heterocyclyl or fused ring of formula: 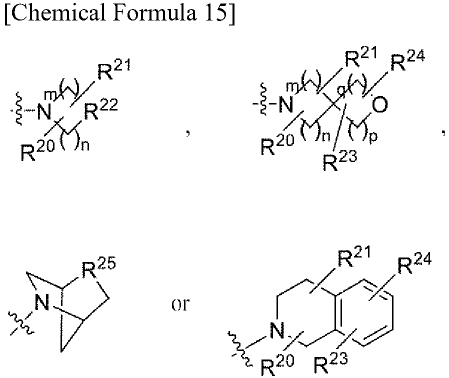
      wherein R²⁰ and R²¹ are each independently selected from the group consisting of hydrogen, C₁₋₃ alkyl, -(CH₂)₁₋₃OH, -NR³¹₂, -CHO, -C(O)C(O)R³³, -C(O)NR³¹₂, - C(O)N(R³¹)(CH₂)₁₋₅ 3- to 10-membered cycloaliphatic hydrocarbon and acetyl, preferably selected from the group consisting of hydrogen, C₁₋₃ alkyl, -NR³¹₂, -C(O)N(R³¹)(CH₂)₁₋₅ 3- to 10-membered cycloaliphatic hydrocarbon and acetyl,
      R²³ and R²⁴ are each independently selected from the group consisting of hydrogen, a C₁₋₁₂ chain aliphatic hydrocarbon, an acetamide group (-NHAc) or acetyl,
      R²² is selected from the group consisting of O, SO₂, CR³¹₂, CR³¹C(O)N(R³¹)(CH₂)₁₋₅ 3-to 10-membered cycloaliphatic hydrocarbon, NR³¹, and NCHO, preferably O, CR³¹₂, NR³¹, or NCHO, R³¹ is preferably hydrogen or C₁₋₃ alkyl,
      R²⁵ is selected from the group consisting of O, SO₂, CR³¹₂, and NR³¹, preferably NR³¹, R³¹ is preferably hydrogen or methyl, more preferably hydrogen, and
      m, n, p and q are each independently an integer of 1 to 4;
   (9) -C(O)OR³¹;
   (10)
      (i)-C(O)NR³¹₂,
      (ii)-C(O)N(R³¹)(CH₂)₀₋₅R³²⁻⁸, where R³²⁻⁸ is an optionally substituted 3- to 10-membered cycloaliphatic hydrocarbon or an optionally substituted 4- to 10-membered heterocyclyl having 1 to 3 heteroatoms selected from nitrogen and oxygen as ring-constituting atoms, wherein the cycloaliphatic hydrocarbon and the heterocyclyl are preferably each independently optionally substituted with C₁₋₃ alkyl, -(CH₂)₁₋₃OH, -NR³¹₂, -CHO, -C(O)C(O)R³³, -C(O)NR³¹₂, -C(O)N(R³¹)(CH₂)₁₋₅ 3- to 10-membered cycloaliphatic hydrocarbon or acetyl, and R³²⁻⁸ is preferably a cycloaliphatic hydrocarbon or heterocyclyl of formula: 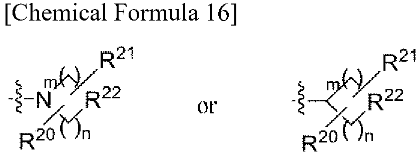
         wherein R²⁰ and R²¹ are each independently selected from the group consisting of hydrogen, C₁₋₃ alkyl, -(CH₂)₁₋₃OH, -NR³¹₂, -CHO, -C(O)C(O)R³³, -C(O)NR³¹₂, - C(O)N(R³¹)(CH₂)₁₋₅ 3- to 10-membered cycloaliphatic hydrocarbon and acetyl, preferably hydrogen or methyl,
         R²² is selected from the group consisting of O, SO₂, CR³¹₂, and NR³¹, preferably O or NR³¹,
         R³¹ is preferably hydrogen or methyl,
         m and n are each independently an integer of 1 to 4;
   (11)
      (i) -OC(R³¹)₂C(O)N(R³¹)(CH₂)₁₋₅OR³¹,
      (ii) -OC(R³¹)₂C(O)OR³¹,
      (iii) -OC(R³¹)₂C(O)NR³¹₂,
      (iv) -OC(R³¹)₂C(O)R³²⁻⁹, where R³²⁻⁹ is an optionally substituted 4- to 10-membered heterocyclyl having 1 to 3 heteroatoms selected from nitrogen and sulfur as ring-constituting atoms, wherein the heterocyclyl is preferably optionally substituted with C₁₋₃ alkyl, -(CH₂)₁₋₃OH, -CHO, -C(O)C(O)R³³, -C(O)NR³¹₂, -C(O)N(R³¹)(CH₂)₁₋₅ 3- to 10-membered cycloaliphatic hydrocarbon or acetyl, and R³²⁻⁹ is preferably a heterocyclyl of formula: 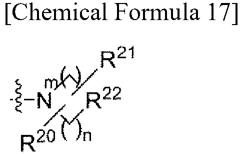
         wherein R²⁰ and R²¹ are each independently selected from the group consisting of hydrogen, C₁₋₃ alkyl, -(CH₂)₁₋₃OH, -NR³¹₂, -CHO, -C(O)C(O)R³³, -C(O)NR³¹₂, - C(O)N(R³¹)(CH₂)₁₋₅ 3- to 10-membered cycloaliphatic hydrocarbon and acetyl, preferably selected from the group consisting of hydrogen, C₁₋₃ alkyl, and -(CH₂)₁₋₃OH,
         R²² is selected from the group consisting of O, SO₂, CR³¹₂, and NR³¹, preferably selected from the group consisting of SO₂, CR³¹₂, and NR³¹,
         R³¹ is preferably hydrogen or methyl,
         m and n are each independently an integer of 1 to 4;
   (12)
      (i) -O(CH₂)₁₋₆R³¹,
      (ii) -(O(CH₂)₁₋₅)₁₋₅(CH₂)₀₋₃NR³¹₂,
      (iii) -(O(CH₂)₁₋₅)₁₋₅(CH₂)₀₋₃NR³¹C(O)R³¹,
      (iv) -(O(CH₂)₁₋₅)₁₋₅NR³¹C(NH)NR³¹₂,
      (v) -O((CH₂)₁₋₅O)₁₋₅R³¹,
   (13) -OC(O)C(R³¹)₂(O(CH₂)₁₋₅)₁₋₅OR³¹, and
   (14) a 5- or 6-membered nitrogen-containing heterocycle optionally substituted with C₁₋₃ alkyl, preferably a nitrogen-containing heterocycle of formula: 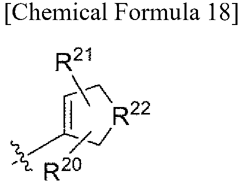
      wherein R²⁰ and R²¹ are each independently selected from the group consisting of hydrogen and C₁₋₃ alkyl, preferably hydrogen or methyl, and
      R²² is NR³¹.

R⁹, R¹⁰, R¹¹ and R¹² are each independently selected from the group consisting of hydrogen, halogen, cyano, hydroxy, C₁₋₅ alkyl, 3- to 6-membered cycloalkyl, C₁₋₅ alkoxy, - N(R³¹)C(O)R³¹, -N(R³¹)C(O)(CH₂)₀₋₃NR³¹₂, -N(R³¹)(CH₂)₁₋₅NR³¹₂, -(CH₂)₁₋₅OR³¹, -(CH₂)₁₋₅O(CH₂)₁₋₅OR³¹, -(CH₂)₁₋₅R³⁵, -(CH₂)₁₋₅N(R³¹)C(O)R³¹, -(CH₂)₁₋₅C(O)NR³¹₂, -C(R³¹)₂NR³¹₂, - (CH₂)₁₋₅NR³¹₂, -C(O)R³⁶, -C(O)OR³¹, -C(O)NR³¹₂, -NR³¹₂, -C(H)₂R³⁵, -C(H)R³⁵₂, and -CR³⁵₃,
R³¹ is each independently hydrogen or a C₁₋₄ chain aliphatic hydrocarbon;
R³⁵ is each independently halogen, cyano, a 3- to 10-membered cycloaliphatic hydrocarbon optionally substituted with C₁₋₅ alkyl (preferably, an unsubstituted 3- to 5-membered cycloaliphatic hydrocarbon), or -OR³¹; and
R³⁶ is a 4- to 10-membered heterocyclyl having 1 to 3 heteroatoms each independently selected from nitrogen, oxygen and sulfur as ring-constituting atoms and optionally substituted with C₁₋₅ alkyl, preferably a heterocyclyl of formula: 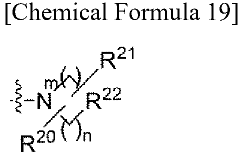
wherein R²⁰ and R²¹ are each independently selected from the group consisting of hydrogen and C₁₋₃ alkyl, preferably hydrogen,
R²² is selected from the group consisting of O, SO₂, CR³¹₂, and NR³¹, preferably NR³¹, and R³¹ is preferably selected from the group consisting of hydrogen and C₁₋₃ alkyl, more preferably hydrogen or methyl,
m and n are each independently an integer of 1 to 4, preferably an integer of 1 to 2.

R¹³ is each independently hydrogen, C₁₋₅ alkyl, -(CH₂)₁₋₅OR³¹, -(CH₂)₁₋₅NR³¹₂, -(CH₂)₁₋₅N(R³¹)C(O)R³¹, -(CH₂)₁₋₅N(R³¹)(CH₂)₀₋₅R³⁸, -C(O)R³¹, -C(O)R³⁷ or -(CH₂)₁₋₅R³⁷;
R³⁷ is a 3- to 10-membered cycloaliphatic hydrocarbon or a 3- to 10-membered heterocyclyl having 1 to 3 heteroatoms selected from nitrogen and oxygen; and
R³⁸ is cyano, hydroxy, or S(O)₂R³¹.

The heterocyclic rings in the structures represented by formulas (I-2), (I-3) and (I-27) to (I-32) and the benzene ring in the structure represented by (I-33) may each independently be optionally substituted with a C₁₋₅ chain aliphatic hydrocarbon, preferably may be optionally substituted with C₁₋₃ alkyl, and are particularly preferably unsubstituted.

In certain embodiments of the present invention, specific examples of R⁶ include the following structures.

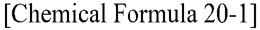

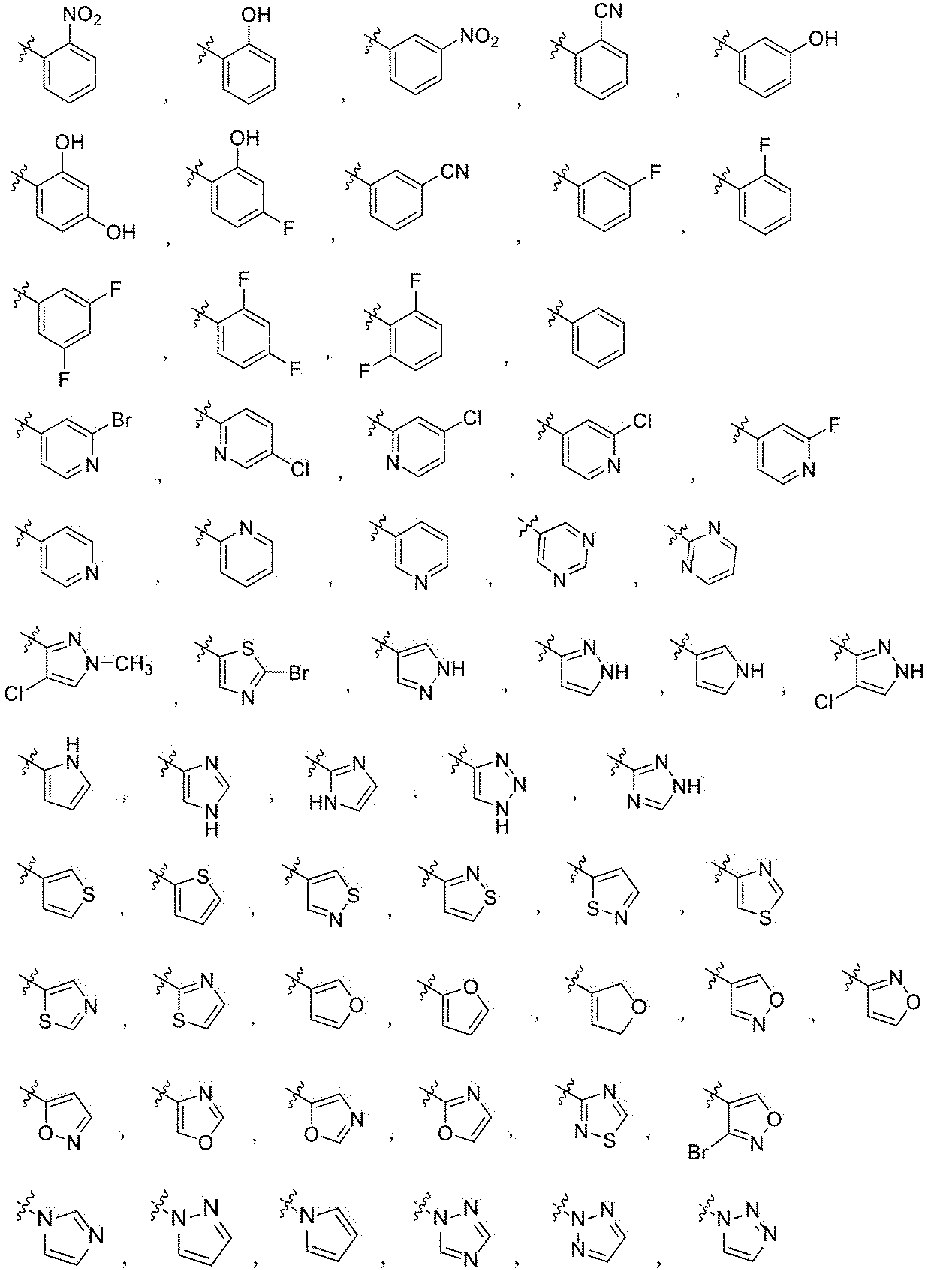

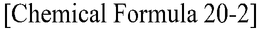

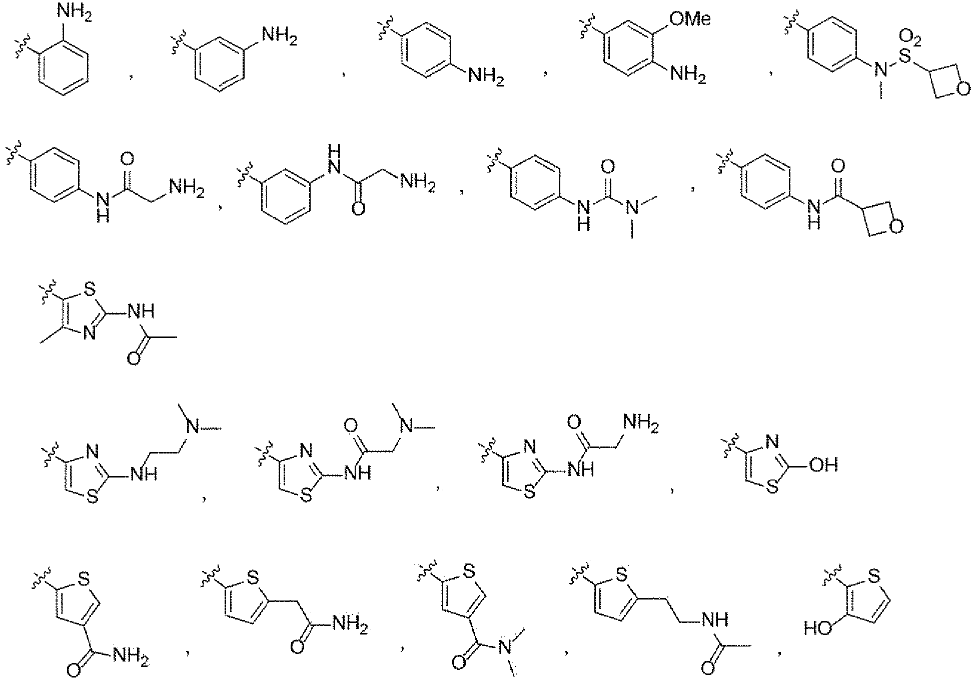

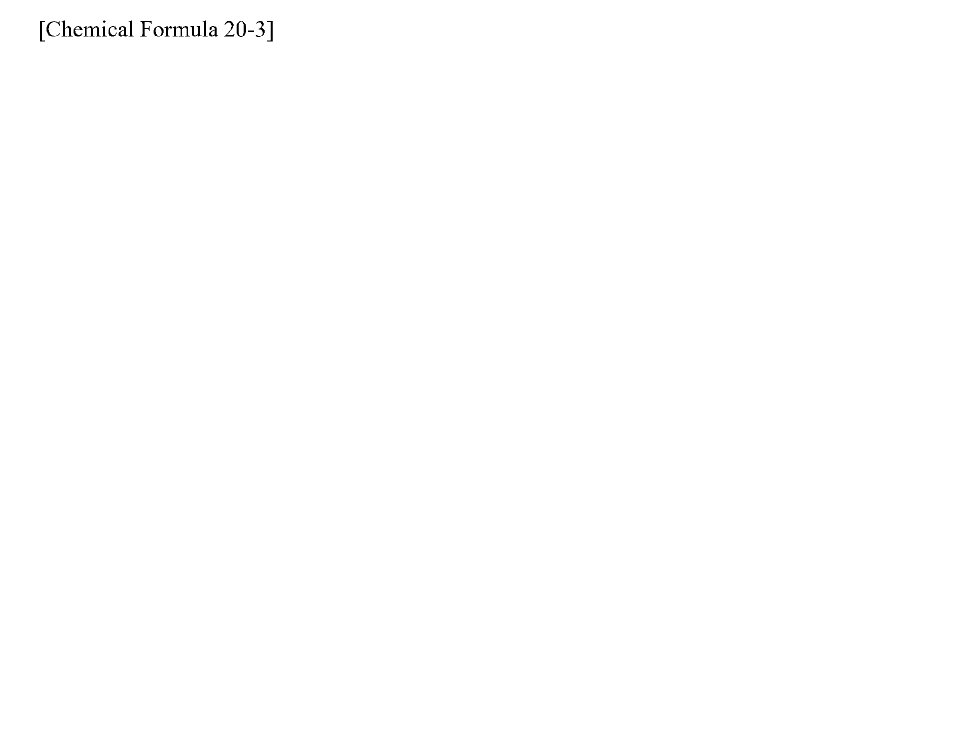

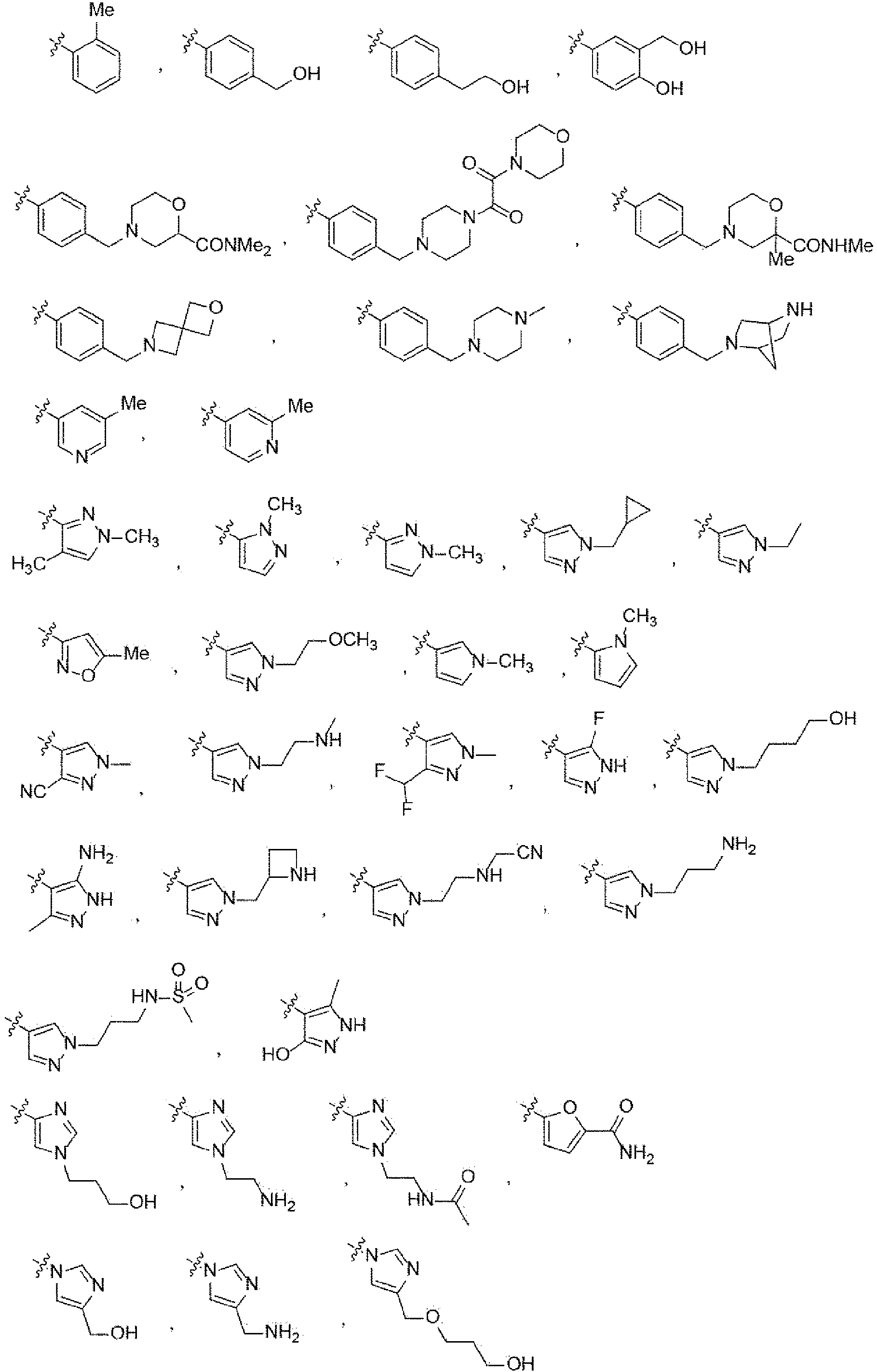

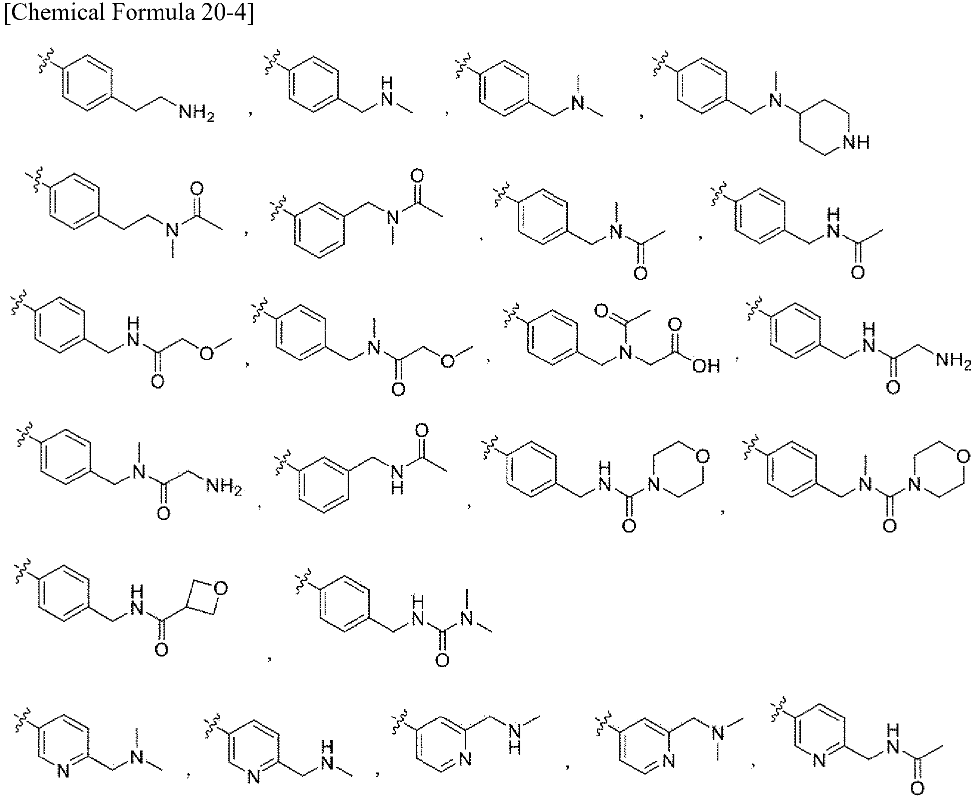

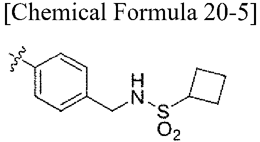

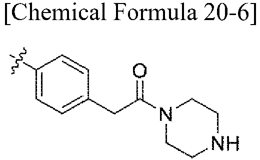

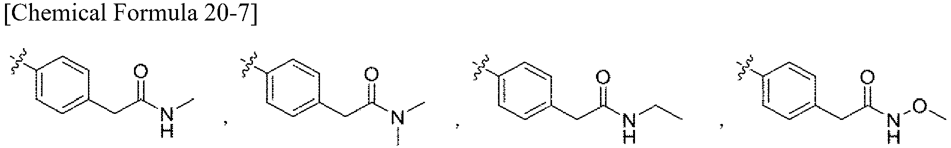

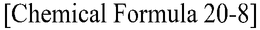

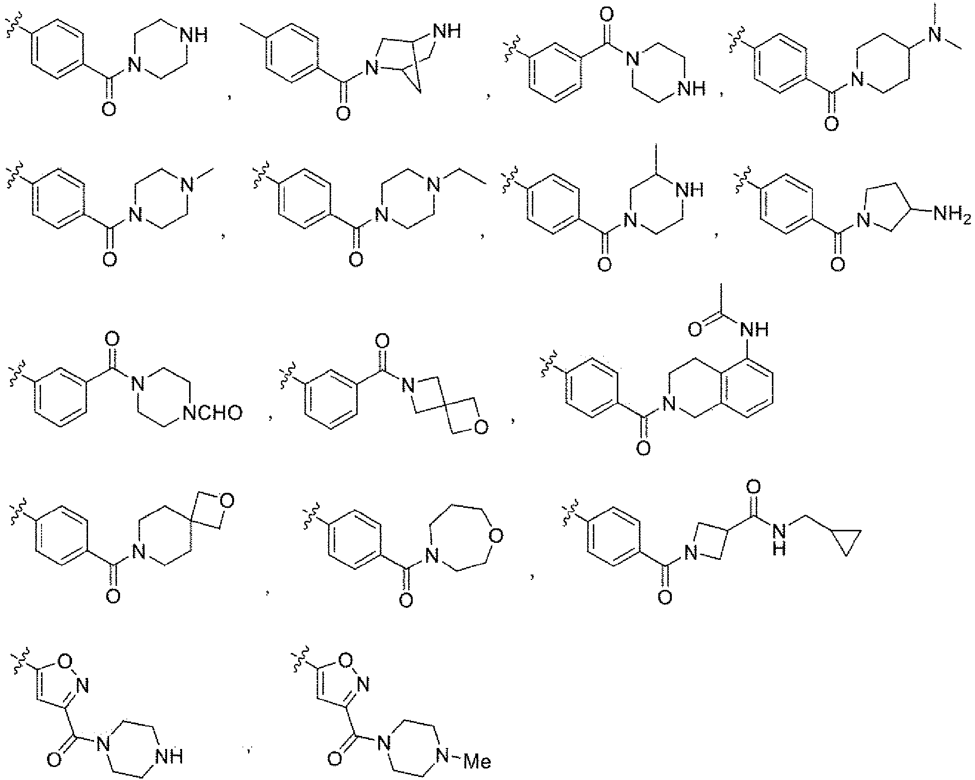

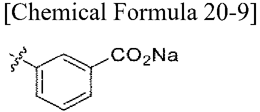

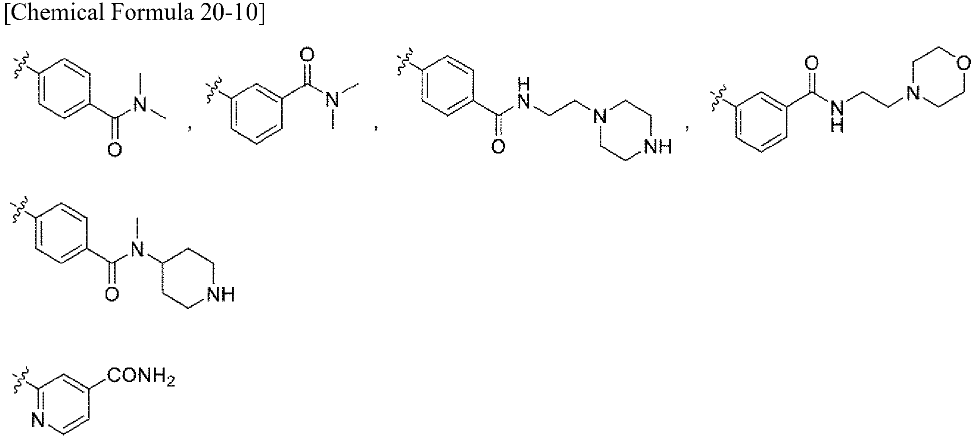

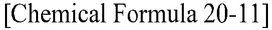

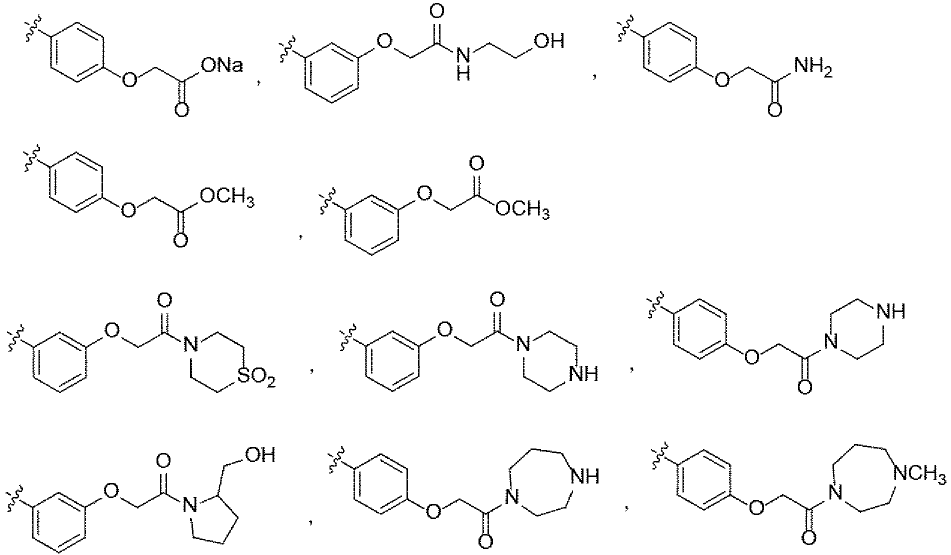

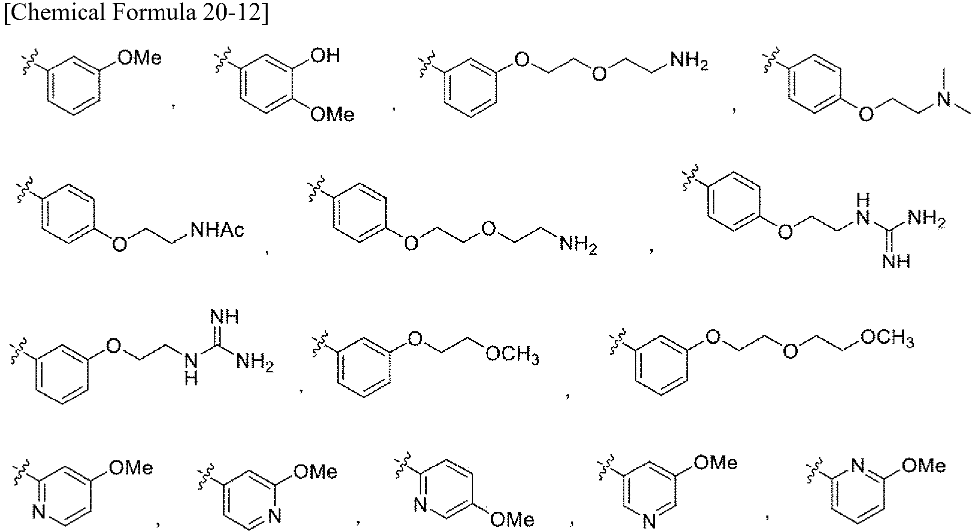

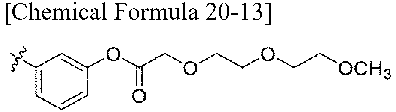

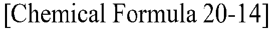

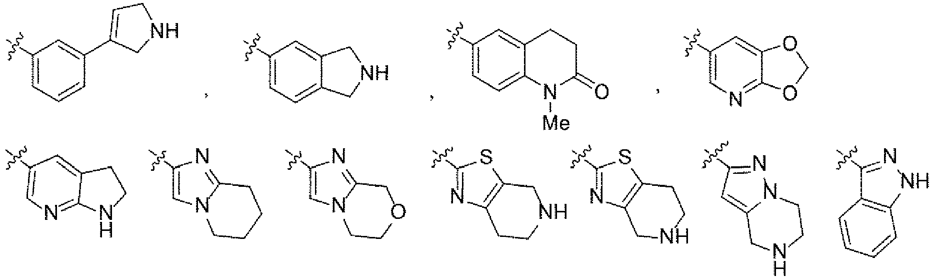

Specific examples of the present compound include the following compounds:
3-(2-(pyrazolo[1,5-a]pyridin-3-yl)thiazol-4-yl)phenol (compound 3),
4-phenyl-2-(pyrazolo[1,5-a]pyridin-3-yl)thiazole (compound 71),
2-methoxy-5-(2-(pyrazolo[1,5-a]pyridin-3-yl)thiazol-4-yl)phenol (compound 79),
4-(3-fluorophenyl)-2-(pyrazolo[1,5-a]pyridin-3-yl)thiazole (compound 112),
4-(2-fluorophenyl)-2-(pyrazolo[1,5-a]pyridin-3-yl)thiazole (compound 122),
4-(2-(pyrazolo[1,5-a]pyridin-3-yl)thiazol-4-yl)benzene-1,3-diol (compound 120),
4-(2,4-difluorophenyl)-2-(pyrazolo[1,5-a]pyridin-3-yl)thiazole (compound 121),
4-(3-methoxyphenyl)-2-(pyrazolo[1,5-a]pyridin-3-yl)thiazole (compound 83),
2-(pyrazolo[1,5-a]pyridin-3-yl)-4-(o-tolyl)thiazole (compound 132),
1-methyl-6-(2-(pyrazolo[1,5-a]pyridin-3-yl)thiazol-4-yl)-3,4-dihydroquinolin-2(1H)-one (compound 131),
N-(4-(2-(pyrazolo[1,5-a]pyridin-3-yl)thiazol-4-yl)benzyl)acetamide (compound 12),
4-(4-methoxypyridin-2-yl)-2-(pyrazolo[1,5-a]pyridin-3-yl)thiazole (compound 7),
2-(pyrazolo[1,5-a]pyridin-3-yl)-4-(pyridin-3-yl)thiazole (compound 15),
2-(pyrazolo[1,5-a]pyridin-3-yl)-4-(pyridin-4-yl)thiazole (compound 92),
2-(pyrazolo[1,5-a]pyridin-3-yl)-4-(pyridin-2-yl)thiazole (compound 93),
4-(2-chloropyridin-4-yl)-2-(pyrazolo[1,5-a]pyridin-3-yl)thiazole (compound 94),
4-(2-methoxypyridin-4-yl)-2-(pyrazolo[1,5-a]pyridin-3-yl)thiazole (compound 95),
4-(6-methoxypyridin-2-yl)-2-(pyrazolo[1,5-a]pyridin-3-yl)thiazole (compound 96),
4-(5-methylpyridin-3-yl)-2-(pyrazolo[1,5-a]pyridin-3-yl)thiazole (compound 97),
6-(2-(pyrazolo[1,5-a]pyridin-3-yl)thiazol-4-yl)-[1,3]dioxolo[4,5-b]pyridine (compound 98),
4-(2-methylpyridin-4-yl)-2-(pyrazolo[1,5-a]pyridin-3-yl)thiazole (compound 99),
4-(2-fluoropyridin-4-yl)-2-(pyrazolo[1,5-a]pyridin-3-yl)thiazole (compound 100),
4-(2-bromopyridin-4-yl)-2-(pyrazolo[1,5-a]pyridin-3-yl)thiazole (compound 101),
4-(5-chloropyridin-2-yl)-2-(pyrazolo[1,5-a]pyridin-3-yl)thiazole (compound 125),
4-(4-chloropyridin-2-yl)-2-(pyrazolo[1,5-a]pyridin-3-yl)thiazole (compound 135),
2'-bromo-2-(pyrazolo[1,5-a]pyridin-3-yl)-4,5'-bithiazole (compound 37),
4-(2-(pyrazolo[1,5-a]pyridin-3-yl)thiazol-4-yl)aniline hydrochloride (compound 1),
4-(3-nitrophenyl)-2-(pyrazolo[1,5-a]pyridin-3-yl)thiazole (compound 4),
3-(2-(pyrazolo[1,5-a]pyridin-3-yl)thiazol-4-yl)aniline hydrochloride (compound 10),
4-(2-nitrophenyl)-2-(pyrazolo[1,5-a]pyridin-3-yl)thiazole (compound 133),
2-(2-(pyrazolo[1,5-a]pyridin-3-yl)thiazol-4-yl)aniline hydrochloride (compound 134),
2-methoxy-4-(2-(pyrazolo[1,5-a]pyridin-3-yl)thiazol-4-yl)aniline (compound 90),
2-(2-(pyrazolo[1,5-a]pyridin-3-yl)thiazol-4-yl)phenol (compound 118),
3-(2-(pyrazolo[1,5-a]pyridin-3-yl)thiazol-4-yl)benzonitrile (compound 115),
2-(2-(pyrazolo[1,5-a]pyridin-3-yl)thiazol-4-yl)benzonitrile (compound 116),
4-(3,5-difluorophenyl)-2-(pyrazolo[1,5-a]pyridin-3-yl)thiazole (compound 119),
4-(2,6-difluorophenyl)-2-(pyrazolo[1,5-a]pyridin-3-yl)thiazole (compound 123),
5-fluoro-2-(2-(pyrazolo[1,5-a]pyridin-3-yl)thiazol-4-yl)phenol (compound 114),
2-(2-(pyrazolo[1,5-a]pyridin-3-yl)thiazol-4-yl)isonicotinamide (compound 126),
4-(5-methoxypyridin-3-yl)-2-(pyrazolo[1,5-a]pyridin-3-yl)thiazole (compound 124),
4-(5-methoxypyridin-2-yl)-2-(pyrazolo[1,5-a]pyridin-3-yl)thiazole (compound 127),
4-(1H-pyrazol-4-yl)-2-(pyrazolo[1,5-a]pyridin-3-yl)thiazole (compound 107),
4-(4-chloro-1-methyl-1H-pyrazol-3-yl)-2-(pyrazolo[1,5-a]pyridin-3-yl)thiazole (compound 53),
4-(1,4-dimethyl-1H-pyrazol-3-yl)-2-(pyrazolo[1,5-a]pyridin-3-yl)thiazole hydrobromide (compound 54),
4-(1-methyl-1H-pyrazol-5-yl)-2-(pyrazolo[1,5-a]pyridin-3-yl)thiazole hydrobromide (compound 106),
4-(1H-pyrazol-3-yl)-2-(pyrazolo[1,5-a]pyridin-3-yl)thiazole (compound 109),
5-methyl-3-(2-(pyrazolo[1,5-a]pyridin-3-yl)thiazol-4-yl)isoxazole hydrobromide (compound 105),
N-methyl-N-(4-(2-(pyrazolo[1,5-a]pyridin-3-yl)thiazol-4-yl)phenyl)oxetane-3-sulfonamide (compound 69),
N-methyl-N-(4-(2-(pyrazolo[1,5-a]pyridin-3-yl)thiazol-4-yl)benzyl)acetamide (compound 16),
N-(4-(2-(pyrazolo[1,5-a]pyridin-3-yl)thiazol-4-yl)benzyl)morpholine-4-carboxamide (compound 72),
N-methyl-1-(4-(2-(pyrazolo[1,5-a]pyridin-3-yl)thiazol-4-yl)phenyl)methanamine hydrochloride (compound 74),
N-methyl-N-(4-(2-(pyrazolo[1,5-a]pyridin-3-yl)thiazol-4-yl)benzyl)morpholine-4-carboxamide (compound 76),
2-methoxy-N-methyl-N-(4-(2-(pyrazolo[1,5-a]pyridin-3-yl)thiazol-4-yl)benzyl)acetamide (compound 78),
1,1-dimethyl-3-(4-(2-(pyrazolo[1,5-a]pyridin-3-yl)thiazol-4-yl)phenyl)urea (compound 59),
1,1-dimethyl-3-(4-(2-(pyrazolo[1,5-a]pyridin-3-yl)thiazol-4-yl)benzyl)urea (compound 75),
2-methoxy-N-(4-(2-(pyrazolo[1,5-a]pyridin-3-yl)thiazol-4-yl)benzyl)acetamide (compound 77),
N-(4-(2-(pyrazolo[1,5-a]pyridin-3-yl)thiazol-4-yl)benzyl)cyclobutanesulfonamide (compound 82),
N-(4-(2-(pyrazolo[1,5-a]pyridin-3-yl)thiazol-4-yl)benzyl)oxetane-3-carboxamide (compound 85),
2-amino-N-(4-(2-(pyrazolo[1,5-a]pyridin-3-yl)thiazol-4-yl)phenyl)acetamide hydrochloride (compound 64),
N-(4-(2-(pyrazolo[1,5-a]pyridin-3-yl)thiazol-4-yl)phenyl)oxetane-3-carboxamide (compound 66),
2-amino-N-(3-(2-(pyrazolo[1,5-a]pyridin-3-yl)thiazol-4-yl)phenyl)acetamide hydrochloride (compound 65),
2-amino-N-methyl-N-(4-(2-(pyrazolo[1,5-a]pyridin-3-yl)thiazol-4-yl)benzyl)acetamide hydrochloride (compound 81),
2-amino-N-(4-(2-(pyrazolo[1,5-a]pyridin-3-yl)thiazol-4-yl)benzyl)acetamide hydrochloride (compound 80),
sodium 2-(4-(2-(pyrazolo[1,5-a]pyridin-3-yl)thiazol-4-yl)phenoxy)acetate (compound 5),
N-acetyl-N-(4-(2-(pyrazolo[1,5-a]pyridin-3-yl)thiazol-4-yl)benzyl)glycine (compound 73),
2-(4-(2-(pyrazolo[1,5-a]pyridin-3-yl)thiazol-4-yl)phenoxy)acetamide (compound 26),
3-(2-(pyrazolo[1,5-a]pyridin-3-yl)thiazol-4-yl)phenyl-2-(2-(2-methoxyethoxy)ethoxy)acetate (compound 13),
4-(3-(2-methoxyethoxy)phenyl)-2-(pyrazolo[1,5-a]pyridin-3-yl)thiazole (compound 14),
4-(3-(2-(2-methoxyethoxy)ethoxy)phenyl)-2-(pyrazolo[1,5-a]pyridin-3-yl)thiazole (compound 22),
4-(1-(cyclopropylmethyl)-1H-pyrazol-4-yl)-2-(pyrazolo[1,5-a]pyridin-3-yl)thiazole (compound 108),
4-(1-ethyl-1H-pyrazol-4-yl)-2-(pyrazolo[1,5-a]pyridin-3-yl)thiazole (compound 110),
4-(1-(2-methoxyethyl)-1H-pyrazol-4-yl)-2-(pyrazolo[1,5-a]pyridin-3-yl)thiazole hydrochloride (compound 111),
2-(2-(4-(2-(pyrazolo[1,5-a]pyridin-3-yl)thiazol-4-yl)phenoxy)ethoxy)ethan-1-amine hydrochloride (compound 32),
2-(2-(3-(2-(pyrazolo[1,5-a]pyridin-3-yl)thiazol-4-yl)phenoxy)ethoxy)ethan-1-amine hydrochloride (compound 20),
N,N-dimethyl-2-(4-(2-(pyrazolo[1,5-a]pyridin-3-yl)thiazol-4-yl)phenoxy)ethan-1-amine hydrochloride (compound 47),
1-(2-(3-(2-(pyrazolo[1,5-a]pyridin-3-yl)thiazol-4-yl)phenoxy)ethyl)guanidine hydrochloride (compound 52),
1-(2-(4-(2-(pyrazolo[1,5-a]pyridin-3-yl)thiazol-4-yl)phenoxy)ethyl)guanidine hydrochloride (compound 51),
N-(2-(4-(2-(pyrazolo[1,5-a]pyridin-3-yl)thiazol-4-yl)phenoxy)ethyl)acetamide (compound 45),
N-(2-hydroxyethyl)-2-(3-(2-(pyrazolo[1,5-a]pyridin-3-yl)thiazol-4-yl)phenoxy)acetamide (compound 19),
methyl 2-(3-(2-(pyrazolo[1,5-a]pyridin-3-yl)thiazol-4-yl)phenoxy)acetate (compound 18),
methyl 2-(4-(2-(pyrazolo[1,5-a]pyridin-3-yl)thiazol-4-yl)phenoxy)acetate (compound 25),
1-(piperazin-1-yl)-2-(4-(2-(pyrazolo[1,5-a]pyridin-3-yl)thiazol-4-yl)phenoxy)ethan-1-one (compound 31),
1-(1,4-diazepan-1-yl)-2-(4-(2-(pyrazolo[1,5-a]pyridin-3-yl)thiazol-4-yl)phenoxy)ethan-1-one hydrochloride (compound 55),
1-(4-methyl-1,4-diazepan-1-yl)-2-(4-(2-(pyrazolo[1,5-a]pyridin-3-yl)thiazol-4-yl)phenoxy)ethan-1-one hydrochloride (compound 102),
1-(2-(hydroxymethyl)pyrrolidin-1-yl)-2-(3-(2-(pyrazolo[1,5-a]pyridin-3-yl)thiazol-4-yl)phenoxy)ethan-1-one (compound 21),
1-(piperazin-1-yl)-2-(3-(2-(pyrazolo[1,5-a]pyridin-3-yl)thiazol-4-yl)phenoxy)ethan-1-one (compound 23),
1-(1,1-dioxidothiomorpholino)-2-(3-(2-(pyrazolo[1,5-a]pyridin-3-yl)thiazol-4-yl)phenoxy)ethan-1-one (compound 24),
(4-methylpiperazin-1-yl)(5-(2-(pyrazolo[1,5-a]pyridin-3-yl)thiazol-4-yl)isoxazol-3-yl)methanone hydrochloride (compound 103),
piperazin-1-yl(5-(2-(pyrazolo[1,5-a]pyridin-3-yl)thiazol-4-yl)isoxazol-3-yl)methanone hydrochloride (compound 104),
N-(4'-methyl-2-(pyrazolo[1,5-a]pyridin-3-yl)-[4,5'-bithiazole]-2'-yl)acetamide (compound 46),
N,N-dimethyl-4-(2-(pyrazolo[1,5-a]pyridin-3-yl)thiazol-4-yl)benzamide (compound 49),
sodium 3-(2-(pyrazolo[1,5-a]pyridin-3-yl)thiazol-4-yl)benzoate (compound 6),
(3-(2-(pyrazolo[1,5-a]pyridin-3-yl)thiazol-4-yl)phenyl)(2-oxa-6-azaspiro[3.3]heptan-6-yl)methanone (compound 33),
N,N-dimethyl-2-(4-(2-(pyrazolo[1,5-a]pyridin-3-yl)thiazol-4-yl)phenyl)acetamide (compound 87),
N-(cyclopropylmethyl)-1-(4-(2-(pyrazolo[1,5-a]pyridin-3-yl)thiazol-4-yl)benzoyl)azetidine-3-carboxamide (compound 9),
(4-(2-(pyrazolo[1,5-a]pyridin-3-yl)thiazol-4-yl)phenyl)(2-oxa-7-azaspiro[3.5]nonan-7-yl)methanone (compound 40),
(1,4-oxazepan-4-yl)(4-(2-(pyrazolo[1,5-a]pyridin-3-yl)thiazol-4-yl)phenyl)methanone (compound 41),
(4-(dimethylamino)piperidin-1-yl)(4-(2-(pyrazolo[1,5-a]pyridin-3-yl)thiazol-4-yl)phenyl)methanone (compound 48),
(4-methylpiperazin-1-yl)(4-(2-(pyrazolo[1,5-a]pyridin-3-yl)thiazol-4-yl)phenyl)methanone (compound 50),
(4-ethylpiperazin-1-yl)(4-(2-(pyrazolo[1,5-a]pyridin-3-yl)thiazol-4-yl)phenyl)methanone (compound 56),
(3-methylpiperazin-1-yl)(4-(2-(pyrazolo[1,5-a]pyridin-3-yl)thiazol-4-yl)phenyl)methanone (compound 58),
N,N-dimethyl-3-(2-(pyrazolo[1,5-a]pyridin-3-yl)thiazol-4-yl)benzamide (compound 61),
4-(3-(2-(pyrazolo[1,5-a]pyridin-3-yl)thiazol-4-yl)benzoyl)piperazine-1-carbaldehyde (compound 68),
N-(2-morpholinoethyl)-3-(2-(pyrazolo[1,5-a]pyridin-3-yl)thiazol-4-yl)benzamide (compound 30),
N-methyl-2-(4-(2-(pyrazolo[1,5-a]pyridin-3-yl)thiazol-4-yl)phenyl)acetamide (compound 88),
N-ethyl-2-(4-(2-(pyrazolo[1,5-a]pyridin-3-yl)thiazol-4-yl)phenyl)acetamide (compound 89),
N-methoxy-2-(4-(2-(pyrazolo[1,5-a]pyridin-3-yl)thiazol-4-yl)phenyl)acetamide (compound 91),
(2,5-diazabicyclo[2.2.1]heptan-2-yl)(4-(2-(pyrazolo[1,5-a]pyridin-3-yl)thiazol-4-yl)phenyl)methanone hydrochloride (compound 43),
N-(2-(piperazin-1-yl)ethyl)-4-(2-(pyrazolo[1,5-a]pyridin-3-yl)thiazol-4-yl)benzamide hydrochloride (compound 11),
(3-aminopyrrolidin-1-yl)(4-(2-(pyrazolo[1,5-a]pyridin-3-yl)thiazol-4-yl)phenyl)methanone hydrochloride (compound 42),
N-methyl-N-(piperidin-4-yl)-4-(2-(pyrazolo[1,5-a]pyridin-3-yl)thiazol-4-yl)benzamide hydrochloride (compound 44),
piperazin-1-yl(4-(2-(pyrazolo[1,5-a]pyridin-3-yl)thiazol-4-yl)phenyl)methanone hydrochloride (compound 57),
piperazin-1-yl(3-(2-(pyrazolo[1,5-a]pyridin-3-yl)thiazol-4-yl)phenyl)methanone hydrochloride (compound 63),
1-(piperazin-1-yl)-2-(4-(2-(pyrazolo[1,5-a]pyridin-3-yl)thiazol-4-yl)phenyl)ethan-1-one hydrochloride (compound 86),
N-(2-(4-(2-(pyrazolo[1,5-a]pyridin-3-yl)thiazol-4-yl)benzoyl)-1,2,3,4-tetrahydroisoquinolin-5-yl)acetamide (compound 60),
2-(hydroxymethyl)-4-(2-(pyrazolo[1,5-a]pyridin-3-yl)thiazol-4-yl)phenol (compound 8),
(4-(2-(pyrazolo[1,5-a]pyridin-3-yl)thiazol-4-yl)phenyl)methanol (compound 2),
2-(4-(2-(pyrazolo[1,5-a]pyridin-3-yl)thiazol-4-yl)phenyl)ethan-1-ol (compound 84),
4-(3-(2,5-dihydro-1H-pyrrol-3-yl)phenyl)-2-(pyrazolo[1,5-a]pyridin-3-yl)thiazole hydrochloride (compound 17),
6-(4-(2-(pyrazolo[1,5-a]pyridin-3-yl)thiazol-4-yl)benzyl)-2-oxa-6-azaspiro[3.3]heptane (compound 29),
N,N-dimethyl-1-(4-(2-(pyrazolo[1,5-a]pyridin-3-yl)thiazol-4-yl)phenyl)methanamine (compound 27),
4-(4-((4-methylpiperazin-1-yl)methyl)phenyl)-2-(pyrazolo[1,5-a]pyridin-3-yl)thiazole (compound 28),
N,N-dimethyl-4-(4-(2-(pyrazolo[1,5-a]pyridin-3-yl)thiazol-4-yl)benzyl)morpholine-2-carboxamide (compound 38),
N,2-dimethyl-4-(4-(2-(pyrazolo[1,5-a]pyridin-3-yl)thiazol-4-yl)benzyl)morpholine-2-carboxamide (compound 39),
1-morpholino-2-(4-(4-(2-(pyrazolo[1,5-a]pyridin-3-yl)thiazol-4-yl)benzyl)piperazin-1-yl)ethane-1,2-dione hydrochloride (compound 36),
4-(4-((2,5-diazabicyclo[2.2.1]heptan-2-yl)methyl)phenyl)-2-(pyrazolo[1,5-a]pyridin-3-yl)thiazole hydrochloride (compound 34),
N-methyl-N-(4-(2-(pyrazolo[1,5-a]pyridin-3-yl)thiazol-4-yl)benzyl)piperidin-4-amine hydrochloride (compound 35),
4-(isoindolin-5-yl)-2-(pyrazolo[1,5-a]pyridin-3-yl)thiazole hydrochloride (compound 117),
N-methyl-N-(4-(2-(pyrazolo[1,5-a]pyridin-3-yl)thiazol-4-yl)phenethyl)acetamide (compound 62),
2-(4-(2-(pyrazolo[1,5-a]pyridin-3-yl)thiazol-4-yl)phenyl)ethan-1-amine hydrochloride (compound 113),
N-(3-(2-(pyrazolo[1,5-a]pyridin-3-yl)thiazol-4-yl)benzyl)acetamide (compound 67),
N-methyl-N-(3-(2-(pyrazolo[1,5-a]pyridin-3-yl)thiazol-4-yl)benzyl)acetamide (compound 70),
N,N-dimethyl-1-(4-(2-(pyrazolo[1,5-a]pyridin-3-yl)thiazol-4-yl)pyridin-2-yl)methanamine (compound 137),
N,N-dimethyl-1-(5-(2-(pyrazolo[1,5-a]pyridin-3-yl)thiazol-4-yl)pyridin-2-yl)methanamine (compound 128),
N-methyl-1-(4-(2-(pyrazolo[1,5-a]pyridin-3-yl)thiazol-4-yl)pyridin-2-yl)methanamine (compound 136),
N-((5-(2-(pyrazolo[1,5-a]pyridin-3-yl)thiazol-4-yl)pyridin-2-yl)methyl)acetamide (compound 129),
N-methyl-1-(5-(2-(pyrazolo[1,5-a]pyridin-3-yl)thiazol-4-yl)pyridin-2-yl)methanamine (compound 130),
2-(pyrazolo[1,5-a]pyridin-3-yl)-4-(1H-pyrrol-3-yl)thiazole (compound 138),
2-(pyrazolo[1,5-a]pyridin-3-yl)-4-(1H-pyrrol-2-yl)thiazole (compound 139),
4-(1H-imidazol-4-yl)-2-(pyrazolo[1,5-a]pyridin-3-yl)thiazole (compound 140),
4-(1H-imidazol-2-yl)-2-(pyrazolo[1,5-a]pyridin-3-yl)thiazole (compound 141),
2-(pyrazolo[1,5-a]pyridin-3-yl)-4-(1H-1,2,3-triazol-4-yl)thiazole (compound 142),
2-(pyrazolo[1,5-a]pyridin-3-yl)-4-(1H-1,2,4-triazol-3-yl)thiazole (compound 143),
2-(pyrazolo[1,5-a]pyridin-3-yl)-4-(thiophen-3-yl)thiazole (compound 144),
2-(pyrazolo[1,5-a]pyridin-3-yl)-4-(thiophen-2-yl)thiazole (compound 145),
3-(2-(pyrazolo[1,5-a]pyridin-3-yl)thiazol-4-yl)isothiazole (compound 146),
4-(2-(pyrazolo[1,5-a]pyridin-3-yl)thiazol-4-yl)isothiazole (compound 147),
5-(2-(pyrazolo[1,5-a]pyridin-3-yl)thiazol-4-yl)isothiazole (compound 148),
2-(pyrazolo[1,5-a]pyridin-3-yl)-4,4'-bithiazole (compound 149),
2-(pyrazolo[1,5-a]pyridin-3-yl)-4,5'-bithiazole (compound 150),
2'-(pyrazolo[1,5-a]pyridin-3-yl)-2,4'-bithiazole (compound 151),
4-(furan-3-yl)-2-(pyrazolo[1,5-a]pyridin-3-yl)thiazole (compound 152),
4-(furan-2-yl)-2-(pyrazolo[1,5-a]pyridin-3-yl)thiazole (compound 153),
4-(2-(pyrazolo[1,5-a]pyridin-3-yl)thiazol-4-yl)isoxazole (compound 154),
3-(2-(pyrazolo[1,5-a]pyridin-3-yl)thiazol-4-yl)isoxazole (compound 155),
5-(2-(pyrazolo[1,5-a]pyridin-3-yl)thiazol-4-yl)isoxazole (compound 156),
4-(2-(pyrazolo[1,5-a]pyridin-3-yl)thiazol-4-yl)oxazole (compound 157),
5-(2-(pyrazolo[1,5-a]pyridin-3-yl)thiazol-4-yl)oxazole (compound 158),
2-(2-(pyrazolo[1,5-a]pyridin-3-yl)thiazol-4-yl)oxazole (compound 159),
4-(1-methyl-1H-pyrazol-3-yl)-2-(pyrazolo[1,5-a]pyridin-3-yl)thiazole (compound 160),
4-(1-methyl-1H-pyrrol-3-yl)-2-(pyrazolo[1,5-a]pyridin-3-yl)thiazole (compound 161),
3-(2-(pyrazolo[1,5-a]pyridin-3-yl)thiazol-4-yl)-1,2,4-thiadiazole (compound 162),
4-(1-methyl-1H-pyrrol-2-yl)-2-(pyrazolo[1,5-a]pyridin-3-yl)thiazole (compound 163),
3-bromo-4-(2-(pyrazolo[1,5-a]pyridin-3-yl)thiazol-4-yl)isoxazole (compound 164),
4-(1H-imidazol-1-yl)-2-(pyrazolo[1,5-a]pyridin-3-yl)thiazole (compound 165),
4-(1H-pyrazol-1-yl)-2-(pyrazolo[1,5-a]pyridin-3-yl)thiazole (compound 166),
2-(pyrazolo[1,5-a]pyridin-3-yl)-4-(1H-pyrrol-1-yl)thiazole (compound 167),
2-(pyrazolo[1,5-a]pyridin-3-yl)-4-(1H-1,2,4-triazol-1-yl)thiazole (compound 168),
2-(pyrazolo[1,5-a]pyridin-3-yl)-4-(2H-1,2,3-triazol-2-yl)thiazole (compound 169),
2-(pyrazolo[1,5-a]pyridin-3-yl)-4-(1H-1,2,3-triazol-1-yl)thiazole (compound 170),
5-(2-(pyrazolo[1,5-a]pyridin-3-yl)thiazol-4-yl)thiophene-3-carboxamide (compound 171),
2-(5-(2-(pyrazolo[1,5-a]pyridin-3-yl)thiazol-4-yl)thiophen-2-yl)acetamide (compound 172),
N,N-dimethyl-5-(2-(pyrazolo[1,5-a]pyridin-3-yl)thiazol-4-yl)thiophene-3-carboxamide (compound 173),
N-(2-(5-(2-(pyrazolo[1,5-a]pyridin-3-yl)thiazol-4-yl)thiophen-2-yl)ethyl)acetamide (compound 174),
4-(3-(difluoromethyl)-1-methyl-1H-pyrazol-4-yl)-2-(pyrazolo[1,5-a]pyridin-3-yl)thiazole (compound 178),
2-(2-(pyrazolo[1,5-a]pyridin-3-yl)thiazol-4-yl)thiophen-3-ol (compound 175),
1-methyl-4-(2-(pyrazolo[1,5-a]pyridin-3-yl)thiazol-4-yl)-1H-pyrazole-3-carbonitrile (compound 176),
N-methyl-2-(4-(2-(pyrazolo[1,5-a]pyridin-3-yl)thiazol-4-yl)-1H-pyrazol-1-yl)ethan-1-amine (compound 177),
4-(5-fluoro-1H-pyrazol-4-yl)-2-(pyrazolo[1,5-a]pyridin-3-yl)thiazole (compound 179),
3-methyl-4-(2-(pyrazolo[1,5-a]pyridin-3-yl)thiazol-4-yl)-1H-pyrazol-5-amine (compound 181),
4-(4-(2-(pyrazolo[1,5-a]pyridin-3-yl)thiazol-4-yl)-1H-pyrazol-1-yl)butan-1-ol (compound 180),
4-(1-(azetidin-2-ylmethyl)-1H-pyrazol-4-yl)-2-(pyrazolo[1,5-a]pyridin-3-yl)thiazole (compound 182),
2-((2-(4-(2-(pyrazolo[1,5-a]pyridin-3-yl)thiazol-4-yl)-1H-pyrazol-1-yl)ethyl)amino)acetonitrile (compound 183),
3-(4-(2-(pyrazolo[1,5-a]pyridin-3-yl)thiazol-4-yl)-1H-pyrazol-1-yl)propan-1-amine (compound 184),
N-(3-(4-(2-(pyrazolo[1,5-a]pyridin-3-yl)thiazol-4-yl)-1H-pyrazol-1-yl)propyl)methanesulfonamide (compound 185),
5-methyl-4-(2-(pyrazolo[1,5-a]pyridin-3-yl)thiazol-4-yl)-1H-pyrazol-3-ol (compound 186),
N¹,N¹-dimethyl-N²-(2'-(pyrazolo[1,5-a]pyridin-3-yl)-[4,4'-bithiazole]-2-yl)ethane-1,2-diamine (compound 187),
2-(dimethylamino)-N-(2'-(pyrazolo[1,5-a]pyridin-3-yl)-[4,4'-bithiazole]-2-yl)acetamide (compound 188),
2-amino-N-(2'-(pyrazolo[1,5-a]pyridin-3-yl)-[4,4'-bithiazole]-2-yl)acetamide hydrochloride (compound 189),
2'-(pyrazolo[1,5-a]pyridin-3-yl)-[4,4'-bithiazole]-2-ol (compound 190),
5-(2-(pyrazolo[1,5-a]pyridin-3-yl)thiazol-4-yl)furan-2-carboxamide (compound 191),
4-(2,5-dihydrofuran-3-yl)-2-(pyrazolo[1,5-a]pyridin-3-yl)thiazole (compound 192),
(1-(2-(pyrazolo[1,5-a]pyridin-3-yl)thiazol-4-yl)-1H-imidazol-4-yl)methanol (compound 193),
(1-(2-(pyrazolo[1,5-a]pyridin-3-yl)thiazol-4-yl)-1H-imidazol-4-yl)methanamine hydrochloride (compound 194),
3-((1-(2-(pyrazolo[1,5-a]pyridin-3-yl)thiazol-4-yl)-1H-imidazol-4-yl)methoxy)propan-1-ol (compound 195),
3-(4-(2-(pyrazolo[1,5-a]pyridin-3-yl)thiazol-4-yl)-1H-imidazol-1-yl)propan-1-ol (compound 196),
2-(4-(2-(pyrazolo[1,5-a]pyridin-3-yl)thiazol-4-yl)-1H-imidazol-1-yl)ethan-1-amine (compound 197),
N-(2-(4-(2-(pyrazolo[1,5-a]pyridin-3-yl)thiazol-4-yl)-1H-imidazol-1-yl)ethyl)acetamide (compound 198),
2-(pyrazolo[1,5-a]pyridin-3-yl)-4-(pyrimidin-5-yl)thiazole (compound 199),
2-(pyrazolo[1,5-a]pyridin-3-yl)-4-(pyrimidin-2-yl)thiazole (compound 200),
4-(2,3-dihydro-1H-pyrrolo[2,3-b]pyridin-5-yl)-2-(pyrazolo[1,5-a]pyridin-3-yl)thiazole (compound 201),
2-(pyrazolo[1,5-a]pyridin-3-yl)-4-(5,6,7,8-tetrahydroimidazo[1,2-a]pyridin-2-yl)thiazole (compound 202),
2-(2-(pyrazolo[1,5-a]pyridin-3-yl)thiazol-4-yl)-5,6-dihydro-8H-imidazo[2,1-c][1,4]oxazine (compound 203),
2-(2-(pyrazolo[1,5-a]pyridin-3-yl)thiazol-4-yl)-4,5,6,7-tetrahydrothiazolo[5,4-c]pyridine (compound 204),
2-(2-(pyrazolo[1,5-a]pyridin-3-yl)thiazol-4-yl)-4,5,6,7-tetrahydrothiazolo[4,5-c]pyridine (compound 205),
2-(pyrazolo[1,5-a]pyridin-3-yl)-4-(4,5,6,7-tetrahydropyrazolo[1,5-a]pyrazin-2-yl)thiazole (compound 206),
4-(1H-indazol-3-yl)-2-(pyrazolo[1,5-a]pyridin-3-yl)thiazole (compound 207),
4-(4-chloro-1H-pyrazol-3-yl)-2-(pyrazolo[1,5-a]pyridin-3-yl)thiazole (compound 208),
2-(6-methylpyrazolo[1,5-a]pyridin-3-yl)-4-phenylthiazole (compound 209),
2-(5-methylpyrazolo[1,5-a]pyridin-3-yl)-4-phenylthiazole (compound 210),
2-(4-methylpyrazolo[1,5-a]pyridin-3-yl)-4-phenylthiazole (compound 211),
4-(4-chloro-1-methyl-1H-pyrazol-3-yl)-2-(5-methylpyrazolo[1,5-a]pyridin-3-yl)thiazole (compound 212), and
N-((5-(2-(5-methylpyrazolo[1,5-a]pyridin-3-yl)thiazol-4-yl)pyridin-2-yl)methyl)acetamide (compound 213).

### 2. Method for producing the present compound

The present compound can be produced by combining known reactions. The following are illustrative synthesis examples of the compounds of the present invention. A person skilled in the art can also synthesize compounds of the formula (I) other than the exemplified compounds and derivatives thereof according to the following description and the description of Examples.

(Abbreviation) The meanings of symbols abbreviated in the following reaction formulas are as follows.
Δ: Heating
Halo: Halogen
DME: dimethoxyethane
EtOH: Ethanol
DMF: dimethylformamide
TEA: triethylamine
DIPEA: diisopropylethylamine
CH₃CN: acetonitrile
Boc₂O: di-tert-butyl dicarbonate
Boc-ON: 2-(tert-butoxycarbonyloxyimino)-2-phenylacetonitrile
aq.: aqueous solution
N-BocGlycine: N-butoxycarbonylglycine
BrCH₂CO₂tBu: tert-butyl bromoacetate
BrCH₂COOBn: benzyl bromoacetate
Boc: tert-butoxycarbonyl
DCM: dichloromethane
EDC·HCl: N-(3-dimethylaminopropyl)-N'-ethylcarbodiimide hydrochloride
DCC: N,N'-dicyclohexylcarbodiimide
HOBt: 1-hydroxybenzotriazole (condensation auxiliary)
HOAt: 1-hydroxy-7-azabenzotriazole (condensation auxiliary)
BOP: 1H-benzotriazol-1-yloxytris(dimethylamino)phosphonium hexafluorophosphate
HATU: 1-[bis(dimethylamino)methylene]-1H-1,2,3-triazolo[4,5-b]pyridinium 3-oxide hexafluorophosphate
TATU: 1-[bis(dimethylamino)methylene]-1H-1,2,3-triazolo[4,5-b]pyridinium 3-oxide tetrafluoroborate
COMU: 1-[bis(cyano-2-ethoxy-2-oxoethylideneaminooxy)dimethylaminomorpholino]uronium hexafluorophosphate
Ac₂O: acetic anhydride
DBU: 1,8-diazabicyclo[5.4.0]-7-undecene
DBN: 1,5-diazabicyclo[4.3.0]nona-5-ene
THF: tetrahydrofuran
Me: methyl
Et: ethyl
MeOH: methanol
LAH: lithium aluminum hydride
DIBAL: diisobutylaluminum hydride
LiBH₄: lithium borohydride
LiBEt₃: lithium triethylboron hydride
AcOEt: ethyl acetate
Pd(PPh₃)₄: tetrakis(triphenylphosphine)palladium(0)
Pd(OAc)₂: palladium acetate
PdCl₂(PPh₃)₂: bis(triphenylphosphine)palladium(II) dichloride
PdCl₂(dppf): [1,1'-bis(diphenylphosphino)ferrocene]dichloropalladium(II)
Pd(acac)₂: palladium(II) acetylacetonate
Pd₂(dba)₃: tris(dibenzylideneacetone)dipalladium(0)
DMAP: 4-dimethylaminopyridine
BocPiperazine: butoxycarbonylpiperazine
NaBH(OAc)₃: sodium triacetoxyborohydride
NaBH₃CN: sodium cyanoborohydride
AcOH: acetic acid
rt: room temperature
XPhos: 2-dicyclohexylphosphino-2',4',6'-triisopropylbiphenyl
Tf: trifluoromethanesulfonyl
DMSO: dimethyl sulfoxide
TBS: tert-butyldimethylsilyl
TBAF: tetrabutylammonium fluoride
TFA: trifluoroacetic acid
t-BuOH: tert-butanol

### (1) Main reaction

The compound of the formula (I) can be synthesized, for example, by the following reaction. 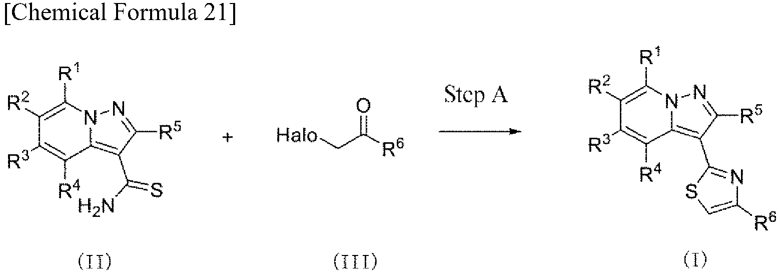 wherein R¹ to R⁶ are as defined in the above formula (I).

Step A: In this reaction, a thioamide of the formula (II) and a halide of the formula (III) having a corresponding substituent or structure may be selected according to the structure of the compound of the formula (I) to be synthesized. The reaction can be performed by dissolving a thioamide in a solvent such as DME, acetone, toluene, hexane, xylene, ethyl acetate, dioxane, THF, ethanol, methanol, dichloroethane, DMF, or water, adding a halide to allow the reaction to proceed, then distilling off the solvent of the reaction mixture, adding a solvent such as ethanol and an excess base such as potassium carbonate, sodium hydroxide, potassium hydroxide, sodium carbonate, triethylamine, DBU, DBN, or pyridine to the residue, and distilling off the solvent of the reaction mixture. If necessary, the obtained crude product may be purified.

By modifying the groups R¹ to R⁶ with protecting groups or the like, or by converting them into different groups, derivatives of the compound of formula (I) can also be obtained, and from this derivative, the compound of the formula (I) can also be synthesized by an additional reaction described later.

The compounds of formula (I) can also be synthesized by the following reaction (Goldberg amination reaction) when R⁶ is an optionally substituted 5- to 10-membered aromatic heterocycle having at least one nitrogen, or a fused ring of the aromatic heterocycle with an optionally substituted 4- to 10-membered heterocyclyl having 1 to 3 heteroatoms independently selected from nitrogen, oxygen and sulfur as ring-constituting atoms. 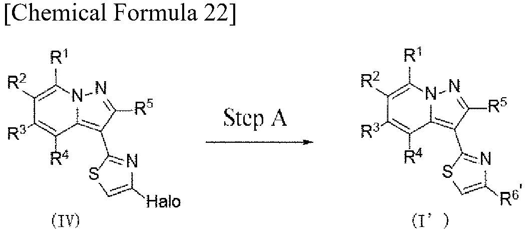 wherein R¹ to R⁵ are as defined in the above formula (I), R⁶' is an optionally substituted 5- to 10-membered heterocycle, preferably a 5- to 10-membered aromatic heterocycle or a 5- or 6-membered heterocycloalkenyl, having at least one nitrogen, or a fused ring of either of these (preferably a 5- to 10-membered aromatic heterocycle) and an optionally substituted 4- to 10-membered heterocyclyl having 1 to 3 heteroatoms independently selected from nitrogen, oxygen and sulfur as ring-constituting atoms, the nitrogen constituting the aromatic heterocyclic ring of R⁶' is linked to a ring-constituting carbon of thiazole, and Halo is a halogen such as iodine, bromine, or chlorine, and preferably bromine.

Step A: A nitrogen-containing heterocyclic ring such as imidazole or pyrazole is heated for a predetermined time in the presence of a copper catalyst such as copper(I) iodide with the aryl halide form represented by the formula (IV) to undergo a Goldberg reaction, whereby a corresponding condensate can be obtained. The solvent to be used varies depending on the starting material, the reagent, and the like, and is not particularly limited as long as the solvent dissolves the starting material to some extent without inhibiting the reaction, and preferred examples thereof include toluene, hexane, xylene, methanol, ethanol, isopropanol, ethylene glycol, acetonitrile, dioxane, THF, dichloromethane, dichloroethane, DME, DMF, and dimethylacetamide. Examples of the base to be used include potassium carbonate, sodium carbonate, sodium bicarbonate, potassium phosphate, potassium acetate, cesium carbonate, potassium phosphate, sodium hydride, sodium methoxide, sodium ethoxide, sodium butoxide, sodium hydroxide, potassium hydroxide, DBU, and DBN. Examples of the copper catalyst to be used include copper(I) chloride, copper(I) bromide, copper(I) iodide, copper, and copper oxide. Furthermore, as the reaction additive, trans-1,2-cyclohexanediamine, N,N'-dimethyl-trans-1,2-cyclohexanediamine, 1,10-phenanthroline, 2,9-dimethyl-1,10-phenanthroline, 3,4,7,8-tetramethyl-1,10-phenanthroline, or the like can also be used. Usually, the reaction temperature is 40 to 200°C and the reaction time is 1 to 24 hours, and it is preferably 180°C for 1 hour.

In this reaction, the groups R¹ to R⁶' of the compounds represented by the formulas (IV) and (I') are modified with a protecting group or the like, or made into different groups, whereby a derivative of the compound of the formula (I') is also obtained, and from this derivative, the compound of the formula (I') can also be synthesized by an additional reaction described later.

The compound of the formula (I) can also be synthesized, for example, by the following reaction. 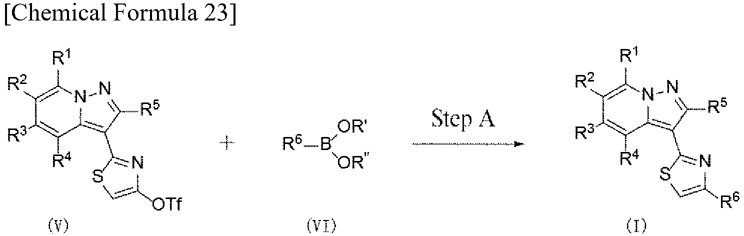 wherein R¹ to R⁶ are as defined in the formula (I) above, Tf represents triflate, and R' and R" each represent a hydrogen atom, or R' and R", together with two adjacent oxygen atoms, form an alkylenedioxy group.

Step A: A triflate compound of the formula (V) and a boronic acid compound of the formula (VI) are heated for a predetermined time in the presence of a palladium catalyst to cause a Suzuki-Miyaura coupling reaction, thereby producing a compound of the formula (I). Examples of the palladium catalyst include palladium acetate, palladium chloride, bis(triphenylphosphine)palladium(II) dichloride, tetrakis(triphenylphosphine)palladium(0), tris(dibenzylideneacetone)dipalladium(0), and [1,1'-bis(diphenylphosphino)ferrocene]palladium(II) dichloride, which are used in the presence of a base such as sodium hydroxide, potassium hydroxide, sodium carbonate, potassium carbonate, tripotassium phosphate, sodium methoxide, or potassium tert-butoxide with addition of water. As the reaction solvent, toluene, hexane, xylene, dioxane, tetrahydrofuran, dimethoxyethane, dichloroethane, N,N-dimethylformamide, or the like can be used. The reaction may be performed at a temperature of 50 to 200°C for about 5 minutes to 20 hours.

The groups R¹ to R⁶ are modified with a protecting group or the like, or made into different groups, whereby a derivative of the compound of the formula (I) is also obtained, and from this derivative, the compound of the formula (I) can also be synthesized by an additional reaction described later.

The compound of the formula (I) can also be synthesized, for example, by the following reaction. 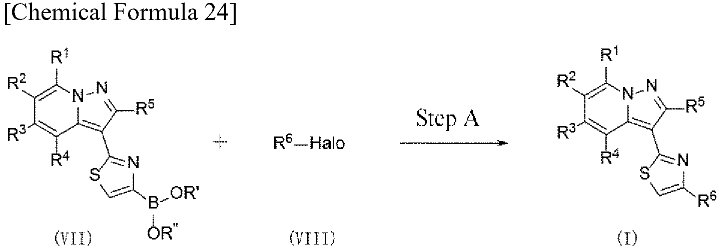 wherein R¹ to R⁶ are as defined in the formula (I) above; R' and R" each represent a hydrogen atom, or R' and R", together with two adjacent oxygen atoms, form an alkylenedioxy group; and Halo is a halogen such as iodine, bromine, or chlorine.

Step A: A boronic acid compound of formula (VII) and a halogen compound of formula (VIII) are heated for a predetermined time in the presence of a palladium catalyst to cause a Suzuki-Miyaura coupling reaction, thereby producing a compound of the formula (I). This reaction can be performed in the same manner as the reaction of the formula (V) and formula (VI) described above.

The groups R¹ to R⁶ are modified with a protecting group or the like, or made into different groups, whereby a derivative of the compound of the formula (I) is also obtained, and from this derivative, the compound of the formula (I) can also be synthesized by an additional reaction described later.

### (2) Additional reaction

It is also possible to produce a compound of the formula (I) or a derivative thereof having a different substituent by applying a known reaction to the substituent of the compound of the formula (I) or a derivative thereof obtained in the above main reaction. Applicable known reactions are described, for example, in general laboratory manuals such as Maruzen's 4th Series of Experimental Chemistry (30 volumes in total), and each reaction can be performed according to this. The contents of these literatures are incorporated herein by reference. Exemplary additional reactions are shown below. In the following reaction formula, the structure represented by a double circle represents a 5- to 10-membered aromatic hydrocarbon, a 5- to 10-membered aromatic heterocycle having 1 to 5 heteroatoms independently selected from nitrogen, oxygen, and sulfur, or a fused ring in which either the aromatic hydrocarbon or the heterocycle is fused with a 4- to 10-membered heterocyclyl having 1 to 3 heteroatoms independently selected from nitrogen, oxygen, and sulfur.

### (Reduction of nitro group with metal, and protection and deprotection reactions of generated amino group)

When a compound having a nitro group is synthesized in a main reaction or in another additional reaction, the compound can be subjected to an additional reaction represented by the following reaction formula to convert the nitro group to an amino group. 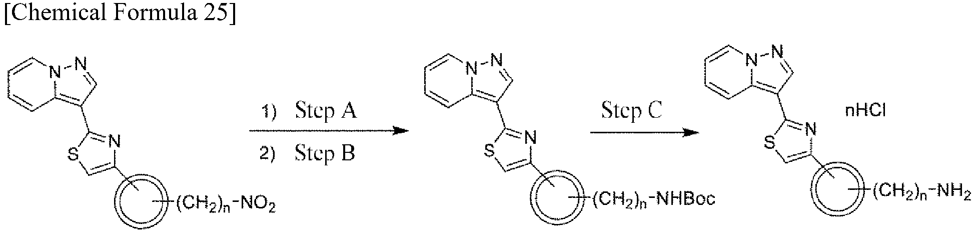 wherein n is an integer of 0 or more, for example, an integer of 0 to 3.

Step A: In this reaction, a nitro compound is dissolved in a solvent such as acetonitrile, and zinc powder and additives such as calcium chloride are added together with water, followed by heating reaction to afford an amine. The solvent to be used varies depending on a starting material, a reagent, and the like, and is not particularly limited as long as the solvent dissolves the starting material to some extent without inhibiting the reaction, and preferred examples thereof include acetone, toluene, hexane, xylene, dioxane, THF, ethanol, methanol, DME, and DMF. Usually, the reaction temperature is 80 to 120°C and the reaction time is 0.5 to 6 hours, and it is preferably 100°C for 1 hour. In this step A, the reduction reaction of the nitro group under neutral conditions was shown, but the present invention is not limited to the present reaction conditions, and as an alternative method, a reduction reaction using a metal such as zinc, iron, or tin under acidic conditions can also be performed.

Step B: In order to protect an amino group having low stability, the amino group is protected with a Boc group. The obtained amine is dissolved in a solvent such as acetonitrile, treated with an excess of a Boc-protecting agent, and stirred to allow the reaction to proceed. After purification, if necessary, the carbamate compound in which the amino group is protected with a Boc group is obtained. The solvent to be used varies depending on a starting material, a reagent, and the like, and is not particularly limited as long as the solvent dissolves the starting material to some extent without inhibiting the reaction, and preferred examples thereof include acetonitrile, acetone, toluene, hexane, xylene, dioxane, THF, ethanol, methanol, DME, and DMF. Examples of the Boc-protecting agent include Boc₂O and Boc-ON. Usually, the reaction temperature is -20 to 80°C and the reaction time is 1 to 48 hours, and it is preferably 24 hours at room temperature. In this step B, a Boc group is used as a protecting group, but the protecting group is not limited thereto, and other protecting groups such as a benzoyl group may be used without any problem.

Step C: A carbamate compound having an amino group protected with a Boc group is added to a solution of hydrogen chloride in dioxane to remove the protecting group, whereby a hydrochloride of an amine can be synthesized. In the present reaction, it is also possible to dissolve a carbamate compound in a solvent and add an acid thereto. The solvent to be used varies depending on a starting material, a reagent, and the like, and is not particularly limited as long as the solvent dissolves the starting material to some extent without inhibiting the reaction, and preferred examples thereof include solvents such as acetonitrile, acetone, toluene, hexane, xylene, dioxane, dichloromethane, ethanol, methanol, DME, DMF, and water. Examples of the acid include acids such as nitric acid, sulfuric acid, acetic acid, and trifluoroacetic acid.
Usually, the reaction temperature is -20 to 80°C and the reaction time is 0.5 to 12 hours, and it is preferably 0°C for 1 hour.

### (Reaction between an amine and halide)

When an amine is synthesized in a main reaction or in another additional reaction, the compound can be subjected to an additional reaction represented by the following reaction formula to synthesize a compound in which a desired substituent (R') is introduced into an amino group. 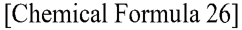 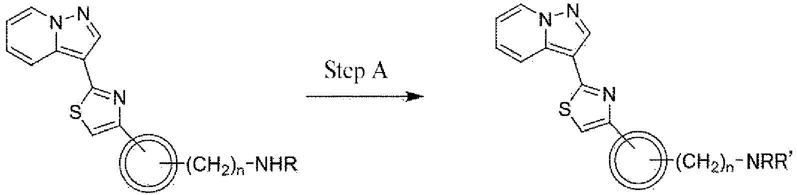 wherein n is an integer of 0 or more (for example, an integer of 0 to 3); R is a hydrogen atom or any substituent, such as a C₁₋₃ alkyl group; and R' is any structure or group to be introduced (for example, R³¹, R³², -C(O)(CH₂)₀₋₃NR³¹₂, -(CH₂)₁₋₃CN, -(CH₂)₁₋₃R³², or - C(O)NR³²).

Step A: In this reaction, an amine hydrochloride is suspended in dichloromethane, a halide is added thereto, and then a base such as triethylamine is added to afford a compound in which a desired substituent (R') is introduced into an amino group. The solvent to be used varies depending on a starting material, a reagent, and the like, and is not particularly limited as long as the solvent dissolves the starting material to some extent without inhibiting the reaction, and preferred examples thereof include solvents such as acetonitrile, acetone, toluene, hexane, xylene, dioxane, THF, dichloromethane, DME, or DMF, or a mixed solvent thereof. Examples of the acid scavenger include an equimolar amount or excess amount of bases such as triethylamine, sodium carbonate, potassium carbonate, sodium bicarbonate, sodium hydride, sodium methoxide, sodium ethoxide, sodium hydroxide, potassium hydroxide, DBU, DBN, or pyridine. Usually, the reaction temperature is -20 to 80°C and the reaction time is 1 to 48 hours, and it is preferably 0°C for 1 hour.

### (Condensation reaction between amine and carboxylic acid)

When an amine is synthesized in a main reaction or in an additional reaction, the compound can be subjected to an additional reaction represented by the following reaction formula to synthesize a compound in which a carbonyl group having a desired substituent (R') is introduced into an amino group. 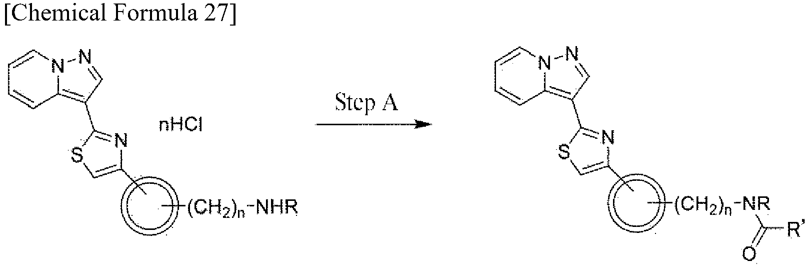 wherein n is an integer of 0 or more (for example, an integer of 0 to 3); R is a hydrogen atom or any substituent, such as a C₁₋₃ alkyl group; and R' is any structure or group to be introduced (for example, R³¹, R³², -(CH₂)₁₋₅C(O)OR³¹, or -(CH₂)₁₋₅NR³¹₂).

Step A: In this condensation reaction, an amine hydrochloride, a carboxylic acid having a desired substituent (R'), and a condensing agent such as EDC·HCl are added to an anhydrous solvent such as dichloromethane, and an equimolar amount to an excess amount of a base such as triethylamine is added as an acid scavenger, and the mixture is allowed to react to afford various amides. The solvent to be used varies depending on a starting material, a reagent, and the like, and is not particularly limited as long as the solvent dissolves the starting material to some extent without inhibiting the reaction, and preferred examples thereof include an anhydrous solvent such as dichloromethane, acetonitrile, acetone, toluene, hexane, xylene, dioxane, THF, DME, or DMF. Examples of the acid scavenger include an equimolar amount or excess amount of triethylamine, sodium carbonate, potassium carbonate, sodium bicarbonate, sodium hydride, sodium methoxide, sodium ethoxide, sodium hydroxide, potassium hydroxide, DBU, DBN, or pyridine. Usually, the reaction temperature is -20 to 80°C and the reaction time is 1 to 48 hours, and it is preferably 24 hours at room temperature. If necessary, a reaction catalyst such as DMAP can be used. Here, EDC is used as the condensing agent, but in addition to this, DCC or the like can be used. In addition, HOBt and HOAt may be added to prevent epimerization. Furthermore, a BOP reagent can also be used as a condensing agent, and HATU, TATU, COMU, and the like are also applicable.

### (Introduction reaction of amino group protected with Boc group)

By utilizing the reaction between an amine and a carboxylic acid, for example, an amino group having a protecting group (Boc) convertible to an amino group (hydrochloride thereof) can be added to a side chain by a reaction represented by the following exemplary reaction formula. 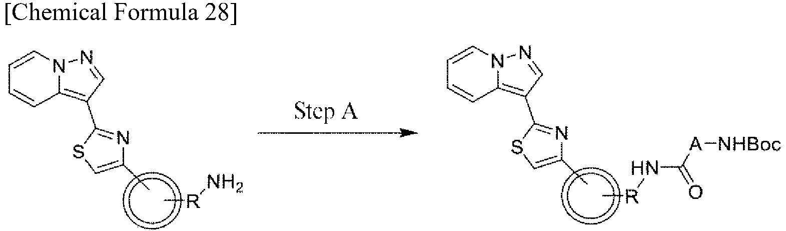 wherein R and A each independently represent a bond or any structure, such as C₁₋₅ alkylene, -C(O)-, -(CH₂)₀₋₅N(R³¹)C(O)(CH₂)₀₋₅-, -C(R³¹)₂C(O)-, -(CH₂)₁₋₅O(CH₂)₁₋₅-, -O(CH₂)₁₋₅-, -(OCH₂)₁₋₅-.

Step A: This reaction is carried out under the same conditions as in step B (of reduction of nitro group with metal, and protection and deprotection reactions of generated amino group) described above.

### (Removal reaction of protecting group (Boc))

When a compound having an amino group protected with Boc is synthesized in a main reaction or in an additional reaction, the compound can be subjected to an additional reaction represented by the following exemplary reaction formula to deprotect the Boc group. 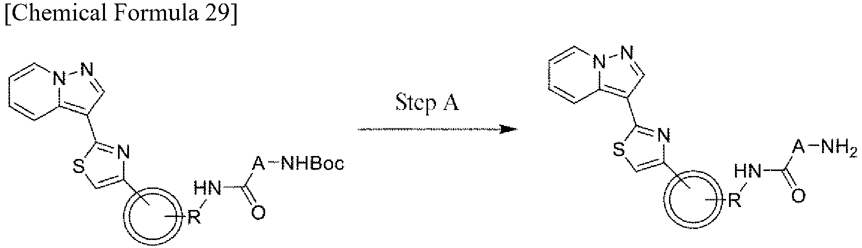 wherein R and A each independently represent a bond or any structure, such as C₁₋₅ alkylene, -C(O)-, -(CH₂)₀₋₅N(R³¹)C(O)(CH₂)₀₋₅-, -C(R³¹)₂C(O)-, -(CH₂)₁₋₅O(CH₂)₁₋₅-, -O(CH₂)₁₋₅-, -(OCH₂)₁₋₅-.

Step A: This reaction is carried out under the same conditions as Step C in (Reduction of nitro group with metal, and protection and deprotection reactions of generated amino group) described above.

### (Acetylation reaction)

When a compound having an amino group is synthesized in a main reaction or in an additional reaction, the compound can be subjected to an additional reaction represented by the following reaction formula to synthesize an amide in which the amino group is protected with an acetyl group. 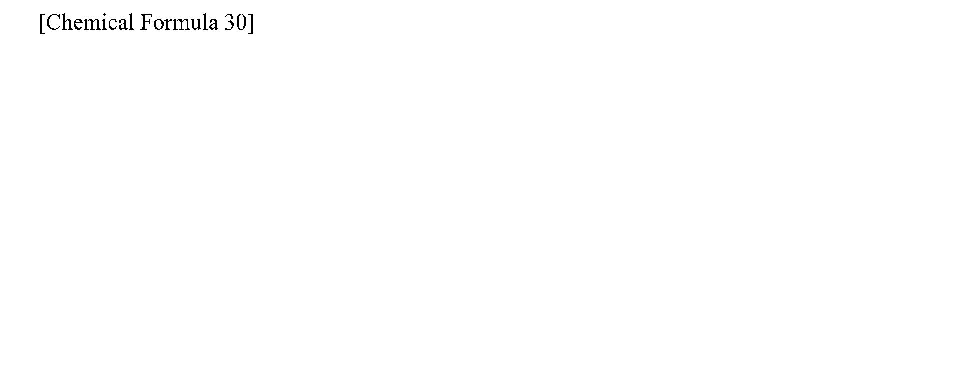 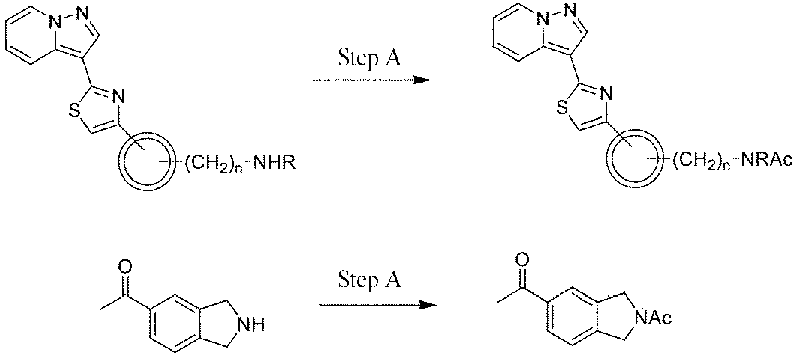 wherein n is an integer of 0 or more, for example, an integer of 0 to 3, and R is a hydrogen atom or any substituent, such as a C₁₋₃ alkyl group.

Step A: In these reactions, an amine is added to a solvent such as acetonitrile, and a catalytic amount of a base such as DMAP or TEA as a catalyst and an acetylating agent are added thereto, and the mixture is allowed to react to afford the acetylated product. The solvent to be used varies depending on a starting material, a reagent, and the like, and is not particularly limited as long as the solvent dissolves the starting material to some extent without inhibiting the reaction, and preferred examples thereof include acetonitrile, acetone, toluene, hexane, xylene, dioxane, THF, dichloromethane, DME, and DMF. Examples of the acid scavenger include bases such as TEA, DIPEA, sodium carbonate, potassium carbonate, sodium bicarbonate, sodium hydride, sodium methoxide, sodium ethoxide, sodium hydroxide, potassium hydroxide, DBU, DBN, or pyridine. Examples of the acetylating agent include acetic anhydride and acetyl chloride. Usually, the reaction temperature is -20 to 80°C and the reaction time is 1 to 48 hours, and it is preferably 24 hours at room temperature.

### (Removal reaction of a protecting group (acetyl group))

When a compound in which an amino group is protected with an acetyl group is synthesized in a main reaction or in an additional reaction, the compound can be subjected to an additional reaction represented by the following exemplary reaction formula to deprotect the acetyl group. 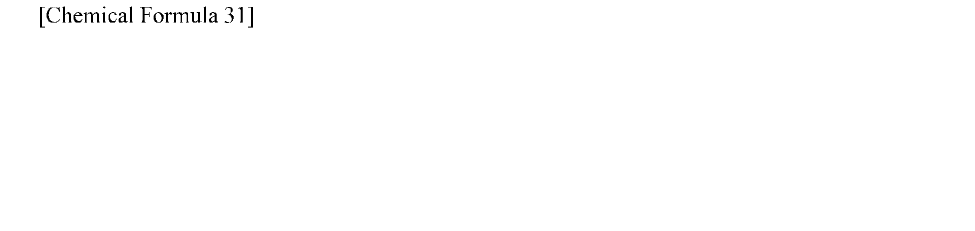 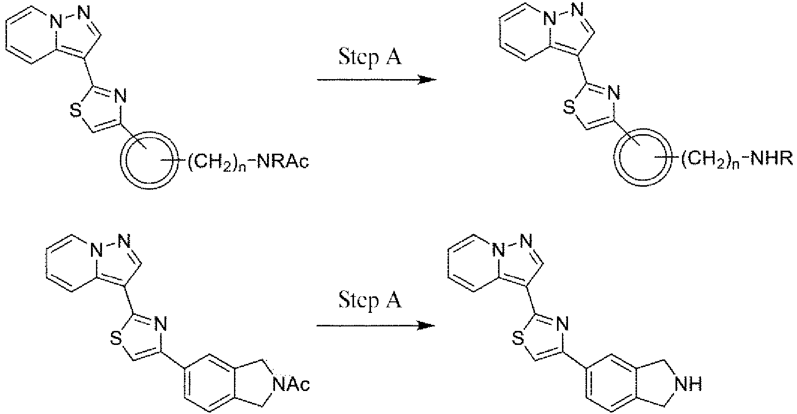 wherein n is an integer of 0 or more, for example, an integer of 0 to 3, and R is a hydrogen atom or any substituent, such as a C₁₋₃ alkyl group.

Step A: In these reactions, a compound in which an amino group is protected with an acetyl group is suspended in a solvent and reacted by heating for a predetermined time under acidic or basic conditions, whereby the acetyl group can be deprotected. The solvent to be used varies depending on a starting material, a reagent, and the like, and is not particularly limited as long as the solvent dissolves the starting material to some extent without inhibiting the reaction, and preferred examples thereof include methanol, ethanol, dioxane, DME, DMF, and water. Examples of the acid include hydrochloric acid, nitric acid, sulfuric acid, trifluoroacetic acid, and hydrobromic acid. Examples of the base include sodium hydroxide, potassium hydroxide, and lithium hydroxide. Usually, the reaction temperature is 50 to 160°C and the reaction time is 0.5 to 24 hours, and it is preferably 100°C for 1 hour.

### (Removal Reaction of Protecting Group (Phthalimide))

When a compound in which an amino group is protected as phthalic anhydride is synthesized in the main reaction, the compound can be subjected to an additional reaction (deprotection) represented by the following exemplary reaction formula to be deprotected.

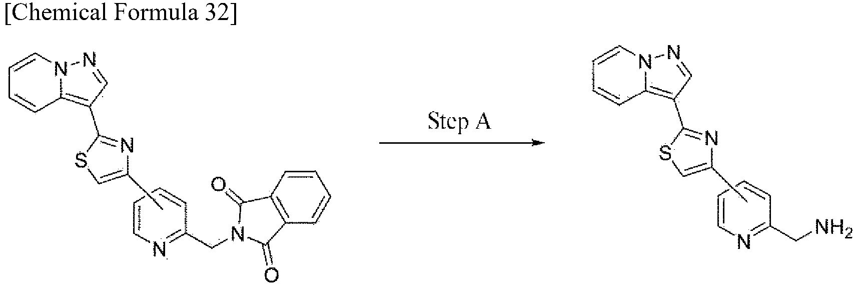

Step A: In this reaction, a phthalimide derivative and hydrazine monohydrate are added to a solvent such as ethanol, heated for a predetermined time to react, and deprotection affords an amine derivative. The solvent to be used varies depending on a starting material, a reagent, and the like, and is not particularly limited as long as the solvent dissolves the starting material to some extent without inhibiting the reaction, and preferred examples thereof include ethanol, methanol, water, dichloromethane, THF, DMF, toluene, chloroform, acetonitrile, diethyl ether, 1-butanol, isopropanol, acetic acid, acetone, benzene, tert-butanol, and DMSO.

### (Alkylation reaction)

When a compound having an amino functional group such as an acetamide group is synthesized in a main reaction or in an additional reaction, the compound can be subjected to an additional reaction represented by the following reaction formula to introduce an alkyl group into the amino group. 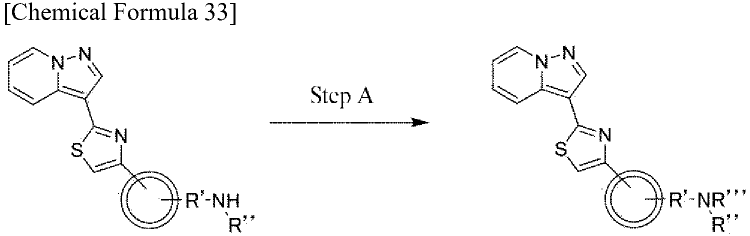 wherein R' is a bond or any structure, such as C₁₋₃ alkylene; R" is any group, such as C₁₋₃ alkyl, C(O)C₁₋₃ alkyl; and R‴ is an alkyl group, such as C₁₋₃ alkyl.

Step A: In this reaction, a compound having an amino functional group such as an acetamide group is added to a suspension obtained by adding sodium hydride to a solvent such as DMF, and an alkyl halide (Halo- R"') is added dropwise and reacted to afford an N-alkylamide. The solvent to be used varies depending on a starting material, a reagent, and the like, and is not particularly limited as long as the solvent dissolves the starting material to some extent without inhibiting the reaction, and preferred examples thereof include THF, DME, and DMF. Further, examples of the strong base include bases such as sodium hydride, sodium methoxide, sodium ethoxide, sodium butoxide, sodium hydroxide, potassium hydroxide, DBU, and DBN. Usually, the reaction temperature is -20 to 50°C and the reaction time is 1 to 24 hours, and it is preferably 0°C for 1 hour.

### (Reductive amination reaction)

When a compound having an amino group is synthesized in a main reaction or in an additional reaction, the compound can be subjected to an additional reaction represented by the following reaction formula to introduce an alkyl group into the amino group. 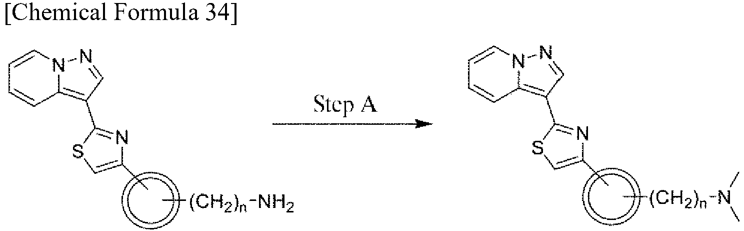 wherein n is an integer of 0 or more, for example, an integer of 0 to 3.

Step A: In this reaction, an amine derivative and an aldehyde are added to a dry solvent such as methanol and stirred, followed by addition of NaBH(OAc)₃ and reaction for a predetermined time to afford an alkylamine derivative. The reaction is carried out in a solvent and in the presence or absence of an acid. The solvent to be used varies depending on a starting material, a reagent, and the like, and is not particularly limited as long as the solvent dissolves the starting material to some extent without inhibiting the reaction, and preferred examples thereof include methanol, ethanol, toluene, benzene, hexane, xylene, dioxane, THF, dichloromethane, dichloroethane, DME, and DMF. The acid to be used varies depending on the starting material, the solvent to be used, and the like, and is not particularly limited as long as the reaction is not inhibited, and preferred examples thereof include acetic acid and formic acid. Furthermore, NaBH(OAc)₃ and NaBH₃CN can be used as the reducing agent, and usually, the reaction temperature is -20 to 100°C, and the reaction time is 1 to 24 hours, and it is preferably 25°C for 3 hours.

### (N-Alkylation reaction of amide group)

When a compound having an amino group is synthesized in a main reaction or in an additional reaction, the compound is subjected to the following additional reaction to form an amide group, and can be further N-alkylated. 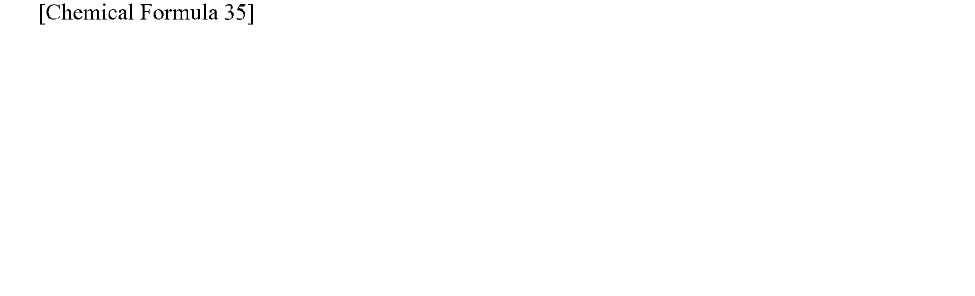 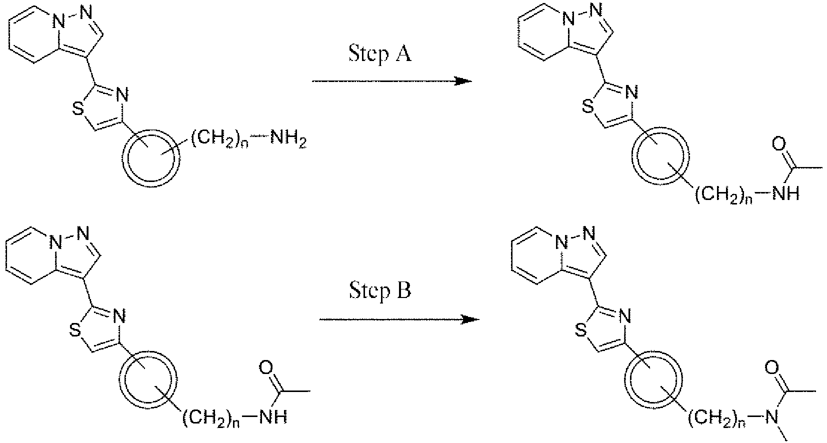 wherein n is an integer of 0 or more, for example, an integer of 0 to 3.

Step A: In this reaction, the solvent to be used varies depending on a starting material, a reagent, and the like, and is not particularly limited as long as the solvent dissolves the starting material to some extent without inhibiting the reaction, and preferred examples thereof include THF, DME, and DMF. Examples of the strong base include sodium hydride, sodium methoxide, sodium ethoxide, sodium hydroxide, and potassium hydroxide, each in an equimolar amount to an excess amount. Usually, the reaction temperature is -20 to 80°C and the reaction time is 1 to 48 hours, and it is preferably 0°C for 24 hours. This reaction is a reaction similar to Step A of the (alkylation reaction) described above.

Step B: An amide-containing compound is treated with a strong base in dry DMF, followed by addition of an alkylhalide, to afford an N-alkylamide.

### (Reaction between hydroxyl group and halide)

When a derivative having a hydroxyl group is synthesized in a main reaction or in an additional reaction, an ether (phenoxy ether) compound can be synthesized by subjecting the derivative to an additional reaction represented by the following reaction formula and introducing a desired substituent (R') into the hydroxyl group. 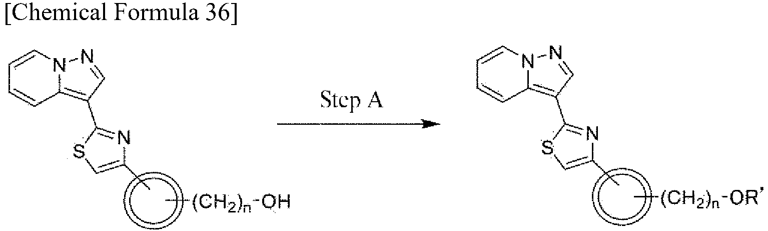 wherein n is an integer of 0 or more (for example, an integer of 0 to 3); R' is any substituent, such as -C(R³¹)₂C(O)(CH₂)₀₋₃0H, -C(R³¹)₂C(O)NR³¹₂, C₁₋₆ alkyl group, -((CH₂)₁₋₅O)₁₋₅R³¹, and -C(O)C(R³¹)₂(O(CH₂)₁₋₅)₁₋₅OR³¹.

Step A: In this reaction, a compound having a hydroxyl group (mainly a phenol derivative) is treated with a base in DMF, followed by reaction with a halide (Halo-R'), to afford an ether (phenoxy ether) derivative. The solvent to be used varies depending on a starting material, a reagent, and the like, and is not particularly limited as long as the solvent dissolves the starting material to some extent without inhibiting the reaction, and preferred examples thereof include THF, DME, DMF, and dichloromethane. Examples of the base include sodium hydride, sodium methoxide, sodium ethoxide, sodium hydroxide, potassium hydroxide, sodium carbonate, potassium carbonate, DBU, DBN, and pyridine, each in an equimolar amount to an excess amount. Usually, the reaction temperature is -20 to 80°C and the reaction time is 1 to 48 hours, and it is preferably 0°C for 24 hours.

### (Alkaline hydrolysis reaction)

When an ester compound is synthesized in a main reaction or in an additional reaction, the ester compound can be subjected to an additional reaction represented by the following reaction formula to undergo alkaline hydrolysis. 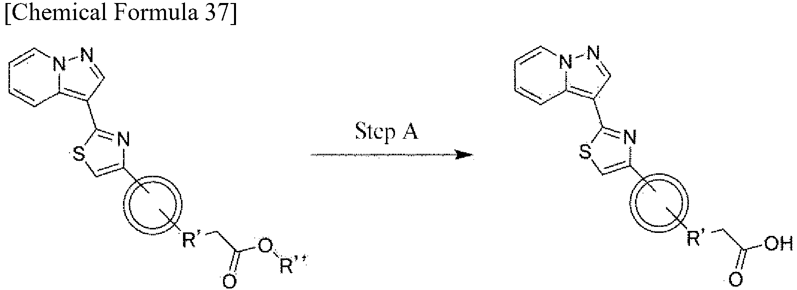 wherein R' is a bond or any structure, such as C₁₋₃ alkylene, -O-C-; and R" is any substituent, such as a chain alkyl group, an aromatic hydrocarbon.

Step A: In this reaction, an ester compound is added to a solvent such as a mixed solvent of an aqueous sodium hydroxide solution and ethanol, reacted by heating and stirring for a predetermined time, and subjected to an acid-base treatment to afford a carboxylic acid. The solvent to be used varies depending on a starting material, a reagent, and the like, and is not particularly limited as long as the solvent dissolves the starting material to some extent without inhibiting the reaction, and preferred examples thereof include methanol, ethanol, dioxane, DME, DMF, and water. Further, examples of the strong base include sodium hydroxide, potassium hydroxide, sodium hydride, sodium methoxide, sodium ethoxide, sodium butoxide, DBU, and DBN. Usually, the reaction temperature is 30 to 140°C and the reaction time is 1 to 24 hours, and it is preferably 100°C for 1 hour.

### (Reaction between ester (and carboxylic acid) and amine)

When an ester compound is synthesized in a main reaction or in an additional reaction, the ester compound can be subjected to an additional reaction represented by the following reaction formula to convert the ester to an aminocarbonyl. 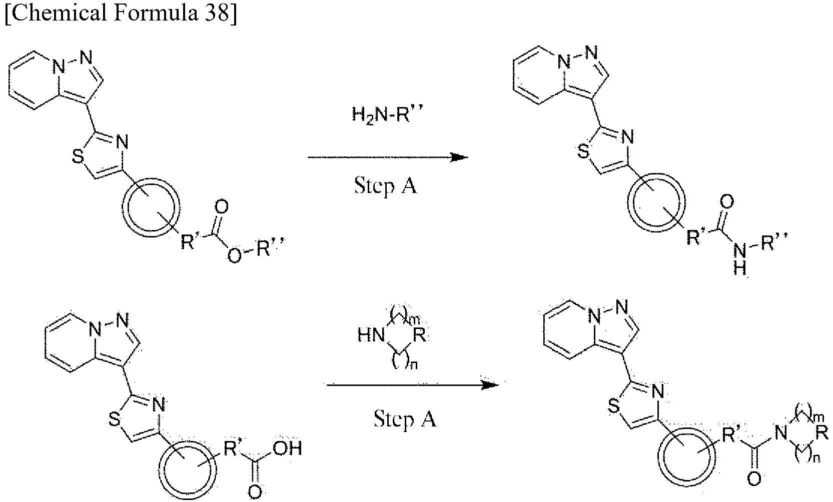 wherein R' is a bond or any structure, such as C₁₋₃ alkylene, -O-C-; R" is hydrogen or any substituent, such as a chain alkyl group, an aromatic hydrocarbon; R is selected from the group consisting of O, SO₂, CR³¹₂, and NR³¹; and m and n are each independently an integer of 1 to 4.

Step A: In these reactions, a predetermined amine is added to the ester compound in an equimolar amount to a large excess, preferably in a large excess, and the reaction is performed without any solvent or with a solvent such as DME, acetone, toluene, hexane, xylene, dioxane, THF, chloroform, dichloromethane, dichloroethane, preferably in the absence of a solvent. The reaction mixture is allowed to react at room temperature or by heating under reflux for 1 hour to 48 hours, preferably under heating and reflux for 12 hours, whereby a desired product can be obtained.

### (Transesterification reaction)

When an ester compound is synthesized in a main reaction or in an additional reaction, the ester compound can be subjected to an additional reaction represented by the following reaction formula to exchange substituents of the ester. 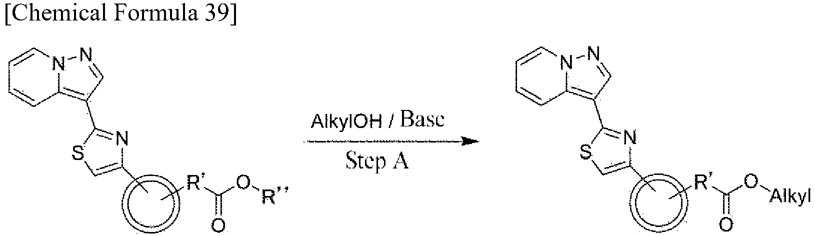 wherein R' is a bond or any structure, such as C₁₋₃ alkylene, -O-C-; R" is any substituent, such as a chain alkyl group, an aromatic hydrocarbon; and Alkyl is an alkyl group, such as a C₁₋₃ alkyl group.

Step A: In these reactions, a predetermined alkyl alcohol is added to the ester compound in an equimolar amount to a large excess, preferably in a large excess, and the reaction is performed without any solvent or with a solvent such as DME, acetone, toluene, hexane, xylene, dioxane, THF, chloroform, dichloromethane, dichloroethane, and DMF and using a base such as piperazine, sodium hydride, piperidine, pyrrolidine, sodium hydroxide, potassium hydroxide, DBU, and DBN, preferably piperazine. The reaction mixture is allowed to react at room temperature or by heating under reflux for 1 hour to 48 hours, preferably under heating and reflux for 12 hours, whereby a desired product can be obtained.

### (Reduction reaction of ester)

When an ester compound is synthesized in a main reaction or in an additional reaction, the ester compound can be subjected to an additional reaction represented by the following reaction formula to reduce the ester compound to an alcohol derivative. 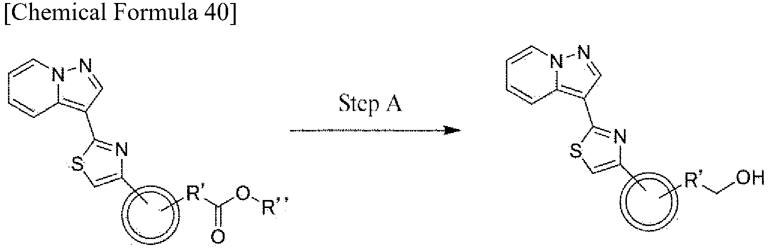 wherein R' is a bond or any structure, such as C1-3 alkylene, -O-C-; and R" is any substituent, such as a chain alkyl group, and an aromatic hydrocarbon.

Step A: In this reaction, a reducing agent such as LAH is added to a solvent such as diethyl ether, an ester compound is added thereto, and the mixture is allowed to react for a predetermined time. The dry solvent to be used varies depending on a starting material, a reagent, and the like, and is not particularly limited as long as the solvent dissolves the starting material to some extent without inhibiting the reaction, and preferred examples thereof include diethyl ether, THF, toluene, and hexane. Further, examples of the reducing agent include LAH, DIBAL, LiBH₄, and LiBEt₃. Usually, the reaction temperature is -20 to 50°C and the reaction time is 1 to 24 hours, and it is preferably 0°C for 2 hours. In addition to the hydride reduction, diborane or the like can also be used for the reduction of the ester.

### (Condensation reaction between a carboxylic acid and an amine)

When a carboxylic acid compound is synthesized in a main reaction or in an additional reaction, the carboxylic acid can be subjected to an additional reaction represented by the following reaction formula to convert the carboxylic acid into an amide compound. 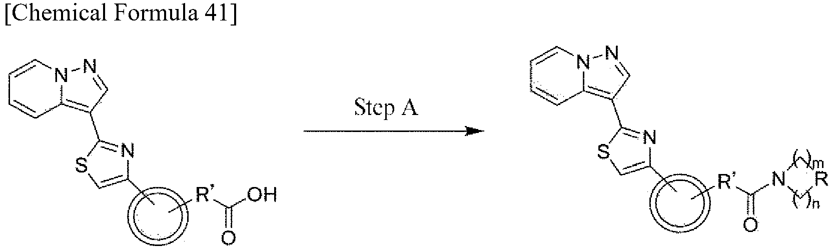 wherein R' is a bond or any structure, such as C₁₋₃ alkylene, -O-C-; R is selected from the group consisting of O, SO₂, CR³¹₂, and NR³¹; and m and n are each independently an integer of 1 to 4.

Step A: Details are the same as those of step A described above (condensation reaction of amine and carboxylic acid).

### (Formation of an alkyl metal salt of a carboxylic acid)

When a carboxylic acid is synthesized in a main reaction or in an additional reaction, the carboxylic acid can be subjected to an additional reaction represented by the following reaction formula to obtain an alkyl metal salt of the carboxylic acid. 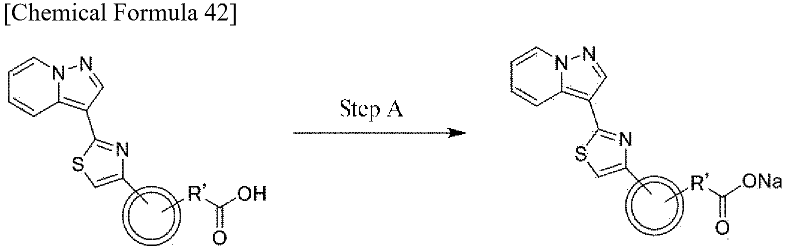 wherein R' is a bond or any structure, such as C₁₋₃ alkyl, -O-C-.

Step A: In this reaction, a carboxylic acid and sodium carbonate are added to purified water and reacted.

Examples of the purified water used here include tap water, ion-exchanged water, and distilled water. Examples of the alkyl metal salt include sodium, potassium, calcium, and barium salts. In particular, in the case of preparing a sodium salt, sodium hydroxide, sodium hydrogen carbonate, or the like can be used in addition to sodium carbonate. Usually, the reaction temperature is -20 to 50°C and the reaction time is 1 to 24 hours, and it is preferably 6 hours at room temperature.

### (Reductive alkyl-amination reaction of formyl group)

When an aldehyde is synthesized in a main reaction or in an additional reaction, the aldehyde can be subjected to an additional reaction represented by the following reaction formula to introduce a desired amino group. 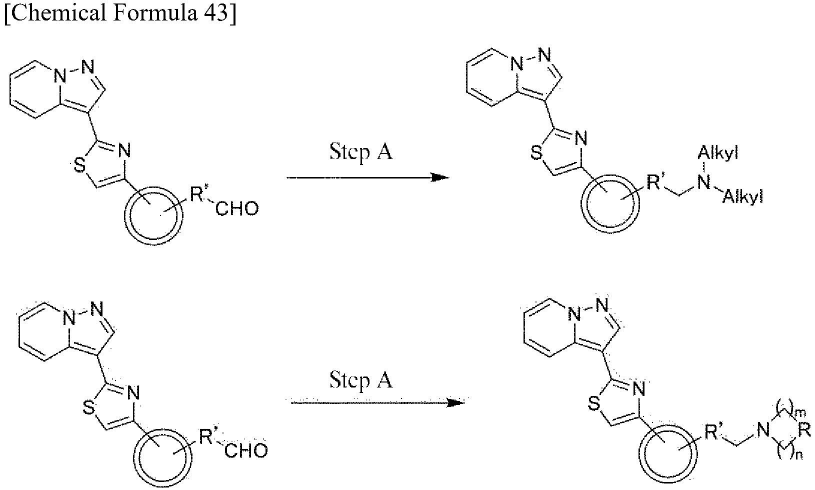 wherein R' is a bond or any structure, such as C₁₋₃ alkylene, -O-C-; Alkyl is an alkyl group (for example, a C₁₋₃ alkyl group); R is selected from the group consisting of O, SO₂, C(R³¹)R³¹', and NR³¹'; R³¹' is each independently hydrogen or any substituent, such as C₁₋₃ alkyl, CHO, -C(O)C(O)R³², -C(O)NR³¹₂, or -(CH₂)₁₋₅ 3- to 10-membered cycloaliphatic hydrocarbon); and m and n are each independently an integer of 1 to 4, preferably an integer of 1 to 2.

Step A: In this reaction, an aldehyde and an amine or a hydrochloride of the amine are added to a dry solvent such as dichloromethane and stirred, followed by addition of NaBH(OAc)₃ and allowing the reaction to proceed for a predetermined time to afford an alkylamine. The solvent to be used varies depending on a starting material, a reagent, and the like, and is not particularly limited as long as the solvent dissolves the starting material to some extent without inhibiting the reaction, and preferred examples thereof include toluene, hexane, xylene, dioxane, THF, dichloromethane, dichloroethane, DME, and DMF. Furthermore, NaBH(OAc)₃ and NaBH₃CN can be used as the reducing agent, and usually, the reaction temperature is -20 to 50°C, and the reaction time is 1 to 24 hours, and it is preferably 0°C for 3 hours.

### (Suzuki-Miyaura coupling reaction)

When a halide is synthesized in a main reaction or an additional reaction, the halide can be subjected to an additional reaction represented by the following reaction formula to introduce a desired substituent. 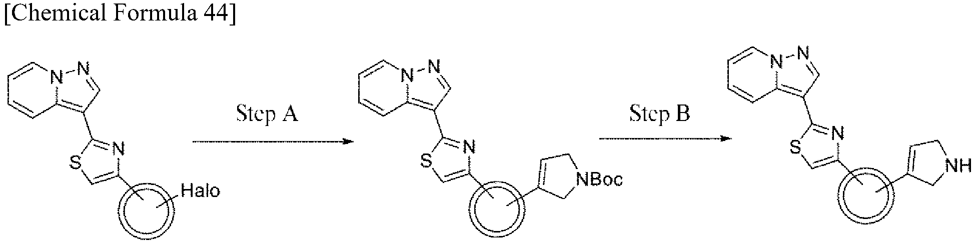 wherein Halo is a halogen such as iodine, bromine, chlorine, or fluorine, preferably bromine.

Step A: In this reaction, a halide, 1-(tert-butoxycarbonyl)-2,5-dihydro-1H-pyrrol-3-yl-boronic acid pinacol ester, Pd(PPh₃)₄ as a catalyst, and sodium carbonate are dissolved in a solvent such as a mixed solvent of DME and water, and reacted by heating for a predetermined time. That is, by reacting the aryl halide with boronic acid, boronic acid pinacol ester, or the like in the presence of a palladium catalyst, the Suzuki-Miyaura coupling reaction proceeds, and a corresponding condensate can be obtained. The solvent to be used varies depending on a starting material, a reagent, and the like, and is not particularly limited as long as the solvent dissolves the starting material to some extent without inhibiting the reaction, and preferred examples thereof include toluene, hexane, xylene, methanol, ethanol, dioxane, THF, dichloromethane, dichloroethane, DME, and DMF. Examples of the base to be used include potassium carbonate, sodium carbonate, sodium bicarbonate, potassium phosphate, potassium acetate, sodium hydride, sodium methoxide, sodium ethoxide, sodium butoxide, sodium hydroxide, potassium hydroxide, DBU, and DBN. Examples of the palladium catalyst to be used include Pd(OAc)₂, Pd(PPh₃)₄, PdCl₂(PPh₃)₂, PdCl₂(dppf), and Pd(acac)₂. Furthermore, XPhos can also be used as the reaction additive. Usually, the reaction temperature is 40 to 150°C and the reaction time is 1 to 24 hours, and it is preferably 80°C for 1 hour.

Step B: When the introduced substituent has a protecting group, it can be converted into an amino group by a deprotection reaction. This reaction is carried out under the same conditions as Step C in (Reduction of nitro group with metal, and protection and deprotection reactions of generated amino group) described above.

### (3) Synthesis of a starting material

A commercially available product may be used as the compound of the formula (III) as a starting material, and for example, as shown in the following reaction formula, the compound can be synthesized by reacting an acetophenone derivative with a halogenating agent in a solvent to obtain an α-halogenated acetophenone. 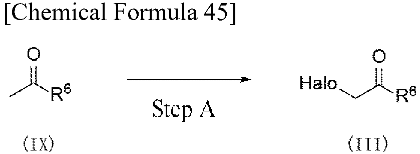 wherein R⁶ is as defined in the formula (I) above, and Halo is a halogen such as iodine, bromine, chlorine, or fluorine, preferably bromine.

Step A: This reaction can be carried out by reacting a compound of formula (IX) with a metal halide such as CuBr₂ in a solvent such as ethyl acetate. When the substituent on the benzene ring is an alkyl group or the like, bromination of the α-position of the acetyl group easily proceeds in about 1 hour by heating with two equivalents of CuBr₂ in ethyl acetate. In this case, when the reaction is performed for a long time, a dibromoacetyl form is produced as a by-product. On the other hand, when the substituent is an electron-withdrawing substituent such as an ester, the reaction time needs to be extended to 2 to 16 hours. In addition, when an acid-sensitive substituent is present, caution is required because a small amount of HBr may be generated during the reaction. Furthermore, in the case of a basic substituent, the reaction with CuBr₂ often does not proceed; therefore, the use of conditions such as bromine-acetic acid allows the synthesis of phenacyl bromide. The bromination conditions are not limited thereto.

This reaction can also be carried out by dissolving the compound of the formula (IX) in a solvent such as acetic acid, adding a halogen such as bromine or a hydrohalic acid such as hydrobromic acid to the solution, and reacting the mixture.

The reaction can also be carried out by dissolving the compound of the formula (IX) in dioxane and adding bromine-1,4-dioxane complex thereto.

It is also possible to synthesize a substituted phenacyl halide by direct haloacetylation, for example by a Friedel-Crafts reaction.

In addition, regarding the synthesis of the compound of the formula (IX) or the derivative thereof, the compound of the formula (III) or the derivative thereof can be synthesized using various reactions described in "Additional reactions", and examples thereof include the following reactions.

### (Protection by phthalic anhydride and synthesis of α-haloacetylpyridine)

The amino group can also be protected with phthalic anhydride before the main reaction, and for example, Step A exemplified below can be utilized. In addition, a compound having a halogen can be subjected to the reaction of Step B exemplified below to convert the halogen into an acetyl group. In addition, a compound having an acetyl group can be subjected to the reaction of Step C exemplified below to synthesize an α-haloacetylpyridine derivative. 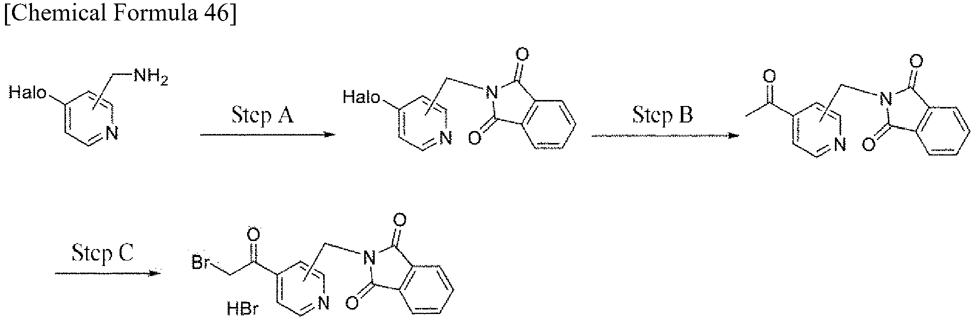 wherein Halo is a halogen such as iodine, bromine, chlorine, or fluorine, preferably bromine.

Step A: In this reaction, the amine derivative is reacted with phthalic anhydride to obtain a phthalimide derivative in which an amino group is protected. The reaction is carried out in a solvent or in the absence of a solvent, in the presence of an acid. The solvent to be used varies depending on a starting material, a reagent, and the like, and is not particularly limited as long as the solvent dissolves the starting material to some extent without inhibiting the reaction, and examples thereof include ethanol, methanol, DMF, toluene, water, and dichloromethane. The acid to be used varies depending on the starting material, the solvent to be used, and the like, and is not particularly limited as long as the reaction is not inhibited, and preferred examples thereof include acetic acid and sulfuric acid. The reaction temperature is 80 to 170°C and the reaction time is 1 to 24 hours, and it is preferably 120°C for 1 hour.

Step B: An acetyl derivative can be obtained by subjecting a halogen derivative to a reaction with tributyl(1-ethoxyvinyl)tin in the presence of a Pd reagent. The solvent to be used varies depending on a starting material, a reagent, and the like, and is not particularly limited as long as the solvent dissolves the starting material to some extent without inhibiting the reaction, and preferred examples thereof include toluene, benzene, dioxane, DMF, THF, ethyl acetate, and acetonitrile. The Pd reagent to be used varies depending on the starting material, the solvent to be used, and the like, and is not particularly limited as long as the reaction is not inhibited, and preferred examples thereof include PdCl₂(PPh₃)₂, PdCl₂(dppf), Pd(PPh₃)₄, and Pd₂(dba)₃. In a normal heating reaction, the reaction temperature is 70 to 110°C and the reaction time is 0.5 to 48 hours, and it is preferably 160°C for 5 minutes using a microwave synthesis apparatus.

Step C: An acetyl derivative is reacted with bromine in the presence of an acid to afford an α-haloacetylpyridine derivative. The reaction is carried out in a solvent or in the absence of a solvent and in the presence of an acid. The solvent to be used varies depending on a starting material, a reagent, and the like, and is not particularly limited as long as the solvent dissolves the starting material to some extent without inhibiting the reaction, and preferred examples thereof include chloroform, dichloromethane, diethyl ether, dioxane, methanol, ethanol, DMF, THF, and acetonitrile. The acid to be used varies depending on the starting material, the solvent to be used, and the like, and is not particularly limited as long as the reaction is not inhibited, and preferred examples thereof include acetic acid, hydrobromic acid, p-toluenesulfonic acid, hydrochloric acid, and sulfuric acid. The reaction temperature is 0 to 90°C and the reaction time is 1 to 12 hours, and it is preferably 70°C for 1 hour.

The compound of the formula (IV), which serves as the starting material, may be commercially available and can also be prepared, for example, by synthesizing, from a thioamide, sequentially a carbonyl compound and a halide as shown in the following reaction formula. 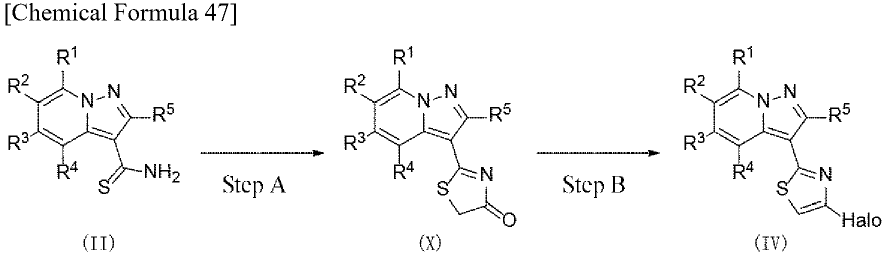 wherein R¹ to R⁵ are as defined in the formula (I) above; and Halo is a halogen such as iodine, bromine, or chlorine, preferably bromine.

Step A: In this reaction, a thioamide of formula (II) having substituent(s) corresponding to those of a compound of formula (I) is dissolved in a solvent such as DME, acetone, toluene, hexane, xylene, acetonitrile, ethyl acetate, dioxane, THF, ethanol, methanol, dichloromethane, chloroform, dichloroethane, DMF, or water, and a haloacetic acid ester such as methyl bromoacetate, ethyl bromoacetate, or ethyl chloroacetate is added thereto and reacted to afford a corresponding carbonyl compound represented by formula (X). Usually, the reaction temperature is 20 to 200°C and the reaction time is 1 to 24 hours, and it is preferably 80°C for 1 hour.

Step B: In this reaction, the compound represented by the formula (X) is dissolved in a solvent such as toluene together with carbon tetrabromide and triphenylphosphine, and reacted by heating for a predetermined time, whereby the Appel reaction proceeds to obtain the corresponding halide. The solvent to be used varies depending on the reagent, and the like, and is not particularly limited as long as the solvent dissolves the starting material to some extent without inhibiting the reaction, and preferred examples thereof include DME, acetone, toluene, hexane, xylene, acetonitrile, ethyl acetate, dioxane, THF, ethanol, methanol, dichloromethane, chloroform, dichloroethane, DMF, and water. As the reaction reagent, carbon tetrachloride or the like can be used in addition to carbon tetrabromide. Usually, the reaction temperature is 0 to 200°C and the reaction time is 1 to 24 hours, and it is preferably 110°C for 1 hour.

The compound of the formula (V), which serves as the starting material, may be commercially available and can also be prepared, for example, by the reaction shown below. 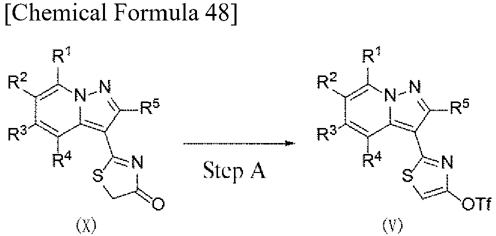 wherein R¹ to R⁵ are as defined in the above formula (I), and Tf represents triflate.

Step A: In this reaction, a compound represented by the formula (X) is dissolved in a solvent such as dichloromethane together with N-phenylbis(trifluoromethanesulfonimide) and a base, and reacted to afford a corresponding triflate compound. Examples of the base to be used include pyridine, 4-dimethylaminopyridine, triethylamine, N,N-diisopropylethylamine, sodium carbonate, potassium carbonate, and tripotassium phosphate. The solvent to be used varies depending on the reagent, and the like, and is not particularly limited as long as the solvent dissolves the starting material to some extent without inhibiting the reaction, and preferred examples thereof include DME, acetone, toluene, hexane, xylene, acetonitrile, ethyl acetate, dioxane, THF, ethanol, methanol, dichloromethane, chloroform, dichloroethane, DMF, and water. Usually, the reaction temperature is -78 to 40°C and the reaction time is 10 minutes to 24 hours, and it is preferably 0°C for 15 minutes.

The compound of the formula (VII), which serves as the starting material, may be commercially available and can also be prepared, for example, by the reaction formula shown below. 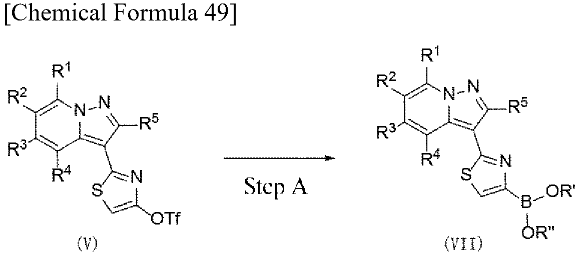 wherein R¹ to R⁶ are as defined in the formula (I) above; and R' and " each represent a hydrogen atom, or R' and R", together with two adjacent oxygen atoms, form an alkylenedioxy group.

Step A: In this reaction, the compound represented by the formula (V) is dissolved in a solvent such as DME, acetone, toluene, hexane, xylene, acetonitrile, ethyl acetate, dioxane, THF, ethanol, methanol, dichloromethane, chloroform, dichloroethane, DMF, water or the like. This mixture is then reacted with a diborane compound such as 4,4,4',4',5,5,5',5'-octamethyl-2,2'-bi(1,3,2-dioxaborolane) or 4,4,4',4',5,5,5',5'-octaethyl-2,2'-bi(1,3,2-dioxaborolane), a palladium catalyst such as palladium acetate, palladium chloride, tris(dibenzylideneacetone)dipalladium(0), and a bidentate ligand such as 2-dicyclohexylphosphino-2',4',6'-triisopropylbiphenyl, 2-di-tert-butylphosphino-3,4,5,6-tetramethyl-2',4',6'-triisopropyl-1,1'-biphenyl, 2,2'-bis(diphenylphosphino)-1,1'-binaphthyl, 4,5-bis (diphenylphosphino)-9,9-dimethylxanthene. Usually, the reaction temperature is 0°C to 150°C and the reaction time is 10 minutes to 24 hours, and it is preferably 18 hours at room temperature.

### 3. Thiazolyl-pyrazolo[1,5-a]pyridine derivative

The thiazolyl-pyrazolo[1,5-a]pyridine derivative may take the form of a hydrate, a solvate or a pharmaceutically acceptable salt (acid or base addition salts), a prodrug, and the like. Thiazolyl-pyrazolo[1,5-a]pyridine or a derivative thereof includes various isomers.

The term "pharmaceutically acceptable salt" means a salt that is pharmaceutically acceptable and capable of exerting the desired pharmacological activity of the parent compound. When the compound according to the present invention is obtained as a free form, it can be converted into a salt according to a conventional method, and conversely, when the compound according to the present invention is obtained as a salt, it can be converted into a free form according to a conventional method.

Pharmaceutically acceptable salts are well known in the art, and are described in detail, for example, in S. M. Berge, et al., J. Pharmaceutical Sciences, 1977, 66, 1-19, and Remington's Pharmaceutical Sciences, 17th ed., Mack Publishing Company, Easton, PA, 1985, the contents of which are incorporated herein by reference.

Examples of the pharmaceutically acceptable acid addition salt include salts formed by addition of inorganic acids such as hydrochloric acid, hydrobromic acid, sulfuric acid, nitric acid, and phosphoric acid; organic acids such as acetic acid, trifluoroacetic acid, propionic acid, hexanoic acid, cyclopentanepropionic acid, glycolic acid, pyruvic acid, lactic acid, oxalic acid, maleic acid, malonic acid, succinic acid, fumaric acid, malic acid, tartaric acid, citric acid, benzoic acid, cinnamic acid, 3-(4-hydroxybenzoyl)benzoic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylene-bis(3-hydroxy-2-en-1-carboxylic acid), 3-phenylpropionic acid, trimethylacetic acid, tert-butylacetic acid, lauryl sulfuric acid, gluconic acid, glutamic acid, hydroxynaphthoic acid, stearic acid, muconic acid, and salicylic acid; and the like.

Examples of the pharmaceutically acceptable base addition salt include salts in which acidic protons present in the parent compound are replaced by metal ions such as sodium, potassium, lithium, ammonium, calcium, magnesium, iron, zinc, copper, manganese, and aluminum. Further examples of the pharmaceutically acceptable base addition salt include salts derived from organic bases such as primary, secondary and tertiary amines, substituted amines, and cyclic amines. Examples of the organic base include isopropylamine, trimethylamine, diethylamine, triethylamine, tripropylamine, ethanolamine, 2-dimethylaminoethanol, dicyclohexylamine, lysine, arginine, histidine, caffeine, procaine, hydrabamine, choline, betaine, ethylenediamine, glucosamine, methylglucamine, theobromine, purine, piperazine, piperidine, N-ethylpiperidine, tromethamine, N-methylglucamine, and a polyamine resin. Representative examples of the organic base include isopropylamine, diethylamine, ethanolamine, trimethylamine, dicyclohexylamine, choline, and caffeine.

Examples of the solvate include, in addition to hydrates, organic solvates such as a dimethyl sulfoxide solvate, a dimethylformamide solvate, or an alcohol solvate such as an ethanol solvate, a methanol solvate, or an n-propyl alcohol solvate.

The present compound or a pharmaceutically acceptable salt or solvate (including hydrate) thereof can exist in an amorphous form. In general, it is known that various crystal polymorphs exist for many organic compounds and salts thereof, and various crystal forms can be produced by various methods well known in the art. Specifically, for example, various crystal forms including amorphous form can be produced by a melting method, an extruder, or the like. The amorphous form can also be provided in the form of a solid dispersion containing an excipient or the like. (However, even for the compounds, raw materials, reagents, and the like described so far, it is essential to address the following points. First, in the use of a compound or the like that increases the "risk of contamination of nitrosamines in pharmaceuticals", sufficient care is required for the use thereof. In addition, it is necessary to pay careful attention to avoid the risk of contamination of various heavy metals and the like on the basis of the "elemental impurity guideline". Even with elemental impurities other than classes 1, 2A, 2B, and 3, zinc and manganese are known to deteriorate the symptoms of patients with liver dysfunction, and aluminum is known to deteriorate the symptoms of patients with renal dysfunction, thus sufficient attention is essential for quality control. Furthermore, it is necessary to pay attention to residual solvents, particularly solvation, of dimethylformamide and methanol of class 2, and dimethyl sulfoxide and ethanol of class 3, based on the "residual solvent guideline").

The above-described thiazolyl-pyrazolo[1,5-a]pyridine derivative or a pharmaceutically acceptable salt, solvate, or hydrate thereof may take the form of a prodrug.

As used herein, the term "prodrug" refers to a compound that is converted into the present compound (compound of the formula (I)) by a reaction such as oxidation, reduction, or hydrolysis by an enzyme or the like under physiological conditions in vivo.

Examples of the prodrug include:
(1) a compound in which amino of the present compound is acylated, alkylated, phosphorylated (for example, a compound in which amino of compound (I) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, tert-butylated, ethoxycarbonylated, tert-butoxycarbonylated, acetylated, or cyclopropylcarbonylated);
(2) a compound in which hydroxy of the present compound is acylated, alkylated, phosphorylated, or borated (for example, a compound in which hydroxy of the compound (I) is acetylated, palmitoylated, propanoylated, pivaloylated, succinylated, fumarylated, alanylated, or dimethylaminomethylcarbonylated); and
(3) a compound in which carboxy of the present compound is esterified or amidated (for example, a compound in which carboxy of the compound (I) is ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonyl ethyl esterified, or methyl amidated). These compounds can be produced from the present compound by a known method.

Details on prodrugs are described in detail, for example, in T. Higuchi and V. Stella, "Pro-drugs as Novel Delivery Systems", Vol. 14 of the A. C. S. Symposium Series, Bioreversible Carriers in Drug Design, ed. Edward B. Roche, American Pharmaceutical Association and Pergamon Press, 1987, and in "Development of Pharmaceuticals", Volume 7, Molecular Design, pp. 163 to 198, published by Hirokawa-Shoten Ltd., 1990, the contents of which are incorporated herein by reference.

Thiazolyl-pyrazolo[1,5-a]pyridine or a derivative thereof can take various isomeric forms, and examples thereof include a geometric isomer, an optical isomer, a rotational isomer, a stereoisomer, and a tautomer. These isomers can be purified and isolated by using ordinary separation means, for example, recrystallization, a diastereomer salt method, an enzyme resolution method, or various chromatography (for example, thin layer chromatography, column chromatography, gas chromatography).

### 4. Pharmaceutical use of thiazolyl-pyrazolo[1,5-a]pyridine and derivatives thereof

The compound of the formula (I) can inhibit myosin light chain kinase 4 (MYLK4) activity, as demonstrated in the Examples below. Thus, the compound of the formula (I) or a pharmaceutically acceptable derivative thereof, or a pharmaceutical composition containing the same can be used to inhibit MYLK4 activity in a biological sample or in vivo. In addition, the compound of the formula (I) or a pharmaceutically acceptable derivative thereof, or a pharmaceutical composition containing the same can be used to prevent, alleviate, or treat a disease, disorder or condition in which MYLK4 is involved, through inhibition of MYLK4 activity.

Examples of the disease, disorder or condition involving myosin light chain kinase 4 (MYLK4) include arteriosclerosis, inflammatory bowel disease, osteosarcoma, ischemic heart disease, glaucoma, ocular hypertension, impaired aqueous humor outflow, dry eye, uveitis, age-related macular degeneration, and diabetic retinopathy. Accordingly, the compound of the formula (I) or a pharmaceutically acceptable derivative thereof, or a pharmaceutical composition containing the same can be used to prevent, alleviate, and/or treat these diseases, disorders, or conditions. Among them, it is effective for preventing, alleviating and/or treating arteriosclerosis, inflammatory bowel disease, osteosarcoma, ischemic heart disease, glaucoma, ocular hypertension, impaired aqueous humor outflow, dry eye, uveitis, age-related macular degeneration, or diabetic retinopathy, particularly glaucoma, ocular hypertension, or impaired aqueous humor outflow, and it is particularly effective to use for reducing the intraocular pressure of a subject.

### 5. Pharmaceutical composition (formulation)

A pharmaceutical composition can be prepared by combining an effective amount of the compound of the formula (I) or a pharmaceutically acceptable derivative thereof with a pharmaceutically acceptable carrier and other auxiliaries as necessary. This does not preclude use of the compound of formula (I) or a derivative thereof in bulk form as a medicament.

The pharmaceutical composition can be administered orally or parenterally, and examples of the dosage form for oral administration include tablets, fine granules, coated tablets, powders, granules, pills, capsules (for example, hard gelatin capsules or soft gelatin capsules), microcapsules, and syrups. Examples of the dosage form for parenteral administration include an injection (including freeze-dried preparations for injection to be dissolved at the time of use), eye drops, a lotion, an aerosol, an ointment, a patch, and a suppository. It can also be prepared as a liposomal preparation. Furthermore, a liquid preparation in which a compound of the formula (I) or a pharmaceutically acceptable derivative thereof is previously dispersed in a pharmaceutically acceptable solvent can also be used, and in this case, for example, a syrup for oral administration, an injection for parenteral administration (including freeze-dried preparations for injection to be dissolved at the time of use), eye drops, or the like can be used.

The pharmaceutical composition described above may also be administered as, for example, a solution, a suspension, an emulsion, a microemulsion, a multiphase emulsion, a foam, a salve, a paste, a plaster, an ointment, a coated tablet, a rinse, a rectal capsule, a drop, a gel, a spray, a powder, an aerosol, an inhalant, eye drops, an ophthalmic ointment, an ophthalmic rinse, an infusion solution, or an implant.

As described above, the pharmaceutical composition can be in various dosage forms, and the preparation thereof can be carried out by blending various carriers (excipients) and various auxiliaries as necessary according to the dosage form, the administration method, and the like.

Examples of the auxiliaries include colorants, sweeteners, flavoring agents, binders, adsorbents, lubricants, disintegrants, softeners, suspending agents, emulsifiers, preservatives, antioxidants, surfactants, stabilizers, pH adjusters, dispersants, isotonic agents, wetting agents, solubilizing agents, solubilizing aids, and/or absorption promoters.

When the pharmaceutical composition is in solid dosage form, examples of the carrier (excipient) include crystalline cellulose, saccharides (glucose, white sugar, lactose, D-mannitol, D-sorbitol, and the like), starches (corn starch, potato starch, wheat starch, rice starch, and the like), magnesium silicate, sodium hydrogen phosphate, calcium hydrogen phosphate, sodium citrate, and talc.

Examples of the disintegrant include sodium carbonate, calcium carbonate, gum arabic, starches (corn starch, potato starch, wheat starch, tapioca starch, rice starch, and the like), agar, alginic acid, a silicate complex, tragacanth, crystalline cellulose, low substituted hydroxypropylcellulose, croscarmellose sodium, carmellose calcium, carmellose sodium, and sodium carboxymethyl starch.

Examples of the binder include cellulose derivatives, starches, alginates, gelatin, polyvinylpyrrolidone, sucrose, and acacia gum.

Examples of the wetting agent include glycerol, cetyl alcohol, glycerol monostearate, magnesium stearate, talc, calcium stearate, solid polyethylene glycol, and sodium lauryl sulfate.

Examples of the absorption promoter include quaternary ammonium compounds.

Examples of the adsorbent include kaolin and bentonite.

Examples of the lubricant include carnauba wax, hardened oil, magnesium stearate, calcium stearate, sodium hydrogen phosphate, calcium hydrogen phosphate, and white beeswax.

Examples of the preservative include parabens, chlorobutanol, phenol, sorbic acid, and benzalkonium chloride.

The pharmaceutical composition in liquid dosage form for oral administration includes pharmaceutically acceptable emulsions, solutions, suspensions, syrups, and elixirs. Pharmaceutical compositions of such dosage forms are generally prepared by dissolving or dispersing the formulation components in a carrier such as a solubilizing agent and an emulsifier, for example, distilled water, saline, aqueous dextrose, glycerol, ethanol, isopropyl alcohol, ethyl carbonate, ethyl acetate, benzyl alcohol, benzyl benzoate, propylene glycol, 1,3-butylene glycol, and dimethylformamide; oils such as cottonseed oil, peanut oil, corn germ oil, olive oil, castor oil, sesame oil, glycerol, tetrahydrofurfuryl alcohol, polyethylene glycols, and sorbitan fatty acid ester oils; or a mixture of these substances to form a solution or suspension.

The pharmaceutical composition in liquid dosage form can optionally contain a suspending agent, a solubilizing agent, or a solubilizing aid. Examples of the suspending agent include ethoxylated isostearyl alcohols, polyoxyethylene sorbitol and sorbitan esters, microcrystalline cellulose, aluminum metahydroxide, bentonite, agar and tragacanth. Examples of the solubilizing agent include ethanol, dilute hydrochloric acid, sodium hydroxide, sodium bicarbonate, olive oil, squalene, squalane, physiological saline, water for injection, rapeseed oil, glucose, propylene glycol, polysorbate, and macrogol. Examples of the solubilizing aid include L-arginine, α-cyclodextrin, β-cyclodextrin, D-sorbitol, soybean oil, urea, white sugar, hydroxypropylcellulose, hypromellose, povidone, and D-mannitol.

The pharmaceutical composition in dosage form suitable for injection is in the form of a physiologically acceptable aqueous or non-aqueous sterile solution, a dispersion, a suspension or emulsion, or a sterile powder which is reconstituted into a sterile injectable solution and/or dispersion at the time of use. Representative examples of suitable aqueous or non-aqueous carriers, diluents, solvents or vehicles include distilled water, ethanol, polyols (propylene glycol, polyethylene glycol, glycerol, and the like), suitable mixtures thereof, vegetable oils such as olive oil, and injectable organic esters such as ethyl oleate, and the like. These aqueous or non-aqueous carriers, diluents, solvents, or vehicles may further contain appropriate salts, pH adjusters, as in physiological saline. The pharmaceutical composition can also contain a suspending agent, a solubilizing agent, or a solubilizing aid, as necessary. As an agent that delays absorption of the injectable agent, for example, aluminum monostearate and gelatin can also be used.

In the case of preparing an injection or eye drops, a pH adjuster, a buffer, a stabilizer, a preservative, and the like are added as necessary to prepare a subcutaneous, intramuscular, or intravenous injection by a conventional method. After the solution is stored in a container, the injection or eye drops may be formed into a solid preparation by freeze-drying or the like to prepare a preparation at the time of use. One dose may be stored in a container, or multiple doses may be stored in the same container.

The pharmaceutical composition in dosage form suitable for rectal administration is usually prepared, for example, as a suppository, using a non-irritating excipient which is solid at room temperature but which melts or dissolves at body temperature and release the active ingredient in a suitable body cavity, and examples of such an excipient include cocoa butter, polyethylene glycol, or a suitable wax.

The pharmaceutical composition in dosage form for topical application includes an ointment, a powder, a spray, and an inhalant. In such dosage forms, the formulation components are usually mixed under sterile conditions with a pharmaceutically acceptable carrier and, if necessary, any preservatives, buffers, or propellants to prepare a pharmaceutical composition. Ophthalmic preparations, ointments, powders, and solutions are typical examples of formulations for topical administration, and suitable carriers (excipients) and auxiliaries are obvious to the person skilled in the art.

Methods for preparing pharmaceutical compositions suitable for each dosage form are known in the art and are described, for example, in Remington's Pharmaceutical Sciences, 18th ed., Mack Publishing Company, Easton, PA, 1990, which is incorporated herein by reference.

When the present compound or a derivative thereof or the pharmaceutical composition is administered to a mammal (for example, mouse, rat, hamster, rabbit, cat, dog, cow, sheep, monkey, human), particularly a human, it can be administered using any dosage form suitable for the desired delivery route, for example, orally, transdermally, intradermally, intrabronchally, intranasally, intraarterially, intravenously, intramuscularly, subcutaneously, intraperitoneally, vaginally, rectally, sublingually, intracranially, epidurally, intratracheally, intraocularly or at other local sites.

### EXAMPLES

The compound of the present invention can be produced, for example, by a method described in the following Examples, and the effect of the compound can be confirmed by a method described in the following Examples. However, these are exemplary, and the present invention is not limited to the following specific examples in any case, and may be changed without departing from the scope of the present invention.

The "silica gel" of "silica gel column chromatography" described in the Examples means Wako Gel C-200 (particle size 75 to 150 µm) manufactured by FUJIFILM Wako Pure Chemical Corporation and the cartridge (UniversalColumn Premium, particle size 30 µm) manufactured by YAMAZEN CORPORATION, unless otherwise specified.

The "TLC" of "preparative TLC" described in the Examples means TLC glass plate silica gel 60F254 (0.5 mm, 1 mm, 2 mm) manufactured by Merck Corporation, unless otherwise specified.

"Under reduced pressure" indicates a reduced-pressure state of about 1 to 50 mmHg using a vacuum pump or the like.

"Room temperature" refers to a range of about 10°C to 30°C. % represents % by weight unless otherwise specified.

### Synthesis of Compounds

### [Example 1] Synthesis of 3-(2-(pyrazolo[1,5-a] pyridin-3-yl)thiazol-4-yl)phenol (compound 3)

[1] Compound (3) was synthesized by reacting a thioamide with a substituted α-bromoacetophenone.

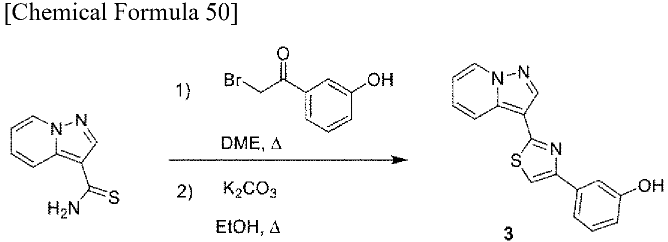

Pyrazolo[1,5-a] pyridine-3-carbothioamide (178 mg, 1.00 mmol) was dissolved in DME (10 mL), and 3-hydroxyphenacyl bromide (228 mg, 1.06 mmol) was added thereto under ice cooling, and the mixture was stirred at room temperature for 16 hours. The reaction mixture was heated and stirred at 50°C for 1 hour and then allowed to cool, and the reaction mixture was concentrated under reduced pressure to remove the solvent. Ethanol (10 mL) was added to the residue, then excess potassium carbonate (327 mg) was added, and the reaction mixture was refluxed at 70°C for 1 hour. After cooling, the reaction mixture was concentrated under reduced pressure to remove the solvent, and the residue was purified by silica gel column chromatography to obtain the title compound (3) (194 mg, 0.661 mmol, 66.1% yield) from a fraction of n-hexane : ethyl acetate (1 : 4).

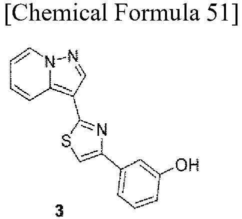

¹H-NMR(CDCl₃) δ:4.93 (1H, brs), 6.83 (1H, d, J=7.8Hz), 6.93 (1H, t, J=6.9Hz), 7.32 (1H, t, J=8.2Hz), 7.36 (1H, s), 7.39 (1H, dd, J=7.1Hz, J=8.5Hz), 7.55 (2H, m), 8.39 (1H, s), 8.46 (1H, d, J=8.7Hz), 8.52 (1H, d, J=6.9Hz).

[Examples 2 to 26] Synthesis of the following compounds:
4-phenyl-2-(pyrazolo[1,5-a]pyridin-3-yl)thiazole (compound 71),
2-methoxy-5-(2-(pyrazolo[1,5-a]pyridin-3-yl)thiazol-4-yl)phenol (compound 79),
4-(3-fluorophenyl)-2-(pyrazolo[1,5-a]pyridin-3-yl)thiazole (compound 112),
4-(2-fluorophenyl)-2-(pyrazolo[1,5-a]pyridin-3-yl)thiazole (compound 122),
4-(2-(pyrazolo[1,5-a]pyridin-3-yl)thiazol-4-yl)benzene-1,3-diol (compound 120),
4-(2,4-difluorophenyl)-2-(pyrazolo[1,5-a]pyridin-3-yl)thiazole (compound 121),
4-(3-methoxyphenyl)-2-(pyrazolo[1,5-a]pyridin-3-yl)thiazole (compound 83),
2-(pyrazolo[1,5-a]pyridin-3-yl)-4-(o-tolyl)thiazole (compound 132),
1-methyl-6-(2-(pyrazolo[1,5-a]pyridin-3-yl)thiazol-4-yl)-3,4-dihydroquinolin-2(1H)-one (compound 131),
N-(4-(2-(pyrazolo[1,5-a]pyridin-3-yl)thiazol-4-yl)benzyl)acetamide (compound 12),
4-(4-methoxypyridin-2-yl)-2-(pyrazolo[1,5-a]pyridin-3-yl)thiazole (compound 7),
2-(pyrazolo[1,5-a]pyridin-3-yl)-4-(pyridin-3-yl)thiazole (compound 15),
2-(pyrazolo[1,5-a]pyridin-3-yl)-4-(pyridin-4-yl)thiazole (compound 92),
2-(pyrazolo[1,5-a]pyridin-3-yl)-4-(pyridin-2-yl)thiazole (compound 93),
4-(2-chloropyridin-4-yl)-2-(pyrazolo[1,5-a]pyridin-3-yl)thiazole (compound 94),
4-(2-methoxypyridin-4-yl)-2-(pyrazolo[1,5-a]pyridin-3-yl)thiazole (compound 95),
4-(6-methoxypyridin-2-yl)-2-(pyrazolo[1,5-a]pyridin-3-yl)thiazole (compound 96),
4-(5-methylpyridin-3-yl)-2-(pyrazolo[1,5-a]pyridin-3-yl)thiazole (compound 97),
6-(2-(pyrazolo[1,5-a]pyridin-3-yl)thiazol-4-yl)-[1,3]dioxolo[4,5-b]pyridine (compound 98),
4-(2-methylpyridin-4-yl)-2-(pyrazolo[1,5-a]pyridin-3-yl)thiazole (compound 99),
4-(2-fluoropyridin-4-yl)-2-(pyrazolo[1,5-a]pyridin-3-yl)thiazole (compound 100),
4-(2-bromopyridin-4-yl)-2-(pyrazolo[1,5-a]pyridin-3-yl)thiazole (compound 101),
4-(5-chloropyridin-2-yl)-2-(pyrazolo[1,5-a]pyridin-3-yl)thiazole (compound 125),
4-(4-chloropyridin-2-yl)-2-(pyrazolo[1,5-a]pyridin-3-yl)thiazole (compound 135),
   and
2'-bromo-2-(pyrazolo[1,5-a]pyridin-3-yl)-4,5'-bithiazole (compound 37)

Compound (71), compound (79), compound (112), compound (122), compound (120), compound (121), compound (83), compound (132), compound (131), compound (12), compound (7), compound (15), compound (92), compound (93), compound (94), compound (95), compound (96), compound (97), compound (98), compound (99), compound (100), compound (101), compound (125), compound (135), and compound (37) were synthesized according to the method for synthesizing compound (3), using a substituted α-bromoacetophenone corresponding to the final compound.

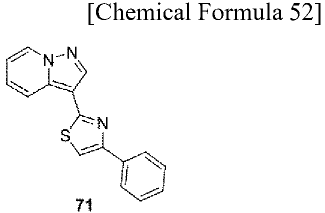

¹H-NMR(CDCl₃) δ:6.92 (1H, dt, J=1.4Hz, 6.9Hz), 7.37 (3H, m), 7.46 (2H, t, J=7.8Hz), 8.01 (2H, d, J=6.9Hz), 8.40 (1H, s), 8.48 (1H, d, J=8.7Hz), 8.52 (1H, d, J=6.9Hz).

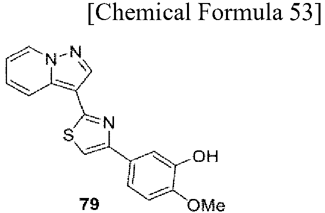

¹H-NMR(CDCl₃) δ:3.78 (3H, s), 6.97 (1H, t, J=8.7Hz), 7.09 (1H, dt, J=1.4Hz, 6.9Hz), 7.44 (1H, dd, J=2.3Hz, 8.2Hz), 7.50 (1H, d, J=2.3Hz), 7.54 (1H, ddd, J=0.9Hz, 6.9Hz, 8.7Hz), 7.73 (1H, s), 8.38 (1H, d, J=8.7Hz), 8.56 (1H, d), 8.81 (1H, d, J=6.9Hz), 9.09 (1H, brs).

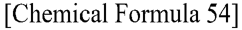

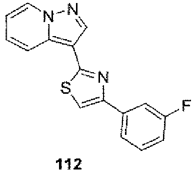

¹H-NMR(CDCl₃) δ:6.94 (1H, dt, J=0.9Hz, 6.9Hz), 7.05 (1H, dt, J=2.3Hz, 8.2Hz), 7.41 (3H, m), 7.76 (2H, m), 8.39 (1H, s), 8.46 (1H, d, J=8.7Hz), 8.52 (1H, d, J=6.9Hz).

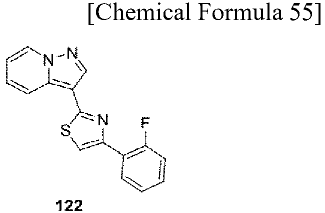

¹H-NMR(CDCl₃) δ:6.93 (1H, dt, J=1.4Hz, 6.9Hz), 7.17 (1H, m), 7.31 (2H, m), 7.40 (1H, ddd, J=0.9Hz, 6.4Hz, 9.2Hz), 7.67 (1H, d, J=2.3Hz), 8.38 (1H, dt, J=2.8Hz, 7.8Hz), 8.41 (1H, s), 8.45 (1H, d, J=8.7Hz), 8.53 (1H, d, J=6.9Hz).

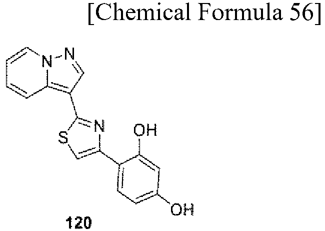

¹H-NMR(CDCl₃) δ:4.85 (1H, s), 6.44 (1H, dd, J=2.8Hz, 8.2Hz), 6.51 (1H, d, J=2.8Hz), 6.96 (1H, t, J=7.3Hz), 7.12 (1H, s), 7.43 (1H, t, J=6.9Hz), 7.54 (1H, d, J=8.7Hz), 8.13 (1H, d, J=9.2Hz), 8.40 (1H, s), 8.55 (1H, d, J=6.9Hz), 12.18(1H, s).

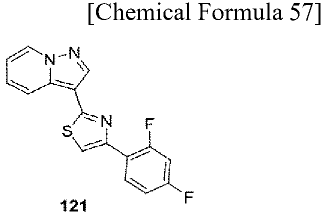

¹H-NMR(CDCl₃) δ:6.93 (2H, m), 7.02 (1H, dt, J=1.8Hz, 7.8Hz), 7.41 (1H, ddd, J=1.4Hz, 6.9Hz, 8.7Hz), 7.60 (1H, d, J=2.3Hz), 8.38 (3H, m), 8.53 (1H, d, J=6.9Hz).

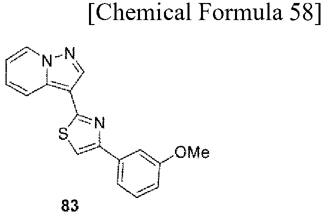

¹H-NMR(CDCl₃) δ:3.90 (3H, s), 6.92 (2H, m), 7.37 (3H, m), 7.58 (2H, m), 8.40 (1H, s), 8.46 (1H, d, J=9.2Hz), 8.52 (1H, d, J=6.9Hz).

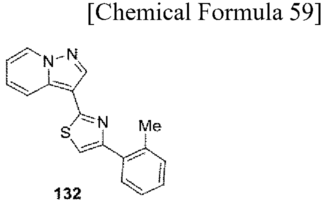

¹H-NMR(CDCl₃) δ:2.55 (3H, s), 6.90 (1H, dt, J=1.4Hz, 6.9Hz), 7.15 (1H, s), 7.29 (3H, m), 7.34 (1H, dd, J=6.0Hz, 6.2Hz), 7.69 (1H, t, J=4.1Hz), 8.39 (2H, m), 8.51 (1H, d, J=7.3Hz).

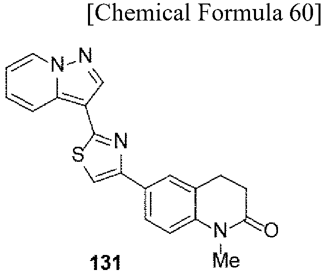

¹H-NMR(CDCl₃) δ:2.71 (2H, t, J=6.9Hz), 3.01 (2H, t, J=7.8Hz), 3.40 (3H, s), 6.93 (1H, dt, J=1.4Hz, 6.9Hz), 7.06 (1H, d, J=8.3Hz), 7.31 (1H, s), 7.39 (1H, ddd, J=0.9Hz, 6.9Hz, 9.2Hz), 7.82 (1H, m), 7.89 (1H, dd, J=1.8Hz, 8.7Hz), 8.41 (1H, s), 8.43 (1H, d, J=9.2Hz), 8.53 (1H, d, J=7.3Hz).

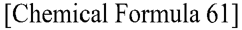

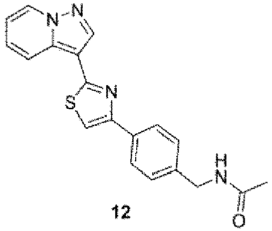

¹H-NMR(CDCl₃) δ:2.05 (3H, s), 4.49 (2H, d, J=6.0Hz), 5.74 (1H, brs), 6.93 (1H, dt, J=1.4Hz, 6.9Hz), 7.36-7.41 (4H, m), 7.98 (2H, d, J=8.2Hz), 8.39 (1H, s), 8.45 (1H, d, J=8.7Hz), 8.52 (1H, d, J=7.3Hz).

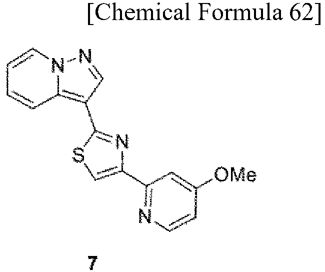

¹H-NMR(CDCl₃) δ:3.97 (3H, s), 6.79 (1H, dd, J=2.3Hz, 5.5Hz), 6.93 (1H, dt, J=1.4Hz, 6.9Hz), 7.39 (1H, ddd, J=0.9Hz, 6.9Hz, 8.7Hz), 7.83 (1H, d, J=2.3Hz), 7.98 (1H, s), 8.41 (2H, m), 8.46 (1H, d, J=5.5Hz), 8.53 (1H, d, J=7.3Hz).

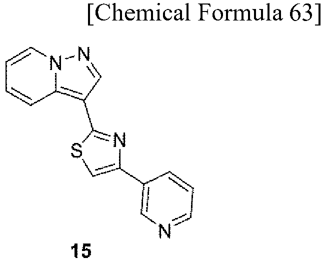

¹H-NMR(CDCl₃) δ:6.94 (1H, dt, J=1.4Hz, 6.9Hz), 7.40 (2H, m), 7.45 (1H, s), 8.28 (1H, dt, J=2.3Hz, 8.2Hz), 8.39 (1H, s), 8.46 (1H, d, J=8.7Hz), 8.52 (1H, d, J=6.0Hz), 8.59 (1H, dd, J=1.4Hz, 5.0Hz), 9.25 (1H, d, J=2.3Hz).

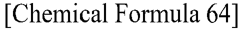

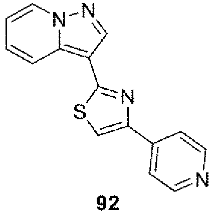

¹H-NMR(CDCl₃) δ:6.96 (1H, ddd, J=1.2Hz, 6.8Hz, 6.8Hz), 7.40-7.47 (1H, m), 7.61 (1H, s), 7.84-7.92 (2H, m), 8.41 (1H, s), 8.46 (1H, d, J=8.8Hz), 8.55 (1H, d, J=6.8Hz), 8.68-8.73 (2H, m).

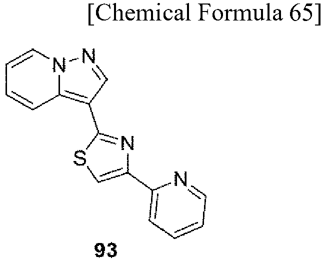

¹H-NMR(CDCl₃) δ:6.94 (1H, ddd, J=1.3Hz, 6.8Hz, 7.0Hz), 7.22-7.30 (1H, m), 7.37-7.44 (1H, m), 7.83 (1H, ddd, J=1.3Hz, 7.8Hz, 7.8Hz), 8.00 (1H, s), 8.26-8.32 (1H, m), 8.43 (1H, s), 8.44-8.49 (1H, m), 8.52-8.57 (1H, m), 8.63-8.67 (1H, m).

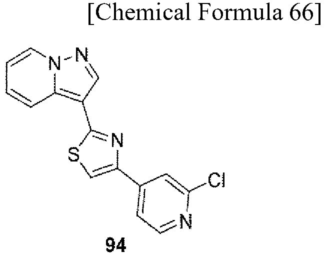

¹H-NMR(CDCl₃) δ:6.98 (1H, ddd, J=1.3Hz, 6.8Hz, 7.0Hz), 7.43-7.50 (1H, m), 7.64 (1H, s), 7.79 (1H, dd, J=1.4Hz, 5.0Hz), 7.95 (1H, s), 8.40-8.46 (2H, m), 8.47 (1H, d, J=5.2Hz), 8.56 (1H, d, J=7.2Hz).

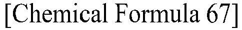

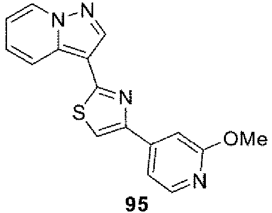

¹H-NMR(CDCl₃) δ:4.02 (3H, s), 6.96 (1H, ddd, J=1.3Hz, 6.8Hz, 7.0Hz), 7.38-7.47 (3H, s), 7.56 (1H, s), 8.25 (1H, d, J=5.6Hz), 8.40 (1H, s), 8.46-8.50 (1H, m), 8.52-8.56 (1H, m).

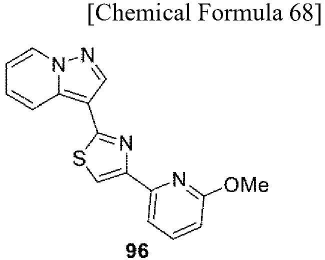

¹H-NMR(CDCl₃) δ:4.04 (3H, s), 6.74 (1H, dd, J=0.8Hz, 2.4Hz), 6.91-6.97 (1H, m), 7.36-7.44 (1H, m), 7.68-7.74 (1H, m), 7.87 (1H, dd, J=0.8Hz, 7.2Hz), 7.97 (1H, s), 8.42 (1H, s), 8.45-8.50 (1H, m), 8.51-8.56 (1H, m).

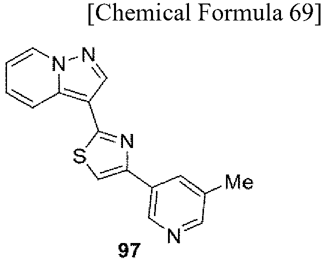

¹H-NMR(CDCl₃) δ:2.45 (3H, s), 6.96 (1H, ddd, J=1.3Hz, 6.8Hz, 7.0Hz), 7.39-7.45 (1H, m), 7.46 (1H, s), 8.11 (1H, s), 8.38-8.49 (3H, m), 8.53-8.57 (1H, m), 9.07 (1H, d, J=2.0Hz).

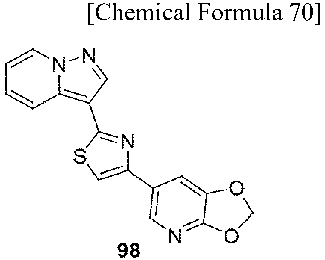

¹H-NMR(CDCl₃) δ:6.14 (2H, s), 6.94 (1H, ddd, J=1.3Hz, 6.8Hz, 7.0Hz), 7.28 (1H, s), 7.37-7.44 (1H, m), 7.64 (1H, d, J=2.0Hz), 8.34 (1H, d, J=2.0 Hz), 8.39 (1H, s), 8.42-8.46 (1H, m), 8.51-8.56 (1H, m).

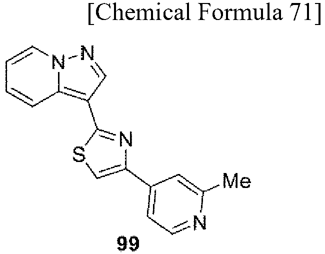

¹H-NMR(CDCl₃) δ:2.66 (3H, s), 6.96 (1H, ddd, J=1.3Hz, 6.8Hz, 7.0Hz), 7.40-7.47 (1H, m), 7.58 (1H, s), 7.66-7.71 (1H, m), 7.76 (1H, s), 8.41 (1H, s), 8.43-8.47 (1H, m), 8.52-8.56 (1H, m), 8.58 (1H, d, J=5.6Hz).

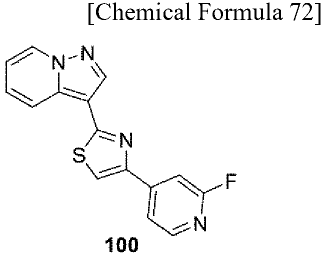

¹H-NMR(CDCl₃) δ:6.94-7.02 (1H, m), 7.41-7.49 (1H, m), 7.57 (1H, s), 7.64 (1H, s), 7.71-7.78 (1H, m), 8.29 (1H, d, J=5.2Hz), 8.41 (1H, s), 8.45 (1H, d, J=9.2Hz), 8.55 (1H, d, J=6.8 Hz).

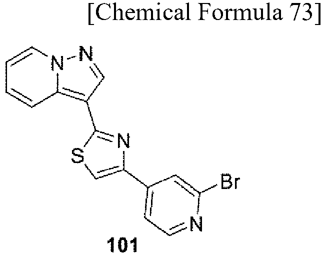

¹H-NMR(CDCl₃) δ:6.98 (1H, ddd, J=1.2Hz, 6.8Hz, 6.8Hz), 7.42-7.50 (1H, m), 7.64 (1H, s), 7.79 (1H, dd, J=1.8Hz, 5.4Hz), 7.95 (1H, s), 8.42 (1H, s), 8.42-8.46 (1H, m), 8.47 (1H, d, J=4.8Hz), 8.54-8.58 (1H, m).

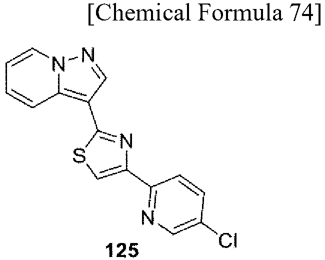

¹H-NMR(CDCl₃) δ:6.92-6.99 (1H, m), 7.38-7.46 (1H, m), 7.79 (1H, dd, J=2.4Hz, 8.4Hz), 7.98 (1H, s), 8.24 (1H, d, J=8.4Hz), 8.30-8.45, (2H, m), 8.53-8.57 (1H, m), 8.59 (1H, d, J=3.2Hz).

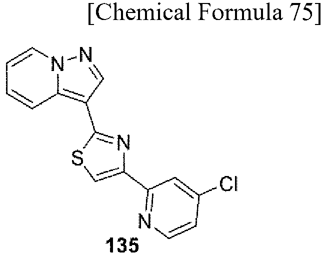

¹H-NMR(CDCl₃) δ:6.96 (1H, ddd, J=1.1Hz, 6.8Hz, 7.0Hz), 7.23-7.29 (1H, m), 7.41-7.48 (1H, m), 8.02 (1H, s), 8.28 (1H, d, J=2.0Hz), 8.40-8.46 (2H, m), 8.51-8.57 (2H, m).

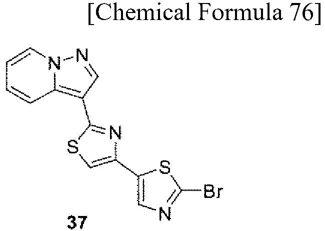

¹H-NMR(CDCl₃) δ:6.95 (1H, t, J=6.9Hz), 7.28 (1H, s), 7.42 (1H, dd, J=0.9Hz, 7.8Hz), 7.93 (1H, s), 8.36 (2H, m), 8.53 (1H, d, J= 6.9Hz).

[Example 27] Synthesis of 4-(2-(pyrazolo[1,5-a]pyridin-3-yl)thiazol-4-yl)aniline hydrochloride (compound 1)

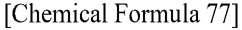

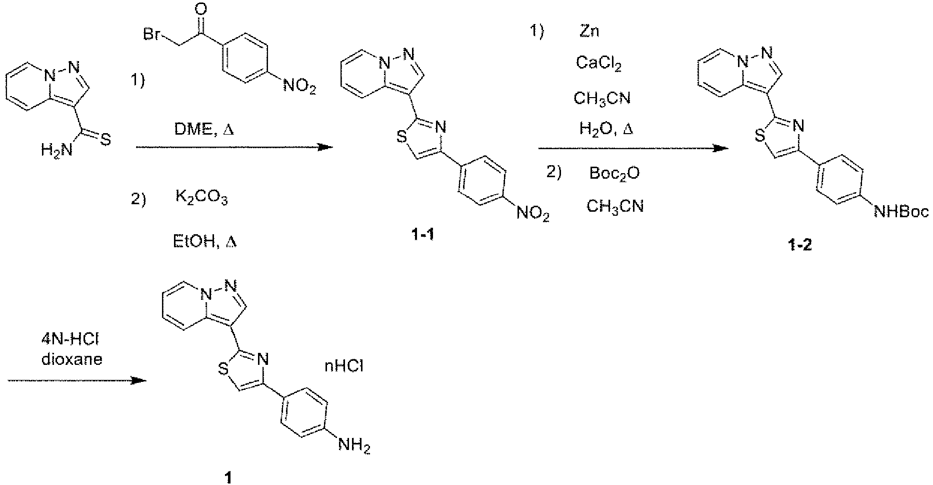

### (1) Synthesis of 4-(4-nitrophenyl)-2-(pyrazolo[1,5-a]pyridin-3-yl)thiazole (compound 1-1)

Pyrazolo[1,5-a]pyridine-3-carbothioamide (887 mg, 5.00 mmol) was dissolved in DME (50 mL), and 4-nitrophenacyl bromide (1.24 g, 5.53 mmol) was added thereto under ice cooling, and the mixture was stirred at room temperature for 16 hours. The reaction mixture was heated and stirred at 50°C for 1 hour and then allowed to cool, and the reaction mixture was concentrated under reduced pressure to remove the solvent. Ethanol (40 mL) was added thereto, then excess potassium carbonate (1.18 g) was added thereto, and the reaction mixture was refluxed at 70°C for 1 hour. After cooling, the reaction mixture was concentrated under reduced pressure to remove the solvent, and the residue was purified by silica gel column chromatography to obtain the title compound (1-1) (1.50 g, 4.64 mmol, 92.8% yield) from a fraction of n-hexane : ethyl acetate (1 : 1).

¹H-NMR(CDCl₃) δ:6.96 (1H, dt, J=1.4Hz, 6.9Hz), 7.44 (1H, ddd, J=0.9Hz, 6.9Hz, 8.7Hz), 7.58 (1H, s),8.18 (2H, d, J=9.2Hz), 8.32 (2H, d, J=9.2Hz), 8.41 (1H, s), 8.44 (1H, d, J=9.2Hz), 8.54 (1H, d, J=6.9Hz).

### (2) Synthesis of tert-butyl (4-(2-(pyrazolo[1,5-a]pyridin-3-yl)thiazol-4-yl)phenyl)carbamate (compound 1-2)

4-(4-Nitrophenyl)-2-(pyrazolo[1,5-a]pyridin-3-yl)thiazole (compound 1-1) (2.62 g, 8.12 mmol) was dissolved in acetonitrile (600 mL), and zinc powder (19.1 g), calcium chloride (3.28 g) and water (8 mL) were added thereto. The reaction mixture was refluxed at 90°C for 1 hour and allowed to cool, then the insoluble matter was filtered through celite, the filtrate was concentrated under reduced pressure to about 50 mL, an excess of di-tert-butyl dicarbonate solution (8 mL) was added thereto under ice cooling, and the mixture was stirred at room temperature for 16 hours. The reaction mixture was concentrated under reduced pressure to remove the solvent, and the residue was purified by silica gel column chromatography to obtain the title compound (1-2) (1.31 g, 3.37 mmol, 41.1% yield) from a fraction of n-hexane : ethyl acetate (1 : 1).

¹H-NMR(CDCl₃) δ:1.53 (9H, s), 6.57 (1H, brs), 6.91 (1H, dd, J=1.4Hz, 6.9Hz), 7.27 (1H, s), 7.37 (1H, dd, J=0.9Hz, 6.9Hz, 8.7Hz), 7.45 (2H, d, J=8.7Hz), 7.93 (2H, d, J=8.7Hz), 8.38 (1H, s), 8.47 (1H, d, J=9.2Hz), 8.51 (1H, d, J=6.9Hz).

### (3) Synthesis of 4-(2-(pyrazolo[1,5-a]pyridin-3-yl)thiazol-4-yl)aniline hydrochloride (compound 1)

Under ice cooling, tert-butyl (4-(2-(pyrazolo[1,5-a]pyridin-3-yl)thiazol-4-yl)phenyl)carbamate (compound 1-2) (1.12 g, 2.84 mmol) was slowly added to 4 N hydrogen chloride in dioxane (15 mL) and the mixture was stirred at room temperature for 2 hours. The reaction mixture was concentrated under reduced pressure to remove the solvent. A solvent was then added, and the resulting solid was collected by filtration, washed well with the solvent, and dried to obtain the title compound (1) (1.04 g, 2.84 mmol, 99.8% yield).

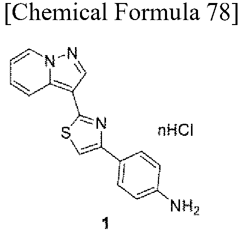

¹H-NMR(D₂O) δ:6.91 (1H, t, J=7.0Hz), 7.27 (2H, d, J=8.4Hz), 7.36 (1H, t, J=7.8Hz), 7.47 (1H, brs), 7.62 (2H, d, J=8.4Hz), 7.77 (1H, d, J=8.9Hz), 8.26 (1H, s), 8.30 (1H, d, J=7.0Hz).

[Examples 28 to 32] Synthesis of the following compounds:
4-(3-nitrophenyl)-2-(pyrazolo[1,5-a]pyridin-3-yl)thiazole (compound 4),
3-(2-(pyrazolo[1,5-a]pyridin-3-yl)thiazol-4-yl)aniline hydrochloride (compound 10),
4-(2-nitrophenyl)-2-(pyrazolo[1,5-a]pyridin-3-yl)thiazole (compound 133),
2-(2-(pyrazolo[1,5-a]pyridin-3-yl)thiazol-4-yl)aniline hydrochloride (compound 134), and
2-methoxy-4-(2-(pyrazolo[1,5-a]pyridin-3-yl)thiazol-4-yl)aniline (compound 90)

Compound (4), compound (10), compound (133), compound (134), and compound (90) were synthesized according to the method for synthesizing compound (1), using a substituted α-bromoacetophenone corresponding to the target compound.

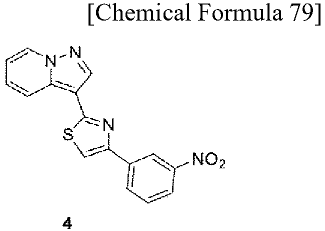

¹H-NMR(CDCl₃) δ:6.96 (1H, t, J=6.9Hz), 7.44 (1H, dd, J=7.5Hz, 8.2Hz), 7.53 (1H, s), 7.63 (1H, t, J=7.8Hz), 8.20 (1H, dt, J=0.9Hz, 7.8Hz), 8.34 (1H, dd, J=0.9Hz, 7.8Hz), 8.42 (1H, s), 8.45 (1H, dt, J=0.9Hz, 8.7Hz), 8.54 (1H, dt, J=1.4Hz, 6.9Hz), 8.84 (1H, t, J=1.8Hz).

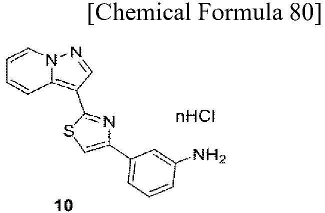

¹H-NMR(D₂O) δ:6.32 (1H, t, J=6.0Hz), 6.75 (3H, m), 6.91 (4H, m), 7.53 (1H, s), 7.56 (1H, d, J=6.9Hz).

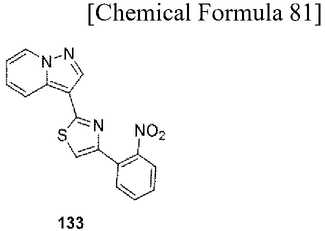

¹H-NMR(CDCl₃) δ:6.92 (1H, m), 7.38 (2H, t, m), 7.50 (1H, t, J=6.0Hz), 7.62 (1H, t, J=6.9Hz), 7.79 (2H, m), 8.33 (1H, s), 8.39 (1H, d, J=9.2Hz), 8.50 (1H, d, J=6.4Hz).

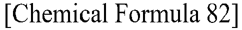

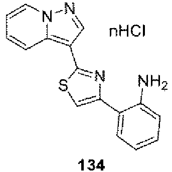

¹H-NMR(D₂O) δ:6.56 (1H, t, J=6.9Hz), 7.02 (2H, m), 7.12 (2H, d, J=6.9Hz), 7.16 (1H, s), 7.20 (2H, t, J=9.2Hz), 7.88 (1H, d, J=6.9Hz), 7.90 (1H, s).

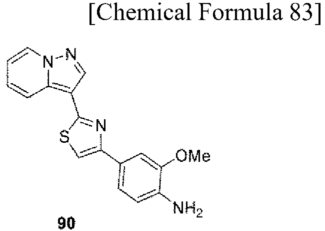

¹H-NMR(CDCl₃) δ:3.67 (3H, s), 6.80 (1H, d, J=7.8Hz), 6.91 (1H, dd, J=1.4Hz, 6.9Hz), 7.17 (1H, s), 7.36 (1H, dd, J=0.9Hz, 6.9Hz), 7.46 (2H, m), 8.40 (1H, s), 8.42 (1H, d, J=8.7Hz), 8.51 (1H, d, J=6.9Hz).

### [Example 33] Synthesis of 2-(2-(pyrazolo[1,5-a]pyridin-3-yl)thiazol-4-yl)phenol (compound 118)

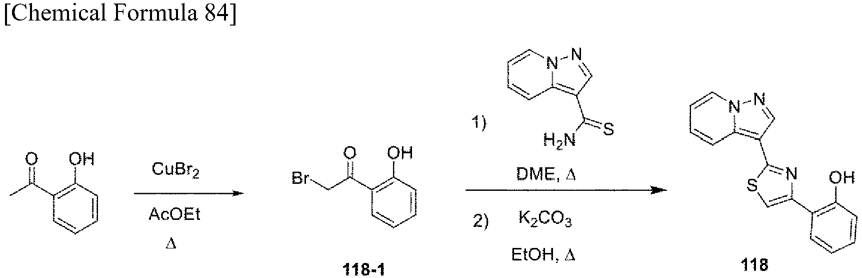

### (1) Synthesis of 2-bromo-1-(2-hydroxyphenyl)ethan-1-one (compound 118-1)

1-(2-Hydroxyphenyl)ethan-1-one (288 mg, 2.11 mmol) was added to ethyl acetate (50 mL), CuBr₂ (929 mg, 4.32 mmol) was added thereto, and the mixture was refluxed for 16 hours. After cooling, the insoluble matter was filtered through celite, the organic solvent was removed under reduced pressure, and the residue was purified by silica gel column chromatography to obtain the title compound (118-1) (438 mg, 2.03 mmol, 79.8% yield) from a fraction of n-hexane : ethyl acetate (1 : 1).

¹H-NMR(CDCl₃) δ:4.44 (2H, s), 6.93 (1H, dt, J=1.4Hz, 8.2Hz), 7.02 (1H, dt, J=1.4Hz, 8.2Hz), 7.52 (1H, dt, J=2.1Hz, 7.3Hz), 7.74 (1H, dd, J=1.8Hz, 7.8Hz), 11.73 (1H, s).

### (2) Synthesis of 2-(2-(pyrazolo[1,5-a]pyridin-3-yl)thiazol-4-yl)phenol (compound 118)

Pyrazolo[1,5-a]pyridine-3-carbothioamide (186 mg, 1.05 mmol) was dissolved in DME (20 mL), and 2-bromo-1-(2-hydroxyphenyl)ethan-1-one (compound 118-1) (218 mg, 1.02 mmol) was added thereto under ice cooling, then the mixture was stirred at room temperature for 16 hours. The reaction mixture was heated and stirred at 50°C for 1 hour and then allowed to cool, and the reaction mixture was concentrated under reduced pressure to remove the solvent. Ethanol (50 mL) was added to the residue, then excess potassium carbonate (301 mg) was added, and the reaction mixture was refluxed at 70°C for 1 hour. After cooling, the reaction mixture was concentrated under reduced pressure to remove the solvent, and the residue was purified by silica gel column chromatography to obtain the title compound (118) (214 mg, 0.729 mmol, 69.4% yield) from a fraction of n-hexane : ethyl acetate (1 : 4).

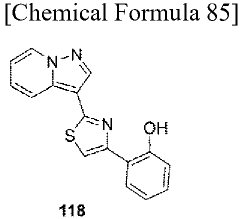

¹H-NMR(CDCl₃) δ:6.93 (2H, m), 7.05 (1H, dd, J=0.9Hz, 8.2Hz), 7.28 (1H, m), 7.43 (2H, m), 7.66 (1H, dd, J=1.8Hz, 7.8Hz), 8.16 (1H, d, J=9.2Hz), 8.42 (1H, s), 8.52 (1H, d, J=6.9Hz), 12.01 (1H, s).

[Examples 34 to 38] Synthesis of the following compounds:
3-(2-(pyrazolo[1,5-a]pyridin-3-yl)thiazol-4-yl)benzonitrile (compound 115),
2-(2-(pyrazolo[1,5-a]pyridin-3-yl)thiazol-4-yl)benzonitrile (compound 116),
4-(3,5-difluorophenyl)-2-(pyrazolo[1,5-a]pyridin-3-yl)thiazole (compound 119),
4-(2,6-difluorophenyl)-2-(pyrazolo[1,5-a]pyridin-3-yl)thiazole (compound 123), and
5-fluoro-2-(2-(pyrazolo[1,5-a]pyridin-3-yl)thiazol-4-yl)phenol (compound 114)

Compound (115), compound (116), compound (119), compound (123), and compound (114) were synthesized according to the method for synthesizing compound (118), using a substituted acetophenone corresponding to the final compound to synthesize a substituted α-bromoacetophenone, which was reacted with a thioamide.

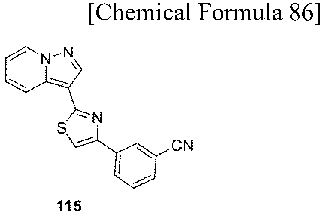

¹H-NMR(CDCl₃) δ:6.96 (1H, t, J=7.3Hz), 7.45 (2H, m), 7.56 (1H, t, J=7.8Hz), 7.63 (1H, d, J=7.8Hz), 8.20 (1H, m), 8.35 (1H, m), 8.40 (1H, s), 8.45 (1H, m), 8.54 (1H, m).

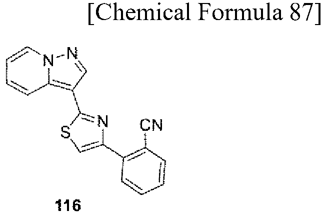

¹H-NMR(CDCl₃) δ:6.94 (1H, dt, J=1.4Hz, 6.9Hz), 7.45 (2H, m), 7.69 (1H, dt, J=1.4Hz, 7.8Hz), 7.79 (1H, dd, J=1.4Hz, 7.8Hz), 7.84 (1H, s), 8.16 (1H, d, J= 7.3Hz), 8.40 (1H, s), 8.52 (1H, d, J=6.9Hz), 8.55 (1H, d, J=9.2Hz).

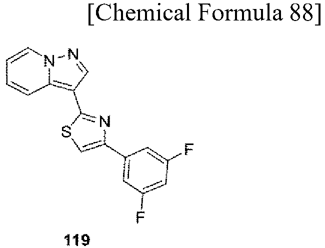

¹H-NMR(CDCl₃) δ:6.79 (1H, tt, J=2.3Hz, 9.2Hz), 6.95 (1H, dt, J=1.4Hz, 6.9Hz), 7.42 (2H, m), 7.54 (2H, d, J=6.4Hz), 8.39 (1H, s), 8.44 (1H, d, J=9.2Hz), 8.53 (1H, d, J=6.9Hz).

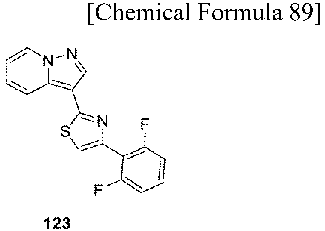

¹H-NMR(CDCl₃) δ:6.91 (1H, dt, J=1.4Hz, 6.9Hz), 7.02 (2H, t, J=8.2Hz), 7.34 (2H, m), 7.46 (1H, t, J=1.4Hz), 8.39 (1H, s), 8.44 (1H, d, J=9.2Hz), 8.51 (1H, d, J=6.9Hz).

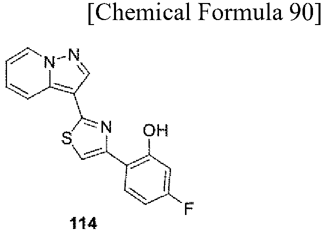

¹H-NMR(CDCl₃) δ:6.64 (1H, t, J=2.8Hz, 8.3Hz), 6.75 (1H, dd, J=2.3Hz, 10.5Hz), 6.97 (1H, dt, J=0.9Hz, 9.2Hz), 7.33 (1H, s), 7.44 (1H, ddd, J=0.9Hz, 6.9Hz, 9.2Hz), 7.61 (1H, dd, J=6.4Hz, 8.7Hz), 8.12 (1H, d, J=8.7Hz), 8.41 (1H, s), 8.56 (1H, d, J=8.7Hz), 12.32 (1H, s).

### [Example 39] Synthesis of 2-(2-(pyrazolo[1,5-a]pyridin-3-yl)thiazol-4-yl)isonicotinamide (compound 126)

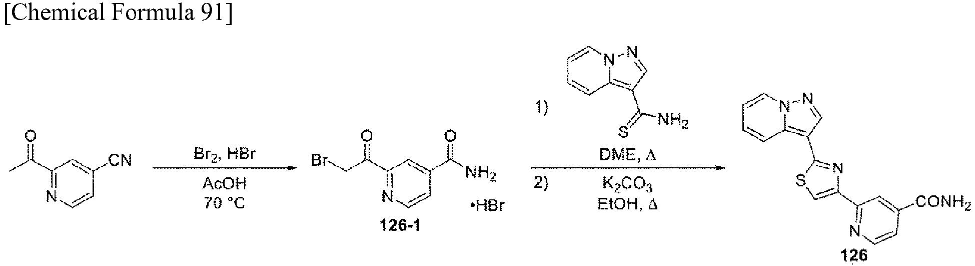

### (1) Synthesis of 2-(2-bromoacetyl)isonicotinamide hydrobromide (compound 126-1)

2-Acetylisonicotinonitrile (51.6 mg, 0.35 mmol) was dissolved in acetic acid (1 mL), and bromine (9 µL, 0.35 mmol) and hydrobromic acid (30% acetic acid solution,
about 5.1 mol/L) (173 µL, 0.88 mmol) were added under ice cooling, and the reaction mixture was heated and stirred at 70°C for 1 hour. After cooling, diethyl ether was added, and the precipitated solid was stirred at room temperature for some time, filtered with suction, then washed with diethyl ether, and dried under reduced pressure to obtain the title compound (126-1) (95.6 mg, 0.30 mmol, 83.6% yield).

¹H-NMR(CD₃OD) δ:3.84 (1H, d, J=11.6Hz), 3.96 (1H, d, J=11.2Hz), 8.37-8.50 (1H, m), 8.59 (1H, d, J=1.6Hz), 8.92-8.99 (1H, m).

### (2) Synthesis of 2-(2-(pyrazolo[1,5-a]pyridin-3-yl)thiazol-4-yl)isonicotinamide (compound 126)

Pyrazolo[1,5-a]pyridine-3-carbothioamide (30.0 mg, 0.17 mmol) was dissolved in DME (2 mL), and 2-(2-bromoacetyl)isonicotinamide hydrobromide (compound 126-1) (82.4 mg, 0.17 mmol) was added thereto under ice cooling, and the mixture was stirred at room temperature for 15 hours. The reaction mixture was heated and stirred at 50°C for 1 hour and then allowed to cool, and the reaction mixture was concentrated under reduced pressure to remove the solvent. Ethanol (10 mL) and potassium carbonate (70.1 mg, 0.51 mmol) were added to the residue, and the mixture was heated at reflux at 80°C for 1 hour. After cooling, the solvent was removed under reduced pressure, and dichloromethane and methanol were added to the residue. The suspended solid was filtered with suction and then dried under reduced pressure to obtain the title compound (126) (7.6 mg, 0.024 mmol, 14.0% yield).

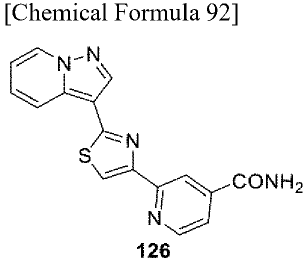

¹H-NMR(DMSO-d₆) δ:7.16 (1H, ddd, J=0.9Hz, 6.8Hz, 7.0Hz), 7.58-7.67 (1H, m), 7.73-7.78 (1H, m), 7.84 (1H, s), 8.27 (1H, s), 8.42-8.52 (2H, m), 8.56 (1H, s), 8.70 (1H, s), 8.78 (1H, d, J=4.4Hz), 8.88 (1H, d, J=6.8Hz).

### [Examples 40 and 41] Synthesis of 4-(5-methoxypyridin-3-yl)-2-(pyrazolo[1,5-a]pyridin-3-yl)thiazole (compound 124) and 4-(5-methoxypyridin-2-yl)-2-(pyrazolo[1,5-a]pyridin-3-yl)thiazole (compound 127)

Compound (124) and compound (127) were synthesized according to the method for synthesizing compound (126).

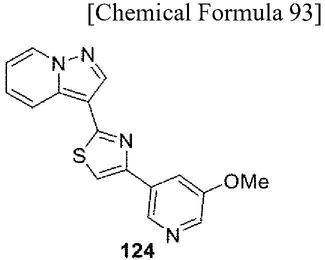

¹H-NMR(CDCl₃) δ:3.98 (3H, s), 6.93-6.99 (1H, m), 7.39-7.45 (1H, m), 7.48 (1H, s), 7.83-7.86 (1H, m), 8.31 (1H, d, J=4.2Hz), 8.42 (1H, s), 8.43-8.48 (1H, m), 8.55 (1H, d, J=6.0Hz), 8.86 (1H, d, J=1.6Hz).

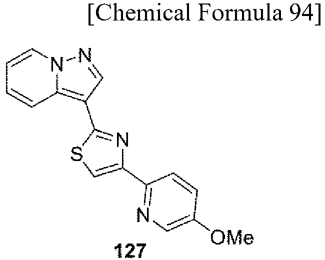

¹H-NMR(CDCl₃) δ:3.92 (3H, s), 6.93 (1H, ddd, J=1.3Hz, 6.8Hz, 7.0Hz), 7.32 (1H, dd, J=3.2Hz, 8.8Hz), 7.36-7.42 (1H, m), 7.82 (1H, s), 8.22 (1H, d, J=8.4Hz), 8.35 (1H, d, J=2.0Hz), 8.40-8.47 (2H, m), 8.51-8.55 (1H, m).

### [Example 42] Synthesis of 4-(1H-pyrazol-4-yl)-2-(pyrazolo[1,5-a]pyridin-3-yl)thiazole (compound 107)

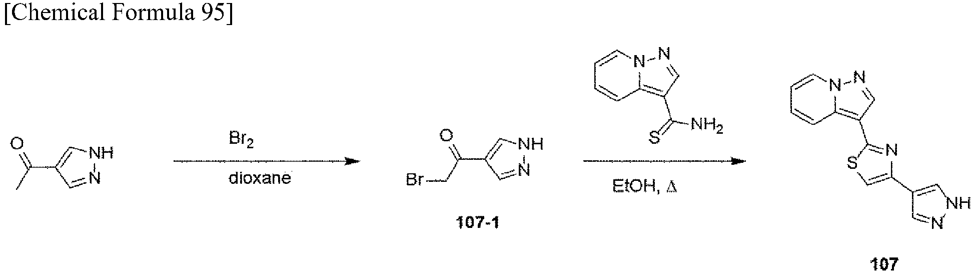

### (1) Synthesis of 2-bromo-1-(1H-pyrazol-4-yl)ethan-1-one (compound 107-1)

Bromine-dioxane complex (1.25 g, 5.00 mmol) was added to 1-(1H-pyrazol-4-yl)ethan-1-one (0.55 g, 5.00 mmol) in dioxane (10 mL), and the mixture was stirred at room temperature for 56 hours. The precipitate was filtered with suction, washed with acetone, and then dried to obtain the title compound (107-1) (1.02 g, 5.00 mmol, 100% yield).

### ¹H-NMR(DMSO-d₆) δ:2.50 (2H, s), 8.22 (2H, s).

### (2) Synthesis of 4-(1H-pyrazol-4-yl)-2-(pyrazolo[1,5-a]pyridin-3-yl)thiazole (compound 107)

A solution of pyrazolo[1,5-a]pyridine-3-carbothioamide (5.40 g, 30.5 mmol) and 2-bromo-1-(1H-pyrazol-4-yl)ethan-1-one (compound 107-1) (5.76 g, 30.5 mmol) in ethanol (200 mL) was stirred at 70°C for 3 hours. The mixture was allowed to cool, diluted with dichloromethane and water, and adjusted to pH 9 by adding a saturated aqueous sodium bicarbonate solution. The mixture was then extracted with dichloromethane. The organic layer was dried over anhydrous sodium sulfate and then filtered, and the solvent was removed under reduced pressure. The residue was suspended in ethyl acetate, then collected by filtration and dried to obtain the title compound (107) (5.00 g, 18.7 mmol, 61.3% yield).

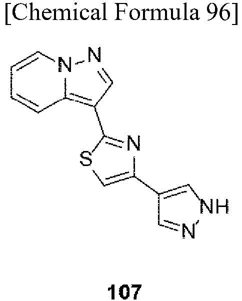

¹H-NMR(DMSO-d₆) δ:7.09-7.13 (1H, m), 7.52-7.56 (1H, m), 7.58 (1H, s), 8.14 (2H, s), 8.43-8.46 (1H, m), 8.59 (1H, s),8.83-8.85 (1H, m).

[Examples 43 to 74] Synthesis of the following compounds:
4-(4-chloro-1-methyl-1H-pyrazol-3-yl)-2-(pyrazolo[1,5-a]pyridin-3-yl)thiazole (compound 53),
4-(1,4-dimethyl-1H-pyrazol-3-yl)-2-(pyrazolo[1,5-a]pyridin-3-yl)thiazole hydrobromide (compound 54),
4-(1-methyl-1H-pyrazol-5-yl)-2-(pyrazolo[1,5-a]pyridin-3-yl)thiazole hydrobromide (compound 106),
4-(1H-pyrazol-3-yl)-2-(pyrazolo[1,5-a]pyridin-3-yl)thiazole (compound 109),
5-methyl-3-(2-(pyrazolo[1,5-a]pyridin-3-yl)thiazol-4-yl)isoxazole hydrobromide (compound 105),
2-(pyrazolo[1,5-a]pyridin-3-yl)-4-(1H-pyrrol-3-yl)thiazole (compound 138),
2-(pyrazolo[1,5-a]pyridin-3-yl)-4-(1H-pyrrol-2-yl)thiazole (compound 139),
4-(1H-imidazol-4-yl)-2-(pyrazolo[1,5-a]pyridin-3-yl)thiazole (compound 140),
4-(1H-imidazol-2-yl)-2-(pyrazolo[1,5-a]pyridin-3-yl)thiazole (compound 141),
2-(pyrazolo[1,5-a]pyridin-3-yl)-4-(1H-1,2,3-triazol-4-yl)thiazole (compound 142),
2-(pyrazolo[1,5-a]pyridin-3-yl)-4-(1H-1,2,4-triazol-3-yl)thiazole (compound 143),
2-(pyrazolo[1,5-a]pyridin-3-yl)-4-(thiophen-3-yl)thiazole (compound 144),
2-(pyrazolo[1,5-a]pyridin-3-yl)-4-(thiophen-2-yl)thiazole (compound 145),
3-(2-(pyrazolo[1,5-a]pyridin-3-yl)thiazol-4-yl)isothiazole (compound 146),
4-(2-(pyrazolo[1,5-a]pyridin-3-yl)thiazol-4-yl)isothiazole (compound 147),
5-(2-(pyrazolo[1,5-a]pyridin-3-yl)thiazol-4-yl)isothiazole (compound 148),
2-(pyrazolo[1,5-a]pyridin-3-yl)-4,4'-bithiazole (compound 149),
2-(pyrazolo[1,5-a]pyridin-3-yl)-4,5'-bithiazole (compound 150),
2'-(pyrazolo[1,5-a]pyridin-3-yl)-2,4'-bithiazole (compound 151),
4-(furan-3-yl)-2-(pyrazolo[1,5-a]pyridin-3-yl)thiazole (compound 152),
4-(furan-2-yl)-2-(pyrazolo[1,5-a]pyridin-3-yl)thiazole (compound 153),
4-(2-(pyrazolo[1,5-a]pyridin-3-yl)thiazol-4-yl)isoxazole (compound 154),
3-(2-(pyrazolo[1,5-a]pyridin-3-yl)thiazol-4-yl)isoxazole (compound 155),
5-(2-(pyrazolo[1,5-a]pyridin-3-yl)thiazol-4-yl)isoxazole (compound 156),
4-(2-(pyrazolo[1,5-a]pyridin-3-yl)thiazol-4-yl)oxazole (compound 157),
5-(2-(pyrazolo[1,5-a]pyridin-3-yl)thiazol-4-yl)oxazole (compound 158),
2-(2-(pyrazolo[1,5-a]pyridin-3-yl)thiazol-4-yl)oxazole (compound 159),
4-(1-methyl-1H-pyrazol-3-yl)-2-(pyrazolo[1,5-a]pyridin-3-yl)thiazole (compound 160),
4-(1-methyl-1H-pyrrol-3-yl)-2-(pyrazolo[1,5-a]pyridin-3-yl)thiazole (compound 161),
3-(2-(pyrazolo[1,5-a]pyridin-3-yl)thiazol-4-yl)-1,2,4-thiadiazole (compound 162),
4-(1-methyl-1H-pyrrol-2-yl)-2-(pyrazolo[1,5-a]pyridin-3-yl)thiazole (compound 163), and
3-bromo-4-(2-(pyrazolo[1,5-a]pyridin-3-yl)thiazol-4-yl)isoxazole (compound 164)

Compound (53), compound (54), compound (106), compound (109), compound (105), compound (138), compound (139), compound (140), compound (141), compound (142), compound (143), compound (144), compound (145), compound (146), compound (147), compound (148), compound (149), compound (150), compound (151), compound (152), compound (153), compound (154), compound (155), compound (156), compound (157), compound (158), compound (159), compound (160), compound (161), compound (162), compound (163), and compound (164) were synthesized according to the method for synthesizing compound (107), using a substituted α-haloketone corresponding to the final compound, in place of 2-bromo-1-(1H-pyrazol-4-yl)ethan-1-one.

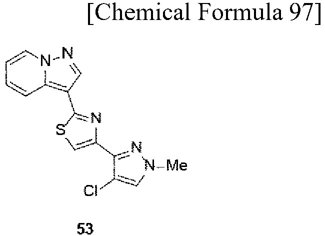

¹H-NMR(DMSO-d₆) δ:3.91 (3H, s), 7.09-7.13 (1H, m), 7.55-7.59 (1H, m), 7.83 (1H, s), 8.08 (1H, s), 8.43-8.46 (1H, m), 8.63 (1H, s), 8.83-8.85 (1H, m).

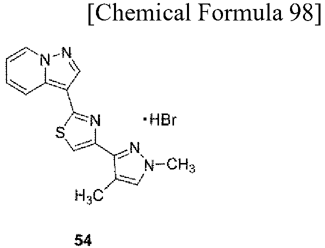

¹H-NMR(DMSO-d₆) δ:2.38 (3H, s), 3.84 (3H, s), 7.06-7.14 (1H, m), 7.52-7.60 (2H, m), 7.64 (1H, s), 8.30-8.37 (1H, m), 8.61 (1H, s), 8.81-8.86 (1H, m).

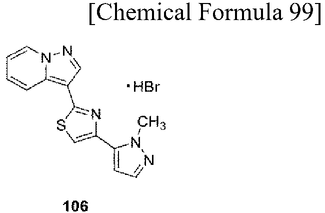

¹H-NMR(DMSO-d₆) δ:4.20 (3H, s), 6.74-6.78 (1H, m), 7.10-7.18 (1H, m), 7.48-7.53 (1H, m), 7.55-7.61 (1H, m), 7.94 (1H, s), 8.33 (1H, d, J=9.2Hz), 8.68 (1H, s), 8.87 (1H, d, J=6.8Hz).

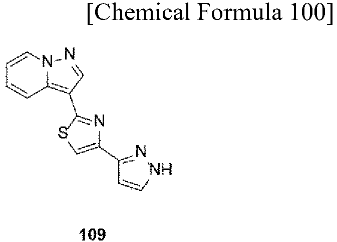

¹H-NMR(CDCl₃) δ:6.72-6.77 (1H, m), 6.94 (1H, ddd, J=1.3Hz, 6.9Hz, 6.9Hz), 7.39 (1H, ddd, J=1.2Hz, 6.8Hz, 8.9Hz), 7.43 (1H, s), 7.66 (1H, d, J=2.1Hz), 8.38 (1H, ddd, J=1.2Hz, 1.2Hz, 8.9Hz), 8.42 (1H, s), 8.54 (1H, ddd, J=1.0Hz, 1.0Hz, 7.0Hz).

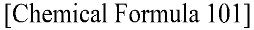

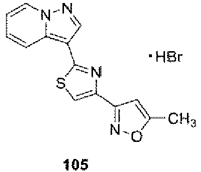

¹H-NMR(DMSO-d₆) δ:2.51 (3H, s), 6.82 (1H, s), 7.12-7.17 (1H, m), 7.55-7.62 (1H, m), 8.14 (1H, s), 8.38-8.44 (1H, m), 8.67 (1H, s), 8.85-8.90 (1H, m).

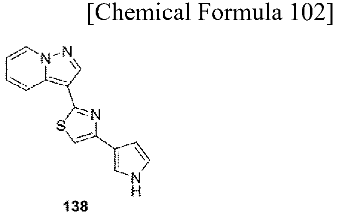

¹H-NMR(CDCl₃) δ:6.63-6.67 (1H, m), 6.83-6.87 (1H, m), 6.90 (1H, ddd, J=1.3Hz, 6.8Hz, 7.0Hz), 7.03 (1H, s), 7.32-7.37 (1H, m), 7.39-7.42 (1H, m), 8.32 (1H, brs), 8.40 (1H, s), 8.41-8.45 (1H, m), 8.49-8.53 (1H, m).

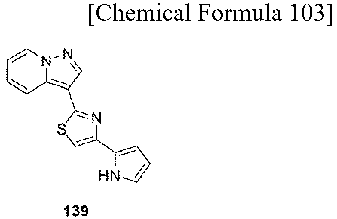

¹H-NMR(DMSO-d₆) δ:6.14 (1H, dd, J=2.4Hz, 6.0Hz), 6.58-6.64 (1H, m), 6.84-6.89 (1H, m), 7.11 (1H, ddd, J=1.2Hz, 6.8Hz, 6.8Hz), 7.48 (1H, s), 7.51-7.58 (1H, m), 8.54-8.62 (2H, m), 8.83 (1H, d, J=6.8Hz), 11.35 (1H, brs).

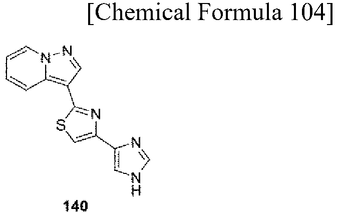

¹H-NMR(DMSO-d₆) δ:7.08-7.14 (1H, m), 7.50-7.58 (2H, m), 7.62 (1H, brs), 7.73 (1H, s), 8.42-8.50 (1H, m), 8.59 (1H, s), 8.82-8.85 (1H, m), 12.26 (1H, brs).

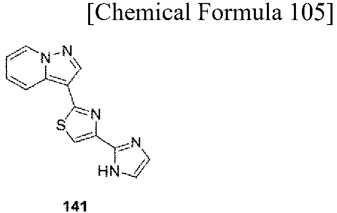

¹H-NMR(DMSO-d₆) δ:7.18 (1H, ddd, J=1.4Hz, 6.9Hz, 6.9Hz), 7.64 (1H, ddd, J=1.0Hz, 6.9Hz, 8.9Hz), 7.87 (2H, s), 8.43-8.44 (1H, m), 8.73-8.77 (1H, m), 8.75 (1H, s), 8.89 (1H, ddd, J=1.0Hz, 1.0Hz, 6.9Hz).

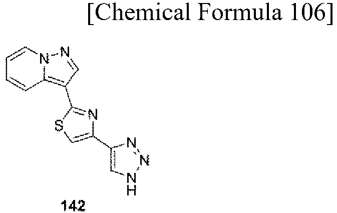

¹H-NMR(DMSO-d₆) δ:7.13 (1H, ddd, J=1.3Hz, 6.8Hz, 7.0Hz), 7.52-7.60 (1H, m), 7.91 (1H, s), 8.30-8.42 (1H, m), 8.45-8.53 (1H, m), 8.64 (1H, s), 8.82-8.88 (1H, m).

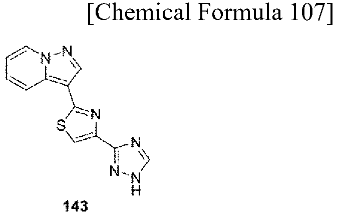

¹H-NMR(CD₃OD) δ:7.09 (1H, ddd, J=1.3Hz, 6.6Hz, 7.2Hz), 7.50-7.57 (1H, m), 8.00 (1H, s), 8.30 (1H, s), 8.54 (1H, s), 8.61-8.68 (2H, m).

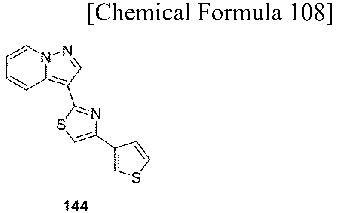

¹H-NMR(DMSO-d₆) δ:7.13 (1H, ddd, J=1.4 Hz, 6.9 Hz, 6.9 Hz), 7.56 (1H, ddd, J=0.9 Hz, 6.9 Hz, 8.8 Hz), 7.66 (1H, dd, J=3.0 Hz, 5.0 Hz), 7.71 (1H, dd, J=1.3 Hz, 5.0 Hz), 7.85 (1H, s), 8.08 (1H, dd, J=1.3 Hz, 3.0 Hz), 8.45-8.49 (1H, m), 8.62 (1H, s), 8.83-8.87 (1H, m).

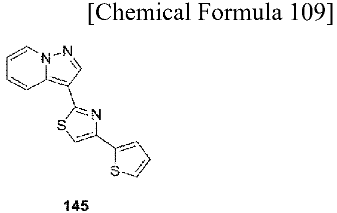

¹H-NMR(DMSO-d₆) δ:7.11-7.18 (2H, m), 7.57 (1H, dd, J=1.2Hz, 5.0Hz), 7.60 (1H, ddd, J=1.2Hz, 6.9Hz, 8.9Hz), 7.66 (1H, dd, J=1.2Hz, 3.6Hz), 7.86 (1H, s), 8.38 (1H, ddd, J=1.2Hz, 1.2Hz, 8.8Hz), 8.63 (1H, s), 8.85-8.88 (1H, m).

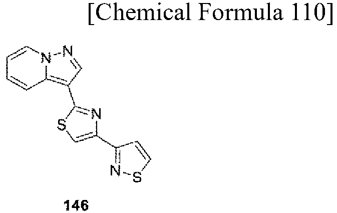

¹H-NMR(DMSO-d₆) δ:7.14 (1H, ddd, J=1.3Hz, 6.9Hz, 6.9Hz), 7.59 (1H, ddd, J=1.1Hz, 6.8Hz, 8.9Hz), 8.03 (1H, d, J=4.6Hz), 8.15 (1H, s), 8.46 (1H, ddd, J=1.2Hz, 1.2Hz, 8.8Hz), 8.67 (1H, s), 8.86 (1H, ddd, J=1.0Hz, 1.0Hz, 7.0Hz), 9.21 (1H, d, J=4.6Hz).

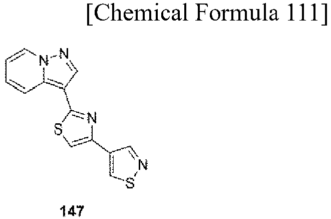

¹H-NMR(DMSO-d₆) δ:7.13 (1H, ddd, J=1.4Hz, 6.9Hz, 6.9Hz), 7.57 (1H, ddd, J=1.0Hz, 6.8Hz, 8.9Hz), 8.04 (1H, s), 8.49 (1H, ddd, J=1.2Hz, 1.2Hz, 8.8Hz), 8.65 (1H, s), 8.86 (1H, ddd, J=1.0Hz, 1.0Hz, 7.0Hz), 9.20 (1H, s), 9.50 (1H, s).

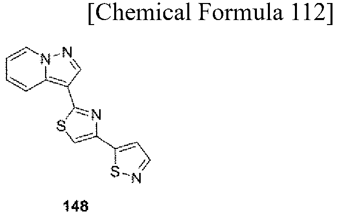

¹H-NMR(DMSO-d₆) δ:7.16 (1H, ddd, J=1.3Hz, 6.9Hz, 6.9Hz), 7.63 (1H, ddd, J=0.9Hz, 6.9Hz, 8.8Hz), 7.93 (1H, d, J=1.7Hz), 8.24 (1H, s), 8.32-8.36 (1H, m), 8.61 (1H, d, J=1.7Hz), 8.68 (1H, s), 8.86-8.90 (1H, m).

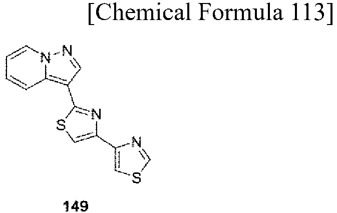

¹H-NMR(CDCl₃) δ:6.93 (1H, ddd, J=1.4Hz, 6.9Hz, 6.9Hz), 7.39 (1H, ddd, J=1.1Hz, 6.8Hz, 8.9Hz), 7.74 (1H, s), 7.98 (1H, d, J=2.1Hz), 8.42 (1H, ddd, J=1.2Hz, 1.2Hz, 8.9Hz), 8.42 (1H, s), 8.53 (1H, ddd, J=1.0Hz, 1.0Hz, 7.0Hz), 8.87 (1H, d, J=2.1Hz).

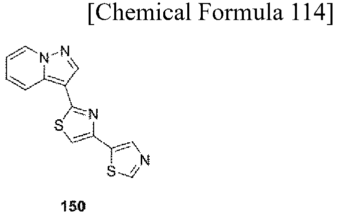

¹H-NMR(CDCl₃) δ:6.95 (1H, ddd, J=1.4Hz, 6.9Hz, 6.9Hz), 7.32 (1H, s), 7.42 (1H, ddd, J=1.0Hz, 6.8Hz, 8.9Hz), 8.32 (1H, d, J=0.5Hz), 8.39 (1H, s), 8.42 (1H, ddd, J=1.2Hz, 1.2Hz, 8.8Hz), 8.54 (1H, ddd, J=1.0Hz, 1.0Hz, 7.0Hz), 8.78 (1H, d, J=0.5Hz).

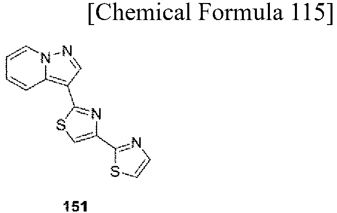

¹H-NMR(DMSO-d₆) δ:7.12-7.18 (1H, m), 7.61-7.66 (1H, m), 7.82-7.84 (1H, m), 7.94-9.96 (1H, m), 8.15 (1H, s), 8.35-8.39 (1H, m), 8.69 (1H, s), 8.87-8.90 (1H, m).

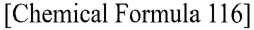

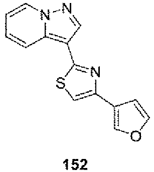

¹H-NMR(DMSO-d₆) δ:7.03 (1H, dd, J=0.8Hz, 1.8Hz), 7.12 (1H, ddd, J=1.3Hz, 6.9Hz, 6.9Hz), 7.55 (1H, ddd, J=1.1Hz, 6.8Hz, 8.9Hz), 7.71 (1H, s), 7.77 (1H, dd, J=1.7Hz, 1.7Hz), 8.27-8.29 (1H, m), 8.44 (1H, ddd, J=1.2Hz, 1.2Hz, 8.8Hz), 8.60 (1H, s), 8.84 (1H, ddd, J=1.0Hz, 1.0Hz, 7.0Hz).

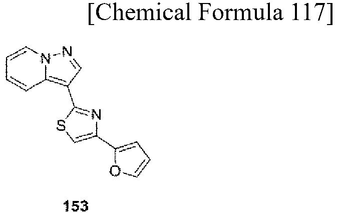

¹H-NMR(DMSO-d₆) δ:6.65 (1H, dd, J=1.8Hz, 3.3Hz), 6.98 (1H, dd, J=0.6Hz, 3.3Hz), 7.13 (1H, ddd, J=1.4Hz, 6.9Hz, 6.9Hz), 7.57 (1H, ddd, J=1.0Hz, 6.8Hz, 8.9Hz), 7.71 (1H, s), 7.79 (1H, dd, J=0.7Hz, 1.8Hz), 8.40 (1H, ddd, J=1.2Hz, 1.2Hz, 8.8Hz), 8.63 (1H, s), 8.85 (1H, ddd, J=1.0Hz, 1.0Hz, 7.0Hz).

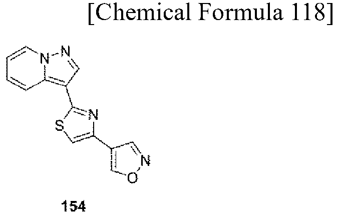

¹H-NMR(DMSO-d₆) δ:7.13 (1H, ddd, J=1.5Hz, 6.8Hz, 7.0Hz), 7.52-7.59 (1H, m), 7.87 (1H, s), 8.46-8.52 (1H, m), 8.63 (1H, s), 8.82-8.87 (1H, m), 9.22 (1H, s), 9.54 (1H, s).

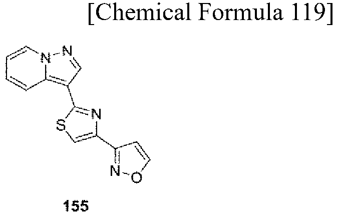

¹H-NMR(DMSO-d₆) δ:7.14 (1H, ddd, J=1.3Hz, 6.8Hz, 7.0Hz), 7.18 (1H, d, J=1.6Hz), 7.55-7.62 (1H, m), 8.21 (1H, s), 8.39-8.45 (1H, m), 8.68 (1H, s), 8.84-8.89 (1H, m), 9.07 (1H, d, J=1.6Hz).

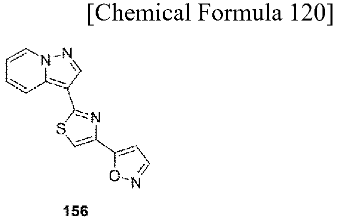

¹H-NMR(DMSO-d₆) δ:7.04 (1H, d, J=2.0Hz), 7.15 (1H, ddd, J=1.3Hz, 6.8Hz, 7.0Hz), 7.56-7.63 (1H, m), 8.23 (1H, s), 8.40-8.46 (1H, m), 8.69 (1H, s), 8.73 (1H, d, J=2.0Hz), 8.87 (1H, d, J=7.2Hz).

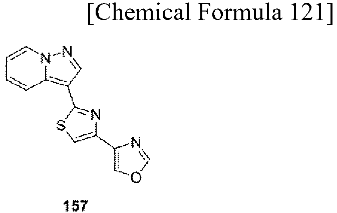

¹H-NMR(DMSO-d₆) δ:7.13 (1H, ddd, J=1.3Hz, 6.9Hz, 6.9Hz), 7.55 (1H, ddd, J=1.1Hz, 6.8Hz, 8.9Hz), 7.78 (1H, d, J=0.4Hz), 8.46 (1H, ddd, J=1.2Hz, 1.2Hz, 8.8Hz), 8.51 (1H, d, J=1.0Hz), 8.63 (1H, s), 8.66 (1H, dd, J=0.4Hz, 1.0Hz), 8.85 (1H, ddd, J=1.0Hz, 1.0Hz, 7.0Hz).

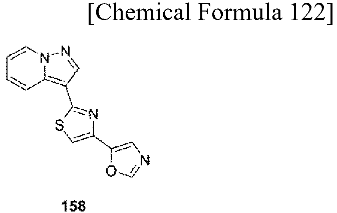

¹H-NMR(DMSO-d₆) δ:7.14 (1H, ddd, J=1.4Hz, 6.9Hz, 6.9Hz), 7.58 (1H, ddd, J=1.0Hz, 6.8Hz, 8.9Hz), 7.70 (1H, s), 7.89 (1H, s), 8.42 (1H, ddd, J=1.2Hz, 1.2Hz, 8.9Hz), 8.50 (1H, s), 8.66 (1H, s), 8.86 (1H, ddd, J=1.0Hz, 1.0Hz, 7.0Hz).

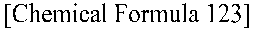

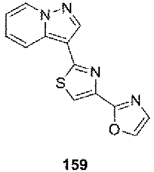

¹H-NMR(DMSO-d₆) δ:7.15 (1H, ddd, J=1.4Hz, 6.9Hz, 6.9Hz), 7.42-7.43 (1H, m), 7.62 (1H, ddd, J=0.9Hz, 6.9Hz, 8.9Hz), 8.25-8.27 (2H, m), 8.37 (1H, ddd, J=1.2Hz, 1.2Hz, 8.9Hz), 8.69 (1H, s), 8.88 (1H, ddd, J=1.0Hz, 1.0Hz, 7.0Hz).

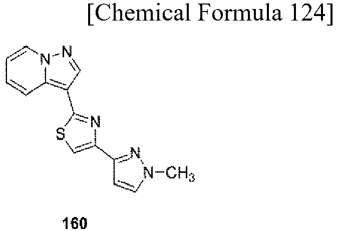

¹H-NMR(DMSO-d₆) δ:3.92 (3H, s), 6.76 (1H, d, J=2.2Hz), 7.12 (1H, ddd, J=1.4Hz, 6.9Hz, 6.9Hz), 7.56 (1H, ddd, J=1.0Hz, 6.8Hz, 8.9Hz), 7.70 (1H, s), 7.78 (1H, d, J=2.2Hz), 8.40 (1H, ddd, J=1.2Hz, 1.2Hz, 8.9Hz), 8.61 (1H, s), 8.84 (1H, ddd, J=1.0Hz, 1.0Hz, 6.9Hz).

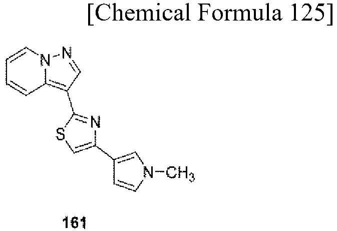

¹H-NMR(CDCl₃) δ:3.72 (3H, s), 6.52-6.55 (1H, m), 6.62-6.65 (1H, m), 6.90 (1H, ddd, J=1.3Hz, 6.8Hz, 7.0Hz), 6.97 (1H, s), 7.19-7.22 (1H, m), 7.32-7.38 (1H, m), 8.39 (1H, s), 8.40-8.44 (1H, m), 8.49-8.53 (1H, m).

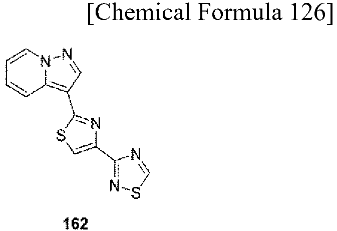

¹H-NMR(DMSO-d₆) δ:7.15 (1H, ddd, J=1.3Hz, 6.8Hz, 7.0Hz), 7.58-7.65 (1H, m), 8.38 (1H, s), 8.40-8.45 (1H, m), 8.69 (1H, s), 8.85-8.90 (1H, m), 10.37 (1H, s).

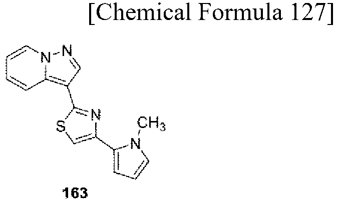

¹H-NMR(CDCl₃) δ:3.98 (3H, s), 6.18-6.21 (1H, m), 6.55 (1H, dd, J=1.8Hz, 3.8Hz), 6.71-6.74 (1H, m), 6.91 (1H, ddd, J=1.3Hz, 6.8Hz, 7.0Hz), 7.10 (1H, s), 7.32-7.38 (1H, m), 8.34-8.38 (1H, m), 8.39 (1H, s), 8.50-8.54 (1H, m).

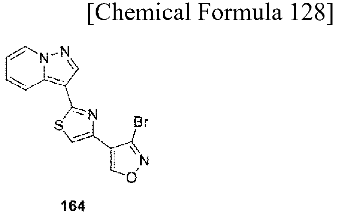

¹H-NMR(CDCl₃) δ:6.96 (1H, ddd, J=1.3Hz, 6.8Hz, 7.0Hz), 7.38-7.44 (1H, m), 7.72 (1H, s), 8.33-8.38 (1H, m), 8.40 (1H, s), 8.52-8.57 (1H, m), 8.96 (1H, s).

### [Example 75] Synthesis of N-methyl-N-(4-(2-(pyrazolo[1,5-a]pyridin-3-yl)thiazol-4-yl)phenyl)oxetane-3-sulfonamide (compound 69)

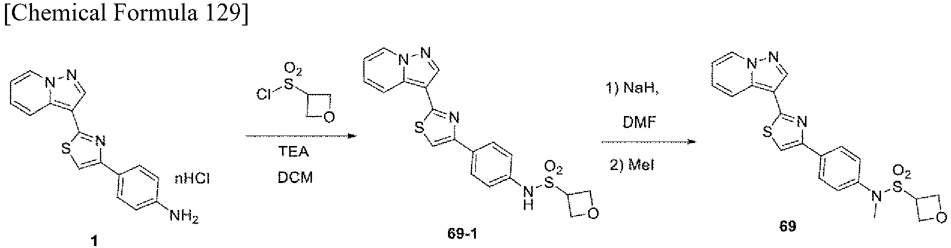

### (1) Synthesis of N-(4-(2-(pyrazolo[1,5-a]pyridin-3-yl)thiazol-4-yl)phenyl)oxetane-3-sulfonamide (compound 69-1)

4-(2-(pyrazolo[1,5-a]pyridin-3-yl)thiazol-4-yl)aniline hydrochloride (compound 1) (50.5 mg, 0.120 mmol) was suspended in an anhydrous dichloromethane solution (10 mL), and under ice cooling, excessive oxetane-3-sulfonyl chloride (0.10 mL) was added, and then excessive triethylamine (0.20 mL) was slowly added. The mixture was gradually allowed to return to room temperature and stirred for 16 hours. Water was added to the reaction mixture under ice cooling, the dichloromethane layer was washed with water several times, the dichloromethane layer was dried over anhydrous magnesium sulfate, and after filtration, the solvent was removed under reduced pressure, and the residue was purified by silica gel column chromatography to obtain the title compound (69-1) (48.5 mg, 0.118 mmol, 98.0% yield) from a fraction of n-hexane : ethyl acetate (1 : 1).

¹H-NMR(CDCl₃) δ:4.94 (4H, m), 5.11 (1H, m), 6.95 (1H, t, J=6.9Hz), 7.31 (2H, d, J=8.2Hz), 7.41 (1H, t, J=8.2Hz), 7.46 (1H, s), 8.09 (2H, d, J=8.7Hz), 8.40 (1H, s), 8.43 (1H, d, J=9.2Hz), 8.53 (1H, d, J=6.9Hz).

### (2) Synthesis of N-methyl-N-(4-(2-(pyrazolo[1,5-a]pyridin-3-yl)thiazol-4-yl)phenyl)oxetane-3-sulfonamide (compound 69)

Sodium hydride (oil dispersion) (21.5 mg) was carefully added to anhydrous DMF (4 mL) to form a suspension solution, and after cooling to -15°C, N-(4-(2-(pyrazolo[1,5-a]pyridin-3-yl)thiazol-4-yl)phenyl)oxetane-3-sulfonamide (compound 69-1) (25.5 mg, 0.0521 mmol) was slowly added. The mixture was once heated to room temperature, then cooled to -15°C, and excess methyl iodide (0.10 mL) was added dropwise. The mixture was stirred for 16 hours while gradually returning to room temperature. The reaction mixture was ice-cooled, and water (about 20 mL) was carefully and gradually added thereto. The resulting precipitate was stirred at room temperature for some time and collected by filtration, then washed thoroughly with n-hexane or a similar solvent and dried, and then the title compound (69) (6.6 mg, 0.0155 mmol, 28.8% yield) was obtained.

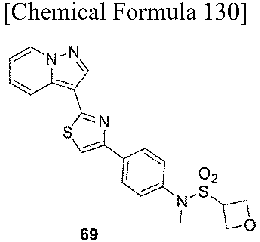

¹H-NMR(CDCl₃) δ:3.37 (3H, s), 4.54 (1H, m), 4.76 (2H, t, J=8.2Hz), 4.86 (2H, t, J=6.9Hz), 6.94 (1H, t, J=5.5Hz), 7.42 (4H, m), 8.02 (2H, d, J=8.2Hz), 8.40 (1H, s), 8.44 (1H, d, J=8.7Hz), 8.53 (1H, d, J=7.3Hz).

### [Example 76] Synthesis of N-methyl-N-(4-(2-(pyrazolo[1,5-a]pyridin-3-yl)thiazol-4-yl)benzyl)acetamide (compound 16)

Compound (16) was synthesized from compound (12), according to the method for synthesizing compound (69) from compound (69-1).

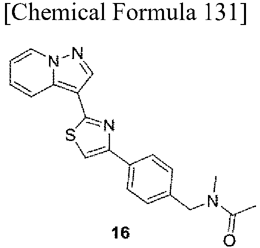

¹H-NMR(CDCl₃) δ:2.18+2.19 (3H, s x2), 2.94+2.97 (3H, s x2), 4.57+4.63 (2H, s x2), 6.93 (1H, m), 7.36 (4H, m), 7.96+8.01 (2H, d x2, J= 6.9Hz), 8.39+8.40 (1H, s x2), 8.46 (1H, d, J=8.7Hz), 8.52 (1H, d, J=6.9Hz).

### [Example 77] Synthesis of N-(4-(2-(pyrazolo[1,5-a]pyridin-3-yl)thiazol-4-yl)benzyl)morpholine-4-carboxamide (compound 72)

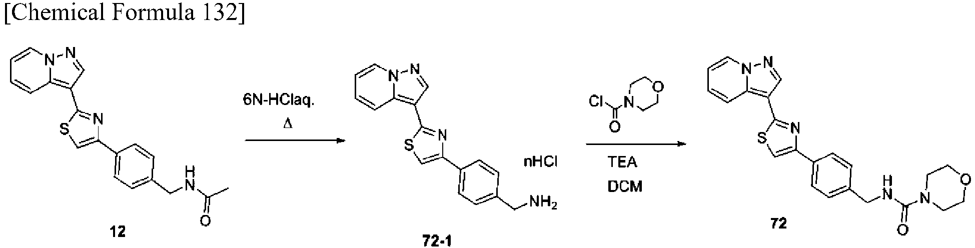

### (1) Synthesis of (4-(2-(pyrazolo[1,5-a]pyridin-3-yl)thiazol-4-yl)phenyl)methanamine hydrochloride (compound 72-1)

N-(4-(2-(Pyrazolo[1,5-a]pyridin-3-yl)thiazol-4-yl)benzyl)acetamide (compound 12) (72.1 mg, 0.21 mmol) was suspended in 6 N hydrochloric acid (1.0 mL), and the mixture was refluxed for 6 hours. After the mixture was allowed to cool, the solvent was removed under reduced pressure, ethanol was added several times, and the mixture was dried to give a residue. The obtained solid was dissolved in water (10 mL), ethyl acetate (10 mL) was added thereto, and the mixture was partitioned. The aqueous layer was concentrated under reduced pressure, ethanol was added several times, and the solvent was removed under reduced pressure to obtain the title compound (72-1) (66.9 mg, 0.180 mmol, 85.5% yield).

¹H-NMR(D₂O) δ:3.91 (2H, s), 6.71 (1H, t, J=6.9Hz), 7.11 (2H, d, J=7.8Hz), 7.16 (2H, d, J=8.7Hz), 7.27 (2H, d, J=7.8Hz), 7.44 (2H, d, J=8.7Hz), 8.02 (1H, s).

### (2) Synthesis of N-(4-(2-(pyrazolo[1,5-a]pyridin-3-yl)thiazol-4-yl)benzyl)morpholine-4-carboxamide (compound 72)

(4-(2-(Pyrazolo[1,5-a]pyridin-3-yl)thiazol-4-yl)phenyl)methanamine hydrochloride (compound 72-1) (20.5 mg, 0.0544 mmol) was suspended in anhydrous dichloromethane (10 mL), and morpholine carbonyl chloride (0.10 mL) was added under ice cooling, then excess triethylamine (0.20 mL) was slowly added. The mixture was gradually allowed to return to room temperature and stirred for 16 hours. Water was added to the reaction mixture under ice cooling, the dichloromethane layer was washed with water several times, the dichloromethane layer was dried over anhydrous magnesium sulfate. After filtration, the solvent was removed under reduced pressure, and the residue was purified by silica gel column chromatography to obtain the title compound (72) (14.3 mg, 0.0341 mmol, 62.7% yield) from a fraction of n-hexane : ethyl acetate (1 : 1).

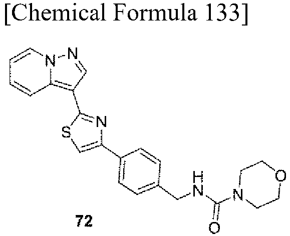

¹H-NMR(CDCl₃) δ:3.38 (4H, t, J=5.0Hz), 3.70 (4H, t, J=5.3Hz), 4.49 (2H, d, J=5.5Hz), 4.71+5.73 (1H, brs x2), 6.93 (1H, t, J=7.1Hz), 7.38 (4H, m), 7.98 (2H, d, J=7.3Hz), 8.39 (1H, s), 8.45 (1H, d, J=8.7Hz), 8.52 (1H, d, J=6.9Hz).

### [Example 78] Synthesis of N-methyl-1-(4-(2-(pyrazolo[1,5-a]pyridin-3-yl)thiazol-4-yl)phenyl)methanamine hydrochloride (compound 74)

Compound (74) was synthesized from compound (16), according to the method for synthesizing compound (72-1) from compound (12).

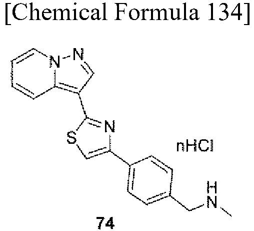

¹H-NMR(D₂O) δ:2.61 (3H, s), 4.07 (2H, s), 6.85 (1H, t, J=6.4Hz), 7.28 (3H, m), 7.40 (1H, brs), 7.62 (2H, m), 7.81 (1H, m), 8.17 (1H, s), 8.27 (1H, d, J=6.9Hz).

### [Example 79] Synthesis of N-methyl-N-(4-(2-(pyrazolo[1,5-a]pyridin-3-yl)thiazol-4-yl)benzyl)morpholine-4-carboxamide (compound 76)

Compound (76) was synthesized from compound (74), according to the method for synthesizing compound (72) from compound (72-1).

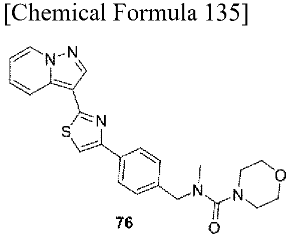

¹H-NMR(CDCl₃) δ:2.79 (3H, s), 3.28 (4H, t, J=5.1Hz), 3.70 (4H, t, J=4.6Hz), 4.44 (2H, s), 6.91 (1H, dt, J=1.4Hz, 6.9Hz), 7.37 (4H, m), 7.98 (2H, d, J=8.2Hz), 8.39 (1H, s), 8.45 (1H, d, J=8.7Hz), 8.51 (1H, d, J=6.9Hz).

### [Example 80] Synthesis of 2-methoxy-N-methyl-N-(4-(2-(pyrazolo[1,5-a]pyridin-3-yl)thiazol-4-yl)benzyl)acetamide (compound 78)

Compound (78) was synthesized from compound (74), according to the method for synthesizing compound (72) from compound (72-1).

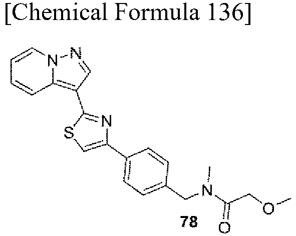

¹H-NMR(CDCl₃) δ:2.92+2.93 (3H, s x2), 3.45+3.48 (3H, s x2), 4.17+4.19 (2H, s x2), 4.58+4.65 (2H, d x2, J=3.7Hz), 6.93 (1H, t, J=6.9Hz), 7.28 (1H, d, J=8.2Hz), 7.38 (3H, m), 7.96+8.01 (2H, d x2, J=7.8Hz), 8.39+8.40 (1H, s x2), 8.45 (1H, d, J=8.7Hz), 8.52 (1H, d, J=6.9Hz).

### [Example 81] Synthesis of 1,1-dimethyl-3-(4-(2-(pyrazolo[1,5-a]pyridin-3-yl)thiazol-4-yl)phenyl)urea (compound 59)

Compound (59) was synthesized from compound (1), according to the method for synthesizing compound (72) from compound (72-1).

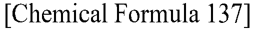

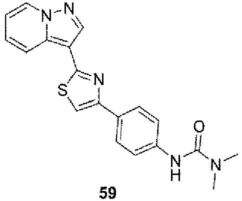

¹H-NMR(CDCl₃) δ:3.06 (6H, s), 6.39 (1H, brs), 6.91 (1H, dt, J=1.4Hz, 6.9Hz), 7.28 (1H, s), 7.38 (1H, ddd, J=0.9Hz, 6.9Hz, 8.7Hz), 7.48 (2H, d, J=8.7Hz), 7.93 (2H, d, J=8.7Hz), 8.39 (1H, s), 8.45 (1H, d, J=8.7Hz), 8.51 (1H, d, J=6.9Hz).

[Examples 82 to 85] Synthesis of the following compounds:
1,1-dimethyl-3-(4-(2-(pyrazolo[1,5-a]pyridin-3-yl)thiazol-4-yl)benzyl)urea (compound 75),
2-methoxy-N-(4-(2-(pyrazolo[1,5-a]pyridin-3-yl)thiazol-4-yl)benzyl)acetamide (compound 77),
N-(4-(2-(pyrazolo[1,5-a]pyridin-3-yl)thiazol-4-yl)benzyl)cyclobutanesulfonamide (compound 82), and
N-(4-(2-(pyrazolo[1,5-a]pyridin-3-yl)thiazol-4-yl)benzyl)oxetane-3-carboxamide (compound 85)

Compound (75), compound (77), compound (82), and compound (85) were synthesized from compound (72-1), according to the method for synthesizing compound (72) from compound (72-1).

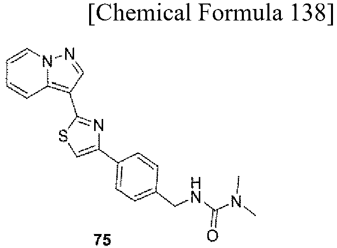

¹H-NMR(CDCl₃) δ:2.94 (6H, s), 4.48 (2H, d, J=5.5Hz), 4.66 (1H, brs), 6.92 (1H, dt, J=1.4Hz, 7.3Hz), 7.38 (4H, m), 7.98 (2H, m), 8.39 (1H, s), 8.46 (1H, d, J=7.8Hz), 8.52 (1H, d, J=6.9Hz).

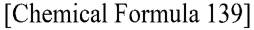

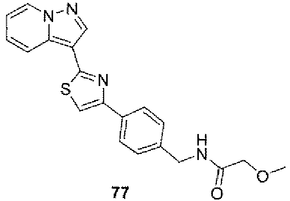

¹H-NMR(CDCl₃) δ:3.41 (3H, s), 3.97 (2H, s), 4.54 (2H, d, J=6.0Hz), 6.85 (1H, brs), 6.93 (1H, t, J=6.9Hz), 7.38 (4H, m), 7.98 (2H, d, J=8.2Hz), 8.39 (1H, s), 8.45 (1H, d, J=8.7Hz), 8.52 (1H, d, J=7.3Hz).

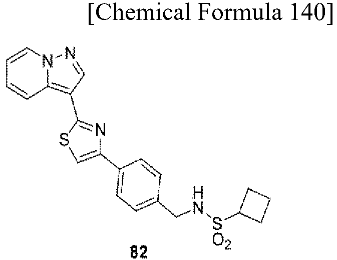

¹H-NMR(CDCl₃) δ:1.99 (2H, m), 2.24 (2H, m), 2.49 (2H, m), 3.72 (1H, m), 4.37 (3H, m), 6.93 (1H, dt, J=1.4Hz, 6.9Hz), 7.39 (4H, m), 8.01 (2H, d, J=8.2Hz), 8.40 (1H, s), 8.46 (1H, d, J=8.7Hz), 8.53 (1H, d, J=6.9Hz).

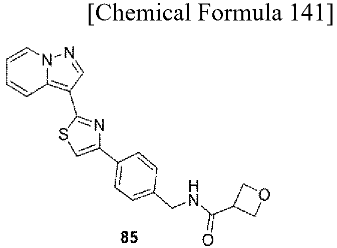

¹H-NMR(CDCl₃) δ:3.73 (1H, m), 4.53 (2H, d, J=5.5Hz), 4.82 (2H, dd, J=6.0Hz, 8.7Hz), 4.91 (2H, t, J=6.4Hz), 5.76 (1H, brs), 6.93 (1H, t, J=6.9Hz), 7.36 (4H, m), 7.98 (2H, d, J=8.2Hz), 8.39 (1H, s), 8.45 (1H, d, J=8.7Hz), 8.52 (1H, d, J=6.9Hz).

### [Example 86] Synthesis of 2-amino-N-(4-(2-(pyrazolo[1,5-a]pyridin-3-yl)thiazol-4-yl)phenyl)acetamide hydrochloride (compound 64)

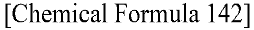

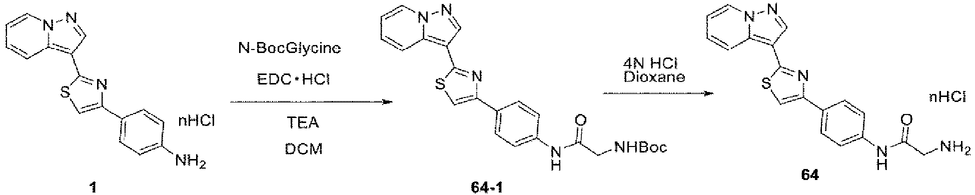

### (1) Synthesis of tert-butyl (2-oxo-2-((4-(2-(pyrazolo[1,5-a]pyridin-3-yl)thiazol-4-yl)phenyl)amino)ethyl)carbamate (compound 64-1)

4-(2-(Pyrazolo[1,5-a]pyridin-3-yl)thiazol-4-yl)aniline hydrochloride (compound 1) (605 mg, 2.07 mmol), N-Boc-glycine (387 mg, 2.21 mmol), and EDC hydrochloride (586 mg, 3.54 mmol) were added to an anhydrous dichloromethane solution (50 mL), and an excess of triethylamine (1.00 mL) was slowly added under ice cooling. The mixture was gradually allowed to return to room temperature and stirred for 16 hours. Water was added to the reaction mixture under ice cooling, the dichloromethane layer was washed several times with water, and dried over anhydrous magnesium sulfate. After filtration, the solvent was removed under reduced pressure, and the residue was purified by silica gel column chromatography to obtain the title compound (64-1) (170 mg, 0.379 mmol, 18.3% yield) from a fraction of n-hexane : ethyl acetate (1 : 1).

¹H-NMR(DMSO-d₆) δ:1.51 (9H, s), 3.71 (2H, d, J=8.2Hz), 7.04 (1H, t, J=6.0Hz), 7.15 (1H, dd, J=1.4Hz, 6.9Hz), 7.55 (1H, t, J=6.0Hz), 7.67 (2H, d, J=8.7Hz), 7.87 (1H, s), 8.00 (2H, d, J=8.7Hz), 8.42 (1H, d, J=9.2Hz), 8.59 (1H, s), 8.81 (1H, d, J=7.3Hz), 10.04 (1H, s).

### (2) Synthesis of 2-amino-N-(4-(2-(pyrazolo[1,5-a]pyridin-3-yl)thiazol-4-yl)phenyl)acetamide hydrochloride (compound 64)

Under ice cooling, tert-butyl (2-oxo-2-((4-(2-(pyrazolo[1,5-a]pyridin-3-yl)thiazol-4-yl)phenyl)amino)ethyl)carbamate (compound 64-1) (170 mg, 0.378 mmol) was slowly added to 4 N hydrogen chloride in dioxane (3.0 mL), and the mixture was stirred at room temperature for 2 hours. The reaction mixture was concentrated under reduced pressure to remove the solvent, and the residue was dried. A solvent was then added, and the resulting solid was collected by filtration, washed well with the solvent, and dried to obtain the title compound (64) (159 mg, 0.378 mmol, 99.8% yield).

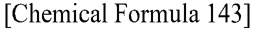

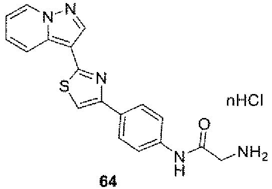

¹H-NMR(D₂O) δ:3.85 (2H, s), 6.85 (1H, t, J=6.2Hz), 7.15 (2H, d, J=7.8Hz), 7.23 (2H, m), 7.34 (2H, d, J=7.3Hz), 7.62 (1H, d, J=8.7Hz), 8.15 (1H, s), 8.23 (1H, d, J=6.9Hz).

### [Example 87] Synthesis of N-(4-(2-(pyrazolo[1,5-a]pyridin-3-yl)thiazol-4-yl)phenyl)oxetane-3-carboxamide (compound 66)

Compound (66) was synthesized from compound (1), according to the method for synthesizing compound (64-1) from compound (1).

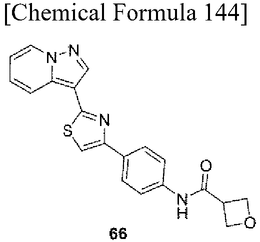

¹H-NMR(CDCl₃) δ:3.88 (1H, m), 4.89 (2H, dd, J=6.4Hz, 8.2Hz), 4.99 (2H, d, J=6.4Hz), 6.92 (1H, t, J=5.5Hz), 7.21 (1H, brs), 7.33 (1H, s), 7.40 (1H, d, J=8.5Hz), 7.65 (2H, d, J=8.7Hz), 8.00 (2H, d, J=8.2Hz), 8.40 (1H, s), 8.45 (1H, d, J=9.2Hz), 8.52 (1H, d, J=6.9Hz).

[Examples 88 to 90] Synthesis of the following compounds:
2-amino-N-(3-(2-(pyrazolo[1,5-a]pyridin-3-yl)thiazol-4-yl)phenyl)acetamide hydrochloride (compound 65),
2-amino-N-methyl-N-(4-(2-(pyrazolo[1,5-a]pyridin-3-yl)thiazol-4-yl)benzyl)acetamide hydrochloride (compound 81), and
2-amino-N-(4-(2-(pyrazolo[1,5-a]pyridin-3-yl)thiazol-4-yl)benzyl)acetamide hydrochloride (compound 80)

Compound (65) was synthesized from compound (10), compound (81) was synthesized from compound (74), and compound (80) was synthesized from compound (72-1), respectively, according to the method for synthesizing compound (64) from compound (1).

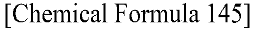

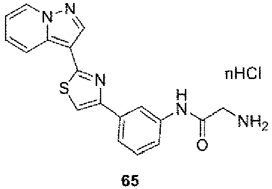

¹H-NMR(D₂O) δ:3.82 (2H, s), 6.70 (1H, t, J=6.9Hz), 7.03 (5H, m), 7.20 (1H, s), 7.37 (1H, d, J=8.2Hz), 7.94 (1H, s), 8.05 (1H, d, J=6.9Hz).

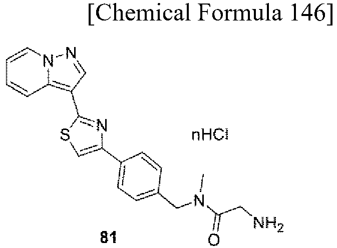

¹H-NMR(D₂O) δ:2.85+2.86 (3H, s x2), 3.95+3.99 (2H, s x2), 4.04+4.45 (2H, s x2), 6.88 (1H, t, J=6.9Hz), 7.06 (2H, d, J=8.2Hz), 7.30 (2H, m), 7.36+7.42 (2H, d x2, J=8.2Hz), 7.61+7.64 (1H, d x2, J=8.2Hz), 8.19 (1H, s), 8.24 (1H, d, J=6.9Hz).

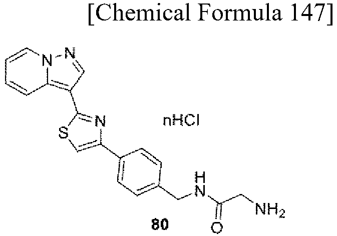

¹H-NMR(D₂O) δ:3.77 (2H, s), 4.25 (2H, s), 6.78 (1H, t, J=8.7Hz), 7.04 (2H, d, J=8.2Hz), 7.14 (1H, brs), 7.17 (1H, t, J=7.8Hz), 7.30 (2H, d, J=7.8Hz), 7.52 (1H, d, J=8.7Hz), 8.06 (1H, s), 8.15 (1H, d, J=6.9Hz).

### [Example 91] Synthesis of sodium 2-(4-(2-(pyrazolo[1,5-a]pyridin-3-yl)thiazol-4-yl)phenoxy)acetate (compound 5)

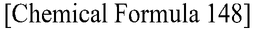

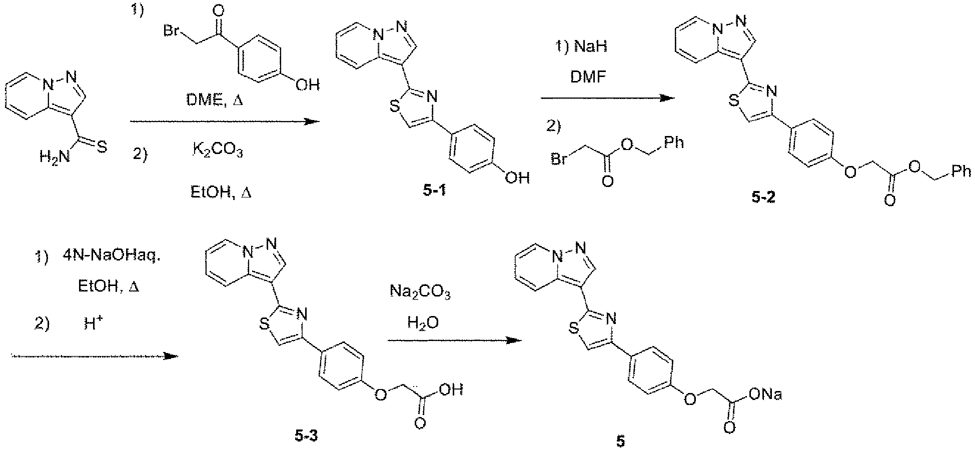

### (1) Synthesis of 4-(2-(pyrazolo[1,5-a]pyridin-3-yl)thiazol-4-yl)phenol (compound 5-1)

Pyrazolo[1,5-a]pyridine-3-carbothioamide (718 mg, 4.05 mmol) was dissolved in DME (40 mL), and 4-hydroxyphenacyl bromide (871 mg, 4.05 mmol) was added thereto under ice cooling, and the mixture was stirred at room temperature for 16 hours. The reaction mixture was heated and stirred at 50°C for 1 hour and then allowed to cool, and the reaction mixture was concentrated under reduced pressure to remove the solvent. Ethanol (40 mL) was added thereto, then excess potassium carbonate (996 mg) was added thereto, and the reaction mixture was refluxed at 70°C for 1 hour. After cooling, the reaction mixture was concentrated under reduced pressure to remove the solvent, and the residue was purified by silica gel column chromatography to obtain the title compound (5-1) (1.03 g, 3.53 mmol, 88.2% yield) from a fraction of n-hexane : ethyl acetate (1 : 1).

¹H-NMR(CDCl₃) δ:6.92 (3H, m), 7.23 (1H, s),7.38 (1H, ddd, J=0.9Hz, 6.4Hz, 8.7Hz), 7.91 (2H, d, J=8.7Hz), 8.39 (1H, s), 8.45 (1H, d, J=7.8Hz), 8.51 (1H, d, J=6.9Hz).

### (2) Synthesis of benzyl 2-(4-(2-(pyrazolo[1,5-a]pyridin-3-yl)thiazol-4-yl)phenoxy)acetate (compound 5-2)

Sodium hydride (oil dispersion) (154 mg) was carefully added to anhydrous DMF (10 mL) to form a suspension solution, and after cooling to -15°C, 4-(2-(pyrazolo[1,5-a]pyridin-3-yl)thiazol-4-yl)phenol (compound 5-1) (153 mg, 0.522 mmol) was slowly added. The mixture was once heated to room temperature, then cooled to -15°C, and then excess benzyl bromoacetate (0.30 mL) was added dropwise thereto. The mixture was stirred for 16 hours while gradually returning to room temperature. The reaction mixture was ice-cooled, and water (about 100 mL) was carefully and gradually added thereto. The resulting solid was stirred at room temperature for some time and collected by filtration, then washed well with a solvent such as n-hexane and then dried to obtain the title compound (5-2) (148 mg, 0.334 mmol, 64.2% yield).

¹H-NMR(CDCl₃) δ:4.72 (2H, s), 5.25 (2H, s), 6.92 (1H, t, J=6.9Hz), 6.98 (2H, d, J=9.2Hz), 7.34 (7H, m), 7.93 (2H, d, J=8.7Hz), 8.38 (1H, s), 8.45 (1H, d, J=9.2Hz), 8.51 (1H, d, J=6.9Hz).

### (3) Synthesis of 2-(4-(2-(pyrazolo[1,5-a]pyridin-3-yl)thiazol-4-yl)phenoxy)acetic acid (compound 5-3)

Benzyl 2-(4-(2-(pyrazolo[1,5-a]pyridin-3-yl)thiazol-4-yl)phenoxy)acetate (compound 5-2) (139 mg, 0.315 mmol) was added to a mixed solvent of a 4 N aqueous sodium hydroxide solution (3 mL) and ethanol (3 mL), and the reaction mixture was heated and stirred at 100°C for 1.5 hours. After the reaction mixture was allowed to cool, it was poured into ice-water (100 mL) and dissolved. The insoluble matter was filtered to afford the title compound (5) (76.3 mg, 0.20 mmol, 64.9% yield). Further, 2 N hydrochloric acid was added to the aqueous layer to make the aqueous layer weakly acidic, and the precipitated solid was stirred for some time and then collected by filtration. The solid was thoroughly washed with water and then dried to obtain the title compound (5-3) (19.0 mg, 0.0256 mmol, 8.1% yield).

¹H-NMR(CDCl₃) δ:4.70 (2H, s), 6.98 (2H, d, J=8.7Hz), 7.08 (1H, t, J=6.9Hz), 7.53 (1H, d, J=7.8Hz), 7.83 (1H, s), 7.97 (2H, d, J=8.7Hz), 8.39 (1H, d, J=8.7Hz), 8.58 (1H, s), 8.81 (1H, d, J=6.9Hz).

### (4) Synthesis of sodium 2-(4-(2-(pyrazolo[1,5-a]pyridin-3-yl)thiazol-4-yl)phenoxy)acetate (compound 5)

2-(4-(2-(pyrazolo[1,5-a]pyridin-3-yl)thiazol-4-yl)phenoxy)acetic acid (compound 5-3) (531 mg, 1.51 mmol) and anhydrous sodium carbonate (80.5 mg, 0.76 mmol) were added to purified water (100 mL), and the mixture was stirred at room temperature to achieve complete dissolution. The faint insoluble matter was removed by filtration, and the resulting pale yellow aqueous solution was freeze-dried to obtain the title compound (5) (592 mg, 1.50 mmol, 99.3% yield).

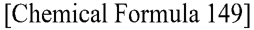

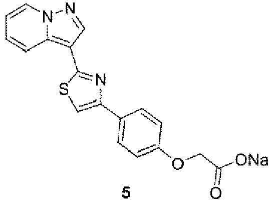

¹H-NMR(D₂O) δ:4.24 (2H, s), 6.61 (2H, d, J=8.7Hz), 6.70 (1H, t, J=6.9Hz), 6.92 (1H, s), 7.30 (2H, d, J=8.7Hz), 7.52 (2H, d, J=8.7Hz), 7.98 (1H, s,), 8.12 (1H, d, J=6.9Hz).

### [Example 92] Synthesis of N-acetyl-N-(4-(2-(pyrazolo[1,5-a]pyridin-3-yl)thiazol-4-yl)benzyl)glycine (compound 73)

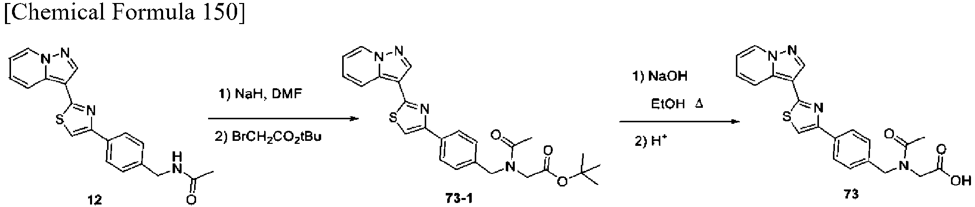

### (1) Synthesis of tert-butyl N-acetyl-N-(4-(2-(pyrazolo[1,5-a]pyridin-3-yl)thiazol-4-yl)benzyl)glycine (compound 73-1)

Sodium hydride (oil dispersion) (48.3 mg) was carefully added to anhydrous DMF (4 mL) to form a suspension solution, and after cooling to -15°C, N-(4-(2-(pyrazolo[1,5-a]pyridin-3-yl)thiazol-4-yl)benzyl)acetamide (compound 12) (106 mg, 0.305 mmol) was slowly added. The mixture was once heated to room temperature, then cooled to -15°C, and then tert-butyl bromoacetate (0.10 mL) was added dropwise thereto. The mixture was stirred for 16 hours while gradually returning to room temperature. The reaction mixture was ice-cooled, and water was carefully and gradually added thereto. Ethyl acetate was added to the reaction mixture, the mixture was washed with saturated saline, and the organic layer was dried over anhydrous magnesium sulfate, then the insoluble matter was filtered, and the organic layer was concentrated under reduced pressure to remove the solvent, and the residue was purified by silica gel column chromatography to obtain the title compound (73-1) (62.8 mg, 0.136 mmol, 64.2% yield) from a fraction of n-hexane : ethyl acetate (1 : 1). From another fraction, the raw material compound (12) (58.0 mg, 0.125 mmol, 55.0% yield) was recovered.

¹H-NMR(CDCl₃) δ:1.56 (9H, s), 2.05 (3H, s), 4.48 (2H, d, J=6.0Hz), 6.60 (2H, s), 6.93 (1H, dt, J=1.4Hz, 6.9Hz), 7.34 (4H, m), 7.98 (2H, d, J=8.2Hz), 8.39 (1H, s), 8.46 (1H, d, J=9.2Hz), 8.52 (1H, d, J=7.3Hz).

### (2) Synthesis of N-acetyl-N-(4-(2-(pyrazolo[1,5-a]pyridin-3-yl)thiazol-4-yl)benzyl)glycine (compound 73)

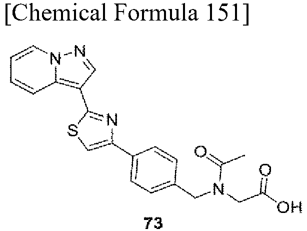

tert-Butyl N-acetyl-N-(4-(2-(pyrazolo[1,5-a]pyridin-3-yl)thiazol-4-yl)benzyl)glycine (compound 73-1) (63.1 mg, 0.136 mmol) was added to a mixed solvent of a 4 N aqueous sodium hydroxide solution (1 mL) and ethanol (1 mL), and the reaction mixture was heated and stirred at 100°C for 3 hours. After the reaction mixture was allowed to cool, it was poured into ice-water (100 mL) and dissolved. The insoluble matter was filtered to give the sodium salt of the title compound (73) (27.4 mg, 0.20 mmol, 64.9% yield). Furthermore, 2 N hydrochloric acid was added to the aqueous layer to make the aqueous layer weakly acidic, and the precipitated solid was stirred for some time, then collected by filtration, washed well with water, and then dried to obtain the title compound (73) (27.4 mg, 0.067 mmol, 49.6% yield).

¹H-NMR(DMSO-d₆) δ:1.99+2.05 (3H, s x2), 3.91+4.04 (2H, s x2), 4.50+4.64 (2H, s x2), 7.09 (1H, t, J=6.9Hz), 7.34+7.55 (3H, m x2), 7.98+8.06 (3H, m x2), 8.40 (1H, d, J=8.7Hz), 8.60 (1H, d, J=5.5Hz), 8.82 (1H, d, J=6.9Hz).

### [Example 93] Synthesis of 2-(4-(2-(pyrazolo[1,5-a]pyridin-3-yl)thiazol-4-yl)phenoxy)acetamide (compound 26)

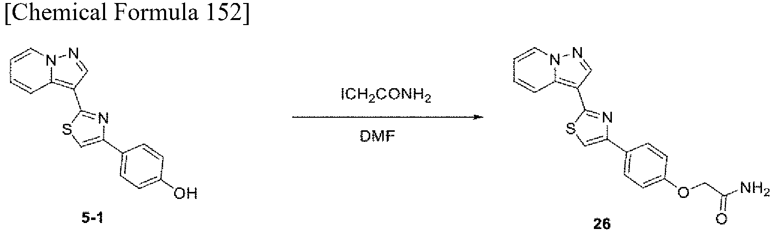

2-Iodoacetamide (0.10 g, 0.54 mmol) and potassium carbonate (0.60 g, 4.34 mmol) were added to a solution of 4-(2-(pyrazolo[1,5-a]pyridin-3-yl)thiazol-4-yl)phenol (compound 5-1) (0.10 g, 0.34 mmol) in N,N-dimethylformamide (2 mL), and the mixture was stirred at room temperature for 2 hours. Water (10 mL) was added, and the resulting precipitate was collected by suction filtration and dried to obtain the title compound (26) (24.0 mg, 0.07 mmol, 20.1% yield).

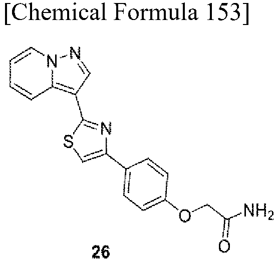

¹H-NMR(DMSO-d₆) δ:4.88 (2H, s), 7.02-7.08 (2H, m), 7.10-7.16 (1H, m), 7.55-7.62 (1H, m), 7.89 (1H, s), 7.99-8.06 (2H, m), 8.44 (1H, d, J=8.4Hz), 8.63 (1H, s), 8.86 (1H, d, J=6.8Hz).

[Examples 94 to 99] Synthesis of the following compounds:
3-(2-(pyrazolo[1,5-a]pyridin-3-yl)thiazol-4-yl)phenyl-2-(2-(2-methoxyethoxy)ethoxy)acetate (compound 13),
4-(3-(2-methoxyethoxy)phenyl)-2-(pyrazolo[1,5-a]pyridin-3-yl)thiazole (compound 14),
4-(3-(2-(2-methoxyethoxy)ethoxy)phenyl)-2-(pyrazolo[1,5-a]pyridin-3-yl)thiazole (compound 22),
4-(1-(cyclopropylmethyl)-1H-pyrazol-4-yl)-2-(pyrazolo[1,5-a]pyridin-3-yl)thiazole (compound 108),
4-(1-ethyl-1H-pyrazol-4-yl)-2-(pyrazolo[1,5-a]pyridin-3-yl)thiazole (compound 110), and
4-(1-(2-methoxyethyl)-1H-pyrazol-4-yl)-2-(pyrazolo[1,5-a]pyridin-3-yl)thiazole hydrochloride (compound 111)

Compound (13), compound (14), and compound (22) were synthesized from compound (3), and compound (108), compound (110), and compound (111) were synthesized from compound (107), according to the method for synthesizing compound (26).

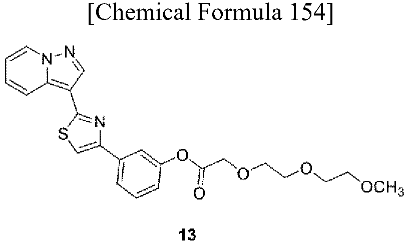

¹H-NMR(DMSO-d₆) δ:3.25 (3H, s), 3.43-3.48 (2H, m), 3.53-3.58 (2H, m), 3.59-3.63 (2H, m), 3.71-3.76 (2H, m), 4.50 (2H, s), 7.11-7.22 (2H, m), 7.52-7.63 (2H, m), 7.85-7.89 (1H, m), 7.99-8.04 (1H, m), 8.11 (1H, s), 8.42 (1H, d, J=9.2Hz), 8.66 (1H, s), 8.86 (1H, d, J=6.8Hz).

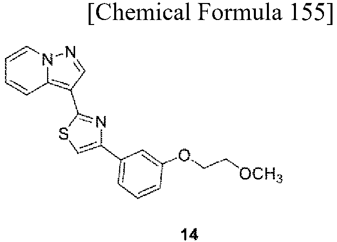

¹H-NMR(CDCl₃) δ:3.49 (3H, s), 3.81 (2H, t, J=4.6Hz), 4.23 (2H, t, J=4.6Hz), 6.88-6.98 (2H, m), 7,30-7.43 (3H, m), 7.56-7.66 (2H, m), 8.40 (1H, s), 8.44-8.56 (2H, m).

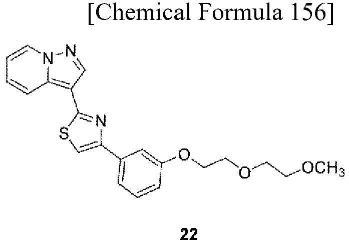

¹H-NMR(DMSO-d₆) δ:3.26 (3H, s), 3.47-3.50 (2H, m), 3.61-3.63 (2H, m), 3.78-3.83 (2H, m), 4.19-4.21 (2H, m), 6.96-6.98 (1H, m), 7.13-7.15 (1H, m), 7.40 (1H, t, J=7.9Hz), 7.60-7.63 (2H, m), 7.69 (1H, t, J=7.8Hz), 8.08 (1H, s), 8.41 (1H, d, J=8.8Hz), 8.64 (1H, s), 8.86 (1H, d, J=7.0Hz).

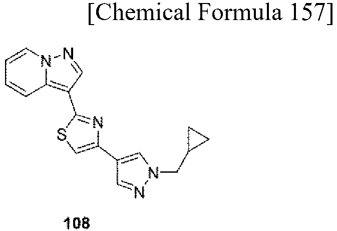

¹H-NMR(DMSO-d₆) δ:0.38-0.45 (2H, m), 0.52-0.60 (2H, m), 1.25-1.38 (1H, m), 4.04 (2H, d, J=6.8Hz), 7.10-7.16 (1H, m), 7.52-7.60 (1H, m), 7.59 (1H, s), 7.97 (1H, s), 8.30 (1H, s), 8.41-8.46 (1H, m), 8.60 (1H, s), 8.82-8.88 (1H, m).

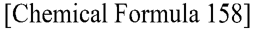

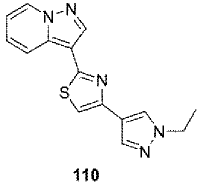

¹H-NMR(DMSO-d₆) δ:1.43 (3H, t, J=7.4Hz), 4.20 (2H, q, J=7.3Hz), 7.08-7.15 (1H, m), 7.52-7.58 (1H, m), 7.56 (1H, s), 7.95 (1H, s), 8.28 (1H, s), 8.42-8.47 (1H, m), 8.59 (1H, s), 8.82-8.87 (1H, m).

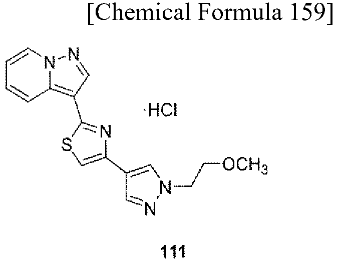

¹H-NMR(DMSO-d₆) δ:3.26 (3H, s), 3.74 (2H, t, J=5.4Hz), 4.33 (2H, t, J=5.2Hz), 7.10-7.16 (1H, m), 7.52-7.60 (1H, m), 7.59 (1H, s), 7.98 (1H, s), 8.25 (1H, s), 8.41-8.47 (1H, m), 8.60 (1H, s), 8.82-8.88 (1H, m).

### [Example 100] Synthesis of 2-(2-(4-(2-(pyrazolo[1,5-a]pyridin-3-yl)thiazol-4-yl)phenoxy)ethoxy)ethan-1-amine hydrochloride (compound 32)

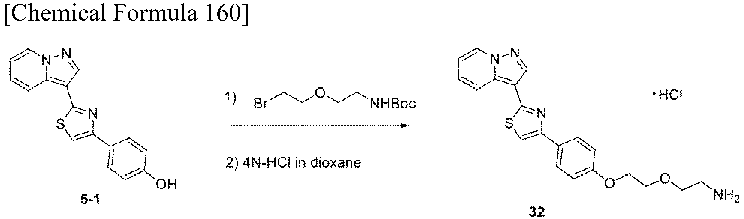

Potassium carbonate (0.5 g) and tert-butyl N-[2-(2-bromoethoxy)ethyl]carbamate (0.2 mL, 0.78 mmol) were added to a solution of 4-(2-(pyrazolo[1,5-a]pyridin-3-yl)thiazol-4-yl)phenol (compound 5-1) (0.10 g, 0.27 mmol) in DMF (1 mL), and the mixture was stirred at room temperature for 2 hours. Saturated saline (5 mL) was added and the resulting oil was separated by decantation. A solution (5 mL) of 4 N hydrogen chloride in dioxane was added thereto, and the mixture was stirred at room temperature for 3 hours. Acetone (20 mL) was added, and the resulting precipitate was filtered with suction, then washed with diethyl ether and dried to obtain the title compound (32) (94.0 mg, 0.225 mmol, 87.2% yield).

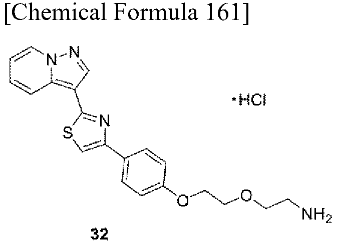

¹H-NMR(DMSO-d₆) δ:2.97-3.06 (2H, m), 3.67-3.73 (2H, m), 3.80-3.87 (2H, m), 4.18-4.24 (2H, m), 7.04-7.16 (3H, m), 7.54-7.62 (1H, m), 7.88 (1H, s), 7.98-8.08 (2H, m), 8.43 (1H, d, J=8.8Hz), 8.63 (1H, s), 8.86 (1H, d, J=6.8Hz).

### [Examples 101 and 102] Synthesis of 2-(2-(3-(2-(pyrazolo[1,5-a]pyridin-3-yl)thiazol-4-yl)phenoxy)ethoxy)ethan-1-amine hydrochloride (compound 20), and N,N-dimethyl-2-(4-(2-(pyrazolo[1,5-a]pyridin-3-yl)thiazol-4-yl)phenoxy)ethan-1-amine hydrochloride (compound 47)

Compound (20) was synthesized from compound (3), and compound (47) was synthesized from compound (5-1), respectively, according to the method for synthesizing compound (32).

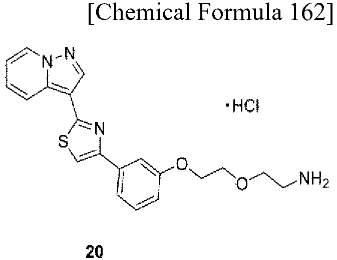

¹H-NMR(DMSO-d₆) δ:2.97-3.07 (2H, m), 3.68-3.75 (2H, m), 3.82-3.88 (2H, m), 4.21-4.28 (2H, m), 6.95-7.01 (1H, m), 7.11-7.17 (1H, m), 7.41 (1H, t, J=8.0Hz), 7.56-7.65 (2H, m), 7.70 (1H, d, J=8.0Hz), 8.10 (1H, s), 8.37-8.44 (1H, m), 8.65 (1H, s), 8.84-8.89 (1H, m).

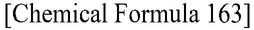

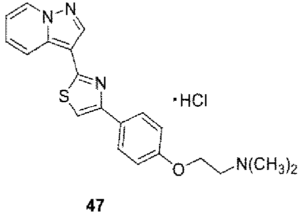

¹H-NMR(DMSO-d₆) δ:2.87 (3H, s), 2.88 (3H, s), 3.51-3.58(2H, m), 4.39-4.45 (2H, m), 7.10-7.16 (3H, m), 7.54-7.62 (1H, m), 7.91 (1H, s), 7.96-8.10 (2H, m), 8.39-8.46 (1H, m), 8.64 (1H, s), 8.84-8.90 (1H, m).

### [Example 103] Synthesis of 1-(2-(3-(2-(pyrazolo[1,5-a]pyridin-3-yl)thiazol-4-yl)phenoxy)ethyl)guanidine hydrochloride (compound 52)

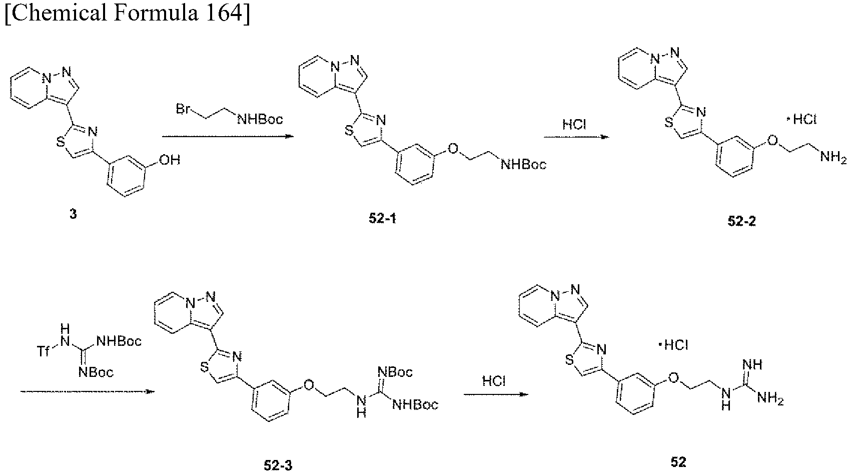

### (1) Synthesis of tert-butyl (2-(3-(2-(pyrazolo[1,5-a]pyridin-3-yl)thiazol-4-yl)phenoxy)ethyl)carbamate (compound 52-1)

Potassium carbonate (0.5 g, 3.60 mmol) was added to a solution of 3-(2-(pyrazolo[1,5-a]pyridin-3-yl)thiazol-4-yl)phenol (compound 3) (0.38 g, 1.00 mmol) in DMF (2 mL), then 2-(tert-butoxycarbonylamino)ethyl bromide (0.30 g, 1.30 mmol) was added, and the mixture was stirred at 70°C for 10 hours. The insoluble matter was filtered, and water (50 mL) was added to the filtrate. The obtained precipitate was collected by filtration and purified by preparative TLC (preparative thin layer chromatography) to obtain the title compound (52-1) (0.33 g, 0.76 mmol, 76.0% yield) from a fraction of chloroform : methanol (98 : 2).

¹H-NMR(DMSO-d₆) δ:1.46 (9H, s), 3.34-3.37 (2H, m), 4.05-4.08 (2H, m), 6.95-6.97 (1H, m), 7.13-7.15 (1H, m), 7.40 (1H, t, J=8.0Hz), 7.57-7.62 (2H, m), 7.67-7.70 (1H, m), 8.08 (1H, s), 8.38-8.44(1H, m), 8.65 (1H, s), 8.84-8.89 (1H, m).

### (2) Synthesis of 2-(3-(2-(pyrazolo[1,5-a]pyridin-3-yl)thiazol-4-yl)phenoxy)ethanamine hydrochloride (compound 52-2)

A solution (5 mL) of 4 N hydrogen chloride in dioxane was added to tert-butyl (2-(3-(2-(pyrazolo[1,5-a]pyridin-3-yl)thiazol-4-yl)phenoxy)ethyl)carbamate (compound 52-1) (0.33 g, 0.76 mmol), and the mixture was stirred at room temperature for 1 hour. Acetone (20 mL) was added, and the resulting precipitate was collected by filtration and dried to obtain the title compound (52-2) (308 mg, 0.76 mmol, quantitative).

¹H-NMR(DMSO-d₆) δ:3.26-3.28 (2H, m), 4.29-4.31 (2H, m), 7.02-7.04 (1H, m), 7.14-7.17 (1H, m), 7.45 (1H, t, J=8.0Hz), 7.58-7.70 (1H, m), 7.70-7.76 (2H, m), 8.10 (1H, s), 8.40-8.46 (1H, m), 8.66 (1H, s), 8.85-8.90(1H, m).

### (3) Synthesis of 1,3-bis(tert-butoxycarbonyl)-2-(2-(3-(2-(pyrazolo[1,5-a]pyridin-3-yl)thiazol-4-yl)phenoxy)ethanguanidine (compound 52-3)

Triethylamine (0.5 mL, 3.60 mmol) and 1,3-bis(tert-butoxycarbonyl)-2-(trifluoromethanesulfonyl)guanidine (0.2 g, 0.51 mmol) were added to a solution of 2-(3-(2-(pyrazolo[1,5-a]pyridin-3-yl)thiazol-4-yl)phenoxy)ethanamine hydrochloride (compound 52-2) (0.1 g, 0.27 mmol) in dichloromethane (2 mL), and the mixture was stirred at room temperature for 4 hours, and the solvent was removed under reduced pressure. Diethyl ether was added to the residue, and the insoluble matter was filtered off. The filtrate was concentrated under reduced pressure to afford the title compound (52-3) (0.15 g, 0.26 mmol, 96.3% yield).

¹H-NMR(DMSO-d₆) δ:1.39-1.47 (18H, m), 3.73-3.76 (2H, m), 4.10-4.21 (2H, m), 7.03-7.06 (1H, m), 7.10-7.15 (1H, m), 7.41 (1H, t, J=8.0Hz), 7.56-7.64 (2H, m), 7.68-7.74 (1H, m), 8.09 (1H, s), 8.38-8.44 (1H, m), 8.57 (1H, t, J=5.6Hz), 8.64 (1H, s), 8.84-8.88 (1H, m), 11.51 (1H, s).

### (4) Synthesis of 1-(2-(3-(2-(pyrazolo[1,5-a]pyridin-3-yl)thiazol-4-yl)phenoxy)ethyl)guanidine hydrochloride (Compound 52)

A solution (5 mL) of 4 N hydrogen chloride in dioxane was added to 1,3-bis(tert-butoxycarbonyl)-2-(2-(3-(2-(pyrazolo[1,5-a]pyridin-3-yl)thiazol-4-yl)phenoxy)ethanguanidine (compound 52-3) (0.15 g, 0.26 mmol), and the mixture was stirred at room temperature for 12 hours. Acetone (10 mL) was added, and the resulting precipitate was collected by filtration, washed with diethyl ether, and then dried to obtain the title compound (52) (85.0 mg, 0.20 mmol, 76.9% yield).

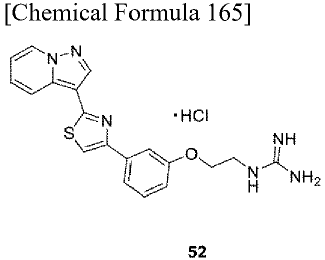

¹H-NMR(DMSO-d₆) δ:3.59-3.61 (2H, m), 4.19 (2H, t, J=5.2Hz), 6.98-7.01(1H, m), 7.12-7.16 (1H, m), 7.43(1H, t, J=8.0Hz), 7.60 (1H, t, J=8.2Hz), 7.65 (1H, s), 7.72 (1H, d, J=7.8Hz), 7.84 (1H, brs), 8.09 (1H, s), 8.39-8.44 (1H, m), 8.65 (1H, s), 8.87 (1H, d, J=6.8Hz).

### [Example 104] Synthesis of 1-(2-(4-(2-(pyrazolo[1,5-a]pyridin-3-yl)thiazol-4-yl)phenoxy)ethyl)guanidine hydrochloride (compound 51)

Compound (51) was synthesized from compound (5-1), according to the method for synthesizing compound (52).

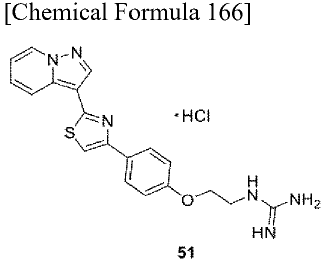

¹H-NMR(DMSO-d₆) δ:3.53-3.61 (2H, m), 4.14 (2H, t, J=5.4Hz), 7.04-7.17 (3H, m), 7.55-7.62 (1H, m), 7.77 (1H, brs), 7.89 (1H, s), 8.02-8.08 (2H, m), 8.40-8.46 (1H, m), 8.63 (1H, s), 8.84-8.88 (1H, m).

### [Example 105] Synthesis of N-(2-(4-(2-(pyrazolo[1,5-a]pyridin-3-yl)thiazol-4-yl)phenoxy)ethyl)acetamide (compound 45)

Compound (45) was synthesized from compound (5-1), according to the method for synthesizing compound (52-2).

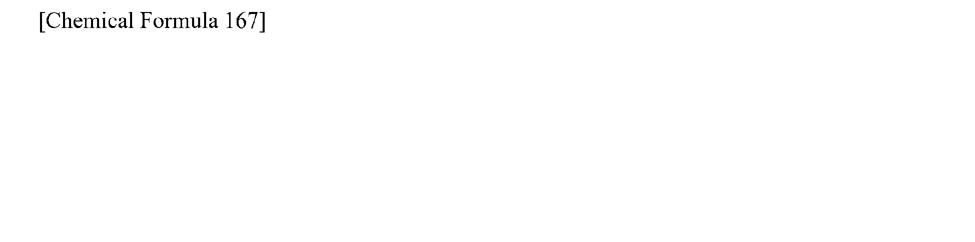

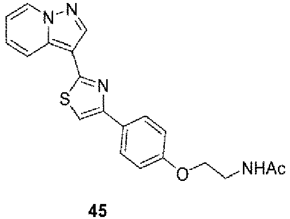

¹H-NMR(DMSO-d₆) δ:2.03 (3H, s), 3.64-3.74 (2H, m), 4.06-4.12 (2H, m), 6.88-7.04 (3H, m), 7.23 (1H, s), 7.27 (1H, s), 7.33-7.42 (1H, m), 7.90-7.98 (2H, m), 8.39 (1H, s), 8.40-8.47 (1H, m), 8.48-8.54 (1H, m).

### [Example 106] Synthesis of N-(2-hydroxyethyl)-2-(3-(2-(pyrazolo[1,5-a]pyridin-3-yl)thiazol-4-yl)phenoxy)acetamide (compound 19)

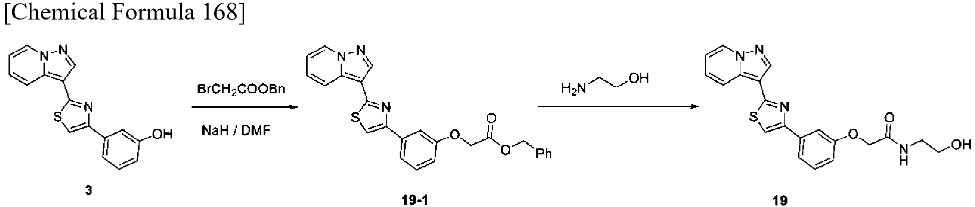

### (1) Synthesis of benzyl 2-(3-(2-pyrazolo[1,5-a]pyridin-3-yl)-4-(thiazol-4-yl)phenoxyacetate (19-1)

3-(2-(pyrazolo[1,5-a]pyridin-3-yl)thiazol-4-yl)phenol (compound 3) (0.30 g, 1.0 mmol) was dissolved in anhydrous DMF (10 mL), and 60% sodium hydride (150 mg, 3.75 mmol) was added thereto. Then, benzyl bromoacetate (0.23 g, 1.0 mmol) was added thereto, and the mixture was stirred at room temperature for 6 hours. Ice water (100 mL) was added, and the precipitate was collected by filtration. The precipitate was washed with methanol to obtain the title compound (19-1) (1.31 g, 2.96 g, 88% yield).

¹H-NMR(DMSO-d₆) δ:5.00 (2H, s), 5.23 (2H, s), 6.95-6.98 (1H, m), 7.10-7.15 (1H, m), 7.31-7.42 (6H, m), 7.55-7.76 (1H, m), 7.64-7.65 (1H, m), 7.72 (1H, d, J=7.8Hz), 8.06 (1H, s), 8.42 (1H, d, J=7.8Hz), 8.65 (1H, s), 8.87 (1H, d, J=7.3Hz).

### (2) Synthesis of N-(2-hydroxyethyl)-2-(3-(2-(pyrazolo[1,5-a]pyridin-3-yl)thiazol-4-yl)phenoxy)acetamide (compound 19)

Ethanolamine (1 mL) was added to benzyl 2-(3-(2-pyrazolo[1,5-a]pyridin-3-yl)-4-(thiazol-4-yl)phenoxyacetate (compound 19-1) (28 mg, 0.063 mmol), and the mixture was stirred at room temperature for 12 hours. Water (10 mL) was added, and the resulting precipitate was filtered with suction, washed with water, methanol, and diethyl ether in this order, and dried to obtain the title compound (19) (28 mg, 0.053 mmol, 84.1% yield).

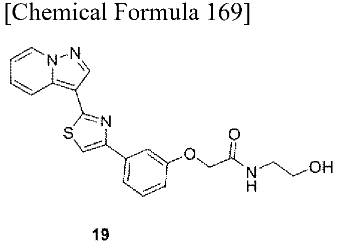

¹H-NMR(DMSO-d₆) δ:3.20-3.29 (2H, m), 3.42-3.49 (2H, m), 4.59 (2H, s), 4.76 (1H, t, J=5.6Hz), 6.98 (1H, dd, J=8.2, 1.8Hz), 7.11-7.18 (1H, m), 7.42 (1H, t, J=7.8Hz), 7.59-7.64 (1H, m), 7.67-7.75 (2H, m), 8.06 (1H, s), 8.10-8.17 (1H, m), 8.44 (1H, d, J=8.4Hz), 8.65 (1H, s), 8.87 (1H, d, J=7.6Hz).

### [Example 107] Synthesis of methyl 2-(3-(2-(pyrazolo[1,5-a]pyridin-3-yl)thiazol-4-yl)phenoxy)acetate (compound 18)

Compound (18) was synthesized from compound (3), according to the method for synthesizing compound (19).

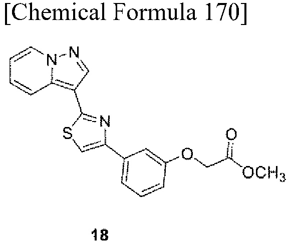

¹H-NMR(DMSO-d₆) δ:3.74 (3H, s), 4.92 (2H, s), 6.95 (1H, dd, J=8.0, 2.4Hz), 7.11-7.18 (1H, m), 7.41 (1H, t, J=8.0Hz), 7.57-7.67 (2H, m), 7.71 (1H, d, J=7.2Hz), 8.09 (1H, s), 8.43 (1H, d, J=8.8Hz), 8.65 (1H, s), 8.87 (1H, d, J=6.8Hz).

### [Example 108] Synthesis of methyl 2-(4-(2-(pyrazolo[1,5-a]pyridin-3-yl)thiazol-4-yl)phenoxy)acetate (compound 25)

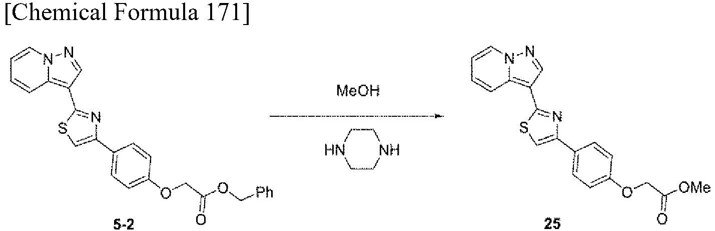

Methanol (20 mL) and piperazine (1.0 g, 11.6 mmol) were added to benzyl 2-(4-(2-(pyrazolo[1,5-a]pyridin-3-yl)thiazol-4-yl)phenoxy)acetate (compound 5-2) (0.3 g, 0.68 mmol) at room temperature, and the mixture was stirred for 12 hours. The resulting precipitate was filtered with suction, washed with methanol and dried to obtain the title compound (25) (226.0 mg, 0.62 mmol, 91.1% yield).

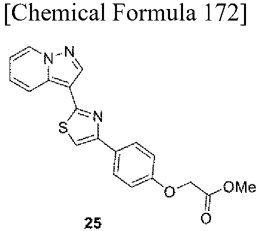

¹H-NMR(DMSO-d₆) δ:3.72 (3H, s), 4.87 (2H, s), 7.01-7.07 (2H, m), 7.09-7.15 (1H, m), 7.54-7.61 (1H, m), 7.88 (1H, s), 7.98-8.04 (2H, m), 8.40-8.46 (1H, m), 8.62 (1H, s), 8.83-8.87 (1H, m).

### [Example 109] Synthesis of 1-(piperazin-1-yl)-2-(4-(2-(pyrazolo[1,5-a]pyridin-3-yl)thiazol-4-yl)phenoxy)ethan-1-one (compound 31)

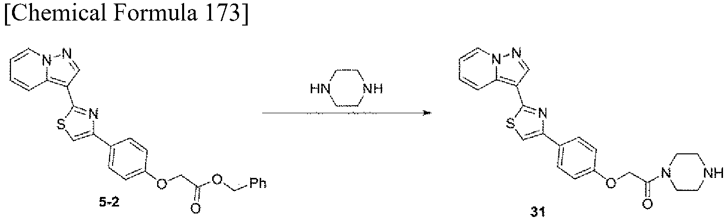

Anhydrous piperazine (3.0 g, 34.8 mmol) was added to benzyl 2-(4-(2-(pyrazolo[1,5-a]pyridin-3-yl)thiazol-4-yl)phenoxy)acetate (compound 5-2) (0.3 g, 0.68 mmol), and the mixture was heated and stirred at 120°C for 1 hour. Water (50 mL) and then saturated saline (5 mL) were added, and the mixture was extracted with chloroform, then dried over anhydrous magnesium sulfate. After filtration, the solvent was removed under reduced pressure, and the residue was dried to obtain the title compound (31) (0.23 g, 0.55 mmol, 80.9% yield).

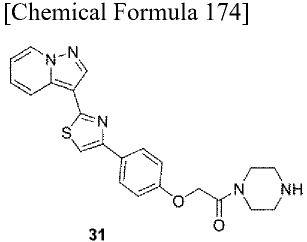

¹H-NMR(DMSO-d₆)δ:3.10-3.20 (4H, m), 3.70-3.80 (4H, m), 5.00 (2H, s), 7.07 (2H, d, J=8.7Hz), 7.11-7.16 (1H, m), 7.57-7.61 (1H, m), 7.89 (1H, s), 8.02 (2H, d, J=9.1Hz), 8.44 (1H, d, J=8.7Hz), 8.64 (1H, s), 8.87 (1H, d, J=6.9Hz).

### [Examples 110 and 111] Synthesis of 1-(1,4-diazepan-1-yl)-2-(4-(2-(pyrazolo[1,5-a]pyridin-3-yl)thiazol-4-yl)phenoxy)ethan-1-one hydrochloride (compound 55), and 1-(4-methyl-1,4-diazepan-1-yl)-2-(4-(2-(pyrazolo[1,5-a]pyridin-3-yl)thiazol-4-yl)phenoxy)ethan-1-one hydrochloride (compound 102)

Compound (55) and compound (102) were synthesized from compound (5-2), respectively, according to the method for synthesizing compound (31).

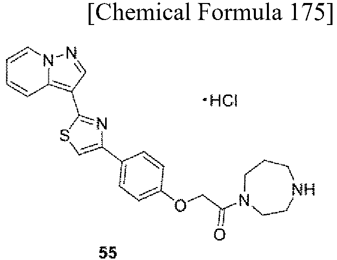

¹H-NMR(DMSO-d₆) δ:3.12-3.82 (10H, m), 4.92 (2H, s), 7.02-7.18 (3H, m), 7.54-7.62 (1H, m), 7.86(1H, s), 8.00 (2H, d, J=8.8Hz), 8.42 (1H, d, J=9.2Hz), 8.62 (1H, s), 8.84 (1H, d, J=7.2Hz).

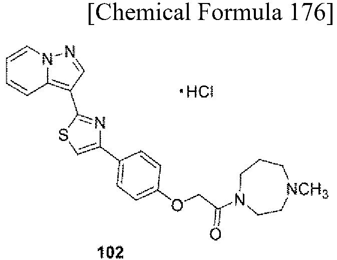

¹H-NMR(DMSO-d₆) δ:2.78-2.80 (2H, m), 3.57 (3H, s), 3.42-3.63 (8H, m), 4.88-4.92 (2H, m), 7.03-7.17 (3H, m), 7.54-7.62 (1H, m), 7.86-7.90 (1H, m), 7.98-8.05 (2H, m), 8.40-8.46 (1H, m), 8.62 (1H, s), 8.84-8.88(1H, m).

[Examples 112 to 114] Synthesis of the following compounds:
1-(2-(hydroxymethyl)pyrrolidin-1-yl)-2-(3-(2-(pyrazolo[1,5-a]pyridin-3-yl)thiazol-4-yl)phenoxy)ethan-1-one (compound 21),
1-(piperazin-1-yl)-2-(3-(2-(pyrazolo[1,5-a]pyridin-3-yl)thiazol-4-yl)phenoxy)ethan-1-one (compound 23), and
1-(1,1-dioxidothiomorpholino)-2-(3-(2-(pyrazolo[1,5-a]pyridin-3-yl)thiazol-4-yl)phenoxy)ethan-1-one (compound 24)

Compound (21), compound (23), and compound (24) were synthesized from compound (19-1), according to the method for synthesizing compound (31).

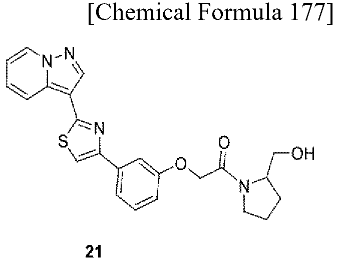

1H-NMR(CDCl₃) δ:1.52-1.68 (1H, m), 1.82-2.12 (3H, m), 3.51-3.66 (2H, m), 3.70-3.79 (2H, m), 4.23-4.32 (1H, m), 4.51-4.59 (1H, m), 4.77 (2H, s), 6.90-6.98 (2H, m), 7.35-7.46 (3H, m), 7.61-7.66 (1H, m), 7.68-7.72 (1H, m), 8.41 (1H, s), 8.46-8.56 (2H, m).

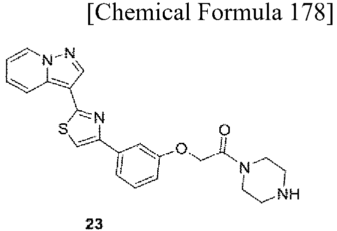

¹H-NMR(DMSO-d₆) δ:2.65-2.76 (4H, m), 3.40-3.44 (4H, m), 4.91 (2H, s), 6.93-6.86 (1H, m), 7.14-7.16 (1H, m), 7.40 (1H, t, J=7.9Hz), 7.60-7.69 (3H, m), 8.05 (1H, s), 8.45-8.47 (1H, d, J=8.9Hz), 8.65 (1H, s), 8.87 (1H, d, J=7.0Hz).

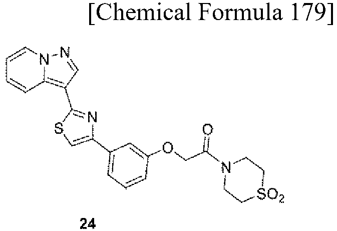

¹H-NMR(DMSO-d₆) δ:3.10-3.20 (2H, m), 3.30-3.46 (2H, m), 3.88-3.94 (4H, m), 5.02 (2H, s), 6.96-7.01 (1H, m), 7.11-7.16 (1H, m), 7.37-7.43 (1H, m), 7.56-7.72 (3H, m), 8.05 (1H, s), 8.41-8.46 (1H, m), 8.64 (1H, s), 8.86 (1H, d, J=6.8Hz).

### [Example 115] Synthesis of (4-methylpiperazin-1-yl)(5-(2-(pyrazolo[1,5-a]pyridin-3-yl)thiazol-4-yl)isoxazol-3-yl)methanone hydrochloride (compound 103)

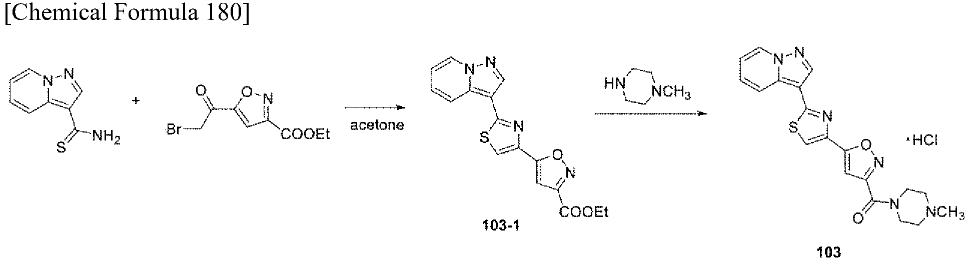

### (1) Synthesis of ethyl 5-(2-(pyrazolo[1,5-a]pyridin-3-yl)thiazol-4-yl)isoxazole-3-carboxylate (compound 103-1)

A solution of pyrazolo[1,5-a]pyridine-3-carbothioamide (0.50 g, 2.82 mmol) and ethyl 5-(2-bromoacetyl)isoxazole-3-carboxylate (0.74 g, 2.82 mmol) in acetone (15 mL) was stirred at room temperature for 4 hours. Water (40 mL) was added, and the precipitate was filtered with suction, washed with methanol and dried to obtain the title compound (103-1) (0.8 g, 1.87 mmol, 66% yield).

¹H-NMR(DMSO-d₆) δ:1.37 (3H, t, J=7.1Hz), 4.45 (2H, q, J=7.1Hz), 7.14-7.18 (1H, m), 7.43 (1H, s), 7.58-7.63 (1H, m), 8.38 (1H, s), 8.48 (1H, t, J=8.8Hz), 8.71 (1H, s), 8.88 (1H, d, J=7.0Hz).

### (2) Synthesis of (4-methylpiperazin-1-yl)(5-(2-(pyrazolo[1,5-a]pyridin-3-yl)thiazol-4-yl)isoxazol-3-yl)methanone hydrochloride (compound 103)

A solution of ethyl 5-(2-(pyrazolo[1,5-a]pyridin-3-yl)thiazol-4-yl)isoxazole-3-carboxylate (compound 103-1) (50 mg, 0.15 mmol) and 1-methylpiperazine (1 mL, 11.0 mmol) in dichloromethane (1 mL) was stirred at room temperature for 16 hours. Water (10 mL) was added to the reaction mixture, the solvent was partially removed under reduced pressure, and the obtained precipitate was filtered with suction and dried to obtain a crude compound. 4 drops of a 4 N hydrochloric acid dioxane solution were added to a solution of the crude compound in dioxane (2 mL), and a precipitate was collected by filtration to obtain the title compound (103) (14 mg, 0.032 mmol, 21.7% yield).

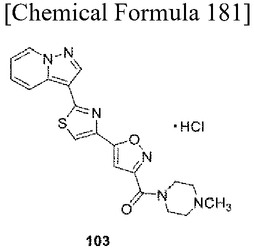

¹H-NMR(DMSO-d₆) δ:2.23 (3H, s), 2.32-2.46 (4H, m), 3.58-3.73 (4H, m), 7.12-7.20 (1H, m), 7.28 (1H, s), 7.56-7.62 (1H, m), 8.32 (1H, s), 8.44-8.52 (1H, m), 8.71 (1H, s), 8.84-8.91 (1H, m).

### [Example 116] Synthesis of piperazin-1-yl(5-(2-(pyrazolo[1,5-a]pyridin-3-yl)thiazol-4-yl)isoxazol-3-yl)methanone hydrochloride (compound 104)

Compound (104) was synthesized from compound (103-1), according to the method for synthesizing compound (103).

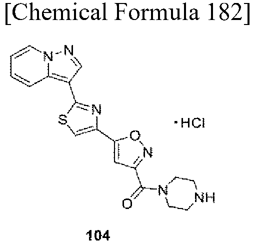

¹H-NMR(DMSO-d₆) δ:3.19-3.30 (4H, m), 3.88-3.96 (4H, m), 7.14-7.21 (1H, m), 7.27-7.32 (1H, m), 7.58-7.66 (1H, m), 8.31-8.36 (1H, m), 8.44 (1H, d, J=8.8Hz), 8.70 (1H, s), 8.86 (1H, d, J=6.8Hz).

### [Example 117] Synthesis of N-(4'-methyl-2-(pyrazolo[1,5-a]pyridin-3-yl)-[4,5'-bithiazole]-2'-yl)acetamide (compound 46)

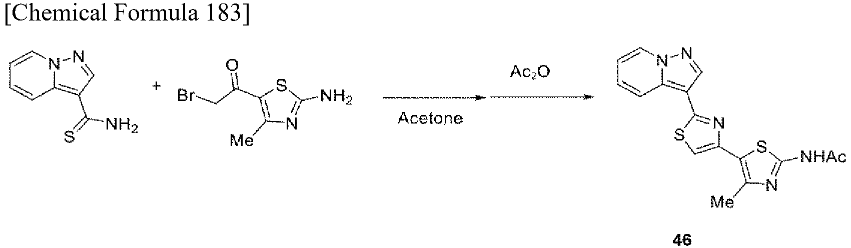

Potassium carbonate (0.1 g, 0.72 mmol) was added to a solution of pyrazolo[1,5-a]pyridine-3-carbothioamide (75 mg, 0.4 mmol) and 1-(2-amino-4-methylthiazol-5-yl)-2-bromoethan-1-one (100 mg, 0.4 mmol) in acetone (3 mL), and the mixture was stirred at room temperature for 12 hours. Water (20 mL) was added, and the resulting precipitate was filtered with suction, washed with methanol and diethyl ether in this order, and dried to obtain a crude compound. Anhydrous DMF (2 mL) was added thereto, followed by the addition of acetic anhydride (1 mL, 10.6 mmol), and the mixture was stirred at room temperature for 2 hours. The reaction mixture was concentrated under reduced pressure, water (20 mL) was added, and the obtained precipitate was collected by suction filtration and dried to obtain the title compound (46) (30.0 mg, 0.08 mmol, 53% yield).

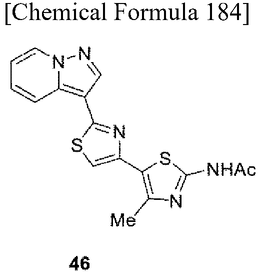

¹H-NMR(DMSO-d₆) δ:2.16 (3H, s), 2.55 (3H, s), 7.10-7.16 (1H, m), 7.57-7.64 (2H, m), 8.27-8.33 (1H, m), 8.63 (1H, s), 8.82-8.88 (1H, m), 12.13 (1H, s).

### [Example 118] Synthesis of N,N-dimethyl-4-(2-(pyrazolo[1,5-a]pyridin-3-yl)thiazol-4-yl)benzamide (compound 49)

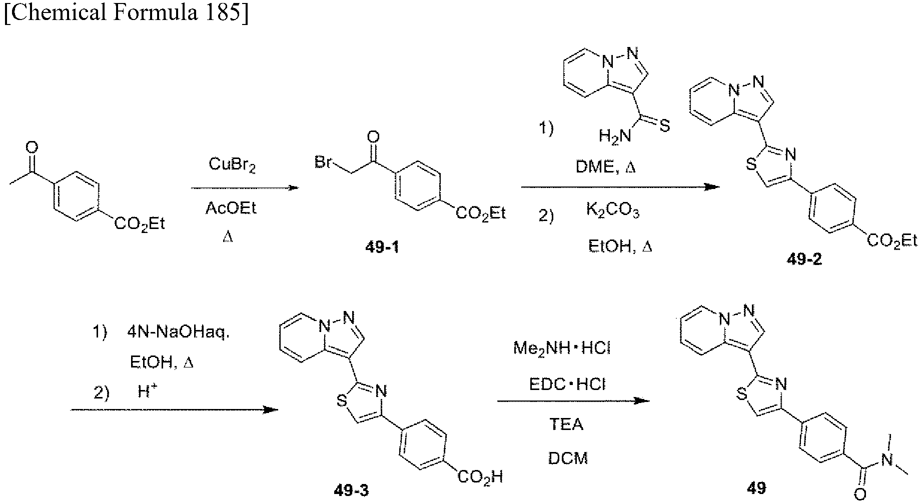

### (1) Synthesis of ethyl 4-(bromoacetyl)benzoate (compound 49-1)

Ethyl 4-acetylbenzoate (5.10 g, 26.5 mmol) was added to ethyl acetate (100 mL), CuBr₂ (12.5 g, 55.9 mmol) was added thereto, and the mixture was refluxed for 16 hours. After cooling, the insoluble matter was filtered through celite, the organic solvent was removed under reduced pressure, and the residue was purified by silica gel column chromatography to obtain the title compound (49-1) (7.11 g, 26.2 mmol, 98.9% yield) from a fraction of n-hexane : ethyl acetate (4 : 1).

¹H-NMR(CDCl₃) δ:1.41 (3H, t, J=6.9Hz), 4.40 (2H, d, J=6.9Hz), 4.46 (2H, s), 8.03 (2H, d, J=8.7Hz), 8.14 (2H, d, J=8.7Hz).

### (2) Synthesis of ethyl 4-(2-(pyrazolo[1,5-a]pyridin-3-yl)thiazol-4-yl)benzoate (compound 49-2)

Pyrazolo[1,5-a]pyridine-3-carbothioamide (711 mg, 4.01 mmol) was dissolved in DME (40 mL), and ethyl 4-(bromoacetyl)benzoate (compound 49-1) (1.08 g, 3.99 mmol) was added thereto under ice cooling, then the mixture was stirred at room temperature for 16 hours. The reaction mixture was heated and stirred at 50°C for 1 hour and then allowed to cool, and the reaction mixture was concentrated under reduced pressure to remove the solvent. Ethanol (40 mL) was added to the residue, then excess potassium carbonate (1.06 g) was added, and the reaction mixture was refluxed at 70°C for 1 hour. After cooling, the reaction mixture was concentrated under reduced pressure to remove the solvent, and the residue was purified by silica gel column chromatography to obtain the title compound (49-2) (1.22 g, 3.50 mmol, 87.4% yield) from a fraction of n-hexane : ethyl acetate (1 : 1).

¹H-NMR(CDCl₃) δ:1.42 (3H, t, J=7.3Hz), 4.40 (2H, q, J=7.3Hz), 6.94 (1H, dt, J=1.4Hz, 6.9Hz), 7.42 (1H, t, J=8.5Hz), 7.50 (1H, s), 8.08 (2H, d, J=8.7Hz), 8.14 (2H, d, J=8.7Hz), 8.40 (1H, s), 8.47 (1H, d, J=9.2Hz), 8.53 (1H, d, J=6.9Hz).

### (3) Synthesis of 4-(2-(pyrazolo[1,5-a]pyridin-3-yl)thiazol-4-yl)benzoic acid (compound 49-3)

Ethyl 4-(2-(pyrazolo[1,5-a]pyridin-3-yl)thiazol-4-yl)benzoate (compound 49-2) (2.63 g, 7.51 mmol) was added to a mixed solvent of 4 N aqueous sodium hydroxide solution (8 mL) and ethanol (4 mL), and the reaction mixture was heated and stirred at 100°C for 1 hour. After the reaction mixture was allowed to cool, it was poured into ice-water (100 mL), then 2 N hydrochloric acid was added to make the mixture weakly acidic, and the precipitated solid was stirred for some time and then collected by filtration. The solid was thoroughly washed with water and then dried to obtain the title compound (49-3) (2.28 g, 7.08 mmol, 94.3% yield).

¹H-NMR(DMSO-d₆) δ:7.10 (1H, dt, J=1.4Hz, 6.9Hz), 7.57 (1H, ddd, J=0.9Hz, 5.5Hz, 8.7Hz), 8.01 (2H, d, J=8.7Hz), 8.19 (3H, m), 8.42 (1H, d, J=9.2Hz), 8.63 (1H, s), 8.83 (1H, d, J=6.9Hz).

### (4) Synthesis of N,N-dimethyl-4-(2-(pyrazolo[1,5-a]pyridin-3-yl)thiazol-4-yl)benzamide (compound 49)

4-(2-(pyrazolo[1,5-a]pyridin-3-yl)thiazol-4-yl)benzoic acid (compound 49-3) (53.8 mg, 0.167 mmol), dimethylamine hydrochloride (56.2 mg, 0.690 mmol) and EDC hydrochloride (174 mg, 0.908 mmol) were added to anhydrous dichloromethane (10 mL), and an excess of TEA (0.20 mL) was slowly added under ice cooling. The mixture was gradually allowed to return to room temperature and stirred for 16 hours. Water was added to the reaction mixture under ice cooling, the dichloromethane layer was washed with water several times, the dichloromethane layer was dried over anhydrous magnesium sulfate, and after filtration, the solvent was removed under reduced pressure, and the residue was purified by silica gel column chromatography to obtain the title compound(49) (44.8 mg, 0.129 mmol, 75.6% yield) from a fraction of n-hexane : ethyl acetate (1 : 1).

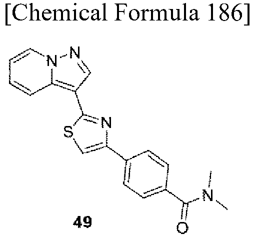

¹H-NMR(CDCl₃) δ:3.03 (3H, s), 3.13 (3H, s), 6.92 (1H, dt, J=1.4Hz, 6.9Hz), 7.39 (1H, ddd, J=1.4Hz, 6.9Hz, 9.2Hz), 7.42 (1H, s), 7.51 (2H, d, J=8.7Hz), 8.04 (2H, d, J=8.7Hz), 8.39 (1H, s), 8.45 (1H, d, J=8.7Hz), 8.51 (1H, d, J=6.9Hz).

### [Examples 119 and 120] Synthesis of sodium 3-(2-(pyrazolo[1,5-a]pyridin-3-yl)thiazol-4-yl)benzoate (compound 6), and (3-(2-(pyrazolo[1,5-a]pyridin-3-yl)thiazol-4-yl)phenyl)(2-oxa-6-azaspiro[3.3]heptan-6-yl)methanone (compound 33)

Compound (6) and compound (33) were synthesized according to the method for synthesizing compound (49).

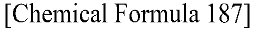

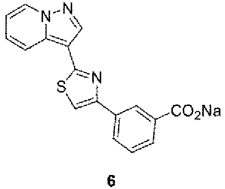

¹H-NMR(D₂O) δ:6.38 (1H, t, J=6.8Hz), 6.65 (1H, s), 6.68-6.74 (1H, m), 6.92 (1H, t, J=7.8Hz), 7.02-7.09 (2H, m), 7.41 (1H, d, J=8.0Hz), 7.57-7.62 (2H, m), 7.74 (1H, d, J=7.2Hz).

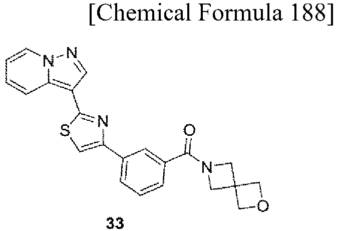

¹H-NMR(CDCl₃) δ:4.39 (2H, s), 4.51 (2H, s), 4.78 (2H, s), 4.83 (2H, s), 6.94 (1H, t, J=6.0Hz), 7.40 (1H, m), 7.42 (1H, s), 7.50 (1H, t, J=7.8Hz), 7.57 (1H, d, J=7.8Hz), 8.12 (1H, d, J=7.8Hz), 8.27 (1H, m), 8.40 (1H, s), 8.45 (1H, d, J=8.7Hz), 8.53 (1H, d, J=6.9Hz).

### [Example 121] Synthesis of N,N-dimethyl-2-(4-(2-(pyrazolo[1,5-a]pyridin-3-yl)thiazol-4-yl)phenyl)acetamide (compound 87)

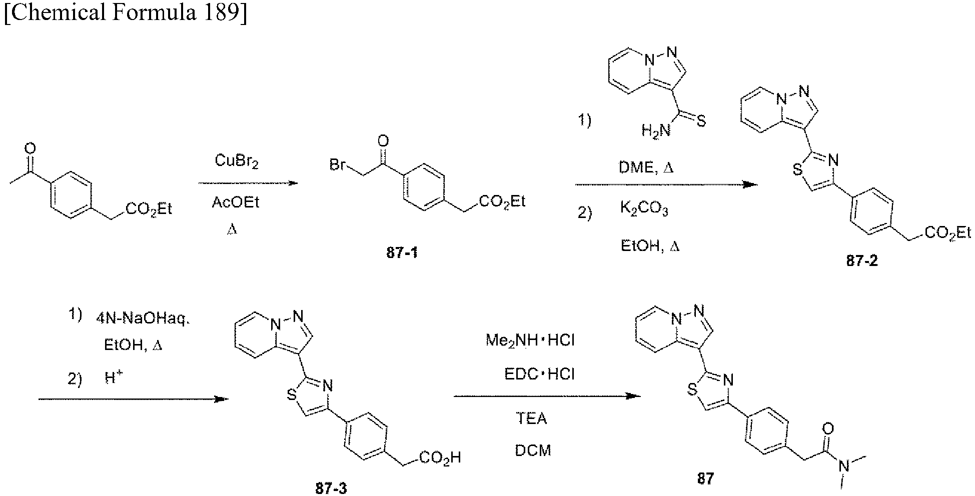

### (1) Synthesis of ethyl 4-(bromoacetyl)phenylacetate (compound 87-1)

Ethyl 4-acetylphenylacetate (425 mg, 2.06 mmol) was added to ethyl acetate (100 mL), CuBr₂ (967 mg, 4.33 mmol) was added thereto, and the mixture was refluxed for 11 hours. After cooling, the insoluble matter was filtered through celite, the organic solvent was removed under reduced pressure, and the residue was purified by silica gel column chromatography to obtain the title compound (87-1) (581 mg, 0.458 mmol, 99.0% yield) from a fraction of n-hexane : ethyl acetate (1 : 1).

¹H-NMR(CDCl₃) δ:1.25 (3H, t, J=7.1Hz), 3.68 (2H, s), 4.15 (2H, d, J=7.1Hz), 4.28 (2H, s), 7.42 (2H, d, J=8.1Hz), 7.94 (2H, d, J=8.5Hz).

### (2) Synthesis of ethyl 2-(4-(2-(pyrazolo[1,5-a]pyridin-3-yl)thiazol-4-yl)phenyl)acetate (compound 87-2)

Pyrazolo[1,5-a]pyridine-3-carbothioamide (379 mg, 2.14 mmol) was dissolved in DME (20 mL), and ethyl 4-(bromoacetyl)phenylacetate (compound 87-1) (621 mg, 2.18 mmol) was added thereto under ice cooling, then the mixture was stirred at room temperature for 16 hours. The reaction mixture was heated and stirred at 50°C for 1 hour and then allowed to cool, and the reaction mixture was concentrated under reduced pressure to remove the solvent. Ethanol (20 mL) was added thereto, then excess potassium carbonate (503 mg) was added thereto, and the reaction mixture was refluxed at 70°C for 1 hour. After cooling, the reaction mixture was concentrated under reduced pressure to remove the solvent, and the residue was purified by silica gel column chromatography to obtain the title compound (87-2) (645 mg, 2.0 mmol, 92.5% yield) from a fraction of n-hexane : ethyl acetate (1 : 1).

¹H-NMR(CDCl₃) δ:1.26 (3H, t, J=7.1Hz), 3.66 (2H, s), 4.16 (2H, q, J=7.1Hz), 6.92 (1H, dt, J=1.4Hz, 6.8Hz), 7.38 (4H, m), 7.97 (2H, d, J=8.4Hz), 8.39 (1H, s), 8.46 (1H, d, J=8.8Hz), 8.51 (1H, d, J=7.0Hz).

### (3) Synthesis of 2-(4-(2-(pyrazolo[1,5-a]pyridin-3-yl)thiazol-4-yl)phenyl)acetic acid (compound 87-3)

Ethyl 2-(4-(2-(pyrazolo[1,5-a]pyridin-3-yl)thiazol-4-yl)phenyl)acetate (compound 87-2) (566 mg, 1.56 mmol) was added to a mixed solvent of a 4 N aqueous sodium hydroxide solution (3 mL) and ethanol (6 mL), and the reaction mixture was heated and stirred at 100°C for 1 hour. After the reaction mixture was allowed to cool, ice water (50 mL) was added thereto, and 2 N hydrochloric acid was added to make the mixture weakly acidic, and the precipitated solid was stirred for some time, then collected by filtration, washed well with water, and then dried to obtain the title compound (87-3) (489 mg, 1.46 mmol, 91.0% yield).

¹H-NMR(CDCl₃) δ:3.72 (2H, s), 6.92 (1H, dt, J=1.3Hz, 6.8Hz), 7.38 (4H, m), 7.99 (2H, d, J=8.4Hz), 8.40 (1H, s), 8.47 (1H, d, J=8.9Hz), 8.54 (1H, d, J=7.0Hz).

### (4) Synthesis of N,N-dimethyl-2-(4-(2-(pyrazolo[1,5-a]pyridin-3-yl)thiazol-4-yl)phenyl)acetamide (compound 87)

2-(4-(2-(pyrazolo[1,5-a]pyridin-3-yl)thiazol-4-yl)phenyl)acetic acid (compound 87-3) (46.3 mg, 0.138 mmol), dimethylamine hydrochloride (58.5 mg, 1.30 mmol) and EDC hydrochloride (129 mg, 0.67 mmol) were added to anhydrous dichloromethane (10 mL), and an excess of triethylamine (0.20 mL) was slowly added thereto under ice cooling. The mixture was gradually allowed to return to room temperature and stirred for 16 hours. Water was added to the reaction mixture under ice cooling, the dichloromethane layer was washed with water several times, the dichloromethane layer was dried over anhydrous magnesium sulfate, and after filtration, the solvent was removed under reduced pressure, and the residue was purified by silica gel column chromatography to obtain the title compound (87) (27.8 mg, 0.0767 mmol, 55.6% yield) from a fraction of n-hexane : ethyl acetate (1 : 1).

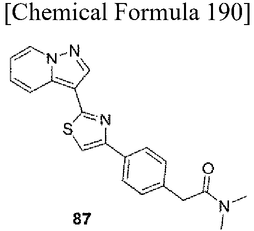

¹H-NMR(CDCl₃) δ:2.98 (3H, s), 3.01 (3H, s), 3.77 (2H, s), 6.92 (1H, t, J=1.4Hz, 6.9Hz), 7.38 (4H, m), 7.96 (2H, d, J=8.2Hz), 8.39 (1H, s), 8.46 (1H, d, J=9.2Hz), 8.52 (1H, d, J=7.3Hz).

[Examples 122 to 128] Synthesis of the following compounds:
N-(cyclopropylmethyl)-1-(4-(2-(pyrazolo[1,5-a]pyridin-3-yl)thiazol-4-yl)benzoyl)azetidine-3-carboxamide (compound 9),
(4-(2-(pyrazolo[1,5-a]pyridin-3-yl)thiazol-4-yl)phenyl)(2-oxa-7-azaspiro[3.5]nonan-7-yl)methanone (compound 40),
(1,4-oxazepan-4-yl)(4-(2-(pyrazolo[1,5-a]pyridin-3-yl)thiazol-4-yl)phenyl)methanone (compound 41),
(4-(dimethylamino)piperidin-1-yl)(4-(2-(pyrazolo[1,5-a]pyridin-3-yl)thiazol-4-yl)phenyl)methanone (compound 48),
(4-methylpiperazin-1-yl)(4-(2-(pyrazolo[1,5-a]pyridin-3-yl)thiazol-4-yl)phenyl)methanone (compound 50),
(4-ethylpiperazin-1-yl)(4-(2-(pyrazolo[1,5-a]pyridin-3-yl)thiazol-4-yl)phenyl)methanone (compound 56), and
(3-methylpiperazin-1-yl)(4-(2-(pyrazolo[1,5-a]pyridin-3-yl)thiazol-4-yl)phenyl)methanone (compound 58)

Compound (9), compound (40), compound (41), compound (48), compound (50), compound (56), and compound (58) were synthesized from compound (49-3), according to the method for synthesizing compound (49).

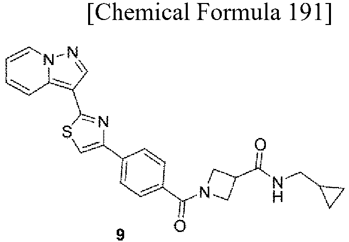

¹H-NMR(CDCl₃) δ:0.21 (2H, m), 0.53 (2H, m), 0.95 (1H, m), 3.15 (2H, dd, J=5.5Hz, 7.3Hz), 3.33 (1H, m), 4.38 (3H, m), 4.66 (1H, t, J=6.9Hz), 5.70 (1H, brs), 6.93 (1H, dt, J=1.4Hz, 7.3Hz), 7.40 (1H, ddd, J=1.4Hz, 7.3Hz), 7.45 (1H, s), 7.73 (2H, d, J=8.2Hz), 8.04 (2H, d, J=8.7Hz), 8.39 (1H, s), 8.44 (1H, d, J=8.7Hz), 8.53 (1H, d, J=6.9Hz).

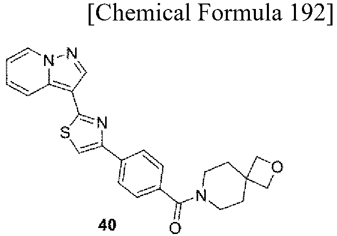

¹H-NMR(CDCl₃) δ:1.91 (4H, m), 3.40 (2H, m), 3.68 (2H, m), 4.48 (4H, s), 6.93 (1H, dt, J=1.4Hz, 6.9Hz), 7.39 (1H, dd, J=7.8Hz, 8.8Hz), 7.42 (1H, s), 7.48 (2H, d, J=8.2Hz), 8.04 (2H, d, J=8.2Hz), 8.40 (1H, s), 8.44 (1H, d, J=9.2Hz), 8.52 (1H, d, J=6.9Hz).

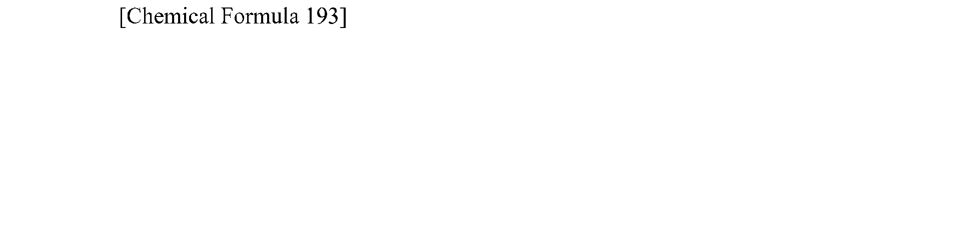

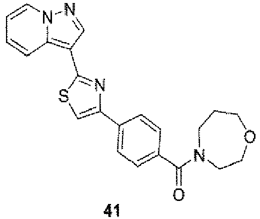

¹H-NMR(CDCl₃) δ:1.84 (1H, m), 2.07 (1H, m), 3.57 (2H, m), 3.67 (1H, m), 3.84 (5H, m), 6.93 (1H, dt, J=0.9Hz, 6.9Hz), 7.39 (1H, m), 7.42 (1H, s), 7.49 (2H, m), 8.05 (2H, d, J=7.8Hz), 8.40 (1H, s), 8.46 (1H, d, J=9.2Hz), 8.53 (1H, d, J=6.9Hz).

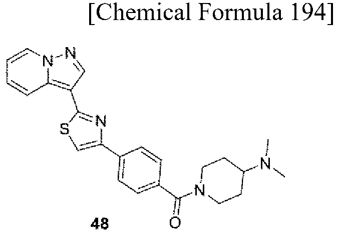

¹H-NMR(CDCl₃) δ:1.45 (2H, m), 1.85 (2H, m), 2.29 (6H, s), 2.38 (2H, m), 2.84 (1H, m), 3.05 (1H, m), 3.20 (1H, m), 6.93 (1H, dt J=1.4Hz, 6.9Hz), 7.40 (1H, ddd, J=1.4Hz, 6.9Hz, 9.2Hz), 7.42 (1H, s), 7.49 (2H, d, J=8.7Hz), 8.04 (2H, d, J=8.2Hz), 8.39 (1H, s), 8.45 (1H, d, J=8.7Hz), 8.53 (1H, d, J=6.9Hz).

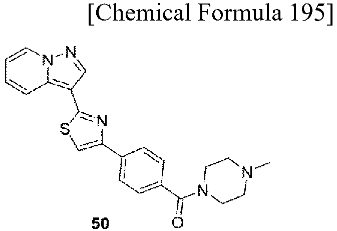

¹H-NMR(CDCl₃) δ:2.33 (3H, s), 2.45 (4H, m), 3.67 (4H, m), 6.93 (1H, dt, J=1.4Hz, 6.9Hz), 7.40 (1H, ddd, J=1.4Hz, 6.9Hz, 9.2Hz), 7.42 (1H, s), 7.50 (2H, d, J=8.2Hz), 8.05 (2H, d, J=8.2Hz), 8.39 (1H, s), 8.45 (1H, d, J=9.2Hz), 8.52 (1H, d, J=7.8Hz).

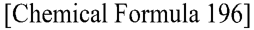

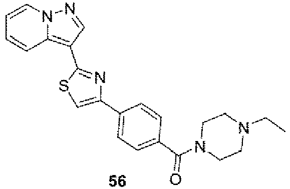

¹H-NMR(CDCl₃) δ:1.09 (3H, t, J=7.3Hz), 2.52(4H, m), 2.46 (2H, q, J=7.3Hz), 3.68 (4H, m), 6.93 (1H, dt, J=1.4Hz, 6.9Hz), 7.39 (1H, ddd, J=0.9Hz, 6.9Hz, 8.7Hz), 7.42 (1H, s), 7.50 (2H, d, J=8.2Hz), 8.04 (2H, d, J=8.2Hz), 8.39 (1H, s), 8.44 (1H, d, J=9.2Hz), 8.53 (1H, d, J=7.3Hz).

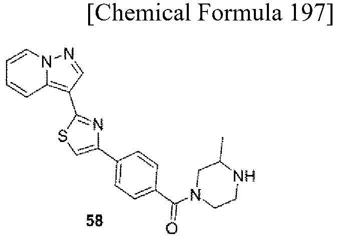

¹H-NMR(CDCl₃) δ:1.24 (3H, m), 2.99 (5H, m), 3.79 (1H, m), 4.67 (1H, m), 6.94 (1H, dt, J=1.4Hz, 6.9Hz), 7.40 (1H, ddd, J=0.9Hz, 6.9Hz, 8.7Hz ), 7.43 (1H, s), 7.50 (2H, d, J=8.2Hz), 8.06 (2H, d, J=8.2Hz), 8.40 (1H, s), 8.46 (1H, d, J=8.9Hz), 8.53 (1H, d, J=6.9Hz).

[Examples 129 to 131] Synthesis of the following compounds:
N,N-dimethyl-3-(2-(pyrazolo[1,5-a]pyridin-3-yl)thiazol-4-yl)benzamide (compound 61),
4-(3-(2-(pyrazolo[1,5-a]pyridin-3-yl)thiazol-4-yl)benzoyl)piperazine-1-carbaldehyde (compound 68), and
N-(2-morpholinoethyl)-3-(2-(pyrazolo[1,5-a]pyridin-3-yl)thiazol-4-yl)benzamide (compound 30)

Compound (61), compound (68), and compound (30) were synthesized from compound (6), according to the method for synthesizing compound (49) from compound (49-3).

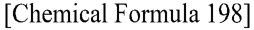

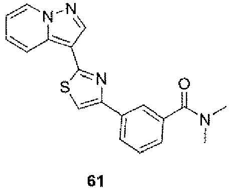

¹H-NMR(CDCl₃) δ:3.02 (3H, s), 3.15 (3H, s), 6.92 (1H, dt, J=1.4Hz, 6.9H), 7.35(1H, s), 7.38 (3H, m), 7.48 (1H, t, J=8.2Hz), 8.05 (1H, m), 8.40 (1H, s), 8.44 (1H, d, J=8.7Hz), 8.51 (1H, d, J=6.9Hz).

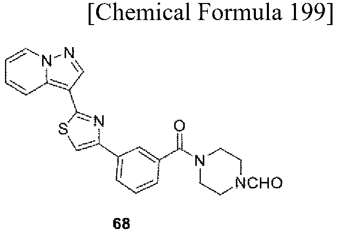

¹H-NMR(CDCl₃) δ:3.65 (8H, m), 6.92 (1H, t, J=6.9Hz), 7.39 (3H, m), 7.49 (1H, d, J=6.9Hz), 8.07 (2H, m), 8.39 (1H, s), 8.43 (1H, t, J=8.7Hz), 8.52 (2H, d, J=6.9Hz).

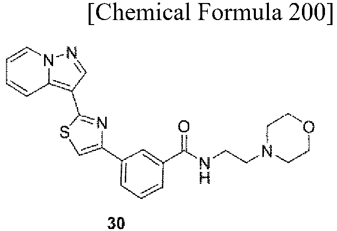

¹H-NMR(D₂O) δ:3.17 (2H, m), 3.31 (2H, t, J=5.7Hz), 3.56 (2H, m), 3.65 (2H, t, J=6.0Hz), 3.75 (2H, m), 4.03 (2H, m), 6.72(1H, t, J=6.6Hz), 7.14 (3H, m), 7.35 (1H, d, J=8.2Hz), 7.48 (1H, d, J=7.8Hz), 7.52 (1H, d, J=8.7Hz), 7.59 (1H, brs), 7.95 (1H, s), 8.08 (1H, d, J=6.9Hz).

[Examples 132 to 134] Synthesis of the following compounds:
N-methyl-2-(4-(2-(pyrazolo[1,5-a]pyridin-3-yl)thiazol-4-yl)phenyl)acetamide (compound 88),
N-ethyl-2-(4-(2-(pyrazolo[1,5-a]pyridin-3-yl)thiazol-4-yl)phenyl)acetamide (compound 89), and
N-methoxy-2-(4-(2-(pyrazolo[1,5-a]pyridin-3-yl)thiazol-4-yl)phenyl)acetamide (compound 91)

Compound (88), compound (89), and compound (91) were synthesized from compound (87-3), according to the method for synthesizing compound (87).

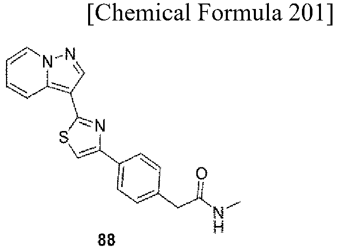

¹H-NMR(CDCl₃) δ:2.77 (3H, d, J=5.0Hz), 3.64 (2H, s), 5.37 (1H, brs), 6.92 (1H, t, J=6.9Hz), 7.37 (4H, m), 8.00 (2H, d, J=8.2Hz), 8.40 (1H, s), 8.45 (1H, d, J=9.2Hz), 8.53 (1H, d, J=6.9Hz).

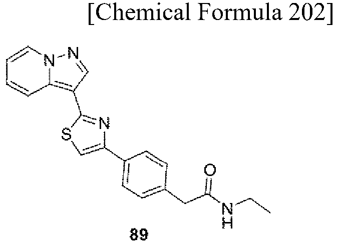

¹H-NMR(CDCl₃) δ:1.06 (3H, t, J=7.3Hz), 3.26 (2H, q, J=7.3Hz), 3.62 (2H, s), 5.36 (1H, brs), 6.93 (1H, dt, J=1.4Hz, 6.9Hz), 7.38 (4H, m), 8.00 (2H, d, J=8.5Hz), 8.40 (1H, s), 8.45 (1H, d, J=8.2Hz), 8.52 (1H, d, J=7.3Hz).

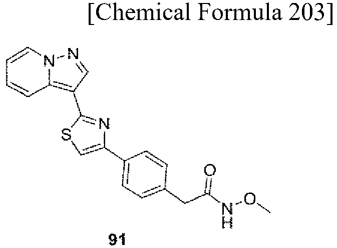

¹H-NMR(CDCl₃) δ:3.60 (3H, s), 3.73 (2H, s), 6.93 (1H, t, J=6.9Hz), 7.38 (4H, m), 7.98 (2H, d, J=8.2Hz), 8.06 (1H, brs), 8.39 (1H, s), 8.45 (1H, d, J=8.7Hz), 8.54 (1H, d, J=6.9Hz).

### [Example 135] Synthesis of (2,5-diazabicyclo[2.2.1]heptan-2-yl)(4-(2-(pyrazolo[1,5-a]pyridin-3-yl)thiazol-4-yl)phenyl)methanone hydrochloride (compound 43)

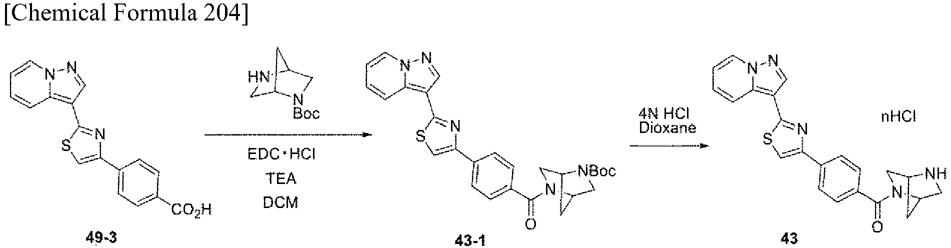

### (1) Synthesis of tert-butyl 5-(4-(2-(pyrazolo[1,5-a]pyridin-3-yl)thiazol-4-yl)benzoyl)-2,5-diazabicyclo[2.2.1]heptane-2-carboxylate (compound 43-1)

4-(2-(pyrazolo[1,5-a]pyridin-3-yl)thiazol-4-yl)benzoic acid (compound 49-3) (53.9 mg, 0.166 mmol), N-(tert-butoxycarbonyl)-2,5-azabicyclo[2.2.1]heptane (53.2 mg, 0.268 mmol) and EDC hydrochloride (141 mg, 0.733 mmol) were added to anhydrous dichloromethane (10 mL), and an excess of TEA (0.50 mL) was slowly added under ice cooling. The mixture was gradually allowed to return to room temperature and stirred for 16 hours. Water was added to the reaction mixture under ice cooling, the dichloromethane layer was washed several times with water, and dried over anhydrous magnesium sulfate. After filtration, the solvent was removed under reduced pressure, and the residue was purified by silica gel column chromatography to obtain the title compound (43-1) (37.2 mg, 0.0742 mmol, 43.6% yield) from a fraction of n-hexane : ethyl acetate (1 : 1).

¹H-NMR(CDCl₃) δ:1.43+1.49 (9H, s x2), 1.87 (2H, m), 3.59 (4H, m), 4.55 (2H, m), 6.93 (1H, t, J=6.9Hz), 7.40 (1H, t, J=8.2Hz), 7.44 (1H, s), 7.61 (2H, t, J=8.2Hz), 8.03+8.07 (2H, d x2, J=8.2Hz), 8.39 (1H, s), 8.45 (1H, d, J=9.2Hz), 8.52 (1H, d, J=7.3Hz).

### (2) Synthesis of (2,5-diazabicyclo[2.2.1]heptan-2-yl)(4-(2-(pyrazolo[1,5-a]pyridin-3-yl)thiazol-4-yl)phenyl)methanone hydrochloride (compound 43)

Under ice cooling, tert-butyl 5-(4-(2-(pyrazolo[1,5-a]pyridin-3-yl)thiazol-4-yl)benzoyl)-2,5-diazabicyclo[2.2.1]heptane-2-carboxylate (compound 43-1) (32.5 mg, 0.0648 mmol) was slowly added to a solution (1.0 mL) of 4 N hydrogen chloride in dioxane, and the mixture was stirred at room temperature for 2 hours. The reaction mixture was concentrated under reduced pressure to remove the solvent, and the residue was dried. Then, a solvent was added, and the solid was collected by filtration, washed thoroughly with the solvent, and dried to obtain the title compound (43) (28.1 mg, 0.281 mmol, 91.3% yield).

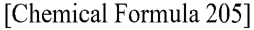

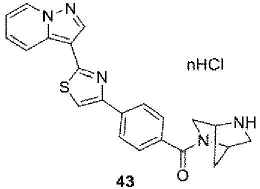

¹H-NMR(D₂O) δ:2.05 (2H, m), 3.35 (1H, m), 3.51 (2H, m), 3.70 (1H, m), 4.54 (2H, m), 6.70 (1H, t, J=6.6Hz), 7.09 (1H, m), 7.25 (3H, m), 7.46 (2H, m), 7.52 (1H, m), 7.93 (1H, d, J=6.0Hz), 8.08 (1H, d, J=6.9Hz).

[Examples 136 to 139] Synthesis of the following compounds:
N-(2-(piperazin-1-yl)ethyl)-4-(2-(pyrazolo[1,5-a]pyridin-3-yl)thiazol-4-yl)benzamide hydrochloride (compound 11),
(3-aminopyrrolidin-1-yl)(4-(2-(pyrazolo[1,5-a]pyridin-3-yl)thiazol-4-yl)phenyl)methanone hydrochloride (compound 42),
N-methyl-N-(piperidin-4-yl)-4-(2-(pyrazolo[1,5-a]pyridin-3-yl)thiazol-4-yl)benzamide hydrochloride (compound 44), and
piperazin-1-yl(4-(2-(pyrazolo[1,5-a]pyridin-3-yl)thiazol-4-yl)phenyl)methanone hydrochloride (compound 57)

Compound (11), compound (42), compound (44), and compound (57) were synthesized from compound (49-3), according to the method for synthesizing compound (43).

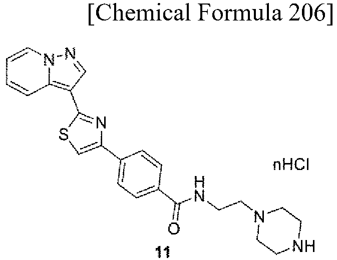

¹H-NMR(D₂O) δ:3.35 (4H, t, J=8.2Hz), 3.45 (4H, t, J=8.6Hz), 3.52 (2H, m), 3.61 (2H, m), 6.59 (1H, t, J=6.9Hz), 7.00 (1H, t, J=8.2Hz), 7.07 (1H, s), 7.12 (2H, d, J=8.2Hz), 7.26 (3H, m), 7.83 (1H, s), 8.90 (1H, d, J=7.3Hz).

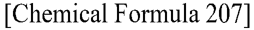

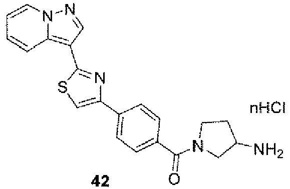

¹H-NMR(D₂O) δ:2.04 (1H, m), 2.31 (1H, m), 3.55 (2H, m), 3.71 (1H, m), 3.89 (2H, m), 6.66 (1H, t, J=6.9Hz), 7.03 (1H, t, J=7.8Hz), 7.09 (1H, d, J=6.5Hz), 7.24 (2H, t, J=8.2Hz), 7.35 (1H, d, J=8.2Hz), 7.43 (2H, m), 7.89 (1H, d, J=10.1Hz), 8.05 (1H, t, J=6.9Hz).

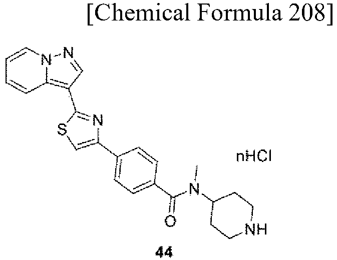

¹H-NMR(D₂O) δ:1.89 (4H, m), 2.65+2.85 (3H, s x2), 3.07 (1H, t, J=11.5Hz), 3.27 (1H, d, J=11.0Hz), 3.48 (1H, d, J=11.9Hz), 3.58 (2H, m), 6.62 (1H, d, J=6.4Hz), 7.10 (6H, m), 7.45 (1H, t, J=9.6Hz), 7.79 (1H, s), 8.02 (1H, d, J=6.4Hz).

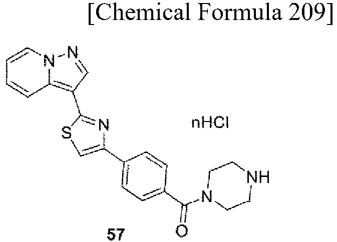

¹H-NMR(D₂O) δ:3.17 (2H, m), 3.30 (2H, m), 3.60 (2H, m), 3.88 (2H, m), 6.69 (1H, t, J=6.9Hz), 7.08 (1H, t, J=6.9Hz), 7.15 (1H, brs), 7.21 (2H, d, J=8.2Hz), 7.41 (2H, d, J=8.2Hz), 7.45 (1H, d, J=9.2Hz), 7.92 (1H, s), 8.06 (1H, d, J=6.9Hz).

### [Example 140] Synthesis of piperazin-1-yl(3-(2-(pyrazolo[1,5-a]pyridin-3-yl)thiazol-4-yl)phenyl)methanone hydrochloride (compound 63)

Compound (63) was synthesized from compound (6), according to the method for synthesizing compound (43).

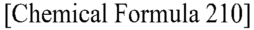

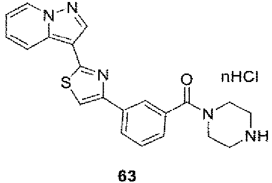

¹H-NMR(D₂O) δ:3.13 (2H, m), 3.30 (2H, m), 3.61 (2H, m), 3.89 (2H, m), 6.66 (1H, dt, J=1.4Hz, 6.9Hz), 7.03 (1H, dd, J=0.9Hz, 6.9Hz), 7.09 (1H, s), 7.22 (2H, m), 7.41 (3H, m), 7.87 (1H, s), 8.03 (1H, d, J=6.9Hz).

### [Example 141] Synthesis of 1-(piperazin-1-yl)-2-(4-(2-(pyrazolo[1,5-a]pyridin-3-yl)thiazol-4-yl)phenyl)ethan-1-one hydrochloride (compound 86)

Compound (86) was synthesized from compound (87-3), according to the method for synthesizing compound (43).

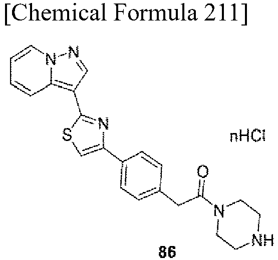

¹H-NMR(D₂O) δ:3.12 (4H, m), 3.63 (2H, s), 3.72 (4H, m), 6.71 (1H, t, J=6.9Hz), 6.94 (2H, d, J=8.2Hz), 7.01 (1H, s), 7.08 (1H, t, J=8.2Hz), 7.24 (2H, d, J=8.2Hz), 7.38 (1H, d, J=9.2Hz), 7.97 (1H, s), 8.08 (1H, d, J=6.9Hz).

### [Example 142] Synthesis of N-(2-(4-(2-(pyrazolo[1,5-a]pyridin-3-yl)thiazol-4-yl)benzoyl)-1,2,3,4-tetrahydroisoquinolin-5-yl)acetamide (compound 60)

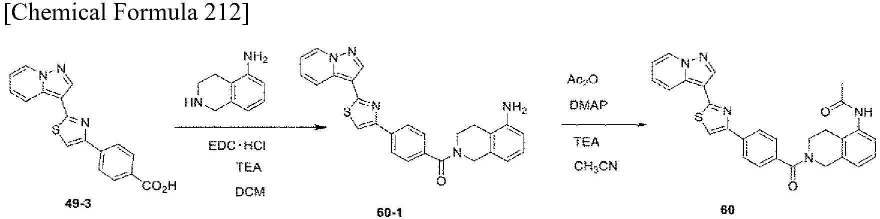

### (1) Synthesis of (5-amino-3,4-dihydroisoquinoline-2(1H)-yl)(4-(2-(pyrazolo[1,5-a]pyridin-3-yl)thiazol-4-yl)phenyl)methanone (compound 60-1)

4-(2-(Pyrazolo[1,5-a]pyridin-3-yl)thiazol-4-yl)benzoic acid (compound 49-3) (53.5 mg, 0.166 mmol), 1,2,3,4-tetrahydroisoquinoline-5-amine (56.5 mg, 0.381 mmol) and EDC hydrochloride (153 mg, 0.800 mmol) were added to anhydrous dichloromethane (10 mL), and an excess of triethylamine (0.20 mL) was slowly added thereto under ice cooling. The mixture was gradually allowed to return to room temperature and stirred for 16 hours. Water was added to the reaction mixture under ice cooling, the dichloromethane layer was washed with water several times, the dichloromethane layer was dried over anhydrous magnesium sulfate, and after filtration, the solvent was removed under reduced pressure, and the residue was purified by silica gel column chromatography to obtain the title compound (60-1) (29.6 mg, 0.0924 mmol, 39.5% yield) from a fraction of n-hexane : ethyl acetate (1 : 1).

¹H-NMR(CDCl₃) δ:2.66 (2H, m), 3.64 (2H, m), 3.74 (1H, m), 4.10 (1H, m), 4.61 (1H, m), 4.68 (1H, m), 6.38 (0.5H, m), 6.59 (1H, d, J=7.8Hz), 6.68 (0.5H, m), 6.94 (1H, dt, J=1.4Hz, 5.5Hz), 7.02 (1H, m), 7.41 (1H, t, J=6.9Hz), 7.44 (1H, s), 7.56 (2H, m), 8.07 (2H, d, J=7.8Hz), 8.41 (1H, s), 8.48 (1H, d, J=9.2Hz), 8.53 (1H, d, J=6.9Hz).

### (2) Synthesis of N-(2-(4-(2-(pyrazolo[1,5-a]pyridin-3-yl)thiazol-4-yl)benzoyl)-1,2,3,4-tetrahydroisoquinolin-5-yl)acetamide (compound 60)

(5-amino-3,4-dihydroisoquinoline-2(1H)-yl)(4-(2-(pyrazolo[1,5-a]pyridin-3-yl)thiazol-4-yl)phenyl)methanone (compound 60-1) (35.2 mg, 0.0780 mmol), acetic anhydride (0.10 mL), excess TEA (0.20 mL), and a catalytic amount of DMAP (5.1 mg) were added to an acetonitrile solution (10 mL) under water cooling, and the mixture was stirred at room temperature for 16 hours. The reaction mixture was concentrated under reduced pressure to remove the solvent, and the residue was purified by silica gel column chromatography to obtain the title compound (60) (26.5 mg, 0.537 mmol, 74.6% yield) from a fraction of n-hexane : ethyl acetate (1 : 1).

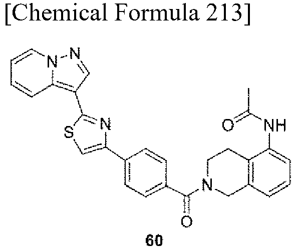

¹H-NMR(CDCl₃) δ:2.20 (3H, s), 2.80 (2H, m), 3.69 (1H, m), 4.02 (1H, m), 4.66 (1H, m), 4.90 (1H, m), 6.94 (1H, t, J=8.2Hz), 7.16 (2H, m), 7.41 (1H, t, J=7.8Hz), 7.43 (1H, s), 7.53 (2H, m), 7.60 (1H, m), 8.06 (2H, d, J=8.7Hz), 8.40 (1H, s), 8.46 (1H, d, J=8.7Hz), 8.53 (1H, d, J=6.9Hz).

### [Example 143] Synthesis of 2-(hydroxymethyl)-4-(2-(pyrazolo[1,5-a]pyridin-3-yl)thiazol-4-yl)phenol (compound 8)

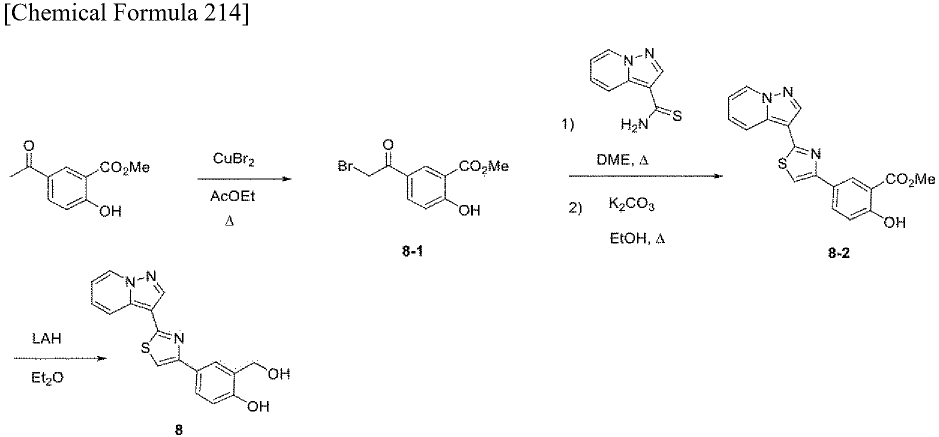

### (1) Synthesis of methyl 5-(2-bromoacetyl)-2-hydroxybenzoate (compound 8-1)

Methyl 5-acetylsalicylate (1.01 g, 5.19 mmol) was added to ethyl acetate (50 mL), CuBr₂ (2.35 g, 10.5 mmol) was added thereto, and the mixture was refluxed for 3 hours. After cooling, the insoluble matter was filtered through celite, the organic solvent was removed under reduced pressure, and the residue was purified by silica gel column chromatography to obtain the title compound (8-1) (1.37 g, 4.98 mmol, 95.8% yield) from a fraction of n-hexane : ethyl acetate (1 : 4).

¹H-NMR(CDCl₃) δ:4.00 (3H, s), 4.39 (2H, s), 7.06 (1H, t, J=8.7Hz), 8.10 (1H, dd, J=2.3Hz, 9.1Hz), 8.51 (1H, d, J=2.3Hz), 11.35 (1H, s).

### (2) Synthesis of methyl 2-hydroxy-5-(2-(pyrazolo[1,5-a]pyridin-3-yl)thiazol-4-yl)benzoate (compound 8-2)

Pyrazolo[1,5-a]pyridine-3-carbothioamide (96.1 mg, 0.542 mmol) was dissolved in DME (20 mL), methyl 5-(2-bromoacetyl)-2-hydroxybenzoate (compound 8-1) (146 mg, 0.534 mmol) was added thereto under ice cooling, and the mixture was stirred at room temperature for 16 hours. The reaction mixture was heated and stirred at 50°C for 1 hour and then allowed to cool, and the reaction mixture was concentrated under reduced pressure to remove the solvent. Ethanol (20 mL) was added thereto, then excess potassium carbonate (246 mg) was added thereto, and the reaction mixture was refluxed at 70°C for 1 hour. After cooling, the reaction mixture was concentrated under reduced pressure to remove the solvent, and the residue was purified by silica gel column chromatography to obtain the title compound (8-2) (76.5 mg, 0.218 mmol, 40.2% yield) from a fraction of n-hexane : ethyl acetate (1 : 1).

¹H-NMR(CDCl₃) δ:4.01 (3H, s), 6.93 (1H, t, J=6.9Hz), 7.08 (1H, d, J=8.7Hz), 7.28 (1H, s), 7.39 (1H, t, J=7.8Hz), 8.14 (1H, dd, J=2.3Hz, 8.7Hz), 8.43 (3H, m), 8.52 (1H, d, J=6.9Hz).

### (3) Synthesis of 2-(hydroxymethyl)-4-(2-(pyrazolo[1,5-a]pyridin-3-yl)thiazol-4-yl)phenol (compound 8)

Excess LAH (138 mg) was carefully added to dry diethyl ether (10 mL), and methyl 2-hydroxy-5-(2-(pyrazolo[1,5-a]pyridin-3-yl)thiazol-4-yl)benzoate (compound 8-2) (98.4 mg, 0.28 mmol) was slowly added at -20°C. The mixture was gradually allowed to return to room temperature and stirred for 16 hours. The reaction mixture was ice-cooled, and a saturated aqueous Na₂SO₄ solution was added thereto. The precipitated insoluble matter was filtered and washed with ethyl acetate. The organic layers were combined and dried over anhydrous magnesium sulfate, and after filtration, the solvent was removed under reduced pressure, and the residue was purified by silica gel column chromatography to obtain the title compound (8) (6.5 mg, 0.0201 mmol, 7.2% yield) from a fraction of n-hexane : ethyl acetate (1 : 4).

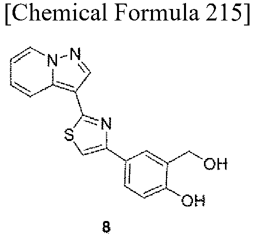

¹H-NMR(CDCl₃) δ:4.97 (2H, d, J=5.5Hz), 6.91 (1H, t, J=5.5Hz), 6.97 (1H, d, J=8.7Hz), 7.20 (1H, s), 7.37 (1H, t, J=7.3Hz), 7.50 (1H, brs), 7.70 (1H, d, J=2.3Hz), 7.83 (1H, dd, J=2.3Hz, 8.7Hz), 8.38 (1H, s), 8.41 (1H, d, J=8.7Hz), 8.50 (1H, d, J=6.9Hz).

### [Examples 144 and 145] Synthesis of (4-(2-(pyrazolo[1,5-a]pyridin-3-yl)thiazol-4-yl)phenyl)methanol (compound 2) and 2-(4-(2-(pyrazolo[1,5-a]pyridin-3-yl)thiazol-4-yl)phenyl)ethan-1-ol (compound 84)

Compound (2) was synthesized from compound (49-2), and compound (84) was synthesized from compound (87-2), according to the method for synthesizing compound (8) from compound (8-2).

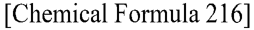

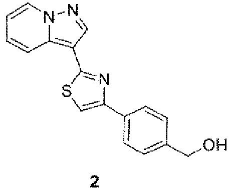

¹H-NMR(CDCl₃) δ:1.68 (1H, t, J=6.0Hz), 4.75 (2H, d, J=5.5Hz), 6.92 (1H, t, J=6.9Hz), 7.36 (1H, s), 7.38 (1H, dd, J=2.3Hz, 6.9Hz), 7.45 (2H, d, J=8.2Hz), 8.00 (2H, d, J=7.8Hz), 8.39 (1H, s), 8.46 (1H, d, J=9.2Hz), 8.52 (1H, d, J=7.3Hz).

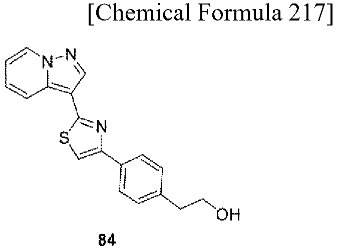

¹H-NMR(CDCl₃) δ:2.92 (2H, t, J=6.4Hz), 3.90 (2H, t, J=5.5Hz), 6.92 (1H, dt, J=1.4Hz, 6.9Hz), 7.33 (3H, m), 7.38 (1H, ddd, J=0.9Hz, 6.9Hz, 7.8Hz), 7.96 (2H, d, J= 8.2Hz), 8.39 (1H, s), 8.46 (1H, d, J=8.7Hz), 8.52 (1H, d, J=7.3Hz).

### [Example 146] Synthesis of 4-(3-(2,5-dihydro-1H-pyrrol-3-yl)phenyl)-2-(pyrazolo[1,5-a]pyridin-3-yl)thiazole hydrochloride (compound 17)

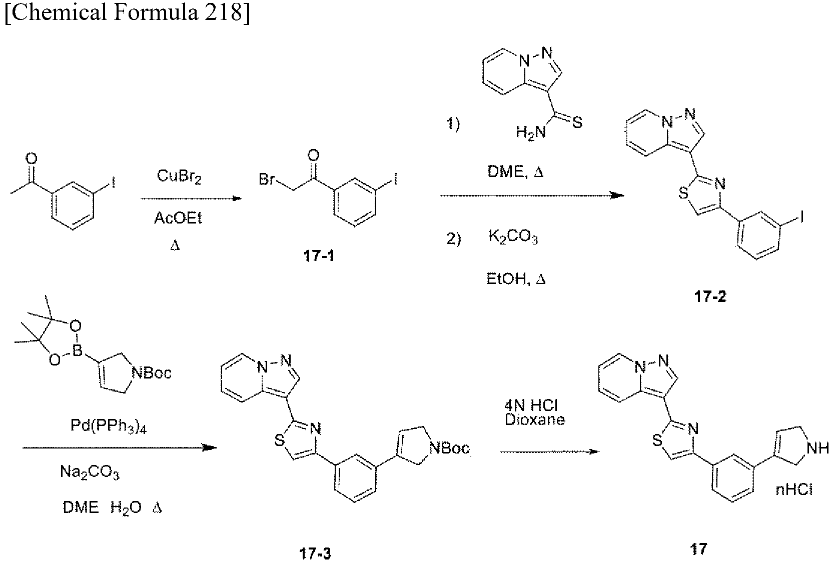

### (1) Synthesis of 3-iodophenacyl bromide (compound 17-1)

3-Iodoacetophenone (5.17 g, 21.0 mmol) was added to ethyl acetate (200 mL), CuBr₂ (12.1 g, 58.4 mmol) was added thereto, and the mixture was refluxed for 3 hours. After cooling, the insoluble matter was filtered through celite, the organic solvent was removed under reduced pressure, and the residue was purified by silica gel column chromatography to obtain the title compound (17-1) (3.69 g, 11.7 mmol, 54.1% yield) from a fraction of n-hexane : ethyl acetate (1 : 1).

¹H-NMR(CDCl₃) δ:4.40 (2H, s), 7.25 (2H, m), 7.94 (1H, dd, J=0.9Hz, 8.2Hz), 8.30 (1H, d, J=1.4Hz).

### (2) Synthesis of 4-(3-iodophenyl)-2-(pyrazolo[1,5-a]pyridin-3-yl)thiazole (compound 17-2)

Pyrazolo[1,5-a]pyridine-3-carbothioamide (273 mg, 1.54 mmol) was dissolved in DME (15 mL), and 3-iodophenacyl bromide (compound 17-1) (494 mg, 1.52 mmol) was added thereto under ice cooling, then the mixture was stirred at room temperature for 16 hours. The reaction mixture was heated and stirred at 50°C for 1 hour and then allowed to cool, and the reaction mixture was concentrated under reduced pressure to remove the solvent. Ethanol (15 mL) was added to the residue, then potassium carbonate (503 mg) was added, and the reaction mixture was refluxed at 70°C for 1 hour. After cooling, the reaction mixture was concentrated under reduced pressure to remove the solvent, and the residue was purified by silica gel column chromatography to obtain the title compound (17-2) (355 mg, 0.880 mmol, 57.9% yield) from a fraction of n-hexane : ethyl acetate (1 : 1).

¹H-NMR(CDCl₃) δ:6.94 (1H, dt, J=1.4Hz, 6.7Hz), 7.18 (1H, t, J=8.7Hz), 7.37 (1H, s), 7.41 (1H, ddd, J=1.4Hz, 6.9Hz, 9.2Hz), 7.67 (1H, dt, J=1.8Hz, 7.8Hz), 7.96 (1H, dt, J=1.8Hz, 8.7Hz), 8.35 (1H, t, J=1.8Hz), 8.40 (1H, s), 8.43 (1H, d, J=7.3Hz), 8.52 (1H, d, J=6.9Hz).

### (3) Synthesis of tert-butyl 3-(3-(2-(pyrazolo[1,5-a]pyridin-3-yl)thiazol-4-yl)phenyl)-2,5-dihydro-1H-pyrrole-1-carboxylate (compound 17-3)

4-(3-Iodophenyl)-2-(pyrazolo[1,5-a]pyridin-3-yl)thiazole (compound 17-2) (50.6 mg, 0.125 mmol), 1-(tert-butoxycarbonyl)-2,5-dihydro-1H-pyrrol-3-yl-boronic acid pinacol ester (55.3 mg, 0.187 mmol), a catalytic amount of Pd(PPh₃)₄ (16.6 mg), and an excess of sodium carbonate (162 mg) were dissolved in a mixed solvent of DME (4 mL) and water (1 mL), and the mixture was refluxed for 1 hour. After cooling, the reaction mixture was concentrated under reduced pressure to remove the solvent, and the residue was purified by silica gel column chromatography to obtain the title compound (17-3) (21.2 mg, 0.0467 mmol, 25.5% yield) from a fraction of n-hexane : ethyl acetate (1 : 1).

¹H-NMR(CDCl₃) δ:1.53 (9H, s), 4.35 (2H, m), 4.58 (2H, m), 6.25 (1H, m), 6.93 (1H, t, J=6.9Hz), 7.41 (4H, m), 7.94 (1H, t, J=8.2Hz), 8.00 (1H, d, J=5.0Hz), 8.41 (1H, s), 8.43 (1H, d, J=9.2Hz), 8.53(1H, d, J=6.9Hz).

### (4) Synthesis of 4-(3-(2,5-dihydro-1H-pyrrol-3-yl)phenyl)-2-(pyrazolo[1,5-a]pyridin-3-yl)thiazole hydrochloride (compound 17)

Under ice cooling, tert-butyl 3-(3-(2-(pyrazolo[1,5-a]pyridin-3-yl)thiazol-4-yl)phenyl)-2,5-dihydro-1H-pyrrole-1-carboxylate (compound 17-3) (16.7 mg, 0.0378 mmol) was slowly added to a 4 N hydrochloric acid dioxane solution (1.0 mL), and the mixture was stirred at room temperature for 2 hours. The solvent was concentrated under reduced pressure to remove the solvent, and the residue was dried. A solvent was added, and the resulting solid was collected by filtration, washed with the solvent, and then dried to obtain the title compound (17) (12.8 mg, 0.308 mmol, 81.5% yield).

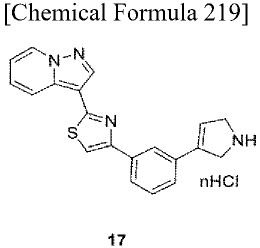

¹H-NMR(D₂O) δ:4.02 (4H, m), 5.81 (1H, s), 6.58 (1H, t, J=6.7Hz), 6.74 (3H, m), 6.91 (3H, m), 7.08 (1H, d, J=8.7Hz), 7.72 (1H, s), 7.89 (1H, d, J=6.9Hz).

### [Example 147] Synthesis of 6-(4-(2-(pyrazolo[1,5-a]pyridin-3-yl)thiazol-4-yl)benzyl)-2-oxa-6-azaspiro[3.3]heptane (compound 29)

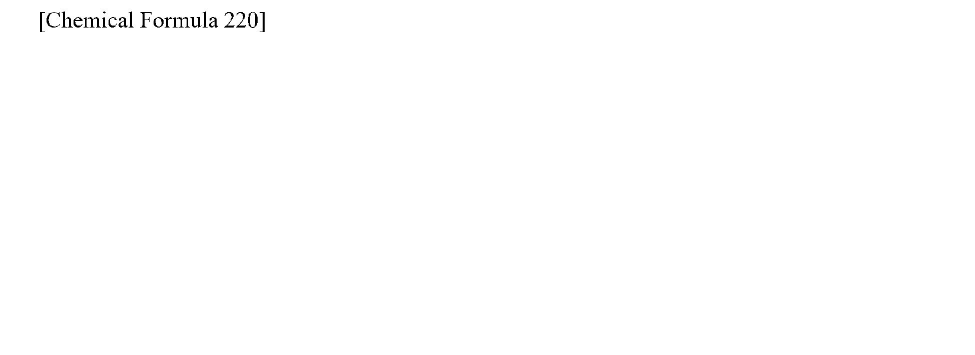

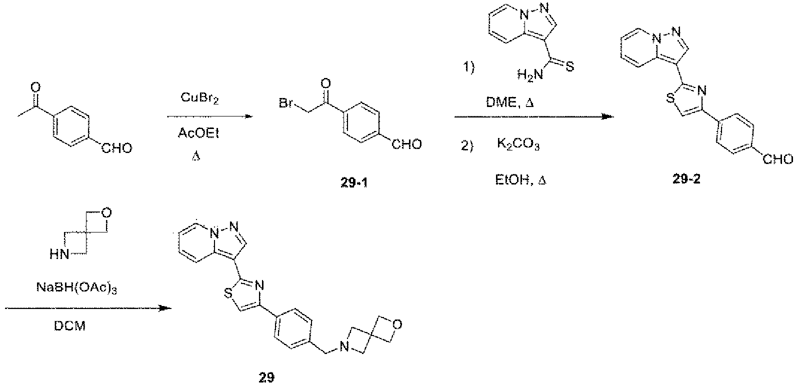

### (1) Synthesis of 4-bromoacetylbenzaldehyde (compound 29-1)

4-Acetylbenzaldehyde (2.24 g, 15.1 mmol) was added to ethyl acetate (75 mL), CuBr₂ (6.89 g, 30.8 mmol) was added thereto, and the mixture was refluxed for 3 hours. After cooling, the insoluble matter was filtered through celite, the organic solvent was removed under reduced pressure, and the residue was purified by silica gel column chromatography to obtain the title compound (compound 29-1) (3.19 g, 14.1 mmol, 93.4% yield) from a fraction of n-hexane : ethyl acetate (4 : 1).

¹H-NMR(CDCl₃) δ:4.42 (2H, s), 8.00 (2H, d, J=8.2Hz), 8.14 (2H, d, J=8.2Hz), 10.10 (1H, s).

### (2) Synthesis of 4-(2-(pyrazolo[1,5-a]pyridin-3-yl)thiazol-4-yl)benzaldehyde (compound 29-2)

Pyrazolo[1,5-a]pyridine-3-carbothioamide (1.23 g, 6.96 mmol) was dissolved in DME (40 mL), and 4-bromoacetylbenzaldehyde (compound 29-1) (1.55 g, 6.83 mmol) was added thereto under ice cooling, then the mixture was stirred at room temperature for 16 hours. The reaction mixture was heated and stirred at 50°C for 1 hour and then allowed to cool, and the reaction mixture was concentrated under reduced pressure to remove the solvent. Ethanol (80 mL) was added to the residue, then excess potassium carbonate (1.25 g) was added, and the reaction mixture was refluxed at 70°C for 1 hour. After cooling, the reaction mixture was concentrated under reduced pressure to remove the solvent, and the residue was purified by silica gel column chromatography to obtain the title compound (29-2) (638 mg, 2.09 mmol, 30.6% yield) from a fraction of n-hexane : ethyl acetate (1 : 1).

¹H-NMR(CDCl₃) δ:6.95 (1H, dt, J=1.4Hz, 6.9Hz), 7.45 (1H, t, J=0.9Hz, 6.9Hz, 8.7Hz), 7.55 (1H, s), 7.97 (2H, d, J=8.2Hz), 8.18 (2H, d, J=8.7Hz), 8.41 (1H, s), 8.46 (1H, d, J=8.7Hz), 8.53 (1H, d, J=7.3Hz), 10.05 (1H, s).

### (3) Synthesis of 6-(4-(2-(pyrazolo[1,5-a]pyridin-3-yl)thiazol-4-yl)benzyl)-2-oxa-6-azaspiro[3.3]heptane (compound 29)

4-(2-(pyrazolo[1,5-a]pyridin-3-yl)thiazol-4-yl)benzaldehyde (compound 29-2) (53.5 mg, 0.175 mmol) and 2-oxa-6-azaspiro[3.3]heptane (62.1 mg, 0.626 mmol) were added to dichloromethane (10 mL), and the mixture was stirred at room temperature for 1 hour. Then, NaBH(OAc)₃ (72.9 mg, 0.433 mmol) was added under ice cooling, and the mixture was stirred for 16 hours while returning to room temperature. Water was added to the reaction mixture under ice cooling, the dichloromethane layer was washed with water several times and dried over anhydrous magnesium sulfate, and after filtration, the solvent was removed under reduced pressure, and the residue was purified by silica gel column chromatography to obtain the title compound (29) (25.2 mg, 0.0649 mmol, 36.0% yield) from a fraction of n-hexane : ethyl acetate (1 : 1).

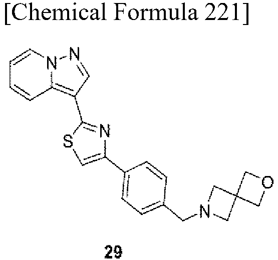

¹H-NMR(CDCl₃) δ:3.39 (4H, s), 3.58 (2H, s), 4.75 (4H, s), 6.92 (1H, dt, J=1.6Hz, 6.9Hz), 7.33 (3H, m), 7.38 (1H, m), 7.95 (2H, d, J=8.2Hz), 8.39 (1H, s), 8.46 (1H, d, J=8.7Hz), 8.51 (1H, d, J=7.3Hz).

[Examples 148 to 151] Synthesis of the following compounds:
N,N-dimethyl-1-(4-(2-(pyrazolo[1,5-a]pyridin-3-yl)thiazol-4-yl)phenyl)methanamine (compound 27),
4-(4-((4-methylpiperazin-1-yl)methyl)phenyl)-2-(pyrazolo[1,5-a]pyridin-3-yl)thiazole (compound 28),
N,N-dimethyl-4-(4-(2-(pyrazolo[1,5-a]pyridin-3-yl)thiazol-4-yl)benzyl)morpholine-2-carboxamide (compound 38), and
N,2-dimethyl-4-(4-(2-(pyrazolo[1,5-a]pyridin-3-yl)thiazol-4-yl)benzyl)morpholine-2-carboxamide (compound 39)

Compound (27), compound (28), compound (38), and compound (39) were synthesized according to the method for synthesizing compound (29), using compound (29-2).

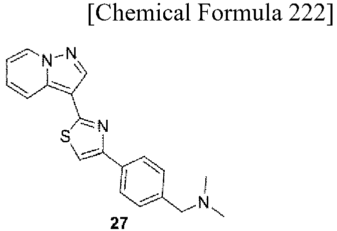

¹H-NMR(CDCl₃) δ:2.27 (6H, s), 3.48 (2H, s), 6.92 (1H, dt, J=1.4Hz, 6.9Hz), 7.38 (4H, m), 7.96 (2H, d, J=8.2Hz), 8.39 (1H, s), 8.47 (1H, d, J=8.7Hz), 8.51 (1H, d, J=7.3Hz).

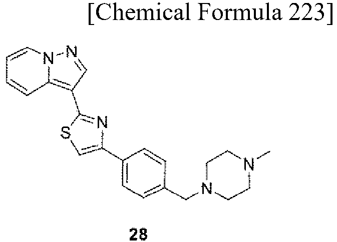

¹H-NMR(CDCl₃) δ:2.28 (3H, s), 2.48 (8H, m), 3.54 (2H, s), 6.91 (1H, dt, J=0.9Hz, 6.9Hz), 7.33 (1H, s), 7.37 (1H, m), 7.40 (2H, d, J=8.2Hz), 7.94 (2H, d, J=8.7Hz), 8.38 (1H, s), 8.46 (1H, d, J=8.7Hz), 8.51 (1H, d, J=7.3Hz).

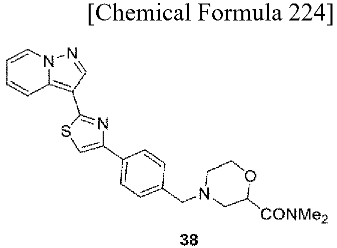

¹H-NMR(CDCl₃) δ:2.29 (1H, t, J=11.5Hz), 2.42 (1H, t, J=11.5Hz), 2.71 (1H, d, J=11.5Hz), 2.92 (1H, d, J=8.2Hz), 2.93 (3H, s), 3.05 (3H, s), 3.60 (2H, d, J=3.1Hz), 3.72 (1H, t, J=11.5Hz), 3.94 (1H, d, J=13.3Hz), 4.29 (1H, d, J=7.8Hz), 6.92 (1H, dt, J=1.8Hz, 6.9Hz), 7.35 (1H, s), 7.39 (1H, t, J=7.8Hz), 7.41 (2H, d, J=7.3Hz), 7.96 (2H, d, J=7.8Hz), 8.40 (1H, s), 8.47 (1H, d, J=8.7Hz), 8.52 (1H, d, J=6.9Hz).

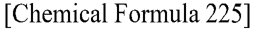

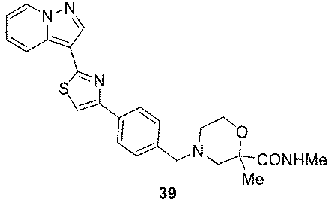

¹H-NMR(CDCl₃) δ:1.44 (3H, s), 2.34 (1H, m), 2.44 (1H, m), 2.50 (1H, d, J=11.5Hz), 2.70 (1H, d, J=10.4Hz), 2.83 (3H, d, J=5.0Hz), 3.54 (2H, s), 3.75 (1H, m), 3.83 (1H, m), 6.71 (1H, m), 6.91 (1H, dt, J=1.4Hz, 6.9Hz), 7.34 (1H, s), 7.38 (3H, m), 7.95 (2H, d, J=8.2Hz), 8.39 (1H, s), 8.47 (1H, d, J=8.7Hz), 8.51 (1H, d, J=6.9Hz).

### [Example 152] Synthesis of 1-morpholino-2-(4-(4-(2-(pyrazolo[1,5-a]pyridin-3-yl)thiazol-4-yl)benzyl)piperazin-1-yl)ethane-1,2-dione hydrochloride (compound 36)

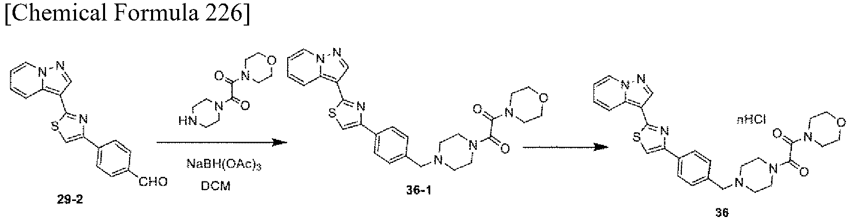

4-(2-(pyrazolo[1,5-a]pyridin-3-yl)thiazol-4-yl)benzaldehyde (compound 29-2) (58.1 mg, 0.190 mmol) and 1-morpholino-2-(piperazin-1-yl)ethane-1,2-dione (50.5 mg, 0.222 mmol) were added to dichloromethane (10 mL), and the mixture was stirred at room temperature for 1 hour. Then, NaBH(OAc)₃ (113 mg, 0.533 mmol) was added under ice cooling, and the mixture was stirred for 16 hours while returning to room temperature. Water was added to the reaction mixture under ice cooling, the dichloromethane layer was washed with water several times and dried over anhydrous magnesium sulfate, and after filtration, the solvent was removed under reduced pressure, and the residue was purified by silica gel column chromatography to obtain the title compound (36-1) (35.5 mg, 0.0687 mmol, 36.2% yield) from a fraction of n-hexane : ethyl acetate (1 : 4).

¹H-NMR(CDCl₃) δ:2.50 (4H, m), 3.43 (4H, m), 3.58 (2H, s), 3.70 (8H, m), 6.92 (1H, dt, J=1.4Hz, 8.5Hz), 7.36 (4H, m), 7.97 (2H, d, J=7.8Hz), 8.40 (1H, s), 8.46 (1H, d, J=8.7Hz), 8.52 (1H, d, J=6.9Hz).

Then, the amine (compound 36-1) was converted to the hydrochloride of the title compound (36) by a conventional method.

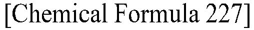

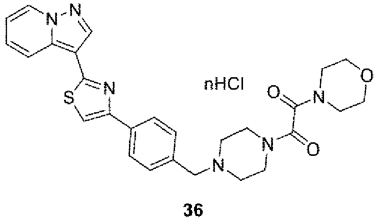

¹H-NMR(D₂O) δ:3.21 (4H, m), 3.34 (2H, m), 3.50 (2H, m), 3.62 (8H, m), 4.17 (2H, s), 6.62 (1H, t, J=6.9Hz), 6.98 (2H, m), 7.14 (2H, d, J=8.2Hz), 7.33 (3H, m), 7.75 (1H, s), 7.99 (1H, d, J=6.9Hz).

### [Examples 153 and 154] Synthesis of 4-(4-((2,5-diazabicyclo[2.2.1]heptan-2-yl)methyl)phenyl)-2-(pyrazolo[1,5-a]pyridin-3-yl)thiazole hydrochloride (compound 34) and N-methyl-N-(4-(2-(pyrazolo[1,5-a]pyridin-3-yl)thiazol-4-yl)benzyl)piperidin-4-amine hydrochloride (compound 35)

Compound (34) and compound (35) were synthesized according to the methods for synthesizing compound (29) and compound (1).

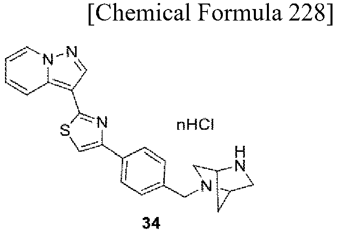

¹H-NMR(D₂O) δ:2.24 (1H, d, J=13.3Hz), 2.56 (1H, d, J=13.3Hz), 3.56 (2H, m), 3.70 (2H, m), 4.30 (1H, d, J=13.3Hz), 4.43 (1H, d, J=13.3Hz), 4.53 (1H, s), 4.59 (1H, s), 6.83 (1H, t, J=6.6Hz), 7.26 (1H, t, J=8.2Hz), 7.35 (2H, d, J=8.2Hz), 7.40 (1H, s), 7.60 (2H, d, J=8.2Hz), 7.76 (1H, d, J=9.2Hz), 8.12 (1H, s), 8.23 (1H, d, J=6.9Hz).

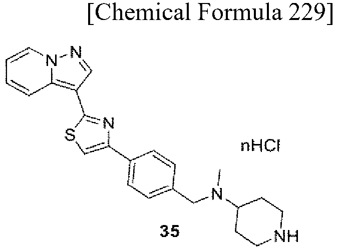

¹H-NMR(D₂O) δ:1.15 (2H, m), 2.00 (2H, m), 2.31 (2H, m), 2.65 (3H, s), 3.03 (2H, t, J=11.5Hz), 3.60 (1H, m), 4.10 (1H, m), 4.39 (1H, m), 6.79 (1H, t, J=6.9Hz), 7.22 (1H, t, J=8.2Hz), 7.28 (1H, s), 7.31 (2H, d, J=9.6Hz), 7.54 (2H, d, J=7.8Hz), 7.68 (1H, d, J=8.7Hz), 8.07 (1H, s), 8.17 (1H, d, J=6.9Hz).

### [Example 155] Synthesis of 4-(isoindolin-5-yl)-2-(pyrazolo[1,5-a]pyridin-3-yl)thiazole hydrochloride (compound 117)

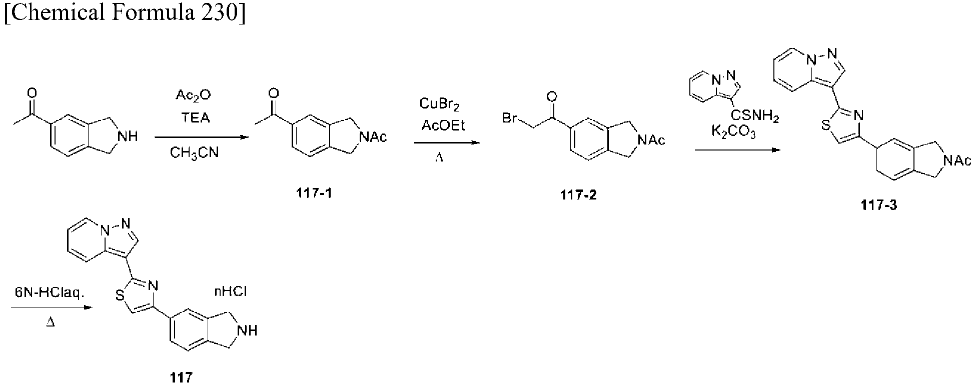

### (1) Synthesis of 1,1'-(isoindoline-2,5-diyl)bis(ethan-1-one) (compound 117-1)

5-Acetylisoindoline (402 mg, 2.49 mmol) was added to acetonitrile (20 mL), acetic anhydride (0.35 mL) and an excess of TEA (0.70 mL) were added thereto under ice cooling, and the mixture was stirred at room temperature for 16 hours. The reaction mixture was concentrated under reduced pressure to remove the solvent, and the residue was purified by silica gel column chromatography to obtain the title compound (117-1) (366 mg, 1.80 mmol, 72.3% yield) from a fraction of n-hexane : ethyl acetate (1 : 4).

¹H-NMR(CDCl₃) δ:2.18 (3H, s), 2.61 (3H, s), 4.83 (2H, s), 4.85 (2H, s), 7.35 (1H, dd, J=8.2Hz, 10.0Hz), 7.87 (1H, d, J=10.0Hz,), 7.90 (1H, d, J=8.2Hz).

### (2) Synthesis of 1-(2-acetylisoindolin-5-yl)-2-bromoethan-1-one (compound 117-2)

1,1'-(isoindoline-2,5-diyl)bis(ethan-1-one) (compound 117-1) (194 mg, 0.952 mmol) was added to ethyl acetate (50 mL), CuBr₂ (522 mg, 2.34 mmol) was added thereto, and the mixture was refluxed for 6.5 hours. After cooling, the insoluble matter was filtered through celite, the organic solvent was removed under reduced pressure, and the residue was purified by silica gel column chromatography to obtain the title compound (117-2) (106 mg, 0.375 mmol, 39.4% yield) from a fraction of n-hexane : ethyl acetate (1 : 4).

¹H-NMR(CDCl₃) δ:2.15 (3H, brs), 4.43 (2H, s), 4.88 (4H, brs), 7.43 (1H, m), 7.91 (2H, m).

### (3) Synthesis of 1-(5-(2-(pyrazolo[1,5-a]pyridin-3-yl)thiazol-4-yl)isoindolin-2-yl) ethan-1-one (compound 117-3)

Pyrazolo[1,5-a]pyridine-3-carbothioamide (73.5 mg, 0.415 mmol) was dissolved in DME (10 mL), and 1-(2-acetylisoindolin-5-yl)-2-bromoethan-1-one (compound 117-2) (106 mg, 0.375 mmol) was added thereto under ice cooling, then the mixture was stirred at room temperature for 16 hours. The reaction mixture was heated and stirred at 50°C for 1 hour and then allowed to cool, and the reaction mixture was concentrated under reduced pressure to remove the solvent. Ethanol (10 mL) was added to the residue, then excess potassium carbonate (141 mg) was added, and the reaction mixture was refluxed at 70°C for 1 hour. After cooling, the reaction mixture was concentrated under reduced pressure to remove the solvent, and the residue was purified by silica gel column chromatography to obtain the title compound (117-3) (66.8 mg, 0.185 mmol, 44.7% yield) from a fraction of n-hexane : ethyl acetate (1 : 4).

¹H-NMR(CDCl₃) δ:2.19 (3H, s), 4.85 (4H, dd, J=10.5Hz, 16.3Hz), 6.93 (1H, dt, J=1.4Hz, 6.9Hz), 7.38 (3H, m), 7.93 (2H, m), 8.42 (2H, m), 8.52 (1H, d, J=6.9Hz).

### (4) Synthesis of 4-(isoindolin-5-yl)-2-(pyrazolo[1,5-a]pyridin-3-yl)thiazole hydrochloride (compound 117)

1-(5-(2-(pyrazolo[1,5-a]pyridin-3-yl)thiazol-4-yl)isoindolin-2-yl)ethan-1-one (compound 117-3) (44.5 mg, 0.123 mmol) was suspended in 6 N hydrochloric acid (1.0 mL), and the mixture was refluxed for 6 hours. After the mixture was allowed to cool, the solvent was removed under reduced pressure, ethanol was added several times, and the mixture was dried to give a residue. The obtained solid was dissolved in water (30 mL), ethyl acetate (30 mL) was added thereto, and the mixture was partitioned. The aqueous layer was concentrated under reduced pressure, ethanol was added several times, and the solvent was removed under reduced pressure and then dried to obtain the title compound (117) (30.1 mg, 0.0773 mmol, 62.8% yield).

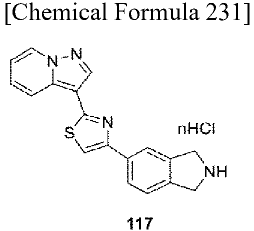

¹H-NMR(D₂O) δ:4.36 (4H, d, J=5.5Hz), 6.66 (1H, t, J=7.1Hz), 6.93 (1H, d, J=8.2Hz), 7.04 (3H, m), 7.15 (1H, brs), 7.38 (1H, d, J=8.7Hz), 7.86 (1H, s), 7.98 (1H, d, J=6.9Hz).

### [Example 156] Synthesis of N-methyl-N-(4-(2-(pyrazolo[1,5-a]pyridin-3-yl)thiazol-4-yl)phenethyl)acetamide (compound 62)

Compound (62) was synthesized according to the methods for synthesizing compound (117-1), compound (69), and compound (118).

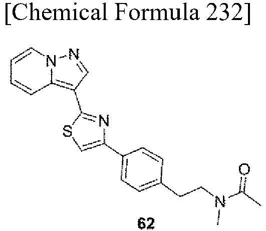

¹H-NMR(CDCl₃) δ:1.95 (3H, s), 2.86 (2H, t, J=6.9Hz), 2.98 (3H, s), 3.55 (2H, q, J=6.9Hz), 6.92 (1H, dt, J=1.4Hz, 6.9Hz), 7.28 (2H, d, J=8.2Hz), 7.34 (1H, s), 7.38 (1H, ddd, J=0.9Hz, 6.9Hz, 8.7Hz), 7.95 (2H, d, J=8.2Hz), 8.39 (1H, s), 8.45 (1H, d, J=9.2Hz), 8.51 (1H, d, J=7.3Hz).

### [Example 157] Synthesis of 2-(4-(2-(pyrazolo[1,5-a]pyridin-3-yl)thiazol-4-yl)phenyl)ethan-1-amine hydrochloride (compound 113)

Compound (113) was synthesized according to the method for synthesizing compound

(118).

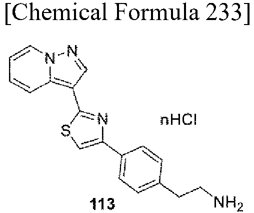

¹H-NMR(D₂O) δ:2.87 (2H, t, J=8.2Hz), 3.07 (2H, m), 7.09 (1H, t, J=6.9Hz), 7.35 (2H, d, J=8.2Hz), 7.55 (1H, d, J=7.8Hz), 7.75 (2H, brs), 7.98 (1H, s), 8.02 (2H, d, J=8.2Hz), 8.39 (1H, d, J=8.7Hz), 8.61 (1H, s), 8.83 (1H, d, J=9.2Hz).

### [Examples 158 and 159] Synthesis of N-(3-(2-(pyrazolo[1,5-a]pyridin-3-yl)thiazol-4-yl)benzyl)acetamide (compound 67) and N-methyl-N-(3-(2-(pyrazolo[1,5-a]pyridin-3-yl)thiazol-4-yl)benzyl)acetamide (compound 70)

Compound (67) was synthesized according to the method for synthesizing compound (117-1) and compound (118), and compound (70) was synthesized from the compound (67) according to the method for synthesizing compound (69).

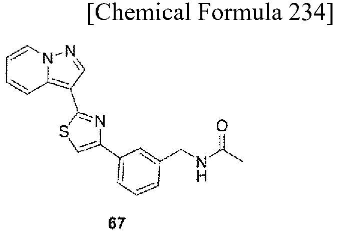

¹H-NMR(CDCl₃) δ:2.06 (3H, s), 4.53 (2H, d, J=5.5Hz), 5.76 (1H, brs), 6.93 (1H, dt, J=1.1Hz, 6.9Hz), 7.29 (1H, d, J=7.3Hz), 7.43 (3H, m), 7.93 (2H, m), 8.40 (1H, s), 8.45 (1H, d, J=9.2Hz), 8.52 (1H, d, J=6.9Hz).

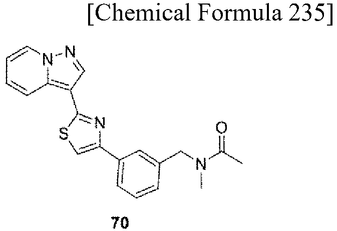

¹H-NMR(CDCl₃) δ:2.19+2.21 (3H, s x2), 2.96+3.00 (3H, s x2), 4.62+6.42 (2H, s x2), 6.93 (1H, m), 7.18 (1H, d, J=7.8Hz), 7.42 (3H, m), 7.86 (1H, d, J=10.4Hz), 7.92 (1H, m), 8.40 (1H, s), 8.44 (1H, d, J=9.2Hz), 8.52 (1H, d, J=6.9Hz).

### [Example 160] Synthesis of N,N-dimethyl-1-(4-(2-(pyrazolo[1,5-a]pyridin-3-yl)thiazol-4-yl)pyridin-2-yl)methanamine (compound 137)

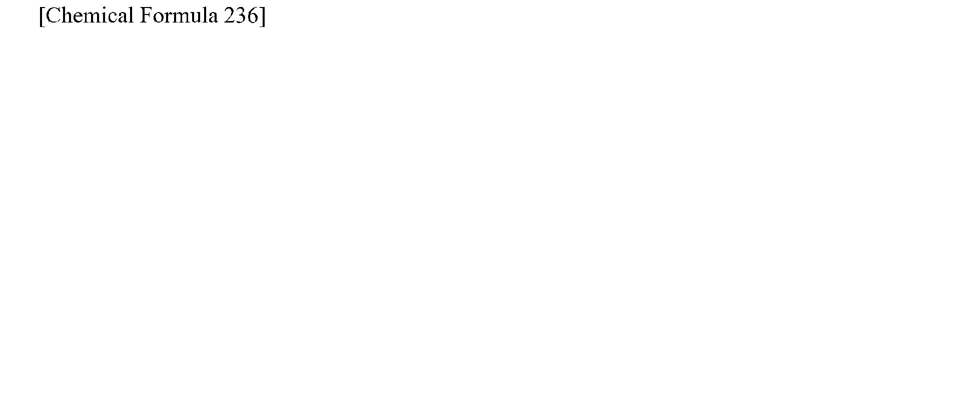

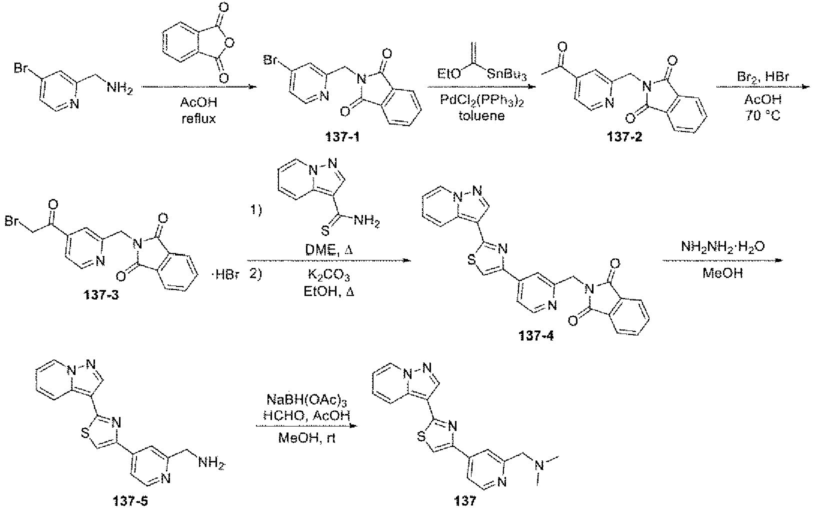

### (1) Synthesis of 2-((4-bromopyridin-2-yl)methyl)isoindoline-1,3-dione (compound 137-1)

(4-Bromopyridin-2-yl)methanamine (2.0 g, 11 mmol) was dissolved in acetic acid (25 mL), phthalic anhydride (2.3 g, 16 mmol) was added thereto, and the mixture was refluxed at 130°C for 3 hours. After cooling, acetic acid was removed under reduced pressure without further treatment, an aqueous potassium carbonate solution was added to the residue, and the mixture was extracted with dichloromethane, then dried over anhydrous magnesium sulfate. After filtration, the solvent was removed under reduced pressure, and dichloromethane and methanol were added to the residue. The suspended solid was filtered with suction and then dried under reduced pressure to obtain the title compound (137-1) (1.9 g, 6.0 mmol, 57.3% yield).

¹H-NMR(CDCl₃) δ:4.99 (2H, s), 7.34 (1H, dd, J=1.8Hz, 5.4Hz), 7.46 (1H, d, J=2.0Hz), 7.73-7.79 (2H, m), 7.87-7.94 (2H, s), 8.34 (1H, d, J=5.6Hz).

### (2) Synthesis of 2-((4-acetylpyridin-2-yl)methyl)isoindoline-1,3-dione (compound 137-2)

2-((4-Bromopyridin-2-yl)methyl)isoindoline-1,3-dione (compound 137-1) (1.9 g, 6.0 mmol) was dissolved in toluene (12 mL), tributyl(1-ethoxyvinyl)tin (2.4 mL, 7.2 mmol) and PdCl₂(PPh₃)₂ (43.7 mg, 0.062 mmol) were added thereto, and the mixture was heated at 160°C for 5 minutes using a microwave synthesis apparatus. After the reaction mixture was allowed to cool, 2 N hydrochloric acid (12 mL) was added thereto, and the mixture was stirred at room temperature for 1 hour. An aqueous sodium hydroxide solution was added to the obtained reaction mixture, and the mixture was extracted with ethyl acetate and then dried over anhydrous magnesium sulfate. After filtration, the solvent was removed under reduced pressure, and the residue was purified by silica gel column chromatography to obtain the title compound (137-2) (0.9 g, 3.3 mmol, 54.5% yield) from a fraction of n-hexane : ethyl acetate (1 : 1).

¹H-NMR(CDCl₃) δ:2.62 (3H, s), 5.10 (2H, s), 7.59-7.63 (1H, m), 7.72-7.74 (1H, m), 7.75-7.78 (2H, m), 7.87-7.94 (2H, m), 8.71 (1H, d, J=5.2Hz).

### (3) Synthesis of 2-((4-(2-bromoacetyl)pyridin-2-yl)methyl)isoindoline-1,3-dione hydrobromide salt (compound 137-3)

2-((4-Acetylpyridin-2-yl)methyl)isoindoline-1,3-dione (compound 137-2) (919.1 mg, 3.3 mmol) was dissolved in acetic acid (10 mL), and bromine (169 µL, 3.3 mmol) and hydrobromic acid (30% acetic acid solution, about 5.1 mol/L) (1.6 mL, 8.2 mmol) were added thereto under ice cooling, then the mixture was heated and stirred at 70°C for 1 hour. After cooling, diethyl ether was added, and the precipitated solid was stirred at room temperature for some time, filtered with suction, then washed with diethyl ether, and dried under reduced pressure to obtain the title compound (137-3) (1.3 g, 3.0 mmol, 92.9% yield).

¹H-NMR(CD₃OD) δ:3.68 (1H, d, J=10.8Hz), 3.77 (1H, d, J=10.8Hz), 5.28 (2H, s), 7.85-7.90 (2H, m), 7.94-7.97 (2H, m), 8.14 (1H, d, J=5.6Hz), 8.18-8.21 (1H, m), 8.78 (1H, d, J=6.0Hz).

### (4) Synthesis of 2-((4-(2-(pyrazolo[1,5-a]pyridin-3-yl)thiazol-4-yl)pyridin-2-yl)methyl)isoindoline-1,3-dione (compound 137-4)

Pyrazolo[1,5-a]pyridine-3-carbothioamide (479.0 mg, 2.7 mmol) was dissolved in DME (20 mL), and 2-((4-(2-bromoacetyl)pyridin-2-yl)methyl)isoindoline-1,3-dione hydrobromide salt (compound 137-3) (1.3 g, 2.7 mmol) was added thereto under ice cooling, then the mixture was stirred at room temperature for 15 hours. The reaction mixture was heated and stirred at 50°C for 1 hour, then allowed to cool, and the reaction mixture was concentrated under reduced pressure to remove the solvent. Ethanol (20 mL) and potassium carbonate (1.1 g, 8.1 mmol) were added to the residue, and the mixture was refluxed at 80°C for 1 hour. After the reaction mixture was allowed to cool, dichloromethane and methanol were added thereto, and the mixture was filtered with suction. The filtrate was then dried under reduced pressure to obtain the title compound (137-4) (917.4 mg, 2.1 mmol, 77.7% yield).

¹H-NMR(CDCl₃) δ:5.12 (2H, s), 6.96 (1H, ddd, J=1.1Hz, 6.8Hz, 6.8Hz), 7.36-7.43 (1H, m), 7.60 (1H, s), 7.72-7.81 (3H, m), 7.91-7.95 (3H, m), 8.39 (1H, s), 8.43 (1H, d, J=9.2Hz), 8.54 (1H, d, J=6.8Hz), 8.61 (1H, d, J=5.2Hz).

### (5) Synthesis of (4-(2-(pyrazolo[1,5-a]pyridin-3-yl)thiazol-4-yl)pyridin-2-yl)methanamine (compound 137-5)

2-((4-(2-(pyrazolo[1,5-a]pyridin-3-yl)thiazol-4-yl)pyridin-2-yl)methyl)isoindoline-1,3-dione (compound 137-4) (721.9 mg, 1.7 mmol) was dissolved in ethanol (15 mL), hydrazine monohydrate (0.3 mL, 6.6 mmol) was added thereto, and the mixture was heated at 160°C for 10 minutes using a microwave synthesis apparatus. After the reaction mixture was allowed to cool, dichloromethane and methanol were added thereto. The suspended solid was filtered with suction and then dried under reduced pressure to obtain the title compound (137-5) (373.3 mg, 1.2 mmol, 73.6% yield).

¹H-NMR(CD₃OD) δ:4.19 (2H, s), 7.10 (1H, ddd, J=1.0Hz, 7.0Hz, 7.0Hz), 7.53-7.59 (1H, m), 7.81-7.87 (2H, m), 7.99 (1H, dd, J=1.6Hz, 5.2Hz), 8.12 (1H, s), 8.16-8.22 (2H, m), 8.50 (1H, d, J=8.8Hz), 8.63 (1H, d, J=5.2Hz), 8.67 (1H, d, J=7.2Hz).

### (6) Synthesis of N,N-dimethyl-1-(4-(2-(pyrazolo[1,5-a]pyridin-3-yl)thiazol-4-yl)pyridin-2-yl)methanamine (compound 137)

(4-(2-(pyrazolo[1,5-a]pyridin-3-yl)thiazol-4-yl)pyridin-2-yl)methanamine (compound 137-5) (131.9 mg, 0.43 mmol) was dissolved in methanol (4 mL), formaldehyde (342 µL, 3.4 mmol), acetic acid (59 µL, 1.0 mmol) and NaBH(OAc)₃ (727.0 mg, 3.4 mmol) were added thereto, and the mixture was stirred at room temperature for 4 hours. An aqueous sodium bicarbonate solution was added to the obtained reaction mixture, and the mixture was extracted with dichloromethane and then dried over anhydrous magnesium sulfate. After filtration, the solvent was removed under reduced pressure, and the residue was purified by silica gel column chromatography to obtain the title compound (137) (30.0 mg, 0.089 mmol, 20.8% yield) from a fraction of dichloromethane : methanol (40 : 1).

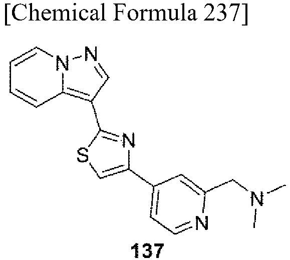

¹H-NMR(CDCl₃) δ:2.36 (6H, s), 3.68 (2H, s), 6.96 (1H, ddd, J=1.1Hz, 6.8Hz, 7.0Hz), 7.40-7.47 (1H, m), 7.64 (1H, s), 7.81-7.87 (1H, m), 7.91 (1H, s), 8.42 (1H, s), 8.45 (1H, d, J=9.2Hz), 8.55 (1H, d, J=7.6Hz), 8.66 (1H, d, J=4.8Hz).

### [Example 161] Synthesis of N,N-dimethyl-1-(5-(2-(pyrazolo[1,5-a]pyridin-3-yl)thiazol-4-yl)pyridin-2-yl)methanamine (compound 128)

Compound (128) was synthesized according to the method for synthesizing compound (137).

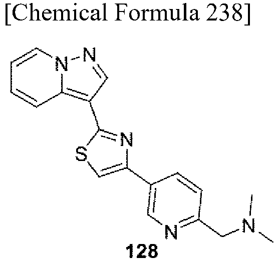

¹H-NMR(CDCl₃) δ:2.37 (6H, s), 3.65 (2H, s), 6.94 (1H, ddd, J=1.3Hz, 6.8Hz, 7.0Hz), 7.36-7.42 (1H, m), 7.43 (1H, s), 7.49 (1H, d, J=8.4Hz), 8.26 (1H, dd, J=2.2Hz, 8.4Hz), 8.40 (1H, s), 8.45 (1H, d, J=8.8Hz), 8.53 (1H, d, J=6.8Hz), 9.17-9.23 (1H, m).

### [Example 162] Synthesis of N-methyl-1-(4-(2-(pyrazolo[1,5-a]pyridin-3-yl)thiazol-4-yl)pyridin-2-yl)methanamine (compound 136)

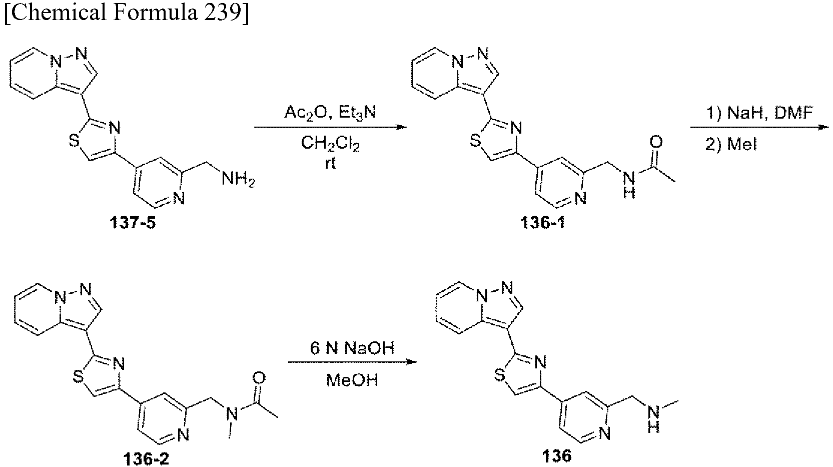

### (1) Synthesis of N-((4-(2-(pyrazolo[1,5-a]pyridin-3-yl)thiazol-4-yl)pyridin-2-yl)methyl)acetamide (compound 136-1)

(4-(2-(Pyrazolo[1,5-a]pyridin-3-yl)thiazol-4-yl)pyridin-2-yl)methanamine (compound 137-5) (101.9 mg, 0.33 mmol) was dissolved in dichloromethane (3 mL), acetic anhydride (63 µL, 0.66 mmol) and TEA(93 µL, 0.66 mmol) were added thereto, and the mixture was stirred at room temperature for 2 hours. An aqueous sodium bicarbonate solution was added to the obtained reaction mixture, and the mixture was extracted with dichloromethane and then dried over anhydrous magnesium sulfate. After filtration, the solvent was removed under reduced pressure, and the residue was purified by silica gel column chromatography to obtain the title compound (136-1) (74.1 mg, 0.21 mmol, 63.9% yield) from a fraction of dichloromethane : methanol (40 : 1).

¹H-NMR(CD₃OD) δ:2.11 (3H, s), 4.57 (2H, s), 7.10 (1H, ddd, J=1.6Hz, 6.8Hz, 6.8Hz), 7.52-7.59 (1H, m), 7.94 (1H, dd, J=1.4Hz, 5.4Hz), 8.05 (1H, s), 8.10 (1H, s), 8.46-8.53 (2H, m), 8.54 (1H, d, J=5.6Hz), 8.66 (1H, d, J=7.2Hz).

### (2) Synthesis of N-methyl-N-((4-(2-(pyrazolo[1,5-a]pyridin-3-yl)thiazol-4-yl)pyridin-2-yl)methyl)acetamide (compound 136-2)

Sodium hydride (oil dispersion) (87.8 mg, 1.8 mmol) was carefully added to anhydrous DMF (4 mL) to form a suspension solution, and after cooling to 0°C, N-((4-(2-(pyrazolo[1,5-a]pyridin-3-yl)thiazol-4-yl)pyridin-2-yl)methyl)acetamide (compound 136-1) (159.8 mg, 0.46 mmol) was slowly added. The mixture was once heated to room temperature, then cooled to 0°C, and methyl iodide (31 µL, 0.50 mmol) was added dropwise thereto. The mixture was stirred for 1 hour while gradually returning to room temperature. The reaction mixture was ice-cooled, and water was carefully and gradually added thereto. An aqueous sodium bicarbonate solution was added to the obtained reaction mixture, and the mixture was extracted with dichloromethane and then dried over anhydrous magnesium sulfate. After filtration, the solvent was removed under reduced pressure, and the residue was subjected to preparative TLC (preparative thin layer chromatography) to obtain the title compound (136-2) (120.4 mg, 0.33 mmol, 72.5% yield) from a fraction of dichloromethane : methanol (20 : 1).

¹H-NMR(CDCl₃) δ:2.22+2.23 (3H, s x2), 3.09+3.11 (3H, s x2), 4.71+4.80 (2H, s x2), 6.93-7.00 (1H, m), 7.40-7.51 (1H, m), 7.61+7.62 (1H, s x2), 7.73-7.85 (2H, m x2), 8.38-8.48 (2H, m), 8.52-8.58 (1H, m), 8.61-8.69 (1H, m x2).

### (3) Synthesis of N-methyl-1-(4-(2-(pyrazolo[1,5-a]pyridin-3-yl)thiazol-4-yl)pyridin-2-yl)methanamine (compound 136)

N-Methyl-N-((4-(2-(pyrazolo[1,5-a]pyridin-3-yl)thiazol-4-yl)pyridin-2-yl)methyl)acetamide (compound 136-2) (51.6 mg, 0.14 mmol) was dissolved in methanol (3 mL), a 6 N aqueous sodium hydroxide solution (3 mL) was added thereto, and the mixture was heated at 120°C for 30 minutes and then at 140°C for 10 minutes using a microwave synthesis apparatus. Water was added to the obtained reaction mixture, and the mixture was extracted with dichloromethane and then dried over anhydrous magnesium sulfate. After filtration, the solvent was removed under reduced pressure, and the residue was subjected to preparative TLC (preparative thin layer chromatography) to obtain the title compound (136) (37.7 mg, 0.12 mmol, 82.7% yield) from a fraction of dichloromethane : methanol (40 : 1).

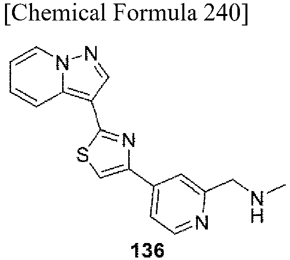

¹H-NMR(CDCl₃) δ:2.55 (3H, s), 3.97 (2H, s), 6.95 (1H, ddd, J=1.2Hz, 6.8Hz, 6.8Hz), 7.38-7.48 (1H, m), 7.61 (1H, s), 7.79 (1H, dd, J=1.8Hz, 5.0Hz), 7.87 (1H, s), 8.41 (1H, s), 8.44 (1H, d, J=9.2Hz), 8.54 (1H, d, J=7.2Hz), 8.61 (1H, d, J=4.8Hz).

### [Examples 163 and 164] Synthesis of N-((5-(2-(pyrazolo[1,5-a]pyridin-3-yl)thiazol-4-yl)pyridin-2-yl)methyl)acetamide (compound 129), and N-methyl-1-(5-(2-(pyrazolo[1,5-a]pyridin-3-yl)thiazol-4-yl)pyridin-2-yl)methanamine (compound 130)

Compound (129) and compound (130) were synthesized according to the method for synthesizing compound (136).

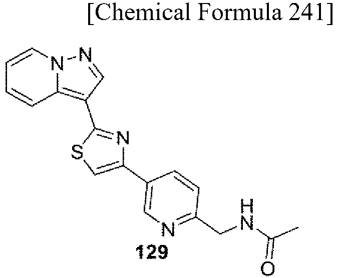

¹H-NMR(CDCl₃) δ:2.12 (3H, s), 4.63 (2H, d, J=5.2Hz), 6.76 (1H, brs), 6.96 (1H, ddd, J=1.3Hz, 6.8Hz, 7.0Hz), 7.37 (1H, d, J=8.0Hz), 7.39-7.44 (1H, m), 7.46 (1H, s), 8.27 (1H, dd, J=2.2Hz, 8.2Hz), 8.41 (1H, s), 8.47 (1H, d, J=8.4Hz), 8.55 (1H, d, J=6.8Hz), 9.20 (1H, d, J=2.0Hz).

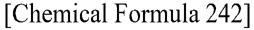

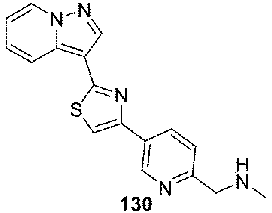

¹H-NMR(CDCl₃) δ:2.53 (3H, s), 3.94 (2H, s), 6.95 (1H, ddd, J=1.3Hz, 6.8Hz, 7.2Hz), 7.38-7.44 (2H, m), 7.44 (1H, s), 8.26 (1H, dd, J=2.2Hz, 8.2Hz), 8.41 (1H, s), 8.47 (1H, d, J=8.8Hz), 8.54 (1H, d, J=7.2Hz), 9.20 (1H, d, J=1.2Hz).

### [Example 165] Synthesis of 4-(1H-imidazol-1-yl)-2-(pyrazolo[1,5-a]pyridin-3-yl)thiazole (compound 165)

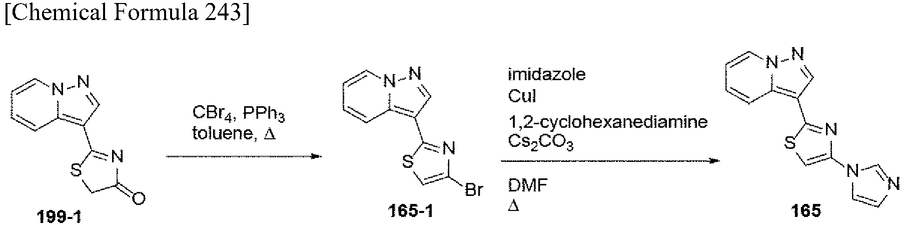

### (1) Synthesis of 4-bromo-2-(pyrazolo[1,5-a]pyridin-3-yl)thiazole (compound 165-1)

Triphenylphosphine (30.9 g, 118 mmol) and carbon tetrabromide (19.6 g, 59.1 mmol) were added to a solution of 2-(pyrazolo[1,5-a]pyridin-3-yl)thiazole-4(5H)-one (compound 199-1) (12.8 g, 58.9 mmol) in toluene (500 mL), and the mixture was stirred at reflux for 1 hour. After the reaction mixture was allowed to cool, water was added thereto, and the mixture was extracted with dichloromethane. The organic layer was dried over anhydrous sodium sulfate and then filtered, and the solvent was removed under reduced pressure. The residue was purified by silica gel column chromatography to obtain the title compound (165-1) (6.92 g, 24.7 mmol, 41.9% yield) from a fraction of n-hexane : ethyl acetate (1 : 1).

¹H-NMR(DMSO-d₆) δ:7.12-7.16 (1H, m), 7.56-7.61 (1H, m), 7.72 (1H, s), 8.19-8.22 (1H, m), 8.64 (1H, s), 8.85-8.89 (1H, m).

### (2) Synthesis of 4-(1H-imidazol-1-yl)-2-(pyrazolo[1,5-a]pyridin-3-yl)thiazole (compound 165)

4-Bromo-2-(pyrazolo[1,5-a]pyridin-3-yl)thiazole (compound 165-1) (16.8 mg, 0.0600 mmol) was dissolved in DMF (1 mL), and imidazole (10.9 mg, 0.160 mmol), copper(I) iodide (16.0 mg, 0.0840 mmol), trans-1,2-cyclohexanediamine (7.20 µL, 0.0599 mmol) and cesium carbonate (49.1 mg, 0.151 mmol) were added thereto. The reaction mixture was heated at 180°C for 20 minutes using a microwave heating apparatus. Water (3 mL) was added to the reaction mixture, and the obtained precipitate was collected by suction filtration to obtain a crude product. The crude product was purified by preparative TLC (preparative thin layer chromatography) to obtain the title compound (165) (6.40 mg, 0.0240 mmol, 40.1% yield) from a fraction of dichloromethane : methanol (30 : 1).

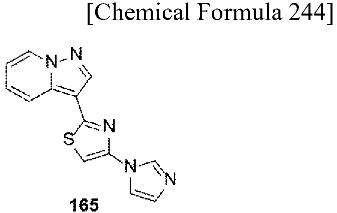

¹H-NMR(CDCl₃) δ:6.88 (1H, s), 6.79 (1H, ddd, J=1.4Hz, 6.9Hz, 6.9Hz), 7.20-7.22 (1H, m), 7.43 (1H, ddd, J=1.1Hz, 6.8Hz, 8.9Hz), 7.53 (1H, dd, J=1.3Hz, 1.3Hz), 8.25-8.27 (1H, m), 8.35 (1H, ddd, J=1.2Hz, 1.2Hz, 8.9Hz), 8.39 (1H, s), 8.55 (1H, ddd, J=1.0Hz, 1.0Hz, 7.0Hz).

[Examples 166 to 170] Synthesis of the following compounds:
4-(1H-pyrazol-1-yl)-2-(pyrazolo[1,5-a]pyridin-3-yl)thiazole (compound 166),
2-(pyrazolo[1,5-a]pyridin-3-yl)-4-(1H-pyrrol-1-yl)thiazole (compound 167),
2-(pyrazolo[1,5-a]pyridin-3-yl)-4-(1H-1,2,4-triazol-1-yl)thiazole (compound 168),
2-(pyrazolo[1,5-a]pyridin-3-yl)-4-(2H-1,2,3-triazol-2-yl)thiazole (compound 169), and
2-(pyrazolo[1,5-a]pyridin-3-yl)-4-(1H-1,2,3-triazol-1-yl)thiazole (compound 170)

Compound (166), compound (167), compound (168), compound (169), and compound (170) were synthesized according to the method for synthesizing compound (165), using pyrazole, pyrrole, 1,2,4-triazole, and 1,2,3-triazole corresponding to the target compounds.

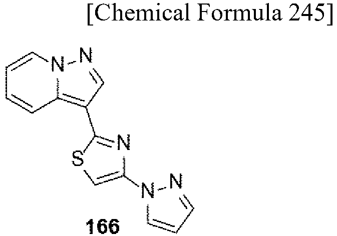

¹H-NMR(CDCl₃) δ: 6.46 (1H, dd, J=1.8Hz, 2.4Hz), 6.95 (1H, ddd, J=1.4Hz, 6.9Hz, 6.9Hz), 7.27 (1H, s), 7.41 (1H, ddd, J=1.2Hz, 6.8Hz, 8.9Hz), 7.72-7.74 (1H, m), 8.35 (1H, ddd, J=1.2Hz, 1.2Hz, 8.9Hz), 8.40-8.42 (2H, m), 8.54 (1H, ddd, J=1.0Hz, 1.0Hz, 7.0Hz).

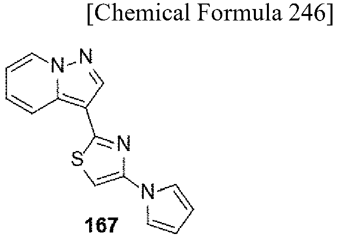

¹H-NMR(CDCl₃) δ:6.33-6.35 (2H, m), 6.71 (1H, s), 6.94 (1H, ddd, J=1.3Hz, 6.9Hz, 6.9Hz), 7.37-7.42 (3H, m), 8.36-8.40 (2H, m), 8.35 (1H, ddd, J=1.0Hz, 1.0Hz, 7.0Hz).

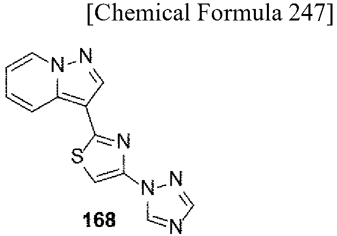

¹H-NMR(CDCl₃) δ:6.98 (1H, ddd, J=1.4Hz, 6.9Hz, 6.9Hz), 7.35 (1H, s), 7.44 (1H, ddd, J=1.2Hz, 6.8Hz, 8.9Hz), 8.10 (1H, s), 8.31 (1H, ddd, J=1.2Hz, 1.2Hz, 8.9Hz), 8.41 (1H, s), 8.56 (1H, ddd, J=1.0Hz, 1.0Hz, 7.0Hz), 9.00 (1H, s).

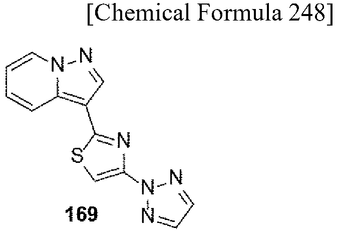

¹H-NMR(CDCl₃) δ:6.95 (1H, ddd, J=1.4Hz, 6.9Hz, 6.9Hz), 7.41 (1H, ddd, J=1.1Hz, 6.8Hz, 8.9Hz), 7.49 (1H, s), 7.88 (2H, s), 8.39 (1H, ddd, J=1.2Hz, 1.2Hz, 8.9Hz), 8.49 (1H, s), 8.54 (1H, ddd, J=1.0Hz, 1.0Hz, 7.0Hz).

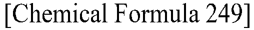

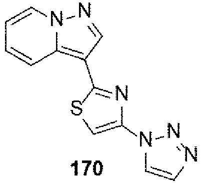

NMR(CDCl₃) δ:6.98 (1H, ddd, J=1.4Hz, 6.9Hz, 6.9Hz), 7.44 (1H, ddd, J=1.1Hz, 6.8Hz, 8.9Hz), 7.63 (1H, s), 7.85 (1H, d, J=1.0Hz), 8.31 (1H, ddd, J=1.2Hz, 1.2Hz, 8.8Hz), 8.43 (1H, s), 8.45 (1H, d, J=0.7Hz), 8.56 (1H, ddd, J=1.0Hz, 1.0Hz, 7.0Hz).

[Examples 171 to 175] Synthesis of the following compounds:
5-(2-(pyrazolo[1,5-a]pyridin-3-yl)thiazol-4-yl)thiophene-3-carboxamide (compound 171),
2-(5-(2-(pyrazolo[1,5-a]pyridin-3-yl)thiazol-4-yl)thiophen-2-yl)acetamide (compound 172),
N,N-dimethyl-5-(2-(pyrazolo[1,5-a]pyridin-3-yl)thiazol-4-yl)thiophene-3-carboxamide (compound 173),
N-(2-(5-(2-(pyrazolo[1,5-a]pyridin-3-yl)thiazol-4-yl)thiophen-2-yl)ethyl)acetamide (compound 174), and
4-(3-(difluoromethyl)-1-methyl-1H-pyrazol-4-yl)-2-(pyrazolo[1,5-a]pyridin-3-yl)thiazole (compound 178)

Compound (171), compound (172), compound (173), compound (174), and compound (178) were synthesized according to the method for synthesizing compound (3), using α-haloketones corresponding to the target compounds.

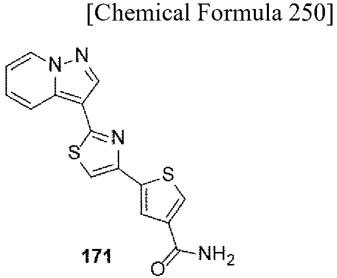

¹H-NMR(DMSO-d₆) δ:7.14 (1H, ddd, J=1.2Hz, 6.8Hz, 6.8Hz), 7.30 (1H, brs), 7.61 (1H, ddd, J=1.2Hz, 7.2Hz, 8.8Hz), 7.87 (1H, brs), 7.91 (1H, s), 7.99 (1H, d, J=1.6Hz), 8.13 (1H, d, J=1.2Hz), 8.36-8.40 (1H, m), 8.64 (1H, s), 8.85-8.89 (1H, m).

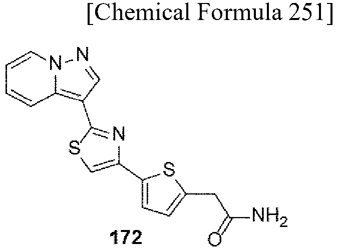

¹H-NMR(DMSO-d₆) δ:3.65 (2H, s), 6.90-6.94 (1H, m), 7.03 (1H, brs), 7.13 (1H, ddd, J=1.2Hz, 6.8Hz, 6.8Hz), 7.46 (1H, d, J=3.6Hz), 7.52-7.64 (2H, m) 7.77 (1H, s), 8.37 (1H, J=1.2Hz, 1.2Hz, 8.8Hz), 8.62 (1H, s), 8.86 (1H, ddd, J=1.2Hz, 1.2Hz, 6.8Hz).

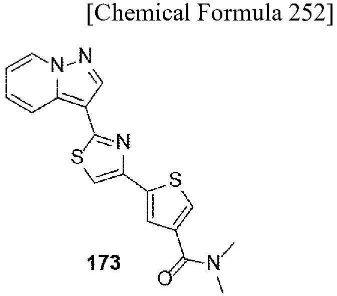

¹H-NMR(DMSO-d₆) δ:2.93-3.15 (6H, m), 7.14 (1H, ddd, J=1.2Hz, 6.8Hz, 6.8Hz), 7.61 (1H, ddd, J=0.8Hz, 6.4 Hz, 8.8Hz), 7.76 (1H, d, J=1.6Hz), 7.80 (1H, d, J=1.6Hz), 7.96 (1H, s), 7.37 (1H, ddd, J=1.2Hz, 1.2Hz, 8.8Hz), 8.64 (1H, s), 7.86 (1H, ddd, J=0.8Hz, 0.8Hz, 7.2Hz).

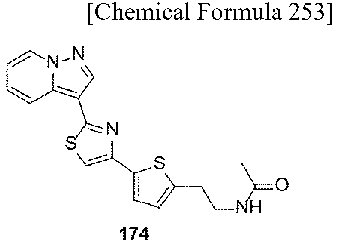

¹H-NMR(CDCl₃) δ:1.99 (3H, s), 3.07 (2H, t, J=6.4Hz), 3.55-3.62 (2H, m), 6.83 (1H, d, J=3.6Hz), 6.94 (1H, ddd, J=1.2Hz, 6.8Hz, 6.8Hz), 7.18 (1H, s), 7.36 (1H, d, J=3.6Hz), 7.40-7.45 (1H, m), 8.38 (1H, s), 8.42-8.47 (1H, m), 8.50-8.55 (1H, m).

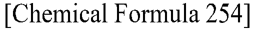

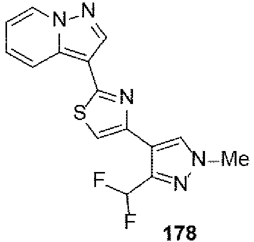

¹H-NMR(DMSO-d₆) δ:3.96 (3H, s), 7.12 (1H, dt, J=1.2Hz, 7.0Hz), 7.36 (1H, t, 53.8Hz), 7.53-7.59 (1H, m), 7.57 (1H, s), 8.38 (1H, ddd, J=1.2Hz, 1.2Hz, 8.8Hz), 8.41 (1H, s), 8.62 (1H, s), 8.85 (1H, ddd, J=1.1Hz, 1.1Hz, 7.2Hz).

### [Example 176] Synthesis of 2-(2-(pyrazolo[1,5-a]pyridin-3-yl)thiazol-4-yl)thiophen-3-ol (compound 175)

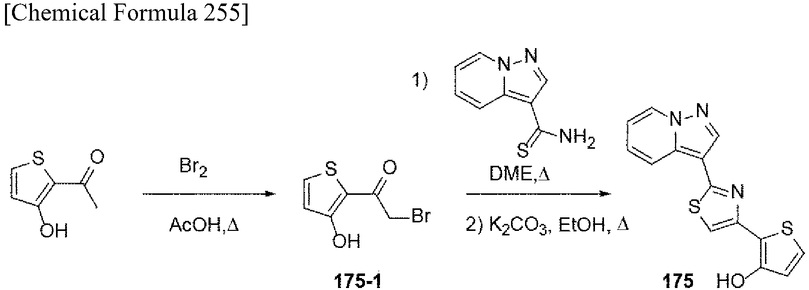

### (1) Synthesis of 2-bromo-1-(3-hydroxythiophen-2-yl)ethan-1-one (compound 175-1)

Bromine (30 µL, 0.584 mmol) was added to a solution of 1-(3-hydroxythiophen-2-yl)ethan-1-one (70.9 mg, 0.499 mmol) in acetic acid (0.7 mL), and the mixture was heated and stirred at 70°C for 4 hours. After cooling, the reaction mixture was neutralized with a saturated aqueous sodium bicarbonate solution, and then extracted with ethyl acetate. The resulting organic layer was washed with water and saturated saline, dried over sodium sulfate, and then filtered, and the solvent was removed under reduced pressure. The residue was purified by silica gel column chromatography to obtain the title compound (175-1) (23.5 mg, 0.106 mmol, 21.3% yield) from a fraction of n-heptane : ethyl acetate (2 : 1).

¹H-NMR(CDCl₃) δ:4.18 (2H, s), 6.80 (1H, d, J=5.6Hz), 7.53 (1H, d, J=5.2Hz), 11.17 (1H, s).

### (2) Synthesis of 2-(2-(pyrazolo[1,5-a]pyridin-3-yl)thiazol-4-yl)thiophen-3-ol (compound 175)

Pyrazolo[1,5-a]pyridine-3-carbothioamide (52.5 mg, 0.296 mmol) and 2-bromo-1-(3-hydroxythiophen-2-yl)ethan-1-one (compound 175-1) (69.3 mg, 0.313 mmol) were dissolved in DME (2 mL), and the mixture was stirred at reflux for 1 hour. After cooling, the reaction mixture was concentrated under reduced pressure. Ethanol (1 mL) and potassium carbonate (52.1 mg, 0.378 mmol) were added to the residue, and the mixture was refluxed for 1 hour. After cooling, the reaction mixture was concentrated under reduced pressure. The residue was dissolved in ethyl acetate, then 0.5 M hydrochloric acid was added, and the mixture was extracted with ethyl acetate. The organic layer was washed with water and saturated saline, dried over sodium sulfate, and then filtered, and the solvent was removed under reduced pressure. The residue was purified by silica gel column chromatography, and a fraction of n-heptane : ethyl acetate (2 : 1) containing the title compound was concentrated under a reduced pressure. The residue was reprecipitated using dichloromethane and n-heptane to obtain the title compound (175) (18.5 mg, 0.0618 mmol, 20.8% yield).

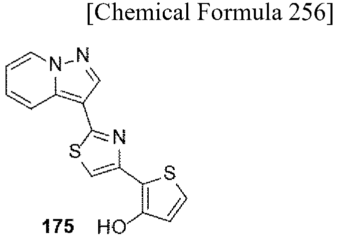

¹H-NMR(DMSO-d₆) δ: 6.78 (1H, d, J=5.6Hz), 7.12 (1H, ddd, J=1.6Hz, 6.8Hz, 6.8Hz), 7.33 (1H, d, J=5.6Hz), 7.54-7.64 (2H, m), 7.33 (1H, ddd, J=1.2Hz, 1.2Hz, 8.8Hz), 8.60 (1H, s), 8.85 (1H, ddd, J=1.2Hz, 1.2Hz, 7.2Hz), 10.33 (1H, s).

### [Example 177] Synthesis of 1-methyl-4-(2-(pyrazolo[1,5-a]pyridin-3-yl)thiazol-4-yl)-1H-pyrazole-3-carbonitrile (compound 176)

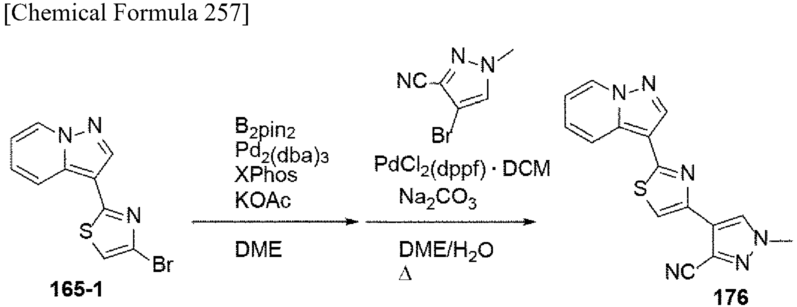

Bis(pinacolato)diboron (176 mg, 0.693 mmol) was dissolved in DME (10 mL), Pd₂(dba)₃ (33.0 mg, 0.0360 mmol), XPhos (18.2 mg, 0.0382 mmol) and potassium acetate (118 mg, 1.20 mmol) were added thereto, and the mixture was refluxed. 4-Bromo-2-(pyrazolo[1,5-a]pyridin-3-yl)thiazole (compound 165-1) (94.0 mg, 0.336 mmol) in DME (3 mL) was added dropwise thereto over 1 hour. After completion of the dropwise addition, the mixture was refluxed for 3 hours. Ethyl acetate (20 mL) was added to the reaction mixture, and the insoluble matter was filtered. The organic layer was concentrated under reduced pressure to remove the solvent, diethyl ether (30 mL) was added to the residue, the precipitated insoluble matter was filtered, and the organic layer was concentrated under reduced pressure to remove the solvent to obtain a crude product. The obtained crude product was dissolved in DME (3 mL), and 4-bromo-1-methyl-1H-pyrazole-3-carbonitrile (52.3 mg, 0.281 mmol), PdCl₂(dppf)·DCM (17.0 mg, 0.0208 mmol), sodium carbonate (49.4 mg, 0.466 mmol) and water (0.5 mL) were added, and the mixture was refluxed for 4 hours. Water (10 mL) was added to the reaction mixture, and the mixture was extracted with ethyl acetate. The organic layer was dried over sodium sulfate, then insoluble matter was removed by filtration, and the solvent was removed from the organic layer under reduced pressure. The residue was purified by silica gel column chromatography to obtain the title compound (176) (3.80 mg, 0.0124 mmol, 3.7% yield over two steps) from a fraction of n-heptane : ethyl acetate (2 : 1).

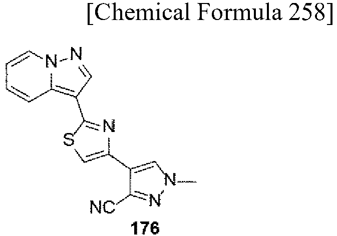

¹H-NMR(CDCl₃) δ:4.04 (3H, s), 6.93 (1H, ddd, J=1.4Hz, 6.9Hz, 6.9Hz), 7.40 (1H, ddd, J=1.1Hz, 6.8Hz, 8.9Hz), 7.53 (1H, s), 7.97 (1H, s), 8.38 (1H, s), 8.46 (1H, ddd, J=1.2Hz, 1.2Hz, 8.8Hz), 8.52 (1H, ddd, J=0.9Hz, 0.9Hz, 6.9Hz).

### [Example 178] Synthesis of N-methyl-2-(4-(2-(pyrazolo[1,5-a]pyridin-3-yl)thiazol-4-yl)-1H-pyrazol-1-yl)ethane-1-amine (compound 177)

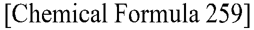

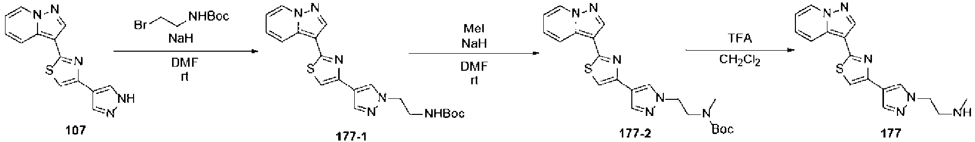

### (1) Synthesis of tert-butyl (2-(4-(2-(pyrazolo[1,5-a]pyridin-3-yl)thiazol-4-yl)-1H-pyrazol-1-yl)ethyl)carbamate (compound 177-1)

Under an argon atmosphere, 4-(1H-pyrazol-4-yl)-2-(pyrazolo[1,5-a]pyridin-3-yl)thiazole (compound 107) (298 mg, 1.11 mmol) and sodium hydride (oil dispersion) (58.4 mg, 1.46 mmol) were suspended in anhydrous DMF (3 mL), and 2-(tert-butoxycarbonylamino)ethyl bromide (277 mg, 1.24 mmol) was added thereto under ice cooling, then the mixture was stirred at room temperature for 5 hours. A saturated aqueous ammonium chloride solution was added to the reaction mixture, and then the mixture was extracted with ethyl acetate. The organic layer was washed with water and saturated saline, dried over sodium sulfate, and then filtered, and the solvent was removed under reduced pressure. The residue was purified by silica gel column chromatography to obtain the title compound (177-1) (274 mg, 0.667 mmol, 59.8% yield) from a fraction of dichloromethane : methanol (10 : 1).

¹H-NMR(CDCl₃) δ:1.45 (9H, s), 3.59-3.68 (2H, m), 4.31 (2H, t, J=5.6Hz), 4.92 (1H, brs), 6.93 (1H, ddd, J=1.2Hz, 6.8Hz, 6.8Hz), 7.08 (1H, s), 7.34-7.42 (1H, m), 7.90 (1H, s), 7.91-7.97 (1H, m), 8.37-8.43 (2H, m), 8.51-8.56 (1H, m).

### (2) Synthesis of tert-butyl methyl (2-(4-(2-(pyrazolo[1,5-a]pyridin-3-yl)thiazol-4-yl)-1H-pyrazol-1-yl)ethyl)carbamate (compound 177-2)

tert-Butyl (2-(4-(2-(pyrazolo[1,5-a]pyridin-3-yl)thiazol-4-yl)-1H-pyrazol-1-yl)ethyl)carbamate (compound 177-1) (39.1 mg, 0.0952 mmol) and sodium hydride (oil dispersion) (8.20 mg, 0.205 mmol) were suspended in anhydrous DMF (1 mL) under an argon atmosphere, methyl iodide (7.50 µL, 0.120 mmol) was added thereto under ice cooling, and the mixture was stirred at room temperature for 1 hour. A saturated aqueous ammonium chloride solution was added to the reaction mixture, and then the mixture was extracted with ethyl acetate. The organic layer was washed with water and saturated saline, dried over sodium sulfate, and then filtered, and the solvent was removed under reduced pressure. The residue was purified by column chromatography (amino silica gel) to obtain the title compound (177-2) (31.0 mg, 0.0730 mmol, 76.7% yield) from a fraction of n-heptane : ethyl acetate (10 : 1).

¹H-NMR(CDCl₃) δ:1.46 (9H, s), 2.62-2.79 (3H, m), 3.69 (2H, t, J=6.0Hz), 4.26-4.38 (2H, m), 6.89-6.96 (1H, m), 7.07 (1H, s), 7.34-7.43 (1H, m), 7.80-7.91 (1H, m), 7.94 (1H, s), 8.34-8.44 (2H, m), 8.50-8.56 (1H, m).

### (3) Synthesis of N-methyl-2-(4-(2-(pyrazolo[1,5-a]pyridin-3-yl)thiazol-4-yl)-1H-pyrazol-1-yl)ethan-1-amine (compound 177)

tert-Butyl methyl (2-(4-(2-(pyrazolo[1,5-a]pyridin-3-yl)thiazol-4-yl)-1H-pyrazol-1-yl)ethyl)carbamate (compound 177-2) (31.0 mg, 0.0755 mmol) was dissolved in dichloromethane (1 mL), then trifluoroacetic acid (0.5 mL) was added thereto, and the mixture was stirred at room temperature for 1.5 hours. The reaction mixture was neutralized with a saturated aqueous sodium bicarbonate solution, and then extracted with dichloromethane. The organic layer was washed with saturated saline, dried over sodium sulfate, and then filtered, and the solvent was removed under reduced pressure. The residue was purified by silica gel column chromatography to obtain the title compound (177) (13.0 mg, 0.0401 mmol, 53.1% yield) from a fraction of dichloromethane : methanol (5 : 1).

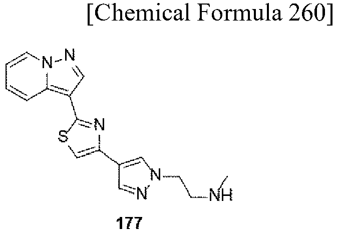

¹H-NMR (DMSO-d₆) d:2.30 (3H, s), 2.91 (2H, t, J=6.4Hz), 4.22 (2H, t, J=6.4Hz), 7.11 (1H, ddd, J=1.2Hz, 6.8Hz, 6.8Hz), 7.52-7.60 (2H, m), 7.94-7.98 (1H, m), 8.25 (1H, s), 8.40-8.46 (1H, m), 8.58 (1H, s), 8.82-8.87 (1H, m).

### [Example 179] Synthesis of 4-(5-fluoro-1H-pyrazol-4-yl)-2-(pyrazolo[1,5-a]pyridin-3-yl)thiazole (compound 179)

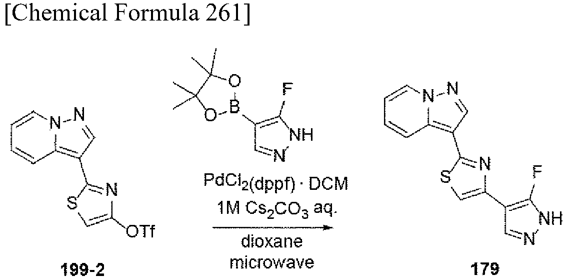

5-fluoro-4-(4,4,5,5-tetramethyl-1,3,2-dioxaborolan-2-yl)-1H-pyrazole (44.1 mg, 0.208 mmol), PdCl₂(dppf)·DCM (14.1 mg, 17.3 µmol) and a 1 M cesium carbonate aqueous solution (0.5 mL) were added to a solution of 2-(pyrazolo[1,5-a]pyridin-3-yl)thiazol-4-yl trifluoromethanesulfonate (compound 199-2) (60.5 mg, 0.173 mmol) in 1,4-dioxane (1.25 mL), and the mixture was heated at 140°C for 10 minutes using a microwave synthesis apparatus. After the reaction mixture was allowed to cool, water was added thereto, and the mixture was extracted with ethyl acetate. The organic layer was dried over anhydrous sodium sulfate and then filtered, and the solvent was removed under reduced pressure. The residue was purified by silica gel column chromatography to obtain the title compound (179) (21.8 mg, 76.4 µmol, 44.2% yield) from a fraction of n-heptane : ethyl acetate (1 : 1).

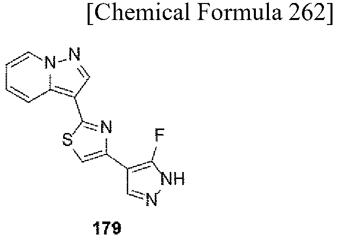

¹H-NMR(DMSO-d₆) δ:7.12 (1H, dt, J=1.5Hz, 6.9Hz), 7.44-7.46 (1H, m), 7.54 (1H, ddd, J=0.9Hz, 6.7Hz, 8.8Hz), 8.26 (1H, s), 8.45 (1H, ddd, J=1.2Hz, 1.2Hz, 8.8Hz), 8.61 (1H, s), 8.84 (1H, ddd, J=0.9Hz, 0.9Hz, 6.8Hz), 12.6-12.8(1H, m).

### [Example 180] Synthesis of 3-methyl-4-(2-(pyrazolo[1,5-a]pyridin-3-yl)thiazol-4-yl)-1H-pyrazol-5-amine (compound 181)

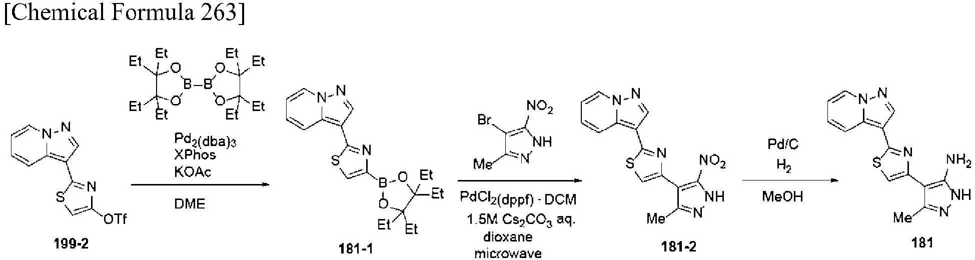

### (1) Synthesis of 2-(pyrazolo[1,5-a]pyridin-3-yl)-4-(4,4,5,5-tetraethyl-1,3,2-dioxaborolan-2-yl)thiazole (compound 181-1)

2-(pyrazolo[1,5-a]pyridin-3-yl)thiazol-4-yl trifluoromethanesulfonate (compound 199-2) (3.50 g, 10.0 mmol) was dissolved in DME (100 mL), and 4,4,4',4',5,5,5',5'-octaethyl-2,2'-bi(1,3,2-dioxaborolane) (7.32 g, 20.0 mmol), Pd₂(dba)₃ (932 mg, 1.02 mmol), XPhos (497 mg, 1.04 mmol), and potassium acetate (2.96 g, 30.2 mmol) were added while bubbling Ar, and the mixture was stirred under an Ar atmosphere at room temperature for 18 hours. Water (50 mL) and ethyl acetate (50 mL) were added to the reaction mixture, and the mixture was separated. The organic layer was washed with saturated saline, then dried over sodium sulfate, and the insoluble matter was filtered, and the solvent was removed under reduced pressure. The residue was purified by silica gel chromatography, and the title compound (181-1) (2.43 g, 6.34 mmol, 63.3% yield) was obtained from a fraction of n-heptane : ethyl acetate (3 : 1).

¹H-NMR(CDCl₃) δ:1.01 (12H, t, J=7.5Hz), 1.72-1.92 (8H, m), 6.87 (1H, ddd, J=1.4Hz, 6.8Hz, 6.8Hz), 7.34 (1H, ddd, J=1.0Hz, 6.8Hz, 8.9Hz), 7.88 (1H, s), 8.36 (1H, ddd, J=1.2Hz, 1.2Hz, 8.9Hz), 8.44 (1H, s), 8.50 (1H, ddd, J=1.1Hz, 1.1Hz, 7.0Hz).

### (2) Synthesis of 4-(3-methyl-5-nitro-1H-pyrazol-4-yl)-2-(pyrazolo[1,5-a]pyridin-3-yl)thiazole (compound 181-2)

4-Bromo-3-methyl-5-nitro-1H-pyrazole (193 mg, 0.936 mmol), PdCl₂(dppf)·DCM (58.8 mg, 72.0 µmol) and a 1.5 M cesium carbonate aqueous solution (1.5 mL) were added to a solution of 2-(pyrazolo[1,5-a]pyridin-3-yl)-4-(4,4,5,5-tetraethyl-1,3,2-dioxaborolan-2-yl)thiazole (compound 181-1) (276 mg, 0.720 mmol) in 1,4-dioxane (4.5 mL), and the mixture was heated at 140°C for 20 minutes using a microwave synthesis apparatus. After the reaction mixture was allowed to cool, water was added thereto, and the mixture was extracted with ethyl acetate. The organic layer was dried over anhydrous sodium sulfate and then filtered, and the solvent was removed under reduced pressure. The residue was purified by silica gel column chromatography to obtain the title compound (181-2) (87.2 mg, 0.267 mmol, 37.1% yield) from a fraction of n-heptane : ethyl acetate (1 : 2).

¹H-NMR(DMSO-d₆) δ:2.50 (3H, s), 7.10 (1H, dt, J=1.3Hz, 7.2Hz), 7.50-7.56 (1H, m), 7.74 (1H, s), 8.18-8.23 (1H, m), 8.61 (1H, s), 8.82-8.86 (1H, m), 13.9 (1H, s).

### (3) Synthesis of 3-methyl-4-(2-(pyrazolo[1,5-a]pyridin-3-yl)thiazol-4-yl)-1H-pyrazol-5-amine (compound 181)

Palladium-activated carbon (Pd 20%) (11.8 mg, 56.2 µmol) was added to a solution of 4-(3-methyl-5-nitro-1H-pyrazol-4-yl)-2-(pyrazolo[1,5-a]pyridin-3-yl)thiazole (compound 181-2) (58.9 mg, 0.180 mmol) in methanol (2 mL), and the mixture was stirred under a hydrogen atmosphere for 23 hours. The reaction mixture was filtered through celite, and the solvent was removed under reduced pressure. The residue was purified by silica gel column chromatography (amino silica gel), and the title compound (181) (5.50 mg, 18.6 µmol, 10.3% yield) was obtained from a fraction of dichloromethane : methanol (20 : 1).

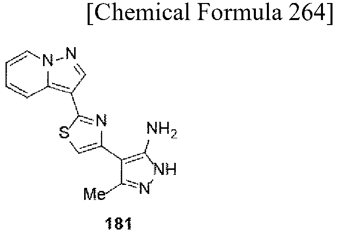

¹H-NMR(DMSO-d₆) δ:2.40 (3H, s), 5.03 (2H, s), 7.08 (1H, dt, J=1.6Hz, 7.0Hz), 7.17-7.25 (1H, m), 7.52 (1H, ddd, J=1.0Hz, 6.8Hz, 8.0Hz), 8.17-8.23 (1H, m), 8.56 (1H, s), 8.80 (1H, ddd, J=1.0Hz, 1.0Hz, 6.8Hz), 11.6 (1H, s).

### [Example 181] Synthesis of 4-(4-(2-(pyrazolo[1,5-a]pyridin-3-yl)thiazol-4-yl)-1H-pyrazol-1-yl)butan-1-ol (compound 180)

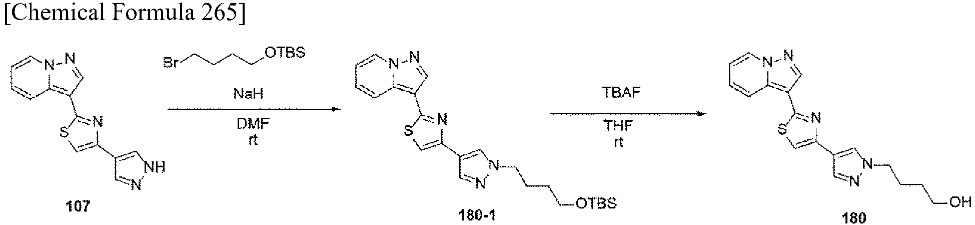

### (1) Synthesis of 4-(1-(4-((tert-butyldimethylsilyl)oxy)butyl)-1H-pyrazol-4-yl)-2-(pyrazolo[1,5-a]pyridin-3-yl)thiazole (compound 180-1)

Under an argon atmosphere, 4-(1H-pyrazol-4-yl)-2-(pyrazolo[1,5-a]pyridin-3-yl)thiazole (compound 107) (52.5 mg, 0.196 mmol) and sodium hydride (oil dispersion) (15.6 mg, 0.390 mmol) were suspended in anhydrous DMF (1.5 mL), and 1-bromo-4-(tert-butyldimethylsilyloxy)butane (74.3 mg, 0.279 mmol) was added thereto, then the mixture was stirred at room temperature for 2 hours. A saturated aqueous ammonium chloride solution was added to the reaction mixture, and the mixture was extracted with ethyl acetate. The organic layer was washed with water and saturated saline, dried over sodium sulfate, and then filtered, and the solvent was removed under reduced pressure. The residue was purified by silica gel column chromatography to obtain the title compound (180-1) (78.9 mg, 0.173 mmol, 88.7% yield) from a fraction of dichloromethane : methanol (10 : 1).

¹H-NMR(CDCl₃) δ:0.05 (6H, s), 0.90 (9H, s), 1.50-1.62 (2H, m), 1.95-2.05 (2H, m), 3.66 (2H, t, J=6.0Hz), 4.22 (2H, t, J=7.2Hz), 6.92 (1H, ddd, J=1.2Hz, 6.8Hz, 6.8Hz), 7.07 (1H, s), 7.34-7.41 (1H, m), 7.88-7.92 (2H, m), 8.37-8.42 (2H, m), 8.50-8.55 (1H, m).

### (2) Synthesis of 4-(4-(2-(pyrazolo[1,5-a]pyridin-3-yl)thiazol-4-yl)-1H-pyrazol-1-yl)butan-1-ol (compound 180)

Under an argon atmosphere, 4-(1-(4-((tert-butyldimethylsilyl)oxy)butyl)-1H-pyrazol-4-yl)-2-(pyrazolo[1,5-a]pyridin-3-yl)thiazole (compound 180-1) (78.9 mg, 0.174 mmol) was dissolved in anhydrous THF (1 mL), then a tetrabutylammonium fluoride THF solution (525 µL, 0.525 mmol) was added thereto under ice cooling, and the mixture was stirred at room temperature for 1 hour. A saturated aqueous ammonium chloride solution was added to the reaction mixture, and the mixture was extracted with ethyl acetate. The organic layer was washed with water and saturated saline, dried over sodium sulfate, and then filtered, and the solvent was removed under reduced pressure. The residue was purified by column chromatography (amino silica gel), and a fraction of dichloromethane : methanol (10 : 1) containing the title compound was concentrated under reduced pressure. The obtained residue was reprecipitated with ethyl acetate and n-heptane, collected by suction filtration, and dried to obtain the title compound (180) (35.3 mg, 0.104 mmol, 59.7% yield).

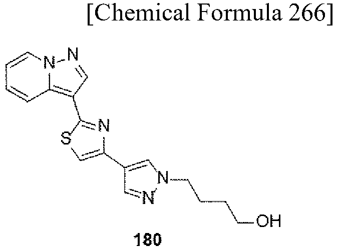

¹H-NMR (DMSO-d₆) d:1.36-1.48 (2H, m), 1.80-1.92 (2H, m), 3.38-3.48 (2H, m), 4.17 (2H, t, J=6.8Hz), 4.44 (1H, t, J=5.2Hz), 7.11 (1H, ddd, J=1.2Hz, 6.8Hz, 6.8Hz), 7.50-7.59 (2H, m), 7.92-7.98 (1H, m), 8.26 (1H, s), 8.39-8.47 (1H, m), 8.58 (1H, s), 8.80-8.87 (1H, m).

### [Example 182] Synthesis of 4-(1-(azetidin-2-ylmethyl)-1H-pyrazol-4-yl)-2-(pyrazolo[1,5-a]pyridin-3-yl)thiazole (compound 182)

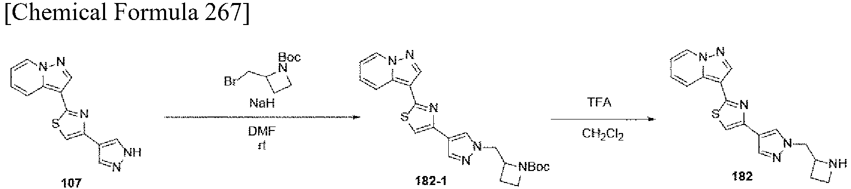

### (1) Synthesis of tert-butyl 2-((4-(2-(pyrazolo[1,5-a]pyridin-3-yl)thiazol-4-yl)-1H-pyrazol-1-yl)methyl)azetidine-1-carboxylate (compound 182-1)

Under an argon atmosphere, 4-(1H-pyrazol-4-yl)-2-(pyrazolo[1,5-a]pyridin-3-yl)thiazole (compound 107) (52.3 mg, 0.196 mmol) and sodium hydride (oil dispersion, 10.5 mg, 0.263 mmol) were suspended in anhydrous DMF (1.5 mL), and tert-butyl 2-(bromomethyl)azetidine-1-carbochelate (68.9 mg, 0.275 mmol) was added thereto, then the mixture was stirred at room temperature for 6 hours. A saturated aqueous ammonium chloride solution was added to the reaction mixture, and then the mixture was extracted with ethyl acetate. The organic layer was washed with water and saturated saline, dried over sodium sulfate, and then filtered, and the solvent was removed under reduced pressure. The residue was purified by column chromatography (amino silica gel) to obtain the title compound (182-1) (55.5 mg, 0.127 mmol, 65.0% yield) from a fraction of n-heptane : ethyl acetate (1 : 3).

H-NMR (DMSO-d₆) δ:1.36 (9H, s), 1.99-2.08 (1H, m), 2,12-2.22 (1H, m), 3.36-3.46 (1H, m), 3.61-3.70 (1H, m), 4.34-4.50 (3H, m), 7.04-7.10 (1H, m), 7.46-7.52 (1H, m), 7.56 (1H, s), 7.97-7.99 (1H, m), 8.11-8.13 (1H, m), 8.32-8.38 (1H, m), 8.54 (1H, s), 8.78-8.82 (1H, m).

### (2) Synthesis of 4-(1-(azetidin-2-ylmethyl)-1H-pyrazol-4-yl)-2-(pyrazolo[1,5-a]pyridin-3-yl)thiazole (compound 182)

tert-Butyl 2-((4-(2-(pyrazolo[1,5-a]pyridin-3-yl)thiazol-4-yl)-1H-pyrazol-1-yl)methyl)azetidine-1-carboxylate (compound 182-1) (55.5 mg, 0.127 mmol) was dissolved in dichloromethane (1 mL), trifluoroacetic acid (0.5 mL) was added thereto, and the mixture was stirred at room temperature for 2.5 hours. The reaction mixture was neutralized with a saturated aqueous sodium bicarbonate solution, and then extracted with dichloromethane. The organic layer was dried over sodium sulfate and filtered, and then the solvent was removed under reduced pressure. The residue was purified by column chromatography (amino silica gel), and the title compound (182) (29.2 mg, 0.0868 mmol, 68.2% yield) was obtained from a fraction of dichloromethane : methanol (20 : 1).

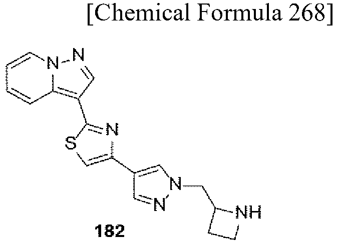

¹H-NMR(CDCl₃) δ:2.14-2.26 (1H, m), 2.30-2.40 (1H, m), 3.31-3.39 (1H, m), 3.62-3.72 (1H, m), 4.24-4.28 (2H, m), 4.32-4.40 (1H, m), 6.92 (1H, ddd, J=1.2Hz, 6.8Hz, 6.8Hz), 7.06 (1H, s), 7.35-7.41 (1H, m), 7.90-7.94 (1H, m), 7.96-8.00 (1H, m), 8.36-8.42 (2H, m), 8.52 (1H, ddd, J=1.2Hz, 1.2Hz, 7.2Hz).

### [Example 183] Synthesis of 2-((2-(4-(2-(pyrazolo[1,5-a]pyridin-3-yl)thiazol-4-yl)-1H-pyrazol-1-yl)ethyl)amino)acetonitrile (compound 183)

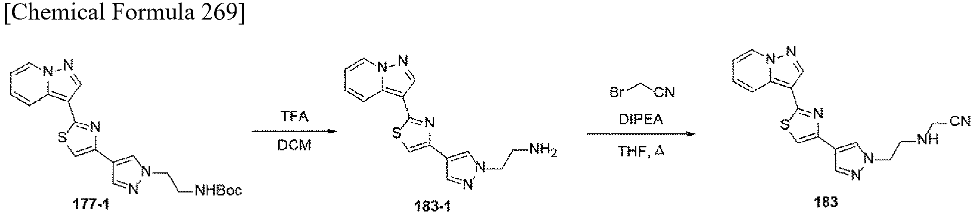

### (1) Synthesis of 2-(4-(2-(pyrazolo[1,5-a]pyridin-3-yl)thiazol-4-yl)-1H-pyrazol-1-yl)ethane-1-amine (compound 183-1)

TFA (1.3 mL) was added at room temperature to a solution of tert-butyl (2-(4-(2-(pyrazolo[1,5-a]pyridin-3-yl)thiazol-4-yl)-1H-pyrazol-1-yl)ethyl)carbamate (compound 177-1) (264 mg, 0.644 mmol) in dichloromethane (6.4 mL). The mixture was stirred for 15.5 hours, then neutralized with a saturated aqueous sodium bicarbonate solution, and extracted with dichloromethane 5 times. The organic layer was dried over anhydrous sodium sulfate and then filtered, and the solvent was removed under reduced pressure. The residue was purified by silica gel column chromatography to obtain the title compound (183-1) (41.3 mg, 0.133 mmol, 20.7% yield) from a fraction of dichloromethane : methanol (10 : 1).

¹H-NMR(CDCl₃) δ:3.22 (2H, t, J=5.6Hz), 4.25 (2H, t, J=5.6Hz), 6.92 (1H, ddd, J=1.2Hz, 7.2Hz, 7.2Hz), 7.08 (1H, s), 7.36-7.40 (1H, m), 7.95 (2H, d, J=3.2Hz), 8.38-8.41 (2H, m), 8.52-8.54 (1H, m).

### (2) Synthesis of 2-((2-(4-(2-(pyrazolo[1,5-a]pyridin-3-yl)thiazol-4-yl)-1H-pyrazol-1-yl)ethyl)amino)acetonitrile (compound 183)

To a solution of 2-(4-(2-(pyrazolo[1,5-a]pyridin-3-yl)thiazol-4-yl)-1H-pyrazol-1-yl)ethan-1-amine (compound 183-1) (40.0 mg, 0.129 mmol) in THF (1.3 mL), DIPEA (65.8 µL, 0.387 mmol) and bromoacetonitrile (10.3 µL, 0.155 mmol) were added, and the mixture was stirred at 50°C for 8 hours. The reaction mixture was concentrated under reduced pressure, and the residue was subjected to preparative TLC (preparative thin layer chromatography) to obtain the title compound (183) (40.0 mg, 0.114 mmol, 88.7% yield) from a fraction of dichloromethane : methanol (10 : 1).

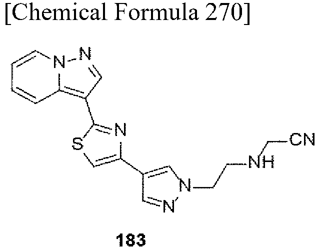

¹H-NMR(CDCl₃) δ:3.24 (2H, t, J=5.2Hz), 3.60 (2H, s), 4.33-4.36 (2H, m), 6.92 (1H, ddd, J=1.2Hz, 6.8Hz, 6.8Hz), 7.09 (1H, s), 7.38 (1H, ddd, J=0.8Hz, 6.4Hz, 8.8Hz), 7.94 (2H, s), 8.37-8.40 (2H, m), 8.53 (1H, ddd, J=0.8Hz, 0.8Hz, 7.2Hz).

### [Example 184] Synthesis of 3-(4-(2-(pyrazolo[1,5-a]pyridin-3-yl)thiazol-4-yl)-1H-pyrazol-1-yl)propan-1-amine (compound 184)

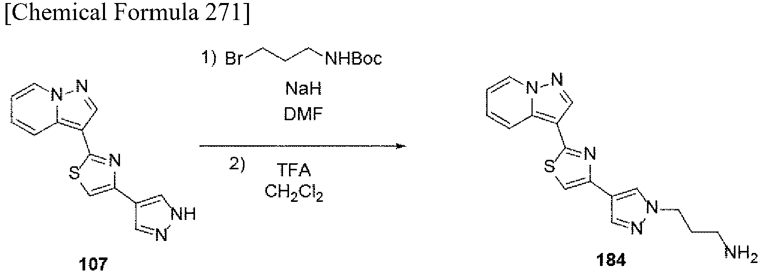

Under an argon atmosphere, 4-(1H-pyrazol-4-yl)-2-(pyrazolo[1,5-a]pyridin-3-yl)thiazole (compound 107) (56.9 mg, 0.213 mmol) and sodium hydride (oil dispersion) (13.3 mg, 0.333 mmol) were suspended in anhydrous DMF, and tert-butyl N-(3-bromopropyl)carbamate (72.8 mg, 0.306 mmol) was added thereto, then the mixture was stirred at room temperature for 4 hours. A saturated aqueous ammonium chloride solution was added to the reaction mixture, and the mixture was extracted with ethyl acetate. The organic layer was washed with water and saturated saline, dried over sodium sulfate, and then filtered, and the solvent was removed under reduced pressure. The residue was dissolved in dichloromethane (1 mL), then trifluoroacetic acid (0.5 mL) was added, and the mixture was stirred at room temperature for 21 hours. The reaction mixture was neutralized with a saturated aqueous sodium bicarbonate solution, and then extracted with dichloromethane. The organic layer was dried over sodium sulfate and filtered, and then the solvent was removed under reduced pressure. The residue was purified by column chromatography (amino silica gel) to obtain the title compound (184) (56.1 mg, 0.173 mmol, 81.1% yield over two steps) from a fraction of dichloromethane : methanol (20 : 1).

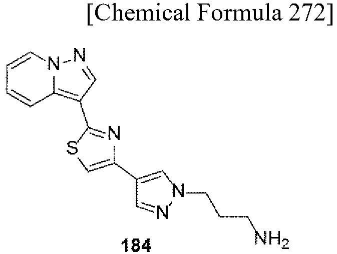

¹H-NMR(CD₃OD) δ: 2.04-2.14 (2H, m), 2.72 (2H, t, 6.8Hz), 4.30 (2H, t, J=6.8Hz), 7.07 (1H, ddd, J=1.2Hz, 6.8Hz, 6.8Hz), 7.41 (1H, s), 7.48-7.55 (1H, m), 7.96-8.01 (1H, m), 8.17 (1H, s), 8.37-8.45 (1H, m), 8.47 (1H, s), 8.62-8.68(1H, m).

### [Example 185] Synthesis of N-(3-(4-(2-(pyrazolo[1,5-a]pyridin-3-yl)thiazol-4-yl)-1H-pyrazol-1-yl)propyl)methanesulfonamide (compound 185)

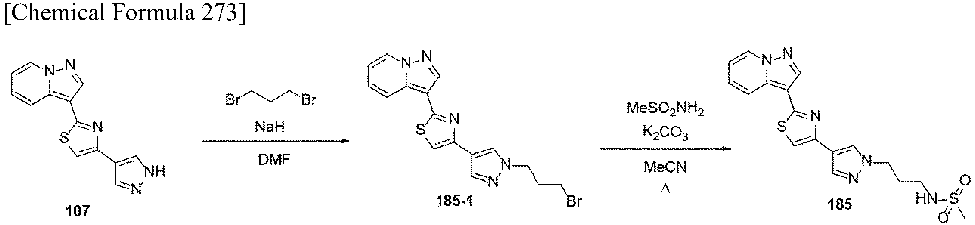

### (1) Synthesis of 4-(1-(3-bromopropyl)-1H-pyrazol-4-yl)-2-(pyrazolo[1,5-a]pyridin-3-yl)thiazole (compound 185-1)

Under an argon atmosphere, 4-(1H-pyrazol-4-yl)-2-(pyrazolo[1,5-a]pyridin-3-yl)thiazole (compound 107) (155 mg, 0.579 mmol) and sodium hydride (oil dispersion, 37.9 mg, 0.948 mmol) were suspended in anhydrous DMF (1.5 mL), and 1,3-dibromopropane (290 µL, 2.94 mmol) was added thereto, then the mixture was stirred at room temperature for 2 hours. A saturated aqueous ammonium chloride solution was added to the reaction mixture, and then the mixture was extracted with ethyl acetate. The organic layer was washed with water and saturated saline, dried over sodium sulfate, and then filtered, and the solvent was removed under reduced pressure. The residue was purified by column chromatography (amino silica gel) to obtain the title compound (185-1) (170 mg, 0.439 mmol, 75.7% yield) from a fraction of n-heptane : ethyl acetate (1 : 3).

¹H-NMR(DMSO-d₆) δ: 2.33-2.42 (2H, m), 3.52 (2H, t, J=6.4Hz), 4.30 (2H, t, J=6.8Hz), 7.08-7.15 (1H, m), 7.52-7.57 (1H, m), 7.58 (1H, s), 7.99 (1H, s), 8.30 (1H, s), 8.44 (1H, ddd, J=1.2Hz, 1.2Hz, 8.8Hz), 8.59 (1H, s), 8.81-8.86 (1H, m).

### (2) Synthesis of N-(3-(4-(2-(pyrazolo[1,5-a]pyridin-3-yl)thiazol-4-yl)-1H-pyrazol-1-yl)propyl)methanesulfonamide (compound 185)

4-(1-(3-Bromopropyl)-1H-pyrazol-4-yl)-2-(pyrazolo[1,5-a]pyridin-3-yl)thiazole (compound 185-1) (60.2 mg, 0.155 mmol), potassium carbonate (48.6 mg, 0.352 mmol) and methanesulfonamide (29.3 mg, 0.308 mmol) were added to acetonitrile (1.5 mL), and the mixture was refluxed for 1.5 hours. After the mixture was allowed to cool, water was added thereto, and the mixture was extracted with ethyl acetate. The organic layer was washed with water and saturated saline, dried over sodium sulfate, and then filtered, and the solvent was removed under reduced pressure. The residue was purified by column chromatography (amino silica gel), and a fraction of dichloromethane : methanol (20 : 1) containing the title compound was concentrated under reduced pressure. The residue was purified by preparative TLC (preparative thin layer chromatography) to obtain the title compound (185) (9.60 mg, 0.0239 mmol, 15.3% yield) from a fraction of dichloromethane : methanol (20 : 1).

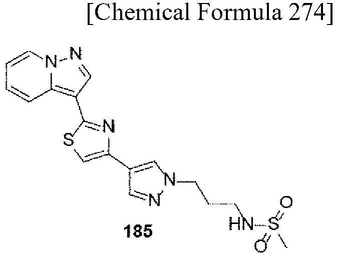

¹H-NMR(DMSO-d₆) δ:1.97-2.07 (2H, m), 2.90 (3H, s), 2.92-3.01 (2H, m), 4.23 (2H, t, J=6.8Hz), 7.08-7.16 (2H, m), 7.50-7.59 (2H, m), 7.98 (1H, s), 8.27 (1H, s), 8.40-8.47 (1H, m), 8.59 (1H, s), 8.81-8.86 (1H, m).

### [Example 186] 5-methyl-4-(2-(pyrazolo[1,5-a]pyridin-3-yl)thiazol-4-yl)-1H-pyrazol-3-ol (compound 186)

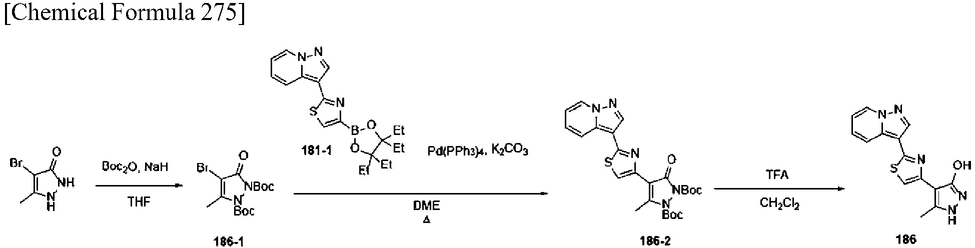

### (1) Synthesis of di-tert-butyl 4-bromo-5-methyl-3-oxo-1H-pyrazole-1,2(3H)-dicarboxylate (compound 186-1)

Under an argon atmosphere, 4-bromo-5-methyl-1,2-dihydro-3H-pyrazol-3-one (351 mg, 1.98 mmol) and sodium hydride (oil dispersion) (270 mg, 6.75 mmol) were suspended in anhydrous THF (5 mL), Boc₂O (1.55 g, 7.12 mmol) was added thereto, and the mixture was stirred at room temperature for 4.5 hours. A saturated aqueous ammonium chloride solution was added to the reaction mixture, and the mixture was extracted with ethyl acetate. The organic layer was washed with water and saturated saline, dried over sodium sulfate, and then filtered, and the solvent was removed under reduced pressure. The residue was purified by silica gel column chromatography to obtain the title compound (186-1) (382 mg, 1.01 mmol, 50.9% yield) from a fraction of n-heptane : dichloromethane (1 : 3).

¹H-NMR(CDCl₃) δ:1.55 (9H, s), 1.63 (9H, s), 2.54(3H, s).

### (2) Synthesis of di-tert-butyl 5-methyl-3-oxo-4-(2-(pyrazolo[1,5-a]pyridin-3-yl)thiazol-4-yl)-1H-pyrazole-1,2(3H)-dicarboxylate (compound 186-2)

DME (6 mL) was added to 2-(pyrazolo[1,5-a]pyridin-3-yl)-4-(4,4,5,5-tetraethyl-1,3,2-dioxaborolan-2-yl)thiazole (compound 181-1) (326 mg, 0.850 mmol), di-tert-butyl 4-bromo-5-methyl-3-oxo-1H-pyrazole-1,2(3H)-dicarboxylate (compound 186-1) (306 mg, 0.812 mmol), potassium carbonate (310 mg, 2.25 mmol), and Pd(PPh₃)₄ (90.4 mg, 0.0782 mmol), and the mixture was heated at 120°C for 20 minutes using a microwave synthesis apparatus. Water was added to the reaction mixture, and the mixture was extracted with ethyl acetate. The organic layer was washed with water and saturated saline, dried over sodium sulfate, and then filtered, and the solvent was removed under reduced pressure. The residue was purified by silica gel column chromatography, and a fraction of n-heptane : ethyl acetate (1 : 1) containing the title compound was concentrated under reduced pressure. The residue was purified by silica gel column chromatography to obtain the title compound (186-2) (86.9 mg, 0.175 mmol, 21.5% yield) from a fraction of dichloromethane : ethyl acetate (4 : 1).

¹H-NMR(DMSO-d₆) δ:1.25 (9H, s), 1.61 (9H, s), 2.80 (3H, s), 7.08-7.15 (1H, m), 7.34-7.41 (1H, m), 8.28-8.30 (1H, m), 8.31-8.34 (1H, m), 8.63 (1H, s), 8.81-8.87(1H, m).

### (3) Synthesis of 5-methyl-4-(2-(pyrazolo[1,5-a]pyridin-3-yl)thiazol-4-yl)-1H-pyrazol-3-ol (compound 186)

Di-tert-butyl 5-methyl-3-oxo-4-(2-(pyrazolo[1,5-a]pyridin-3-yl)thiazol-4-yl)-1H-pyrazole-1,2(3H)-dicarboxylate (compound 186-2) (86.9 mg, 0.175 mmol) was dissolved in dichloromethane (1 mL), and trifluoroacetic acid (0.5 mL) was added thereto, and then the mixture was stirred at room temperature for 1.5 hours. The reaction mixture was neutralized with a saturated aqueous sodium bicarbonate solution, and then extracted with dichloromethane. The organic layer was dried over sodium sulfate and filtered, and then the solvent was removed under reduced pressure. The residue was washed with a mixed solution of dichloromethane and n-heptane and dried to obtain the title compound (compound 186) (23.2 mg, 0.0780 mmol, 44.7% yield).

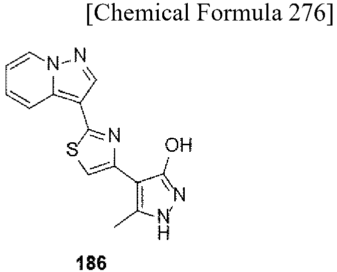

¹H-NMR(DMSO-d₆) δ:2.59 (3H, s), 7.05-7.12 (1H, m), 7.39 (1H, s), 7.50-7.59 (1H, m), 8.28-8.32 (1H, m), 8.58 (1H, s), 8.80-8.86 (1H, m), 10.21 (1H, brs), 11.57 (1H, brs).

### [Example 187] Synthesis of N¹,N¹-dimethyl-N²-(2'-(pyrazolo[1,5-a]pyridin-3-yl)-[4,4'-bithiazole]-2-yl)ethane-1,2-diamine (compound 187)

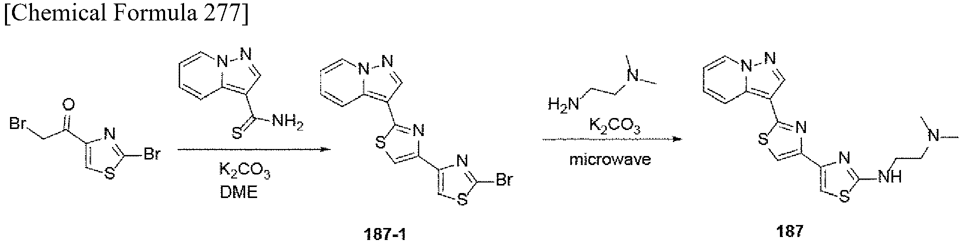

### (1) Synthesis of 2-bromo-2'-(pyrazolo[1,5-a]pyridin-3-yl)-4,4'-bithiazole (compound 187-1)

2-Bromo-1-(2-bromothiazol-4-yl)ethan-1-one (97.2 mg, 0.341 mmol) was added to a solution of pyrazolo[1,5-a]pyridine-3-carbothioamide (60.5 mg, 0.341 mmol) in DME (3.5 mL), and the mixture was stirred at room temperature for 18 hours. The reaction mixture was concentrated under reduced pressure to remove the solvent. The residue was purified by silica gel column chromatography to obtain the title compound (187-1) (92.3 mg, 0.254 mmol, 74.5% yield) from a fraction of n-heptane : ethyl acetate (1 : 1).

¹H-NMR(DMSO-d₆) δ:7.10 (1H, dt, J=1.3Hz, 6.8Hz), 7.53 (1H, ddd, J=1.0Hz, 6.8Hz, 8.0Hz), 7.88 (1H, s), 8.19 (1H, s), 8.42 (1H, ddd, J=1.1Hz, 1.2Hz, 8.8Hz), 8.61 (1H, s), 8.82 (1H, ddd, J=0.9Hz, 0.9Hz, 6.8Hz).

### (2) Synthesis of N¹,N¹-dimethyl-N²-(2'-(pyrazolo[1,5-a]pyridin-3-yl)-[4,4'-bithiazole]-2-yl)ethane-1,2-diamine (compound 187)

Potassium carbonate (28.8 mg, 0.208 mmol) was added to a solution of 2-bromo-2'-(pyrazolo[1,5-a]pyridin-3-yl)-4,4'-bithiazole (compound 187-1) (37.7 mg, 0.104 mmol) in N,N-dimethylethylenediamine (1 mL), and the mixture was heated at 130°C for 70 minutes using a microwave synthesis apparatus. After the reaction mixture was allowed to cool, water was added thereto, and the mixture was extracted with dichloromethane. The organic layer was dried over anhydrous sodium sulfate and then filtered, and the solvent was removed under reduced pressure. The residue was purified by silica gel column chromatography (amino silica gel), and the title compound (187) (27.1 mg, 73.1 µmol, 70.3% yield) was obtained from a fraction of n-heptane : ethyl acetate (1 : 1).

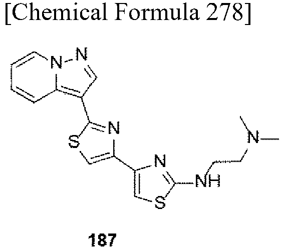

¹H-NMR(CDCl₃) δ :2.27 (6H, s), 2.58 (2H, t, J=5.8Hz), 3.50-3.42 (2H, m), 5.73-5.80 (1H, m), 6.90 (1H, dt, J=1.3Hz, 7.0Hz), 7.14 (1H, s), 7.35 (1H, ddd, J=1.2Hz, 6.8Hz, 8.8Hz), 7.50 (1H, s), 8.38 (1H, s), 8.40 (1H, ddd, J=1.2Hz, 1.2Hz, 9.2Hz), 8.51 (1H, ddd, J=1.0Hz, 1.0Hz, 6.8Hz).

### [Example 188] Synthesis of 2-(dimethylamino)-N-(2'-(pyrazolo[1,5-a]pyridin-3-yl)-[4,4'-bithiazole]-2-yl)acetamide (compound 188)

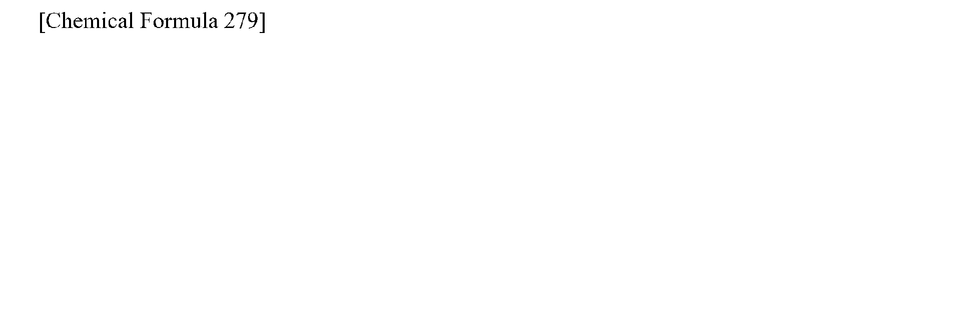

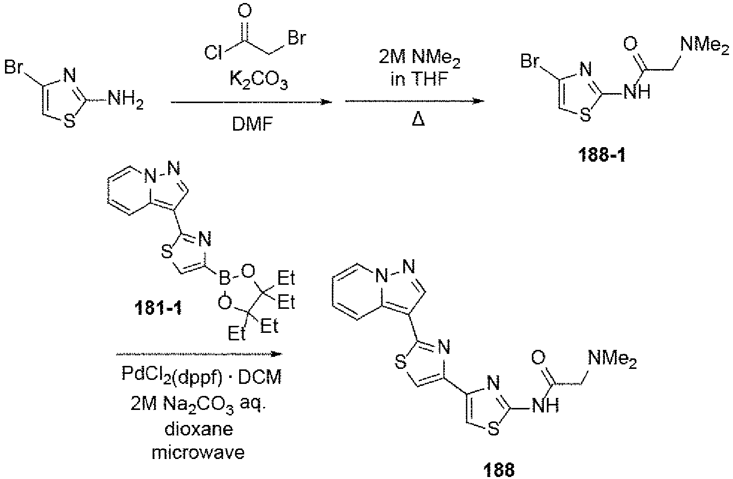

### (1) Synthesis of N-(4-bromothiazol-2-yl)-2-(dimethylamino)acetamide (compound 188-1)

Bromoacetyl chloride (93.6 µL, 1.18 mmol) and potassium carbonate (237 mg, 1.72 mmol) were added to a solution of 2-amino-4-bromothiazole (154 mg, 0.860 mmol) in DMF (6 mL), and the mixture was stirred at room temperature for 14 hours. Water was added to the reaction mixture, and the mixture was extracted with chloroform. The organic layer was dried over anhydrous sodium sulfate and then filtered, and the solvent was removed under reduced pressure. Dimethylamine (2 M in THF) (1 mL) was added to a suspension of the crude product in THF (3 mL), and the mixture was stirred at room temperature for 20 hours and then refluxed for 2 hours. The reaction mixture was concentrated under reduced pressure to remove the solvent. The residue was purified by silica gel column chromatography to obtain the title compound (188-1) (37.2 mg, 0.141 mmol, 16.4% yield over two steps) from a fraction of n-heptane : ethyl acetate (1 : 1).

¹H-NMR(CDCl₃) δ:2.40 (6H, s), 3.17 (2H, s), 6.86 (1H, s), 10.5 (1H, brs).

### (2) Synthesis of 2-(dimethylamino)-N-(2'-(pyrazolo[1,5-a]pyridin-3-yl)-[4,4'-bithiazole]-2-yl)acetamide (compound 188)

N-(4-bromothiazol-2-yl)-2-(dimethylamino)acetamide (compound 188-1) (35.6 mg, 0.135 mmol), PdCl₂(dppf)·DCM (8.50 mg, 10.4 µmol) and a 2 M aqueous sodium carbonate solution (0.1 mL) were added to a solution of 2-(pyrazolo[1,5-a]pyridin-3-yl)-4-(4,4,5,5-tetraethyl-1,3,2-dioxaborolan-2-yl)thiazole (compound 181-1) (39.8 mg, 0.104 mmol) in 1,4-dioxane (1 mL), and the mixture was heated at 120°C for 15 minutes using a microwave synthesis apparatus. After the reaction mixture was allowed to cool, water was added thereto, and the mixture was extracted with dichloromethane. The organic layer was dried over anhydrous sodium sulfate and then filtered, and the solvent was removed under reduced pressure. The residue was subjected to preparative TLC (preparative thin layer chromatography), and the title compound (188) (9.30 mg, 24.2 µmol, 23.3% yield) was obtained using ethyl acetate.

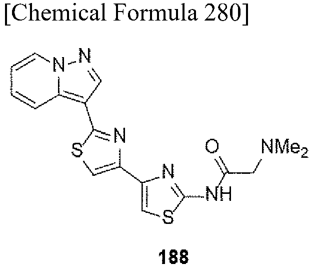

¹H-NMR(CDCl₃) δ:2.42 (6H, s), 3.21 (2H, s), 6.92 (1H, dt, J=1.5Hz, 6.8Hz), 7.38 (1H, ddd, J=1.0Hz, 6.8Hz, 8.0Hz), 7.54 (1H, s), 7.58 (1H, s), 8.40 (1H, s), 8.42 (1H, ddd, J=1.2Hz, 1.2Hz, 8.8Hz), 8.52 (1H, ddd, J=1.0Hz, 1.0Hz, 7.2Hz), 10.5 (1H, brs).

### [Example 189] Synthesis of 2-amino-N-(2'-(pyrazolo[1,5-a]pyridin-3-yl)-[4,4'-bithiazole]-2-yl)acetamide hydrochloride (compound 189)

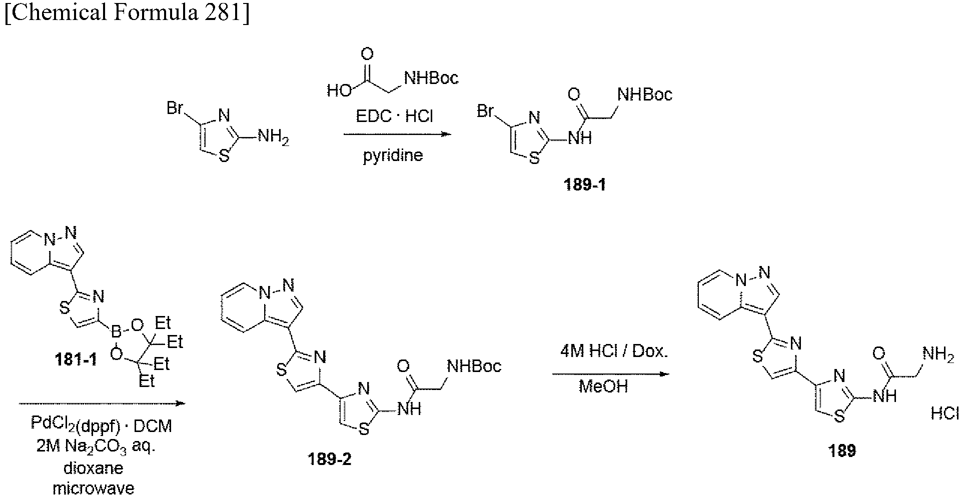

### (1) Synthesis of tert-butyl (2-((4-bromothiazol-2-yl)amino)-2-oxoethyl)carbamate (compound 189-1)

2-Amino-4-bromothiazole (313 mg, 1.75 mmol) and EDC·HCl (1.68 g, 8.75 mmol) were added to a solution of [(tert-butoxycarbonyl)amino]acetic acid (460 mg, 2.62 mmol) in pyridine (5 mL), and the mixture was stirred at room temperature for 22 hours. The reaction mixture was concentrated under reduced pressure with toluene azeotrope to remove the solvent, then brine was added, and the mixture was extracted with chloroform. The organic layer was dried over anhydrous sodium sulfate and then filtered, and the solvent was removed under reduced pressure. The residue was purified by silica gel column chromatography to obtain the title compound (189-1) (295 mg, 0.876 mmol, 50.1% yield) from a fraction of n-heptane : ethyl acetate (1 : 1).

¹H-NMR(CDCl₃) δ:1.48 (9H, s), 4.08 (2H, d, J=6.0Hz), 5.05-5.25 (1H, brs), 6.88(1H, s), 10.0 (1H, brs).

### (2) Synthesis of tert-butyl (2-oxo-2-((2'-(pyrazolo[1,5-a]pyridin-3-yl)-[4,4'-bithiazole]-2-yl)amino)ethyl)carbamate (compound 189-2)

tert-Butyl (2-((4-bromothiazol-2-yl)amino)-2-oxoethyl)carbamate (compound 189-1) (201 mg, 0.598 mmol), PdCl₂(dppf)·DCM (44.4 mg, 54.4 µmol) and a 2 M aqueous sodium carbonate solution (0.1 mL) were added to a solution of 2-(pyrazolo[1,5-a]pyridin-3-yl)-4-(4,4,5,5-tetraethyl-1,3,2-dioxaborolan-2-yl)thiazole (compound 181-1) (208 mg, 0.544 mmol) in 1,4-dioxane (6 mL), and the mixture was heated at 140°C for 20 minutes using a microwave synthesis apparatus. After the reaction mixture was allowed to cool, water was added thereto, and the mixture was extracted with dichloromethane. The organic layer was dried over anhydrous sodium sulfate and then filtered, and the solvent was removed under reduced pressure. The residue was purified by silica gel column chromatography to obtain the title compound (189-2) (180 mg, 0.394 mmol, 72% yield) from a fraction of n-heptane : ethyl acetate (1 : 1).

¹H-NMR(DMSO-d₆) δ:1.37 (9H, s), 3.83 (2H, d, J=6.0Hz), 7.07-7.16 (2H, m), 7.49-7.55 (1H, m), 7.63 (1H, s), 7.70 (1H, s), 8.42-8.47 (1H, m), 8.59 (1H, s), 8.79-8.84 (1H, m), 12.3 (1H, s).

### (3) Synthesis of 2-amino-N-(2'-(pyrazolo[1,5-a]pyridin-3-yl)-[4,4'-bithiazole]-2-yl)acetamide hydrochloride (compound 189)

A 4 M hydrochloric acid/dioxane solution (0.175 mL, 0.700 mmol) was added to a suspension of tert-butyl (2-oxo-2-((2'-(pyrazolo[1,5-a]pyridin-3-yl)-[4,4'-bithiazole]-2-yl)amino)ethyl)carbamate (compound 189-2) (64.0 mg, 0.140 mmol) in methanol (2 mL), and the mixture was stirred at room temperature for 2 hours and then stirred at 50°C for 2 hours. The reaction mixture was concentrated under reduced pressure to remove the solvent. The solid was collected by filtration with a mixed solvent of methanol and ethyl acetate to obtain the title compound (189) (27.1 mg, 69.0 µmmol, 49.3% yield).

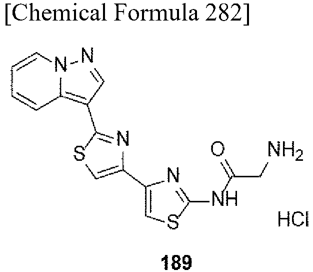

¹H-NMR(DMSO-d₆) δ:3.84-3.92 (2H, m), 7.10 (1H, dt, J=1.3Hz, 6.9Hz), 7.53 (1H, ddd, J=1.1Hz, 6.8Hz, 10.0Hz), 7.66(1H, s), 7.78(1H, s), 8.22-8.33 (2H, m), 8.40-8.45 (1H, m), 8.60 (1H, s), 8.80-8.84 (1H, m), 12.8 (1H, s).

### [Example 190] Synthesis of 2'-(pyrazolo[1,5-a]pyridin-3-yl)-[4,4'-bithiazole]-2-ol (compound 190)

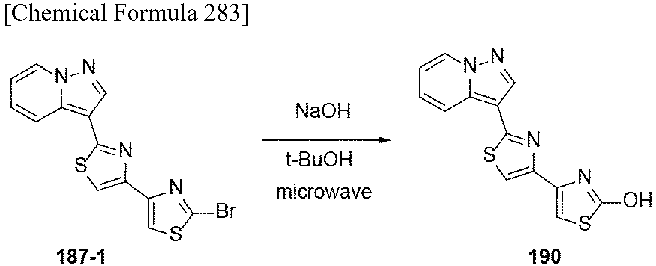

Sodium hydroxide (68.0 mg, 1.70 mmol) was added to a solution of 2-bromo-2'-(pyrazolo[1,5-a]pyridin-3-yl)-4,4'-bithiazole (compound 187-1) (206 mg, 0.567 mmol) in tert-butanol (5.5 mL), and the mixture was heated at 150°C for 75 minutes using a microwave synthesis apparatus. After the reaction mixture was allowed to cool, the reaction mixture was neutralized with 2 M hydrochloric acid under ice cooling, and the precipitated solid was collected by filtration. This solid was washed with a mixed solvent of dichloromethane and methanol to obtain the title compound (190) (150 mg, 0.449 mmol, 88.1% yield).

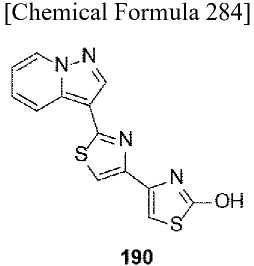

¹H-NMR(DMSO-d₆) δ:8.48 (1H, d, J=8.8Hz), 8.61 (1H, s), 8.84 (1H, d, J=7.2Hz), 6.96 (1H, s), 7.12 (1H, dt, J=1.0Hz, 7.0Hz), 7.51-7.58 (1H, m), 7.72 (1H, s), 12.0 (1H, brs).

### [Example 191] Synthesis of 5-(2-(pyrazolo[1,5-a]pyridin-3-yl)thiazol-4-yl)furan-2-carboxamide (compound 191)

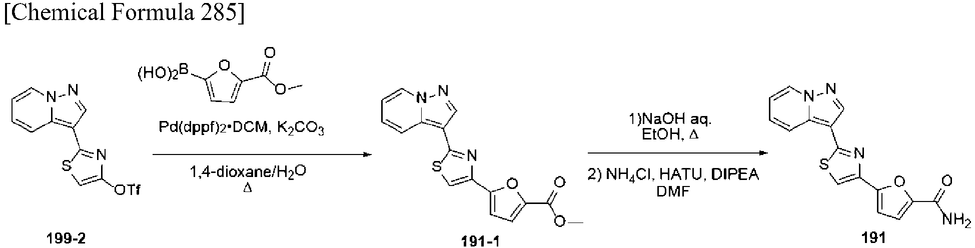

### (1) Synthesis of methyl 5-(2-(pyrazolo[1,5-a]pyridin-3-yl)thiazol-4-yl)furan-2-carboxylate (compound 191-1)

1,4-dioxane (2.5 mL) and water (0.7 mL) were added to 2-(pyrazolo[1,5-a]pyridin-3-yl)thiazol-4-yl trifluoromethanesulfonate (compound 199-2) (204 mg, 0.584 mmol), (5-(methoxycarbonyl)furan-2-yl) boronic acid (121 mg, 0.714 mmol), sodium carbonate (191 mg, 1.80 mmol), and Pd(dppf)₂·DCM (53.4 mg, 0.0653 mmol), and the mixture was heated at 120°C for 20 minutes using a microwave synthesis apparatus. Water was added to the reaction mixture, and the mixture was extracted with ethyl acetate. The organic layer was washed with water and saturated saline, dried over sodium sulfate, and then filtered, and the solvent was removed under reduced pressure. The residue was purified by column chromatography (amino silica gel) to obtain the title compound (191-1) (70.4 mg, 0.216 mmol, 37.0% yield) from a fraction of n-heptane : ethyl acetate (1 : 1).

¹H-NMR(DMSO-d₆) δ:3.87 (3H, s), 7.14 (1H, ddd, J=1.2Hz, 6.8Hz, 6.8Hz), 7.18 (1H, d, J=3.6Hz), 7.48 (1H, d, J=3.6Hz), 7.54-7.62 (1H, m), 7.99 (1H,s), 8.42 (1H, ddd, J=1.6Hz, 1.6Hz, 8.8Hz), 8.67 (1H, s), 8.87 (1H, ddd, J=0.8Hz, 0.8Hz, 7.2Hz).

### (2) Synthesis of 5-(2-(pyrazolo[1,5-a]pyridin-3-yl)thiazol-4-yl)furan-2-carboxamide (compound 191)

Methyl 5-(2-(pyrazolo[1,5-a]pyridin-3-yl)thiazol-4-yl)furan-2-carboxylate (compound 191-1) (34.4 mg, 0.106 mmol) was dissolved in ethanol (2 mL), and a 2 M aqueous sodium hydroxide solution (265 µL, 0.265 mmol) was added, then the mixture was heated and stirred at 50°C for 1 hour. The reaction mixture was neutralized with a 2 M aqueous hydrogen chloride solution, and then the solvent was removed under reduced pressure. HATU (105 mg, 0.277 mmol), ammonium chloride (24.8 mg, 0.463 mmol), DIPEA (100 µL, 0.586 mmol) and DMF (1.5 mL) were added to the residue, and the mixture was stirred at room temperature for 16 hours. Water was added to the reaction mixture, and then the mixture was extracted with ethyl acetate. The organic layer was washed with water and saturated saline, dried over sodium sulfate, and then filtered, and the solvent was removed under reduced pressure. The residue was washed with a mixed solution of dichloromethane and n-heptane and then dried to obtain the title compound (191) (31.1 mg, 0.10 mmol, 94.8% yield).

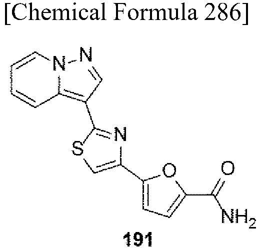

¹H-NMR(DMSO-d₆) δ:7.07 (1H, d, J=3.6Hz), 7.14 (1H, ddd, J=1.2Hz, 6.8Hz, 6.8Hz), 7.22 (1H, d, J=3.6Hz), 7.50 (1H, brs), 7.55-7.62 (1H, m), 7.95 (1H, brs), 7.98 (1H, s), 8.43 (1H, ddd, J=1.2Hz, 1.2Hz, 8.8Hz), 8.65 (1H, s), 8.86 (1H, ddd, J=1.2Hz, 1.2Hz, 7.2Hz).

### [Example 192] Synthesis of 4-(2,5-dihydrofuran-3-yl)-2-(pyrazolo[1,5-a]pyridin-3-yl)thiazole (compound 192)

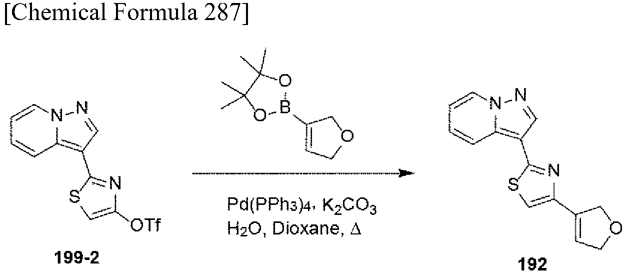

2-(2,5-Dihydrofuran-3-yl)-4,4,5,5-tetramethyl-1,3,2-dioxaborolane (68.0 mg, 0.347 mmol), potassium carbonate (59.9 mg, 0.434 mmol), water (0.6 mL), and Pd(PPh₃)₄ (33.4 mg, 0.0289 mmol) were added to a solution of 2-(pyrazolo[1,5-a]pyridin-3-yl)thiazol-4-yl trifluoromethanesulfonate (compound 199-2) (101 mg, 0.289 mmol) in dioxane (3 mL), and the mixture was heated at 110°C for 10 minutes using a microwave synthesis apparatus. After the reaction mixture was allowed to cool, water was added thereto, and the mixture was extracted with dichloromethane. The organic layer was dried over anhydrous sodium sulfate and then filtered, and the solvent was removed under reduced pressure. The residue was purified by silica gel column chromatography to obtain the title compound (192) (50.0 mg, 0.186 mmol, 64.4% yield) from a fraction of n-heptane : ethyl acetate (1 : 2).

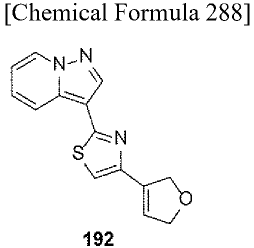

¹H-NMR(DMSO-d₆) δ:4.75-4.78 (2H, m), 4.91-4.93 (2H, m), 6.60-6.61 (1H, m), 7.10-7.14 (1H,), 7.50 (1H, s), 7.53-7.57 (1H, m), 8.35-8.38 (1H, m), 8.60 (1H, s), 8.83-8.85 (1H, m).

### [Example 193] Synthesis of (1-(2-(pyrazolo[1,5-a]pyridin-3-yl)thiazol-4-yl)-1H-imidazol-4-yl)methanol (compound 193)

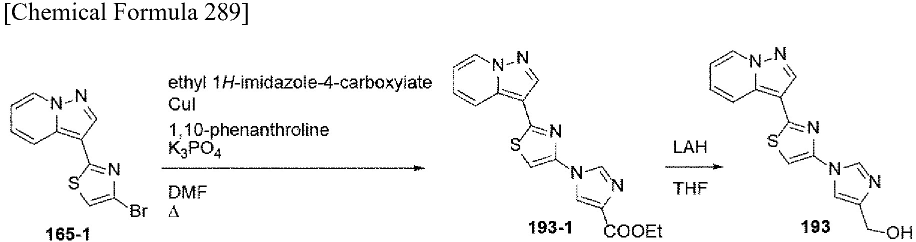

### (1) Synthesis of ethyl 1-(2-(pyrazolo[1,5-a]pyridin-3-yl)thiazol-4-yl)-1H-imidazole-4-carboxylate (compound 193-1)

4-Bromo-2-(pyrazolo[1,5-a]pyridin-3-yl)thiazole (compound 165-1) (340 mg, 1.21 mmol) was dissolved in DMF (5 mL), and ethyl 1H-imidazole-4-carboxylate (337 mg, 2.40 mmol), copper(I) iodide (115 mg, 0.604 mmol), 1,10-phenanthroline (109 mg, 0.605 mmol) and potassium phosphate (521 mg, 0.605 mmol) were added. The reaction mixture was heated at 170°C for 1.5 hours using a microwave heating apparatus. Water (5 mL) was added to the reaction mixture, and the obtained precipitate was collected by suction filtration. The residue was purified by silica gel column chromatography to obtain the title compound (193-1) (117 mg, 0.345 mmol, 28.4% yield) from a fraction of dichloromethane : methanol (20 : 1).

¹H-NMR(CDCl₃) δ:1.44 (3H, t, J=7.1Hz), 4.44 (2H, q, J=7.1Hz), 6.97-7.02 (2H, m), 7.47 (1H, ddd, J=1.1Hz, 6.8Hz, 8.9Hz), 8.21-8.25 (2H, m), 8.34 (1H, ddd, J=1.2Hz, 1.2Hz, 8.8Hz), 8.40 (1H, s), 8.56 (1H, ddd, J=1.0Hz, 1.0Hz, 7.0Hz).

### (2) Synthesis of (1-(2-(pyrazolo[1,5-a]pyridin-3-yl)thiazol-4-yl)-1H-imidazol-4-yl)methanol (compound 193)

Ethyl 1-(2-(pyrazolo[1,5-a]pyridin-3-yl)thiazol-4-yl)-1H-imidazole-4-carboxylate (compound 193-1) (21.4 mg, 0.0631 mmol) was dissolved in dehydrated THF (2 mL), and the solution was ice-cooled under an Ar atmosphere. LAH (8.90 mg, 0.235 mmol) was added, and the reaction mixture was warmed to room temperature and stirred for 2 hours. A saturated aqueous solution of potassium sodium tartrate (3 mL) was added to the reaction mixture, and after stirring, the mixture was extracted with dichloromethane. The organic layer was dried over sodium sulfate, insoluble matter was removed by filtration, and the solvent was removed from the organic layer under reduced pressure. The residue was purified by preparative TLC (preparative thin layer chromatography) to obtain the title compound (193) (10.1 mg, 0.0340 mmol, 53.9% yield) from a fraction of dichloromethane : methanol (20:1).

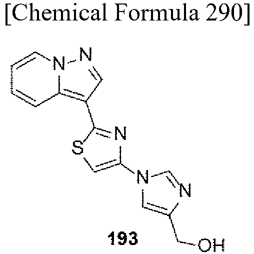

¹H-NMR(DMSO-d₆) δ:4.45 (2H, d, J=5.6Hz), 5.04 (1H, t, J=5.6Hz), 7.12-7.17 (1H, m), 7.56-7.61 (1H, m), 7.66-7.68 (1H, m), 7.72-7.75 (1H, m), 8.40-8.44 (2H, m), 8.67 (1H, s), 8.85-8.88 (1H, m).

### [Example 194] Synthesis of (1-(2-(pyrazolo[1,5-a]pyridin-3-yl)thiazol-4-yl)-1H-imidazol-4-yl)methanamine hydrochloride (compound 194)

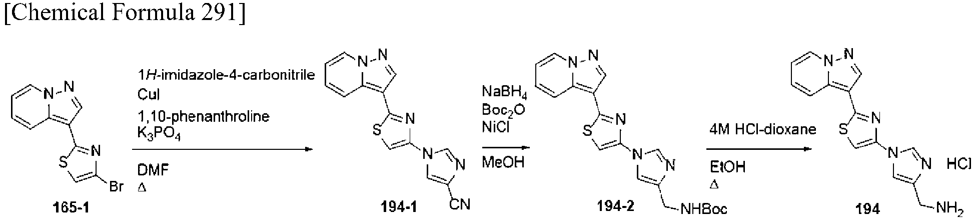

### (1) Synthesis of 1-(2-(pyrazolo[1,5-a]pyridin-3-yl)thiazol-4-yl)-1H-imidazole-4-carbonitrile (compound 194-1)

4-Bromo-2-(pyrazolo[1,5-a]pyridin-3-yl)thiazole (compound 165-1) (500 mg, 1.78 mmol) was dissolved in DMF (10 mL), and 1H-imidazole-4-carbonitrile (336 mg, 3.61 mmol), copper(I) iodide (352 mg, 1.85 mmol), 1,10-phenanthroline (321 mg, 1.78 mmol) and potassium phosphate (792 mg, 3.73 mmol) were added thereto. The reaction mixture was heated at 180°C for 1.5 hours using a microwave heating apparatus. Water (15 mL) was added to the reaction mixture, and the obtained precipitate was collected by suction filtration. The residue was purified by silica gel column chromatography to obtain the title compound (194-1) (131 mg, 0.450 mmol, 25.2% yield) from a fraction of dichloromethane : methanol (20 : 1).

¹H-NMR(DMSO-d₆) δ:7.13-7.19 (1H, m), 7.55-7.61 (1H, m), 7.86 (1H, s), 8.49-8.53 (1H, m), 8.71 (1H, s), 8.74-8.76 (1H, m), 8.85-8.89 (1H, m), 8.95-8.97 (1H, m).

### (2) Synthesis of tert-butyl ((1-(2-(pyrazolo[1,5-a]pyridin-3-yl)thiazol-4-yl)-1H-imidazol-4-yl)methyl)carbamate (compound 194-2)

1-(2-(Pyrazolo[1,5-a]pyridin-3-yl)thiazol-4-yl)-1H-imidazole-4-carbonitrile (compound 194-1) (78.0 mg, 0.267 mmol) was dissolved in methanol (10 mL), and nickel(II) chloride hexahydrate (50.2 mg, 0.211 mmol) and Boc₂O (208 mg, 0.963 mmol) were added thereto. The mixture was ice-cooled, and then sodium borohydride (78.1 mg, 2.06 mmol) was added thereto. The reaction mixture was heated to room temperature and stirred for 5 hours. Water (15 mL) was added to the reaction mixture, and the mixture was extracted with dichloromethane. The organic layer was dried over sodium sulfate, the insoluble matter was removed by filtration, and the solvent was removed from the organic layer under reduced pressure. The residue was purified by preparative TLC (preparative thin layer chromatography) to obtain the title compound (194-2) (16.5 mg, 0.0416 mmol, 15.6% yield) from a fraction of dichloromethane : methanol (30 : 1).

¹H-NMR(DMSO-d₆) δ:1,41 (9H, s), 4.06-4.14 (2H, m), 7.15 (1H, ddd, J=1.3Hz, 6.9Hz, 6.9Hz), 7.17-7.24 (1H, m), 7.57 (1H, ddd, J=1.0Hz, 6.9Hz, 8.9Hz), 7.62-7.69 (1H, m), 7.68 (1H, s), 8.36-8.45 (2H, m), 8.67 (1H, s), 8.85-8.89 (1H, m).

### (3) Synthesis of (1-(2-(pyrazolo[1,5-a]pyridin-3-yl)thiazol-4-yl)-1H-imidazol-4-yl)methanamine hydrochloride (compound 194)

tert-Butyl ((1-(2-(pyrazolo[1,5-a]pyridin-3-yl)thiazol-4-yl)-1H-imidazol-4-yl)methyl)carbamate (compound 194-2) (16.5 mg, 0.0416 mmol) was dissolved in ethanol (2 mL), 4 M hydrochloric acid-dioxane (0.4 mL) was added thereto, and the mixture was stirred at 50°C for 5 hours. Diethyl ether (15 mL) was added to the reaction mixture, and the precipitated solid was collected by filtration and dried to obtain the title compound (194) (8.90 mg, 0.0267 mmol, 64.3% yield).

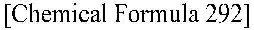

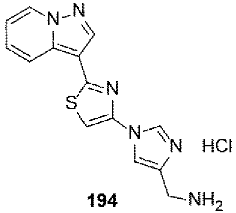

¹H-NMR(CD₃OD) δ:4.42 (2H, s), 7.12-7.17 (1H, m), 7.57-7.62 (1H, m), 7.94 (1H, s), 8.44-8.51 (2H, m), 8.58 (1H, s), 8.69-8.72 (1H, m), 9.84-9.86 (1H, m).

### [Example 195] Synthesis of 3-((1-(2-(pyrazolo[1,5-a]pyridin-3-yl)thiazol-4-yl)-1H-imidazol-4-yl)methoxy)propan-1-ol (compound 195)

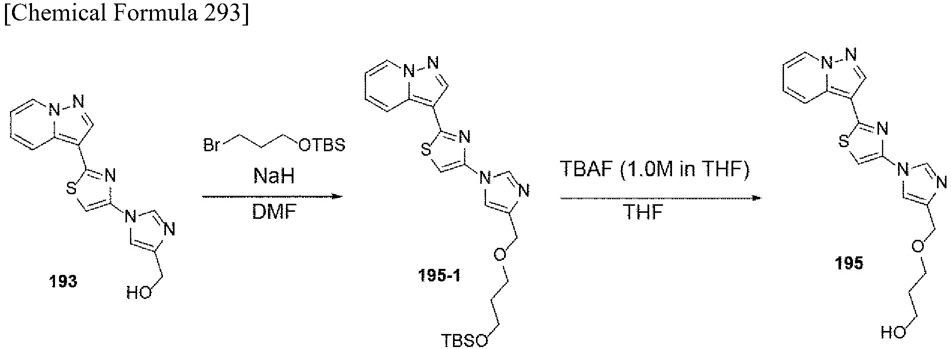

### (1) Synthesis of 4-(4-((3-((tert-butyldimethylsilyl)oxy)propoxy)methyl)-1H-imidazol-1-yl)-2-(pyrazolo[1,5-a]pyridin-3-yl)thiazole (compound 195-1)

Sodium hydride (16.3 mg, 0.678 mmol) was added to a solution of (1-(2-(pyrazolo[1,5-a]pyridin-3-yl)thiazol-4-yl)-1H-imidazol-4-yl)methanol (compound 193) (80.6 mg, 0.271 mmol) in DMF (2.7 mL) under ice cooling, and the mixture was stirred for 30 minutes, then (3-bromopropoxy)(tert-butyl) dimethylsilane (93.7 mg, 0.407 mmol) was added thereto. The mixture was stirred at room temperature for 19 hours, then water was added to stop the reaction, and the mixture was extracted with a mixed solution of n-heptane and ethyl acetate (1 : 1) and DCM. The organic layer was dried over anhydrous sodium sulfate and then filtered, and the solvent was removed under reduced pressure. The residue was purified by silica gel column chromatography to obtain the title compound (195-1) (73.1 mg, 0.156 mmol, 57.4% yield) from a fraction of dichloromethane : methanol (10 : 1).

¹H-NMR(CDCl₃) δ:0.038 (6H, s), 0.87 (9H, s), 1.82-1.88 (2H, m), 3.66 (2H, t, J=6.4), 3.72 (2H, t, J=6.4), 4.54 (2H, s), 6.84 (1H, s), 6.95 (1H, ddd, J=1.2Hz, 6.8Hz, 6.8Hz), 7.41 (1H, ddd, J=1.2Hz, 6.8Hz, 9.2Hz), 7.47-7.48 (1H, m), 8.18 (1H, d, J=1.6Hz), 8.32 (1H, ddd, J=1.2Hz, 1.2Hz, 8.8Hz), 8.37 (1H, s), 8.53 (1H, ddd, J=0.8Hz, 0.8Hz, 6.8Hz).

### (2) Synthesis of 3-((1-(2-(pyrazolo[1,5-a]pyridin-3-yl)thiazol-4-yl)-1H-imidazol-4-yl)methoxy)propan-1-ol (compound 195)

A 1M tetrabutylammonium fluoride THF solution (0.47 mL, 0.467 mmol) was added to a solution of 4-(4-((3-((tert-butyldimethylsilyl)oxy)propoxy)methyl)-1H-imidazol-1-yl)-2-(pyrazolo[1,5-a]pyridin-3-yl)thiazole (compound 195-1) (73.1 mg, 0.156 mmol) in THF (1.6 mL), and the mixture was stirred at room temperature for 2.5 hours. The reaction mixture was diluted with ethyl acetate, and then washed with a saturated aqueous ammonium chloride solution and saturated saline. The organic layer was dried over anhydrous sodium sulfate and then filtered, and the solvent was removed under reduced pressure. The residue was purified by silica gel column chromatography to obtain the title compound (195) (25.2 mg, 0.0709 mmol, 45.5% yield) from a fraction of dichloromethane : methanol (10 : 1).

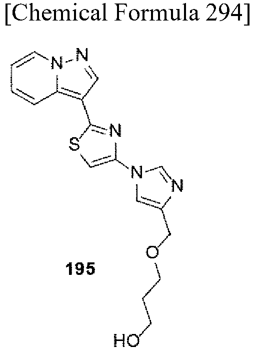

¹H-NMR(CDCl₃) δ:1.86-1.92 (2H, m), 3.81 (4H, ddd, J=2.0Hz, 5.6Hz, 5.6Hz), 4.59 (2H, d, J=0.4Hz), 6.87 (1H, s), 6.97 (1H, ddd, J=1.6Hz, 6.8Hz, 6.8Hz), 7.44 (1H, ddd, J=1.2Hz, 6.8Hz, 9.2Hz), 7.48 (1H, s), 8.22 (1H, d, J=1.2Hz), 8.34 (1H, ddd, J=1.2Hz, 1.2Hz, 8.8Hz), 8.39 (1H, s), 8.55 (1H, ddd, J=0.8Hz, 0.8Hz, 6.8Hz).

### [Example 196] Synthesis of 3-(4-(2-(pyrazolo[1,5-a]pyridin-3-yl)thiazol-4-yl)-1H-imidazol-1-yl)propan-1-ol (compound 196)

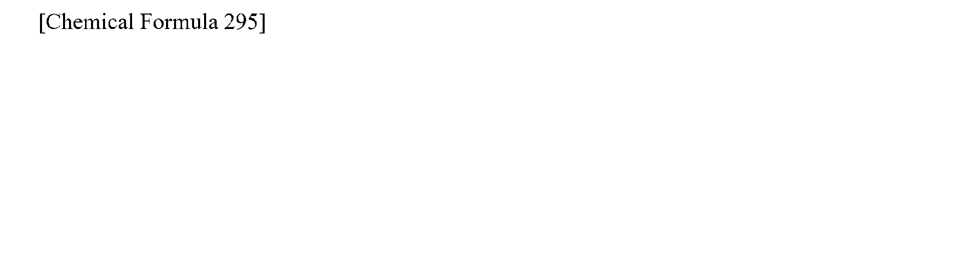

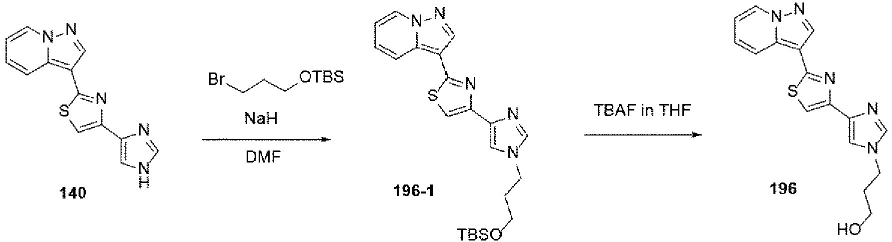

### (1) Synthesis of 4-(1-(3-((tert-butyldimethylsilyl)oxy)propyl)-1H-imidazol-4-yl)-2-(pyrazolo[1,5-a]pyridin-3-yl)thiazole (compound 196-1)

(3-Bromopropoxy)(tert-butyl)dimethylsilane (0.715 mL, 3.10 mmol) and sodium hydride (oil dispersion) (166 mg, 4.14 mmol) were added to a solution of 4-(1H-imidazol-4-yl)-2-(pyrazolo[1,5-a]pyridin-3-yl)thiazole (compound 140) (553.1 mg, 2.07 mmol) in DMF (10 mL) under ice cooling, and the mixture was stirred at 0°C for 2 hours. Water was added to the reaction mixture under ice cooling, and the mixture was extracted with ethyl acetate. The organic layer was dried over anhydrous sodium sulfate and then filtered, and the solvent was removed under reduced pressure. The residue was purified by preparative TLC (preparative thin layer chromatography) to obtain the title compound (196-1) (190 mg, 0.432 mmol, 20.9% yield) from a fraction of dichloromethane : methanol (20 : 1).

¹H-NMR(CDCl₃) δ:0.07 (6H, s), 0.93 (9H, s), 1.96-2.04 (2H, m), 3.62 (2H, t, J=5.6Hz), 4.13 (2H, t, J=6.8Hz), 6.90 (1H, dt, J=1.5Hz, 6.8Hz), 7.34 (1H, ddd, J=0.9Hz, 6.7Hz, 8.0Hz), 7.48-7.51 (3H, m), 8.37 (1H, ddd, J=1.0Hz, 1.0Hz, 8.8Hz), 8.40 (1H, s), 8.51 (1H, ddd, J=0.9Hz, 0.9Hz, 7.2Hz).

### (2) Synthesis of 3-(4-(2-(pyrazolo[1,5-a]pyridin-3-yl)thiazol-4-yl)-1H-imidazol-1-yl)propan-1-ol (compound 196)

Tetrabutylammonium fluoride (1 M THF solution) was added to a solution of 4-(1-(3-((tert-butyldimethylsilyl)oxy)propyl)-1H-imidazol-4-yl)-2-(pyrazolo[1,5-a]pyridin-3-yl)thiazole (compound 196-1) (189 mg, 0.430 mmol) in THF (5 mL) under ice cooling, and the mixture was stirred at room temperature for 2 hours. A saturated aqueous ammonium chloride solution was added to the reaction mixture, and the mixture was extracted with ethyl acetate. The organic layer was dried over anhydrous sodium sulfate and then filtered, and the solvent was removed under reduced pressure. The residue was purified by silica gel column chromatography to obtain the title compound (196) (98.2 mg, 0.302 mol, 70.2% yield) from a fraction of dichloromethane : methanol (10 : 1).

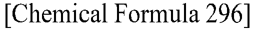

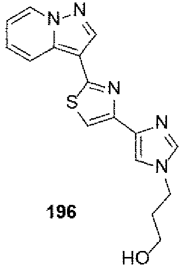

¹H-NMR(DMSO-d₆) δ:1.84-1.93 (2H, m), 3.37 (2H, q, J=5.7Hz), 4.06 (2H, t, J=7.0Hz), 4.62 (1H, t, J=5.0Hz), 7.07 (1H, dt, J=1.5Hz, 6.9Hz), 7.47 (1H, s), 7.50 (1H, ddd, J=1.0Hz, 6.7Hz, 8.0Hz), 7.64-7.67 (2H, m),8.40 (1H, ddd, J=1.2Hz, 1.2Hz, 8.8Hz), 8.54(1H, s), 8.80 (1H, ddd, J=1.0Hz, 1.0Hz, 6.8Hz).

### [Example 197] Synthesis of 2-(4-(2-(pyrazolo[1,5-a]pyridin-3-yl)thiazol-4-yl)-1H-imidazol-1-yl)ethan-1-amine (compound 197)

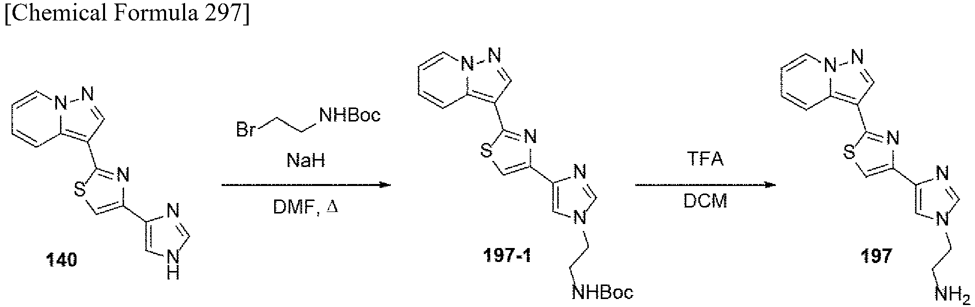

### (1) Synthesis of tert-butyl (2-(4-(2-(pyrazolo[1,5-a]pyridin-3-yl)thiazol-4-yl)-1H-imidazol-1-yl)ethyl)carbamate (compound 197-1)

Sodium hydride (67.2 mg, 2.80 mmol) was added to a solution of 4-(1H-imidazol-4-yl)-2-(pyrazolo[1,5-a]pyridin-3-yl)thiazole (compound 140) (300 mg, 1.12 mmol) in DMF (11.2 mL) under ice cooling, and the mixture was stirred for 30 minutes, then 2-(tert-butoxycarbonylamino)ethyl bromide (251 mg, 1.12 mmol) was added thereto. The mixture was stirred at room temperature for 16 hours, then stirred at 60°C for 2.5 hours, and further stirred at 70°C for 4 hours. After the reaction mixture was allowed to cool, water was added thereto, and the mixture was extracted with a mixed solution of n-heptane and ethyl acetate (1 : 1). The organic layer was dried over anhydrous sodium sulfate and then filtered, and the solvent was removed under reduced pressure. The residue was purified by silica gel column chromatography to obtain the title compound (197-1) (152 mg, 0.370 mmol, 33.1% yield) from a fraction of dichloromethane : methanol (10 : 1).

¹H-NMR(CDCl₃) δ:1.46 (9H, s), 3.48-3.52 (2H, m), 4.14-4.15 (2H, m), 6.90 (1H, ddd, J=1.2Hz, 6.8Hz, 6.8Hz), 7.35 (1H, ddd, J=0.8Hz, 6.8Hz, 8.8Hz), 7.50 (3H, m), 8.34 (1H, d, J=9.2Hz), 8.38 (1H, s), 8.51 (1H, d, J=6.8Hz).

### (2) Synthesis of 2-(4-(2-(pyrazolo[1,5-a]pyridin-3-yl)thiazol-4-yl)-1H-imidazol-1-yl)ethan-1-amine (compound 197)

TFA (0.1 mL) was added to a solution of tert-butyl (2-(4-(2-(pyrazolo[1,5-a]pyridin-3-yl)thiazol-4-yl)-1H-imidazol-1-yl)ethyl)carbamate (compound 197-1) (39.2 mg, 0.0955 mmol) in dichloromethane (1.0 mL), and the mixture was stirred at room temperature for 17.5 hours. The reaction mixture was neutralized with a saturated aqueous sodium bicarbonate solution and extracted twice with dichloromethane. The organic layer was dried over anhydrous sodium sulfate and then filtered, and the solvent was removed under reduced pressure. The residue was purified by silica gel column chromatography to obtain a crude product from a fraction of dichloromethane : methanol (10 : 1). The crude product was subjected to preparative TLC (preparative thin layer chromatography) to obtain the title compound (197) (3.10 mg, 0.00999 mmol, 10.5% yield) from a fraction of dichloromethane : methanol (10 : 1).

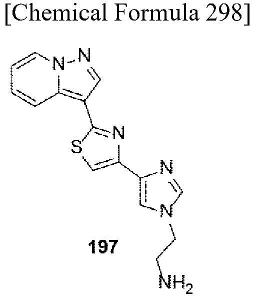

¹H-NMR(CDCl₃) δ:3.14 (2H, t, J=5.6Hz), 4.06 (2H, t, J=5.6Hz), 6.89-6.92 (1H, m), 7.34-7.38 (1H, m), 7.52 (1H, s), 7.55-7.57 (2H, m), 8.35-3.38 (1H, m), 8.41 (1H, s), 8.51-8.53 (1H, m).

### [Example 198] Synthesis of N-(2-(4-(2-(pyrazolo[1,5-a]pyridin-3-yl)thiazol-4-yl)-1H-imidazol-1-yl)ethyl)acetamide (compound 198)

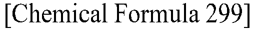

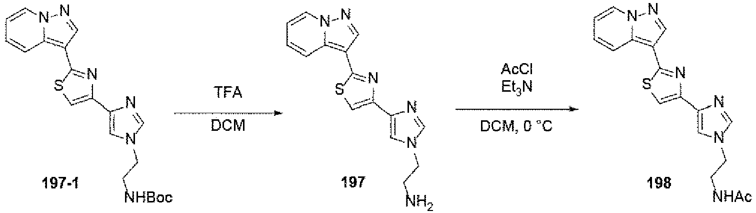

TFA (0.3 mL) was added to a solution of tert-butyl (2-(4-(2-(pyrazolo[1,5-a]pyridin-3-yl)thiazol-4-yl)-1H-imidazol-1-yl)ethyl)carbamate (compound 197-1) (31.2 mg, 0.0760 mmol) in dichloromethane (1.5 mL), and the mixture was stirred at room temperature for 6 hours. Toluene was added to the reaction mixture, and the mixture was concentrated under reduced pressure. The mixture was diluted with dichloromethane, neutralized with a saturated aqueous sodium bicarbonate solution, and extracted three times with dichloromethane. The organic layer was dried over anhydrous sodium sulfate and then filtered, and the solvent was removed under reduced pressure. Triethylamine (31.9 µL, 0.288 mmol) and acetyl chloride (10.8 µL, 0.152 mmol) were added to a solution of the residue in dichloromethane (1.5 mL) under ice cooling, and the mixture was stirred for 25 minutes. The reaction mixture was diluted with water and extracted three times with dichloromethane. The organic layer was dried over anhydrous sodium sulfate and then filtered, and the solvent was removed under reduced pressure. The residue was subjected to preparative TLC (preparative thin layer chromatography) to obtain the title compound (198) (18.1 mg, 0.0514 mmol, 67.6% yield over two steps) from a fraction of dichloromethane : methanol (10 : 1).

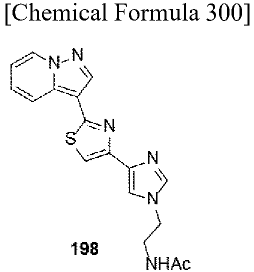

¹H-NMR(CDCl₃) δ:2.01 (3H, s), 3.63 (2H, q, J=6.0Hz, 5.6Hz), 4.17 (2H, t, J=5.2Hz), 6.91 (1H, ddd, J=1.2Hz, 7.2Hz, 7.2Hz), 7.36 (1H, ddd, J=0.8Hz, 6.8Hz, 8.8Hz), 7.46-7.47 (3H, m), 8.32-8.35 (1H, m), 8.38 (1H, s), 8.50-8.52 (1H, m).

### [Example 199] Synthesis of 2-(pyrazolo[1,5-a]pyridin-3-yl)-4-(pyrimidin-5-yl)thiazole (compound 199)

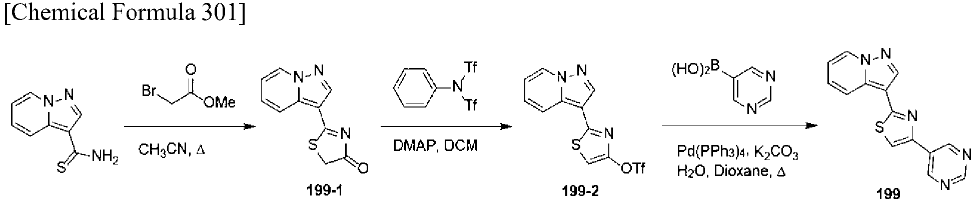

### (1) Synthesis of 2-(pyrazolo[1,5-a]pyridine-3-yl)thiazole-4(5H)-one (compound 199-1)

Methyl bromoacetate (8.98 mL, 58.7 mmol) was added to a solution of pyrazolo[1,5-a]pyridin-3-carbothioamide (10.4 g, 58.7 mmol) in acetonitrile (400 mL), and the mixture was stirred at reflux for 1 hour, then water (300 mL) was added thereto, and the mixture was stirred for 10 minutes. After cooling, the reaction mixture was extracted with dichloromethane. The organic layer was dried over anhydrous sodium sulfate and then filtered, and the solvent was removed under reduced pressure. The residue was suspended in diethyl ether, then collected by filtration, and dried to obtain the title compound (compound 199-1) (12.0 g, 55.2 mmol, 94.0% yield).

¹H-NMR(DMSO-d₆) δ:4.18 (2H, s), 7.32-7.36 (1H, m), 7.79-7.84 (1H, m), 8.35-8.37 (1H, m), 8.88 (1H, s), 9.01-9.03 (1H, m).

### (2) Synthesis of 2-(pyrazolo[1,5-a]pyridin-3-yl)thiazol-4-yl trifluoromethanesulfonate (compound 199-2)

DMAP (5.20 g, 42.6 mmol) was added to a solution of 2-(pyrazolo[1,5-a]pyridin-3-yl)thiazole-4(5H)-one (compound 199-1) (6.16 g, 28.4 mmol) in dichloromethane (300 mL) under ice cooling, then a solution of N-phenylbis(trifluoromethanesulfonimide) (12.2 g, 34.2 mmol) in dichloromethane (100 mL) was added dropwise thereto over 40 minutes, and the mixture was stirred for 15 minutes. Water was added to the reaction mixture, and then the mixture was extracted with dichloromethane. The organic layer was dried over anhydrous sodium sulfate and then filtered, and the solvent was removed under reduced pressure. The residue was purified by silica gel column chromatography (amino silica gel), and the title compound (199-2) (7.49 g, 21.4 mmol, 75.4% yield) was obtained from a fraction of n-heptane : ethyl acetate (2 : 1).

¹H-NMR(DMSO-d₆) δ:7.14-7.18 (1H, m), 7.60-7.65 (1H, m), 7.81 (1H, s), 8.13-8.16 (1H, m), 8.70 (1H, s), 8.87-8.90 (1H, m).

### (3) Synthesis of 2-(pyrazolo[1,5-a]pyridin-3-yl)-4-(pyrimidin-5-yl)thiazole (compound 199)

5-Pyrimidylboronic acid (43.8 mg, 0.354 mmol), potassium carbonate (61.0 mg, 0.442 mmol), water (0.6 mL), and Pd(PPh₃)₄ (34.1 mg, 0.0295 mmol) were added to a solution of 2-(pyrazolo[1,5-a]pyridin-3-yl)thiazol-4-yl trifluoromethanesulfonate (compound 199-2) (103 mg, 0.295 mmol) in dioxane (3 mL), and the mixture was heated at 110°C for 10 minutes using a microwave synthesis apparatus. After the reaction mixture was allowed to cool, water was added thereto, and the resulting solid was collected by filtration and then purified by silica gel column chromatography to obtain the title compound (199) (24.0 mg, 0.0859 mmol, 29.1% yield) from a fraction of n-heptane: ethyl acetate (1 : 1).

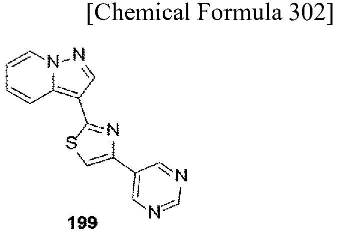

¹H-NMR(DMSO-d₆) δ:7.13-7.17 (1H, m), 7.58-7.62 (1H, m), 8.36 (1H, s), 8.51-8.53 (1H, m), 8.69 (1H, s), 8.86-8.88 (1H, m), 9.20 (1H, s), 9.49 (2H, s).

### [Example 200] Synthesis of 2-(pyrazolo[1,5-a]pyridin-3-yl)-4-(pyrimidin-2-yl)thiazole (compound 200)

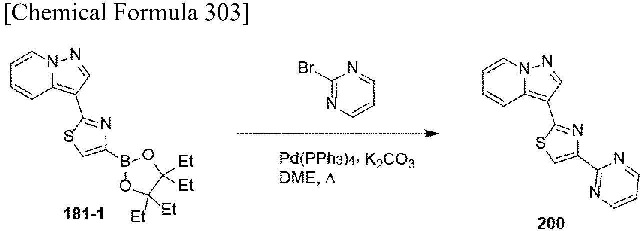

2-Bromopyrimidine (35.6 mg, 0.224 mmol), potassium carbonate (42.2 mg, 0.306 mmol) and Pd(PPh₃)₄ (23.5 mg, 0.0203 mmol) were added to a solution of 2-(pyrazolo[1,5-a]pyridin-3-yl)-4-(4,4,5,5-tetraethyl-1,3,2-dioxaborolan-2-yl)thiazole (compound 181-1) (78.1 mg, 0.204 mmol) in DME (2 mL), and the mixture was heated at 160°C for 10 minutes using a microwave synthesis apparatus. After the reaction mixture was allowed to cool, water was added thereto, and the mixture was extracted with dichloromethane. The organic layer was dried over anhydrous sodium sulfate and then filtered, and the solvent was removed under reduced pressure. The residue was purified by silica gel column chromatography (amino silica gel), and the title compound (200) (12.0 mg, 0.0430 mmol, 21.1% yield) was obtained from a fraction of n-heptane : ethyl acetate (1 : 10).

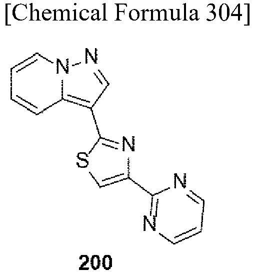

¹H-NMR(DMSO-d₆) δ:7.12-7.16 (1H, m), 7.47-7.50 (1H, m), 7.59-7.63 (1H, m), 8.42-8.44 (1H, m), 8.44 (1H, s), 8.67 (1H, s), 8.85-8.87 (1H, m), 8.93-8.95 (2H, m).

### [Example 201] Synthesis of 4-(2,3-dihydro-1H-pyrrolo[2,3-b]pyridin-5-yl)-2-(pyrazolo[1,5-a]pyridin-3-yl)thiazole (compound 201)

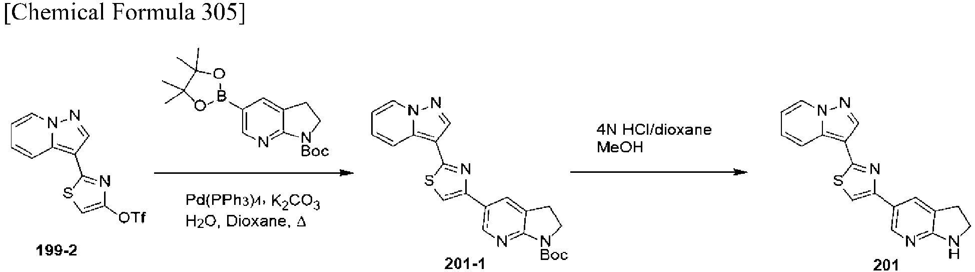

### (1) Synthesis of tert-butyl 5-(2-(pyrazolo[1,5-a]pyridin-3-yl)thiazol-4-yl)-2,3-dihydro-1H-pyrrolo[2,3-b]pyridine-1-carboxylate (compound 201-1)

tert-Butyl 5-(4,4,5,5-tetramethyl-1,3,2-dioxaborolan-2-yl)-2,3-dihydro-1H-pyrrolo[2,3-b]pyridine-1-carboxylate (180 mg, 0.520 mmol), potassium carbonate (95.5 mg, 0.692 mmol), water (0.6 mL), and Pd(PPh₃)₄ (40.0 mg, 0.0346 mmol) were added to a solution of 2-(pyrazolo[1,5-a]pyridin-3-yl)thiazol-4-yl trifluoromethanesulfonate (compound 199-2) (121 mg, 0.346 mmol) in dioxane (3 mL), and the mixture was heated at 100°C for 10 minutes using a microwave synthesis apparatus. After the reaction mixture was allowed to cool, water was added thereto, and the mixture was extracted with dichloromethane. The organic layer was dried over anhydrous sodium sulfate and then filtered, and the solvent was removed under reduced pressure. The residue was purified by silica gel column chromatography (amino silica gel), and the title compound (201-1) (140 mg, 0.334 mmol, 96.5% yield) was obtained from a fraction of n-heptane : ethyl acetate (1 : 2).

¹H-NMR(DMSO-d₆) δ:1.52 (9H, s), 3.13 (2H, t, J=8.7Hz), 3.99 (2H, t, J=8.7Hz), 7.11-7.15 (1H, m), 7.56-7.62 (1H, m), 8.00 (1H, s), 8.19-8.20 (1H, m), 8.44-8.46 (1H, m), 8.64 (1H, s), 8.81-8.82 (1H, m), 8.84-8.86 (1H, m).

### (2) Synthesis of 4-(2,3-dihydro-1H-pyrrolo[2,3-b]pyridin-5-yl)-2-(pyrazolo[1,5-a]pyridin-3-yl)thiazole (compound 201)

A 4 N hydrochloric acid dioxane solution (2 mL) was added to a solution of tert-butyl 5-(2-(pyrazolo[1,5-a]pyridin-3-yl)thiazol-4-yl)-2,3-dihydro-1H-pyrrolo[2,3-b]pyridine-1-carboxylate (compound 201-1) (140 mg, 0.334 mmol) in methanol (4 mL), and the mixture was stirred at 70°C for 1 hour. After the mixture was allowed to cool, a saturated aqueous sodium bicarbonate solution was added to adjust the pH to 9, and then the mixture was extracted with dichloromethane. The organic layer was dried over anhydrous sodium sulfate and then filtered, and the solvent was removed under reduced pressure. The residue was suspended in methanol, then collected by filtration, and dried to give the title compound (201) (82.3 mg, 0.258 mmol, 77.2% yield).

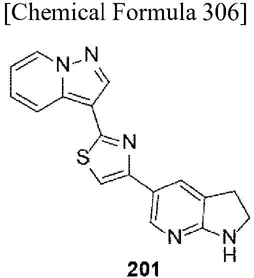

¹H-NMR(DMSO-d₆) δ:3.07 (2H, t, J=8.3Hz), 3.54 (2H, t, J=8.3Hz), 6.63 (1H, s), 7.10-7.14 (1H, m), 7.55-7.60 (1H, m), 7.71 (1H, s), 7.88-7.90 (1H, m), 8.39-8.42 (1H, m), 8.47-8.49 (1H, m), 8.60 (1H, s), 8.83-8.85 (1H, m).

### [Example 202] Synthesis of 2-(pyrazolo[1,5-a]pyridin-3-yl)-4-(5,6,7,8-tetrahydroimidazo[1,2-a]pyridin-2-yl)thiazole (compound 202)

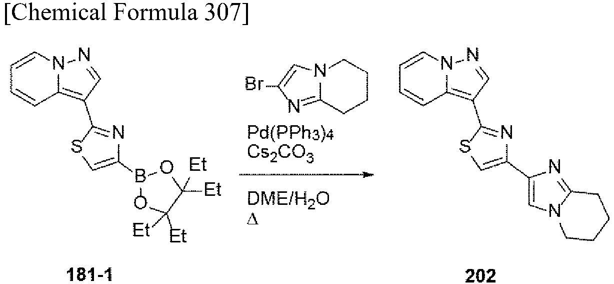

2-(pyrazolo[1,5-a]pyridin-3-yl)-4-(4,4,5,5-tetraethyl-1,3,2-dioxaborolan-2-yl)thiazole (compound 181-1) (60.1 mg, 0.157 mmol) was dissolved in DME (2 mL), and 2-bromo-5,6,7,8-tetrahydroimidazo[1,2-a]pyridine (38.8 mg, 0.193 mmol), Pd(PPh₃)₄ (20.5 mg, 0.0177 mmol), cesium carbonate (107 mg, 0.328 mmol) and water (0.4 mL) were added thereto. The reaction mixture was heated at 130°C for 15 minutes using a microwave heating apparatus. Water (5 mL) was added to the reaction mixture, and the mixture was extracted with ethyl acetate. The organic layer was dried over sodium sulfate, the insoluble matter was removed by filtration, and the solvent was removed from the organic layer under reduced pressure. The residue was purified by silica gel column chromatography, and the title compound (202) (21.9 mg, 0.0681 mmol, 43.5% yield) was obtained from a fraction of n-heptane : ethyl acetate (1 : 1).

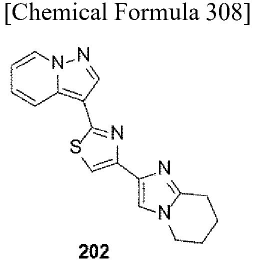

¹H-NMR(CDCl₃) δ:1.92-2.08 (4H, m), 2.91-2.97 (2H, m), 3.99-4.04 (2H, m), 6.89 (1H, ddd, J=1.4Hz, 6.8Hz, 6.8Hz), 7.34 (1H, ddd, J=1.2Hz, 6.8Hz, 8.9Hz), 7.36 (1H, s), 7.47 (1H, s), 8.36 (1H, ddd, J=1.2Hz, 1.2Hz, 8.9Hz), 8.40 (1H, s), 8.50 (1H, ddd, J=1.0Hz, 1.0Hz, 7.0Hz).

### [Example 203] Synthesis of 2-(2-(pyrazolo[1,5-a]pyridin-3-yl)thiazol-4-yl)-5,6-dihydro-8H-imidazo[2,1-c][1,4]oxazine (compound 203)

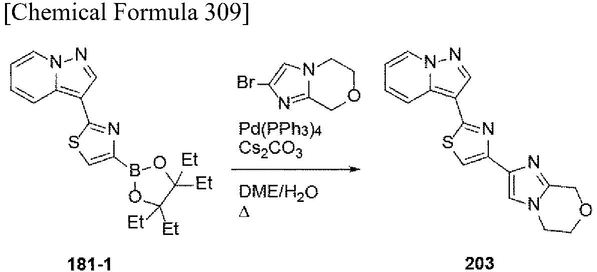

2-(pyrazolo[1,5-a]pyridin-3-yl)-4-(4,4,5,5-tetraethyl-1,3,2-dioxaborolan-2-yl)thiazole (compound 181-1) (61.5 mg, 0.160 mmol) was dissolved in DME (2 mL), and 2-bromo-5,6-dihydro-8H-imidazo[2,1-c][1,4]oxazine (40.6 mg, 0.199 mmol), tetrakis(triphenylphosphine)palladium(0) (20.8 mg, 0.0180 mmol), cesium carbonate (130 mg, 0.399 mmol) and water (0.4 mL) were added thereto. The reaction mixture was heated at 130°C for 15 minutes using a microwave heating apparatus. Water (5 mL) was added to the reaction mixture, and the mixture was extracted with ethyl acetate. The organic layer was dried over sodium sulfate, the insoluble matter was removed by filtration, and the solvent was removed from the organic layer under reduced pressure. The residue was purified by silica gel column chromatography to obtain the title compound (203) (17.2 mg, 0.0532 mmol, 33.2% yield) from a fraction of dichloromethane : methanol (20 : 1).

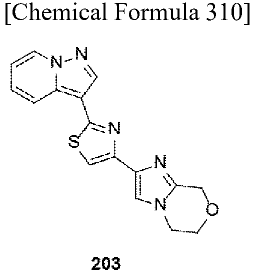

¹H-NMR(DMSO-d₆) δ:4.01-4.16 (4H, m), 4.79 (2H, s), 7.11 (1H, ddd, J=1.4Hz, 6.9Hz, 6.9Hz), 7.49 (1H, s), 7.54 (1H, ddd, J=1.0Hz, 6.8Hz, 8.9Hz), 7.67 (1H, s), 8.41-8.46 (1H, m), 8.58 (1H, s), 8.82-8.86 (1H, m).

### [Example 204] Synthesis of 2-(2-(pyrazolo[1,5-a]pyridin-3-yl)thiazol-4-yl)-4,5,6,7-tetrahydrothiazolo[5,4-c]pyridine (compound 204)

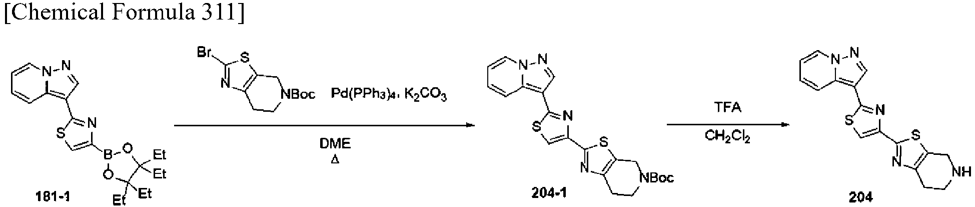

### (1) Synthesis of tert-butyl 2-(2-(pyrazolo[1,5-a]pyridin-3-yl)thiazol-4-yl)-6,7-dihydrothiazolo[5,4-c]pyridine-5(4H)-carboxylate (compound 204-1)

DME (0.8 mL) was added to 2-(pyrazolo[1,5-a]pyridin-3-yl)-4-(4,4,5,5-tetraethyl-1,3,2-dioxaborolan-2-yl)thiazole (compound 181-1) (47.9 mg, 0.125 mmol), tert-butyl 2-bromo-6,7-dihydrothiazolo[5,4-c]pyridine-5(4H)-carboxylate (47.0 mg, 0.147 mmol), potassium carbonate (57.0 mg, 0.412 mmol), and Pd(PPh₃)₄ (15.4 mg, 0.0133 mmol), and the mixture was heated at 120°C for 20 minutes using a microwave synthesis apparatus. Water was added to the reaction mixture, and the mixture was extracted with ethyl acetate. The organic layer was washed with water and saturated saline, dried over sodium sulfate, and then filtered, and the solvent was removed under reduced pressure. The residue was purified by silica gel column chromatography to obtain the title compound (204-1) (20.6 mg, 0.0469 mmol, 37.5% yield) from a fraction of n-heptane : ethyl acetate (1 : 1).

¹H-NMR(CDCl₃) δ:1.51 (9H, s), 2.92-3.01 (2H, m), 3.76-3.84 (2H, m), 4.69-4.75 (2H, m), 6.96 (1H, ddd, J=1.2Hz, 6.8Hz, 6.8Hz), 7.38-7.45 (1H, m), 7.79 (1H, s), 8.39 (1H, s), 8.41-8.46 (1H, m), 8.52-8.56 (1H, m).

### (2) Synthesis of 2-(2-(pyrazolo[1,5-a]pyridin-3-yl)thiazol-4-yl)-4,5,6,7-tetrahydrothiazolo[5,4-c]pyridine (compound 204)

tert-Butyl 2-(2-(pyrazolo[1,5-a]pyridin-3-yl)thiazol-4-yl)-6,7-dihydrothiazolo[5,4-c]pyridine-5(4H)-carboxylate (compound 204-1) (20.6 mg, 0.0468 mmol) was dissolved in dichloromethane (1 mL), trifluoroacetic acid (0.5 mL) was added thereto, and the mixture was stirred at room temperature for 2 hours. The reaction mixture was neutralized with a saturated aqueous sodium bicarbonate solution, and then extracted with dichloromethane. The organic layer was dried over sodium sulfate and then filtered, and the solvent was removed under reduced pressure. The residue was purified by column chromatography (amino silica gel) to obtain the title compound (204) (10.3 mg, 0.0303 mmol, 64.8% yield) from a fraction of dichloromethane : methanol (20 : 1).

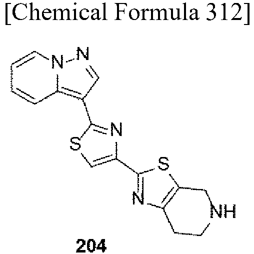

¹H-NMR(CDCl₃) δ:2.88-2.94 (2H, m), 3.25 (2H, t, J=6.0Hz), 4.14 (2H, t, J=3.0Hz), 6.95 (1H, ddd, J=1.2Hz, 6.8Hz, 6.8Hz), 7.38-7.44 (1H, m), 7.79 (1H, s), 8.39 (1H, s), 8.44 (1H, ddd, J=1.2Hz, 1.2Hz, 9.2Hz), 8.54 (1H, ddd, J=1.2Hz, 1.2Hz, 6.8Hz).

### [Example 205] Synthesis of 2-(2-(pyrazolo[1,5-a]pyridin-3-yl)thiazol-4-yl)-4,5,6,7-tetrahydrothiazolo[4,5-c]pyridine (compound 205)

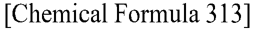

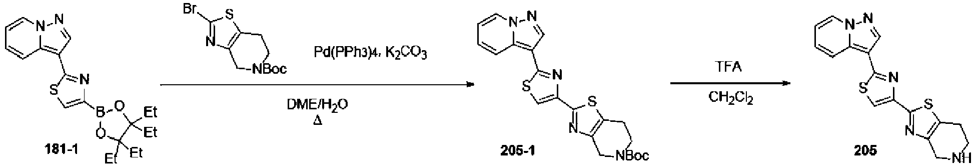

### (1) Synthesis of tert-butyl 2-(2-(pyrazolo[1,5-a]pyridin-3-yl)thiazol-4-yl)-6,7-dihydrothiazolo[4,5-c]pyridine-5(4H)-carboxylate (compound 205-1)

DME (0.8 mL) and water (0.2 mL) were added to 2-(pyrazolo[1,5-a]pyridin-3-yl)-4-(4,4,5,5-tetraethyl-1,3,2-dioxaborolan-2-yl)thiazole (compound 181-1) (45.2 mg, 0.118 mmol), tert-butyl 2-bromo-6,7-dihydrothiazolo[4,5-c]pyridine-5(4H)-carboxylate (47.7 mg, 0.149 mmol), potassium carbonate (56.2 mg, 0.407 mmol), and Pd(PPh₃)₄ (13.7 mg, 0.0119 mmol), and the mixture was heated at 130°C for 20 minutes using a microwave synthesis apparatus. Water was added to the reaction mixture, and the mixture was extracted with ethyl acetate. The organic layer was washed with water and saturated saline, dried over sodium sulfate, and then filtered, and the solvent was removed under reduced pressure. The residue was purified by column chromatography (amino silica gel) to obtain the title compound (205-1) (15.3 mg, 0.0348 mmol, 29.5% yield) from a fraction of n-heptane : ethyl acetate (1 : 1).

¹H-NMR(CDCl₃) δ:1.51 (9H, s), 2.90-2.98 (2H, m), 3.75-3.84 (2H, m), 4.67-4.73 (2H, m), 6.96 (1H, ddd, J=1.2Hz, 6.8Hz, 6.8Hz), 7.38-7.45 (1H, m), 7.78 (1H, s), 8.40 (1H, s), 8.41-8.46 (1H, m), 8.52-8.57 (1H, m).

### (2) Synthesis of 2-(2-(pyrazolo[1,5-a]pyridin-3-yl)thiazol-4-yl)-4,5,6,7-tetrahydrothiazolo[4,5-c]pyridine (compound 205)

tert-Butyl 2-(2-(pyrazolo[1,5-a]pyridin-3-yl)thiazol-4-yl)-6,7-dihydrothiazolo[4,5-c]pyridine-5(4H)-carboxylate (compound 205-1) (28.7 mg, 0.0653 mmol) was dissolved in dichloromethane (1 mL), trifluoroacetic acid (0.5 mL) was added thereto, and the mixture was stirred at room temperature for 2.5 hours. The reaction mixture was neutralized with a saturated aqueous sodium bicarbonate solution, and then extracted with dichloromethane. The organic layer was dried over sodium sulfate and then filtered, and the solvent was removed under reduced pressure. The residue was purified by column chromatography (amino silica gel) to obtain the title compound (205) (10.7 mg, 0.0315 mol, yield 48.2%) from a fraction of dichloromethane : methanol (20 : 1).

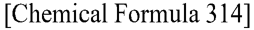

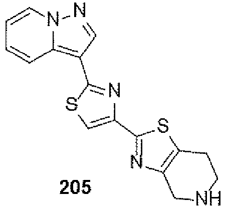

¹H-NMR(DMSO-d₆) δ:2.78-2.84 (2H, m), 3.00 (2H, t, J=5.6Hz), 3.85-3.90 (2H, m), 7.15 (1H, ddd, J=1.2Hz, 6.8Hz, 6.8Hz), 7.59-7.64 (1H, m), 8.05 (1H, s), 8.34-8.38 (1H, m), 8.67 (1H, s), 8.84-8.90 (1H, m).

### [Example 206] Synthesis of 2-(pyrazolo[1,5-a]pyridin-3-yl)-4-(4,5,6,7-tetrahydropyrazolo[1,5-a]pyrazin-2-yl)thiazole (compound 206)

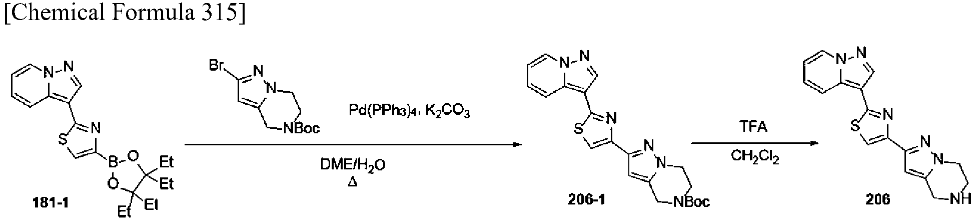

### (1) Synthesis of tert-butyl 2-(2-(pyrazolo[1,5-a]pyridin-3-yl)thiazol-4-yl)-6,7-dihydropyrazolo[1,5-a]pyrazin-5(4H)-carboxylate (compound 206-1)

DME (1.8 mL) and water (0.6 mL) were added to 2-(pyrazolo[1,5-a]pyridin-3-yl)-4-(4,4,5,5-tetraethyl-1,3,2-dioxaborolan-2-yl)thiazole (compound 181-1) (124 mg, 0.323 mmol), tert-butyl 2-bromo-6,7-dihydropyrazolo[1,5-a]pyrazine-5(4H)-carboxylate (81.6 mg, 0.270 mmol), potassium carbonate (117 mg, 0.847 mmol), and Pd(PPh₃)₄ (33.7 mg, 0.0292 mmol), and the mixture was heated at 120°C for 20 minutes using a microwave synthesis apparatus. Water was added to the reaction mixture, and the mixture was extracted with ethyl acetate. The organic layer was washed with water and saturated saline, dried over sodium sulfate, and then filtered, and the solvent was removed under reduced pressure. The residue was purified by column chromatography (amino silica gel) to obtain the title compound (206-1) (68.6 mg, 0.162 mmol, 60.1% yield) from a fraction of n-heptane : ethyl acetate (1 : 3).

¹H-NMR(CDCl₃) δ:1.52 (9H, s), 3.94 (2H, t, J=5.2Hz), 4.26 (2H, t, J=5.2Hz), 4.73 (2H, s), 6.63 (1H, s), 6.92 (1H, ddd, J=1.2Hz, 6.8Hz, 6.8Hz), 7.32-7.41 (1H, m), 7.49 (1H, s), 8.38-8.46 (2H, m), 8.50-8.55 (1H, m).

### (2) Synthesis of 2-(pyrazolo[1,5-a]pyridin-3-yl)-4-(4,5,6,7-tetrahydropyrazolo[1,5-a]pyrazin-2-yl)thiazole (compound 206)

tert-Butyl 2-(2-(pyrazolo[1,5-a]pyridin-3-yl)thiazol-4-yl)-6,7-dihydropyrazolo[1,5-a]pyrazin-5(4H)-carboxylate (compound 206-1) (68.6 mg, 0.162 mmol) was dissolved in dichloromethane (1 mL), trifluoroacetic acid (0.5 mL) was added thereto, and the mixture was stirred at room temperature for 1.5 hours. The reaction mixture was neutralized with a saturated aqueous sodium bicarbonate solution, and then extracted with dichloromethane. The organic layer was dried over sodium sulfate and then filtered, and the solvent was removed under reduced pressure. The residue was purified by preparative TLC (preparative thin layer chromatography) to obtain the title compound (206) (27.9 mg, 0.0865 mmol, 53.3% yield) from a fraction of dichloromethane : methanol (20 : 1).

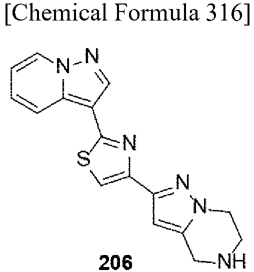

¹H-NMR(CDCl₃) δ:3.36 (2H, t, J=5.6Hz), 4.15 (2H, s), 4.21 (2H, t, J=5.6Hz), 6.55 (1H, s), 6.91 (1H, ddd, J=1.2Hz, 6.8Hz, 6.8Hz), 7.32-7.40 (1H, m), 7.49 (1H, s), 8.41(1H, ddd, J=1.2Hz, 1.2Hz, 8.8Hz), 8.43 (1H, s), 8.52 (1H, ddd, J=1.2Hz, 1.2Hz, 7.2Hz).

### [Example 207] Synthesis of 4-(1H-indazol-3-yl)-2-(pyrazolo[1,5-a]pyridin-3-yl)thiazole (compound 207)

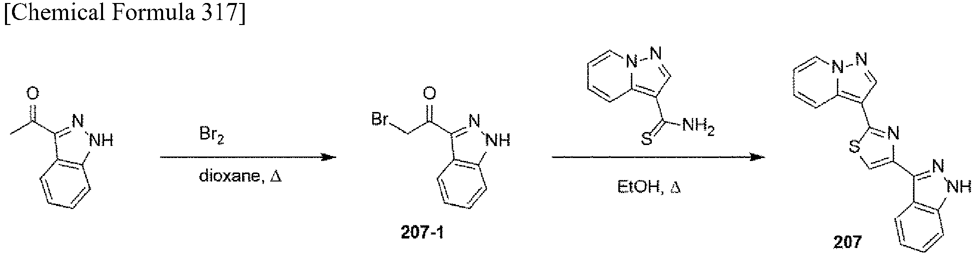

### (1) Synthesis of 2-bromo-1-(1H-indazol-3-yl)ethan-1-one (compound 207-1)

Bromine-dioxane complex (378 mg, 1.52 mmol) was added to 1-(1H-indazol-3-yl)ethan-1-one (233 mg, 1.45 mmol) in dioxane (5 mL), and the mixture was stirred at 70°C for 15 minutes. After the reaction mixture was allowed to cool, the solvent was removed under reduced pressure to obtain the title compound (207-1) (350 mg) as a crude product.

### (2) Synthesis of 4-(1H-indazol-3-yl)-2-(pyrazolo[1,5-a]pyridin-3-yl)thiazole (compound 207)

2-Bromo-1-(1H-indazol-3-yl)ethan-1-one (compound 207-1) (350 mg) was added to an ethanol solution of pyrazolo[1,5-a]pyridine-3-carbothioamide (257 mg, 1.45 mmol), and the mixture was stirred at 50°C for 30 minutes. The mixture was allowed to cool, then diluted with dichloromethane, and a saturated aqueous sodium bicarbonate solution was added to adjust the pH to 9, then the mixture was extracted with dichloromethane. The organic layer was dried over anhydrous sodium sulfate and then filtered, and the solvent was removed under reduced pressure. The residue was suspended in methanol, then collected by filtration and dried to obtain the title compound (207) (180 mg, 0.567 mmol, 39.1% yield over two steps).

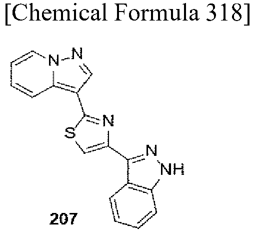

¹H-NMR(DMSO-d₆) δ:7.13-7.17 (1H, m), 7.27-7.31 (1H, m), 7.41-7.46 (1H, m), 7.60-7.66 (2H, m), 8.01 (1H, s), 8.41-8.49 (2H, m), 8.71 (1H, s), 8.88-8.90 (1H, m).

### [Example 208] Synthesis of 4-(4-chloro-1H-pyrazol-3-yl)-2-(pyrazolo[1,5-a]pyridin-3-yl)thiazole (compound 208)

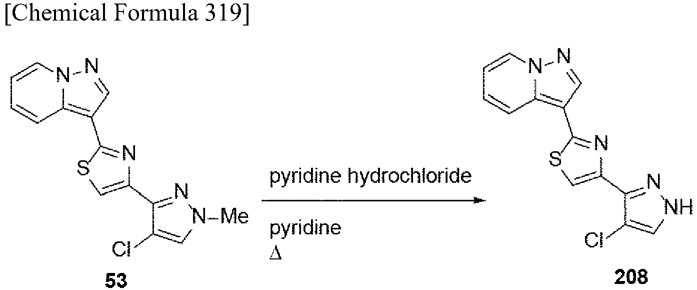

4-(4-Chloro-1-methyl-1H-pyrazol-3-yl)-2-(pyrazolo[1,5-a]pyridin-3-yl)thiazole (compound 53) (67.0 mg, 0.212 mmol) was dissolved in pyridine (0.6 mL), and pyridine hydrochloride (504.4 mg, 4.36 mmol) was added. The reaction mixture was heated at 250°C for 2 hours using a microwave heating apparatus. A saturated aqueous sodium bicarbonate solution was added to the reaction mixture to neutralize the reaction mixture, then water (10 mL) and diethyl ether (15 mL) were added thereto, and the resulting precipitate was collected by suction filtration to obtain a crude product. The crude product was washed with a mixed solution of dichloromethane and diethyl ether to obtain the title compound (208) (19.5 mg, 0.0646 mmol, 30.5% yield).

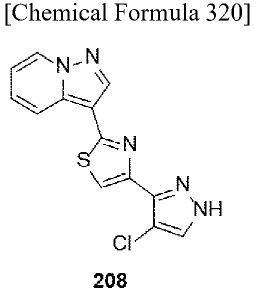

¹H-NMR(CDCl₃) δ: 6.98 (1H, ddd, J=1.3Hz, 6.9Hz, 6.9Hz), 7.44 (1H, ddd, J=1.0Hz, 6.8Hz, 8.9Hz), 7.63 (1H, s), 7.86 (1H, s), 8.32-8.37 (1H, m), 8.45 (1H, s), 8.56-8.59 (1H, m).

### [Example 209] Synthesis of 2-(6-methylpyrazolo[1,5-a]pyridin-3-yl)-4-phenylthiazole (compound 209)

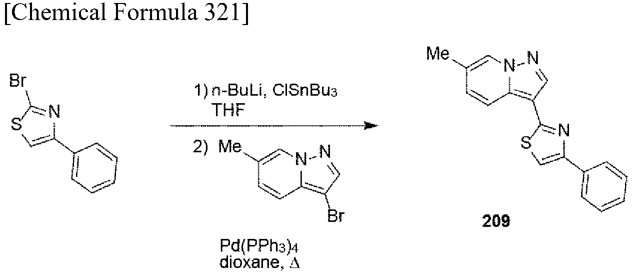

Butyllithium (1.6 M in hexane) (0.490 mL, 0.783 mmol) was added dropwise to a solution of 2-bromo-4-phenylthiazole (171 mg, 0.712 mmol) in THF (3 mL) at -78°C, and the mixture was stirred for 1 hour. Tributyltin chloride (0.232 mL, 0.855 mmol) was added dropwise thereto at the same temperature, and the mixture was stirred for 20 minutes and then stirred at room temperature for 2 hours. A saturated aqueous ammonium chloride solution was added to the reaction mixture, and the mixture was extracted with diethyl ether. The organic layer was dried over anhydrous sodium sulfate and then filtered, and the solvent was removed under reduced pressure. 3-Bromo-6-methylpyrazolo[1,5-a]pyridine (67.5 mg, 0.319 mmol) and Pd(PPh₃)₄ (61.6 mg, 0.0533 mmol) were added to a solution of the residue in dioxane (3 mL), and the mixture was heated at 130°C for 10 minutes using a microwave synthesis apparatus. After the reaction mixture was allowed to cool, water was added thereto, and the mixture was extracted with dichloromethane. The organic layer was dried over anhydrous sodium sulfate and then filtered, and the solvent was removed under reduced pressure. The residue was purified by silica gel column chromatography (amino silica gel), and the title compound (209) (7.70 mg, 0.0264 mmol, 8.28% yield over two steps) was obtained from a fraction of n-heptane : ethyl acetate (1 : 1).

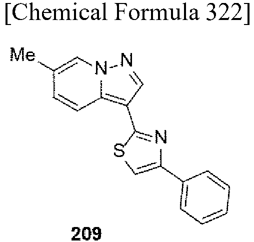

¹H-NMR(DMSO-d₆) δ:2.38 (3H, s), 7.36-7.40 (1H, m), 7.45-7.51 (3H, m), 8.01 (1H, s), 8.07-8.10 (2H, m), 8.32-8.35 (1H, m), 8.55 (1H, s), 8.70 (1H, s).

### [Example 210] Synthesis of 2-(5-methylpyrazolo[1,5-a]pyridin-3-yl)-4-phenylthiazole (compound 210)

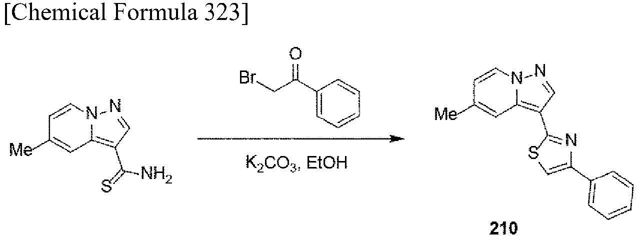

Potassium carbonate (255 mg, 1.63 mmol) and phenacyl bromide (108 mg, 0.544 mmol) were added to a solution of 5-methylpyrazolo[1,5-a]pyridine-3-carbothioamide (104 mg, 0.544 mmol) in ethanol (5 mL), and the mixture was stirred at room temperature for 30 minutes. The reaction mixture was diluted with dichloromethane, then water was added thereto, and the mixture was extracted with dichloromethane. The organic layer was dried over anhydrous sodium sulfate and then filtered, and the solvent was removed under reduced pressure. The residue was suspended in methanol, then collected by filtration and dried to obtain the title compound (210) (119 mg, 0.408 mmol, 75.1% yield).

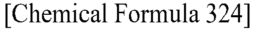

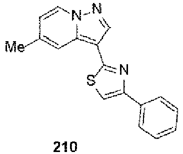

¹H-NMR(CDCl₃) δ:2.52 (3H, s), 6.75 (1H, dd, J=2.0Hz, J=7.0Hz), 7.34-7.39 (2H, m), 7.45-7.50 (2H, m), 8.00-8.04 (2H, m), 8.19-8.21 (1H, m), 8.36 (1H, s), 8.39-8.41 (1H, m).

### [Example 211] Synthesis of 2-(4-methylpyrazolo[1,5-a]pyridin-3-yl)-4-phenylthiazole (compound 211)

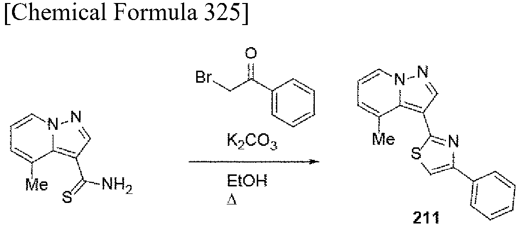

4-Methylpyrazolo[1,5-a]pyridine-3-carbothioamide (30.6 mg, 0.160 mmol) and phenacyl bromide (32.2 mg, 0.162 mmol) were dissolved in ethanol (2 mL), and the mixture was stirred at 50°C for 2 hours. After the reaction mixture was allowed to cool, potassium carbonate (38.0 mg, 0.275 mmol) was added thereto, and the mixture was stirred at room temperature for 30 minutes. The reaction mixture was then concentrated under reduced pressure. The residue was purified by silica gel column chromatography to obtain the title compound (211) (28.1 mg, 0.0964 mmol, 60.3% yield) from a fraction of n-heptane : ethyl acetate (2 : 1).

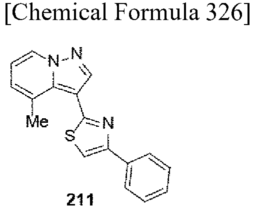

¹H-NMR(CDCl₃) δ:2.72 (3H, s), 6.79-6.84 (1H, m), 7.03-7.07 (1H, m), 7.32-7.38 (1H, m), 7.42-7.47 (2H, m), 7.50 (1H, s), 7.96-8.00 (2H, m), 8.19 (1H, s), 8.38-8.41 (1H, m).

### [Example 212] Synthesis of 4-(4-chloro-1-methyl-1H-pyrazol-3-yl)-2-(5-methylpyrazolo[1,5-a]pyridin-3-yl)thiazole (compound 212)

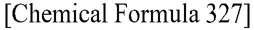

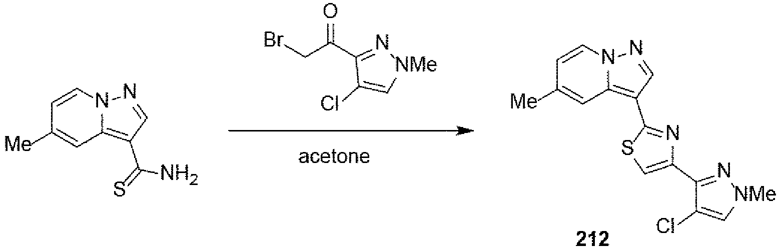

2-Bromo-1-(4-chloro-1-methyl-1H-pyrazol-3-yl)ethane-1-one (76.1 mg, 0.321 mmol) was added to a solution of 5-methylpyrazolo[1,5-a]pyridine-3-carbothioamide (61.3 mg, 0.321 mmol) in acetone (3 mL), and the mixture was stirred at room temperature for 1 hour. A saturated aqueous sodium bicarbonate solution was added to the reaction mixture, and the precipitated solid was collected by suction filtration. The obtained solid was suspended in diethyl ether, collected by filtration, and dried to obtain the title compound (212) (42.0 mg, 0.127 mmol, 40.0% yield).

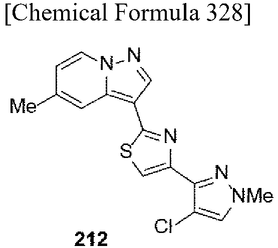

¹H-NMR(DMSO-d₆) δ:2.44 (3H, s), 3.90 (3H, s), 6.95 (1H, dd, J=1.8Hz, 7.0Hz), 7.79 (1H, s), 8.07 (1H, s), 8.23-8.24 (1H, m), 8.54 (1H, s), 8.72 (1H, d, J=7.0Hz).

### [Example 213] Synthesis of N-((5-(2-(5-methylpyrazolo[1,5-a]pyridin-3-yl)thiazol-4-yl)pyridin-2-yl)methyl)acetamide (compound 213)

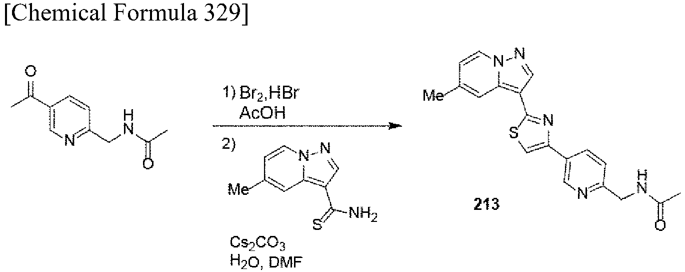

Bromine (18.2 µL, 0.354 mmol) and hydrobromic acid (30% acetic acid solution, about 5.1 mol/L) (173 µL, 0.884 mmol) were added to a solution of N-((5-acetylpyridin-2-yl)methyl)acetamide (68.0 mg, 0.354 mmol) in acetic acid (1 mL), and the mixture was stirred at room temperature for 10 minutes. The reaction mixture was concentrated under reduced pressure, and the resulting residue was dissolved in DMF (3 mL). Water (0.5 mL), 5-methylpyrazolo[1,5-a]pyridine-3-carbothioamide (67.7 mg, 0.354 mmol), and cesium carbonate (231 mg, 0.708 mmol) were added, and the mixture was stirred at 70°C for 1 hour. After the reaction mixture was allowed to cool, water was added thereto, and the precipitated solid was collected by suction filtration. The obtained solid was suspended in diethyl ether, then collected by filtration, and dried to obtain the title compound (213) (16.3 mg, 0.0448 mmol, 12.7% yield).

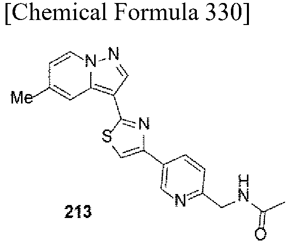

¹H-NMR(DMSO-d₆) δ:1.93 (3H, s), 2.50 (3H, s), 4.40 (2H, d, J=6.0Hz), 6.98 (1H, dd, J=2.0Hz, J=7.0Hz), 7.41 (1H, d, J=8.1Hz), 8.13 (1H, s), 8.17 (1H, s), 8.40 (1H, dd, J=2.2Hz, J=8.1Hz), 8.47-8.51 (1H, m), 8.58 (1H, s), 8.74 (1H, d, J=7.0Hz), 9.21 (1H, d, J=2.2Hz).

### [2] Measurement of MYLK4 inhibitory activity (kinase assay)

(Abbreviation) The meanings of symbols abbreviated in the following tests are as follows.
ATP: adenosine triphosphate
ADP: adenosine diphosphate
BSA: bovine serum albumin
DMSO: dimethyl sulfoxide
DTT: dithiothreitol
EDTA: ethylenediaminetetraacetic acid
EGTA: glycol ether diamine tetraacetic acid
IOP: intraocular pressure
MOPS: 3-morpholinopropanesulfonic acid
MYLK: myosin light chain kinase
ROCK: Rho kinase

### 1. Test Methods

In order to evaluate the inhibitory activity of the test compound on the kinase activity of MYLK4, a kinase assay using MYLK4 recombinant protein and LC20 recombinant protein or MLC Peptide as a substrate was performed. MLC Peptide is a peptide based on the partial sequence of LC20 recombinant protein, and has the same phosphorylation site.

A test compound (final concentration: 1 nM to 10 µM), MYLK4 recombinant protein (SignalChem Lifesciences Corporation, final concentration: 8 µg/mL), LC20 recombinant protein (SignalChem Lifesciences Corporation, final concentration: 20 µg/mL) or MLC Peptide (SignalChem Lifesciences Corporation, final concentration: 40 µg/mL), and ATP (Promega Corporation, final concentration 50 µM) were mixed in 12.5 µL of assay buffer (5 mM MOPS, pH 7.2, 2.5 mM β-glycerol phosphate, 5 mM MgCl₂, 1 mM EGTA, 0.4 mM EDTA, 0.05 mM DTT, 200 µg/mL BSA) using a 96-well half-area microplate (Greiner Bio-One International GmbH), and a kinase reaction was performed at 30°C for 1 hour while stirring. The test compound was pre-diluted in DMSO to 1000 times the final concentration and then serially diluted with assay buffer. For kinase activity measurement, an operation was performed according to the procedure manual using ADP-Glo (registered trademark) Kinase Assay (Promega Corporation). The amount of luminescence of each well obtained by the reaction was measured using SpectraMax (registered trademark) iD3 (Molecular Devices, LLC). When a solvent was added instead of the test compound, the amount of luminescence was set to 100%, and when the kinase reaction did not proceed without addition of MYLK4, the amount of luminescence was set to 0%. Then, the relative luminescence in the presence of the test compound was determined. Relative luminescence was plotted against the concentration of the test compound using statistical analysis software (KaleidaGraph Version 4.1), and the IC50 value, defined as the concentration required to inhibit MYLK4 kinase activity by 50%, was calculated from the obtained regression curve (dosersplgst).

### 2. Test results

The following table shows the IC₅₀ values of each compound for the enzymatic activity of MYLK4. The results using LC20 recombinant protein as a substrate are shown in Table 1-1 to Table 1-2, and the results using MLC Peptide are shown in Table 2.

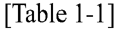

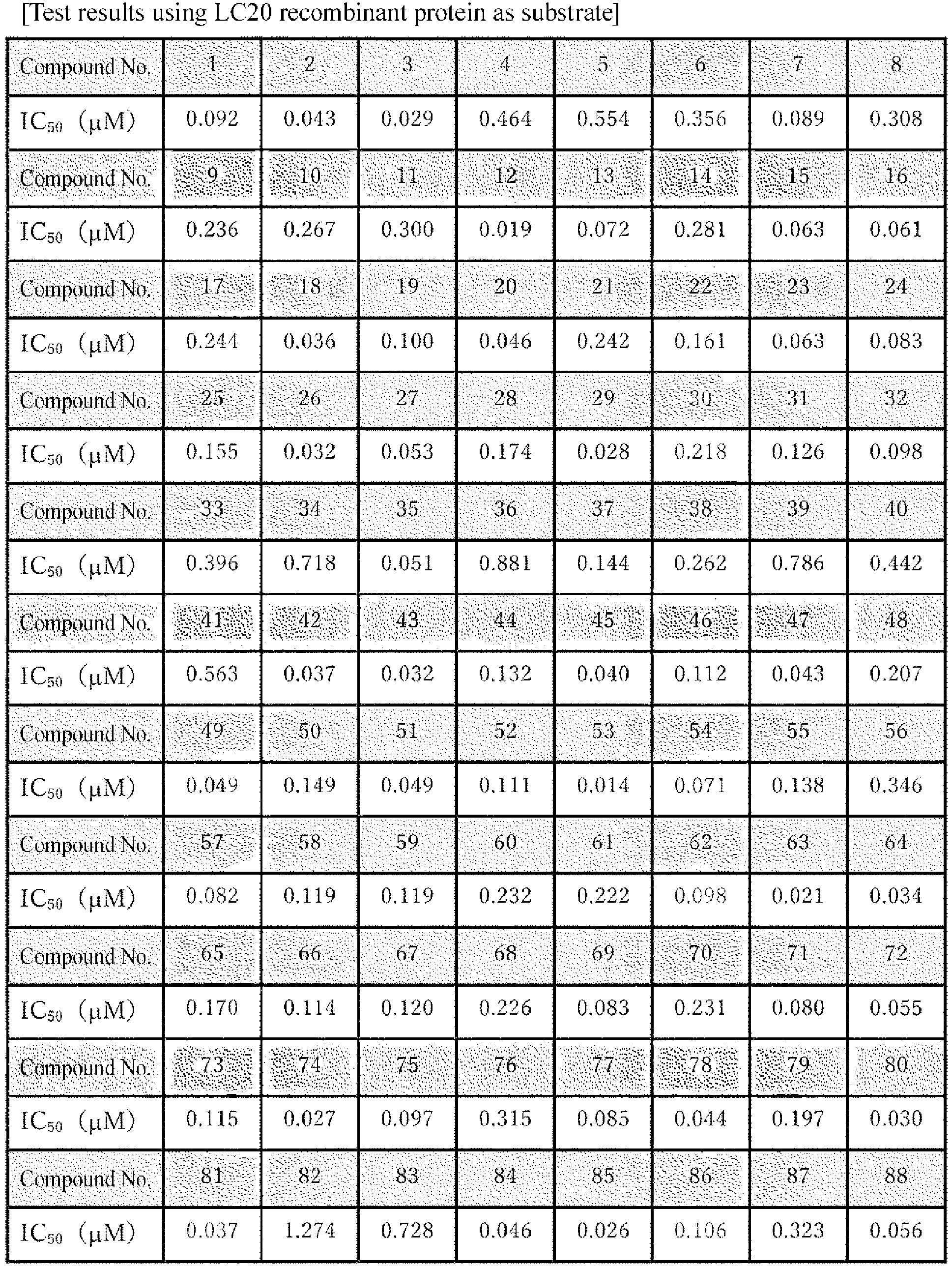

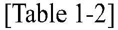

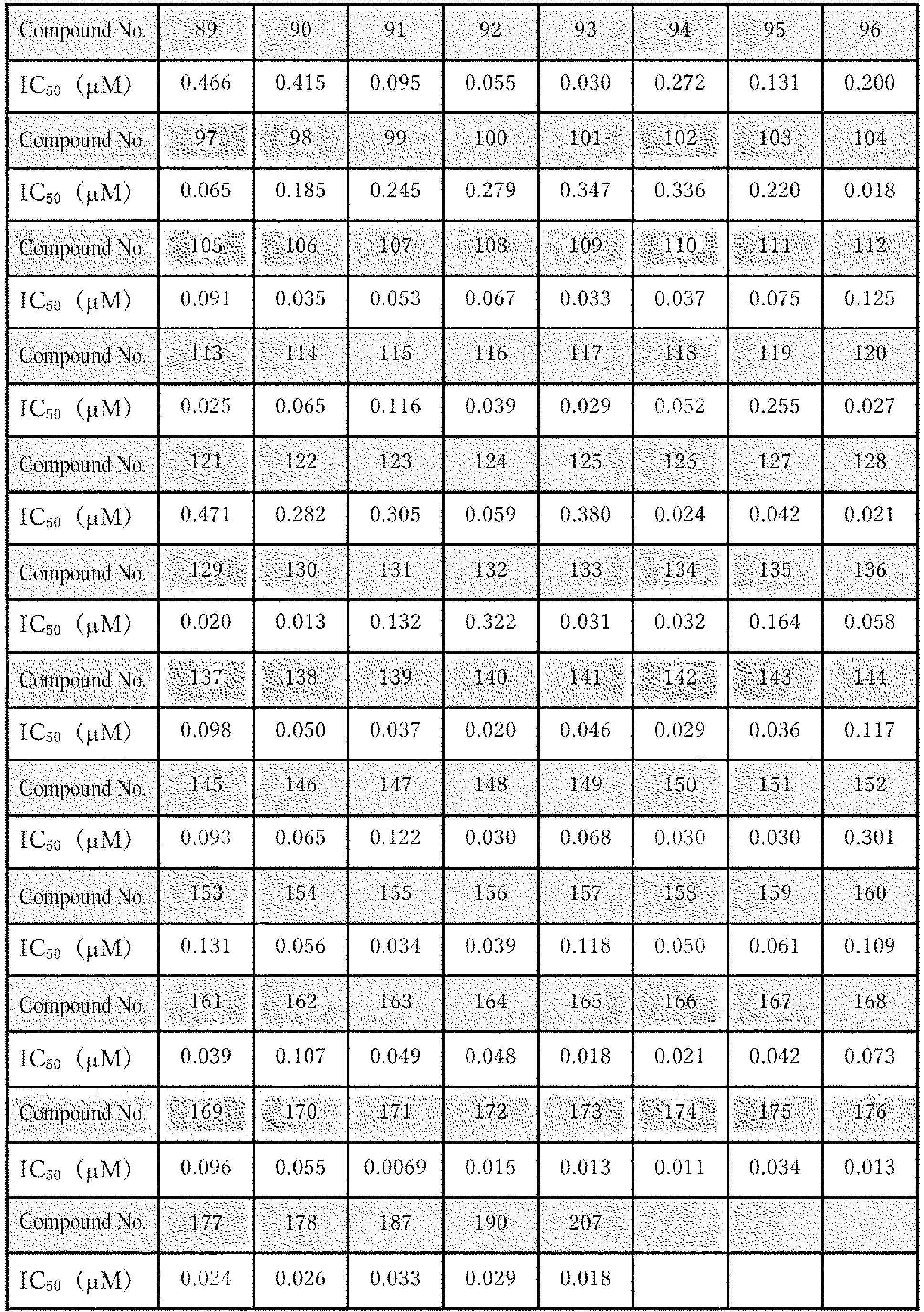

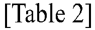

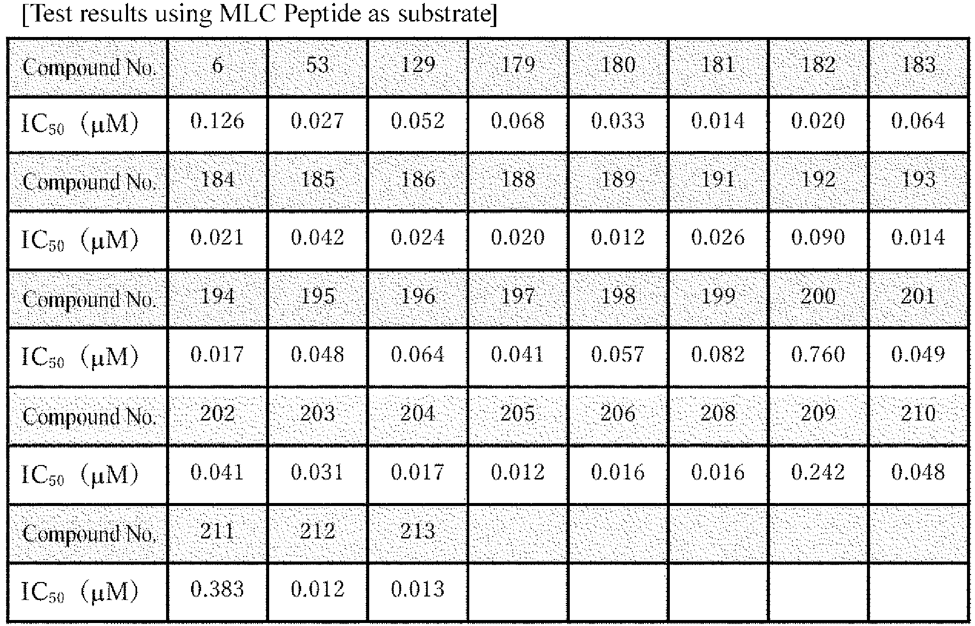

### [3] Study on intraocular pressure lowering effect by single instillation (normotensive rabbits)

### 1. Preparation of administration solution

Compounds 129, 6, 53, and 130, as well as compound 74 in its free form, were formulated at various concentrations with a solubilizing agent, isotonic agent, preservative, and pH adjuster in purified water to prepare eye drops (test compound solutions). In addition, a solution prepared in the same manner, but without the compound, was used as a blank.

### 2. Animals and methods of administration

Normotensive rabbits (Kbl:NZW, male, 11 to 12 weeks old) were used for the experiment. 50 µL of the blank was instilled to the left eye, and 50 µL of the test compound solution was instilled to the right eye. The same test compound solution was instilled in the same animal at increasing concentrations, and each instillation was performed at intervals of one week.

### 3. Intraocular pressure (IOP) measurement method

0.4% benoxyl ophthalmic solution (Santen Pharmaceutical Co., Ltd.) was instilled to achieve topical anesthesia, and then the intraocular pressure was measured using a pneumatic applanation tonometer (Model 30 Classic, Reichert, Inc.). Intraocular pressure measurements were performed before administration (0 hours), and 0.5, 1, 2, 4, 8, and 24 hours after administration of the blank or the test compound solution.

### 4. Test results

Each IOP was an average value of three measurements, and the change in intraocular pressure (ΔIOP) was calculated as the difference between the IOP at each time point and the IOP at 0 h (before instillation) for each individual. The average value (n=4) of ΔIOP of each eye drop is shown in the following table.

**[Table 3]**

| [Compound 129] Average ΔIOP (mmHg) | | | | | | | | |
|---|---|---|---|---|---|---|---|---|
| Eye drop | | Time | | | | | | |
| | | 0h | 0.5h | 1h | 2h | 4h | 8h | 24h |
| 0.05 mg/mL Eye drop | Blank | 0.0 | -0. 7 | -0. 1 | -0.7 | 0.0 | 1.4 | -0.3 |
| | Test compound solution | 0.0 | -0.9 | -1.8 | -2.3 | -0.3 | 1.2 | 0.1 |
| 0. 15 mg/mL Eye drop | Blank | 0.0 | -0.2 | -0.1 | -1.1 | -0.7 | 1.3 | 0.1 |
| | Test compound solution | 0.0 | -1.4 | -2.0 | -3.3 | -2.0 | 0. 0 | -0.9 |
| 1 mg/mL Eye drop | Blank | 0.0 | -0.8 | -0.1 | -0.5 | -0. 1 | 2. 0 | -0.4 |
| | Test compound solution | 0.0 | -1.2 | -0.7 | -2.3 | -2.3 | 1.4 | -0.2 |
| 4 mg/mL Eye drop | Blank | 0.0 | -0. 5 | -0. 2 | -0.5 | -0.8 | 0.6 | -0.7 |
| | Test compound solution | 0.0 | -2.6 | -2.0 | -4.3 | -2.9 | -0.4 | -0.4 |

In all concentrations of the test compound solutions, a reduction in intraocular pressure was observed with a maximum at 2 hours after administration.

**[Table 4]**

| [Compound 6] Average ΔIOP (mmHg) | | | | | | | | |
|---|---|---|---|---|---|---|---|---|
| Eye drop | | Time | | | | | | |
| | | 0h | 0.5h | 1h | 2h | 4h | 8h | 24h |
| 0.05 mg/mL Eye drop | Blank | 0.0 | -0. 1 | -0. 1 | 0. 1 | -0.2 | 0.2 | -0.3 |
| | Test compound solution | 0. 0 | 0. 0 | 0. 1 | -0.3 | -0.3 | 0.3 | 0.1 |
| 0. 15 mg/mL Eye drop | Blank | 0. 0 | -0. 2 | 0.1 | -0.1 | -0.3 | 0. 4 | -0. 2 |
| | Test compound solution | 0.0 | 0.1 | -0. 3 | -0. 7 | -0.9 | -0. 1 | -0. 1 |
| 1 mg/mL Eye drop | Blank | 0. 0 | -0. 4 | 0.0 | -0. 2 | -0.7 | -0. 1 | -0. 3 |
| | Test compound solution | 0. 0 | -0. 7 | -0.4 | -1. 5 | -1.7 | 0.3 | -0.4 |
| 4 mg/mL Eye drop | Blank | 0.0 | -0. 2 | 0.5 | 0. 1 | -0.1 | 0. 1 | -0.2 |
| | Test compound solution | 0. 0 | -0. 8 | -0. 7 | -2. 1 | -1.7 | -0. 4 | -0. 2 |

In the test compound solutions of 1 mg/mL and 4 mg/mL, a reduction in intraocular pressure reaching a maximum at 2 or 4 hours after administration was observed.

**[Table 5]**

| [Compound 53] Average ΔIOP (mmHg) | | | | | | | | |
|---|---|---|---|---|---|---|---|---|
| Eye drop | | Time | | | | | | |
| | | 0h | 0.5h | 1h | 2h | 4h | 8h | 24h |
| 0.05 mg/mL Eye drop | Blank | 0.0 | -0.5 | -0.3 | -0.6 | -0.1 | -0.2 | -0.7 |
| | Test compound solution | 0.0 | -2.0 | -3.2 | -4.1 | -2.2 | -0.5 | -0.9 |
| 0. 15 mg/mL Eye drop | Blank | 0.0 | -0.3 | -0.5 | -0. 7 | -0.9 | 0.0 | -0.7 |
| | Test compound solution | 0.0 | -2.1 | -3.0 | -3.8 | -3.2 | 0.2 | -0.5 |
| 1 mg/mL Eye drop | Blank | 0.0 | -0.2 | -0.2 | -0. 5 | -0. 5 | -0.2 | -0.2 |
| | Test compound solution | 0.0 | -2.0 | -3.0 | -3.9 | -3.8 | -1. 1 | -0.4 |
| 4 mg/mL Eye drop | Blank | 0.0 | -0.4 | -0.4 | -0.2 | -0.6 | 0.6 | 0.3 |
| | Test compound solution | 0.0 | -2.1 | -3.1 | -4.4 | -4.0 | -1.3 | -0.4 |

In all concentrations of the test compound solutions, a reduction in intraocular pressure was observed with a maximum at 2 hours after administration.

**[Table 6]**

| [Compound 130] Average ΔIOP (mmHg) | | | | | | | | |
|---|---|---|---|---|---|---|---|---|
| Eye drop | | Time | | | | | | |
| | | 0h | 0.5h | 1h | 2h | 4h | 8h | 24h |
| 0. 05 mg/mL Eye drop | Blank | 0. 0 | -0.2 | 0.3 | 0.1 | 0.2 | 0.8 | -0.2 |
| | Test compound solution | 0. 0 | 0.2 | 0. 3 | 0.2 | 0. 1 | 0.6 | 0.2 |
| 0. 15 mg/mL Eye drop | Blank | 0.0 | -0.3 | -0.1 | -0.5 | -0.2 | 0.2 | -0.4 |
| | Test compound solution | 0.0 | -0.2 | -0.1 | -0.5 | -0.5 | 0.4 | -0.3 |
| 1 mg/mL Eye drop | Blank | 0.0 | -0.3 | -0.4 | 0.2 | -0.1 | 0.4 | -0.3 |
| | Test compound solution | 0. 0 | -0.4 | -0.8 | -2.0 | -1.4 | 0.0 | -0.5 |
| 4 mg/mL Eye drop | Blank | 0.0 | -0.3 | 0.1 | -0.4 | -0.2 | 0.2 | -0.1 |
| | Test compound solution | 0. 0 | -0.5 | -0.7 | -2.2 | -1.4 | 0.6 | 0.1 |

In the test compound solutions at concentrations of 1 mg/mL and 4 mg/mL, a reduction in intraocular pressure was observed, reaching a maximum at 2 hours after administration.

**[Table 7]**

| [Compound 74] Average ΔIOP (mmHg) | | | | | | | | |
|---|---|---|---|---|---|---|---|---|
| Eye drop | | Time | | | | | | |
| | | 0h | 0. 5h | 1h | 2h | 4h | 8h | 24h |
| 0.05 mg/mL Eye drop | Blank | 0.0 | 0.2 | -0.1 | -0.1 | -0.4 | 0.4 | 0.3 |
| | Test compound solution | 0.0 | 0.1 | 0. 1 | 0.1 | -0.1 | 0.2 | -0. 3 |
| 0. 15 mg/mL Eye drop | Blank | 0. 0 | -0.3 | -0. 2 | -0.4 | -0.5 | 0. 4 | 0.1 |
| | Test compound solution | 0.0 | -0.3 | -0.3 | -0.4 | -0.4 | 0.4 | 0.1 |
| 1 mg/mL Eye drop | Blank | 0.0 | -0.6 | -0.3 | -0.6 | -0.4 | -0.2 | -0.3 |
| | Test compound solution | 0.0 | -0.9 | -0.5 | -1.4 | -2. 0 | -0.3 | -0. 4 |

In the test compound solution of 1 mg/mL, a reduction in intraocular pressure was observed with a maximum at 4 hours after administration.

### [4] Study on intraocular pressure lowering effect by single instillation (normotensive monkeys)

### 1. Preparation of administration solution

The hydrochloride of compound 129 was added to purified water together with a solubilizing agent, an isotonic agent, a preservative, and a pH adjuster at final concentrations of 0.05 mg/mL and 4 mg/mL to prepare eye drops (test compound solutions). In addition, a solution prepared in the same manner, but without the compound, was used as a blank.

### 2. Animals and methods of administration

Cynomolgus normotensive monkeys (male, 7 years old) were used for the experiment. A 30 µL aliquot of the blank was instilled to the left eye, and a 30 µL aliquot of the test compound solution was instilled to the right eye. Test compound solutions at different concentrations were administered to the same animal at one-week intervals.

### 3. Intraocular pressure measurement method

0.4% benoxyl ophthalmic solution (Santen Pharmaceutical Co., Ltd.) was instilled to achieve topical anesthesia, and then the intraocular pressure was measured using a pneumatic applanation tonometer (Model 30 Classic, Reichert, Inc.). Intraocular pressure measurements were performed before administration (0 hours), and 0.5, 1, 2, 4, 8, and 24 hours after administration of the blank or the test compound solution.

### 4. Test results

Each IOP was calculated as an average value of three measurements, and the change in intraocular pressure (ΔIOP) was calculated as the difference between the IOP at each time point and the IOP at 0 h (before administration). The mean ΔIOP (n=2) for each eye-drop formulation is shown in the following table.

**[Table 8]**

| [Hydrochloride of compound 129] Average ΔIOP (mmHg) | | | | | | | | |
|---|---|---|---|---|---|---|---|---|
| Eye drop | | Time | | | | | | |
| | | 0h | 0.5h | 1h | 2h | 4h | 8h | 24h |
| 0. 05 mg/mL Eye drop | Blank | 0.0 | 0.2 | 0. 5 | 0.0 | 0.0 | 0.7 | 0.2 |
| | Test compound solution | 0.0 | -0.8 | -1. 8 | -2.5 | -1. 2 | -0.7 | -0.2 |
| 4 mg/mL Eye drop | Blank | 0.0 | -0.2 | -0.4 | -0.5 | -0.3 | -0.5 | -0.2 |
| | Test compound solution | 0.0 | -0.7 | -2.1 | -3.7 | -2.2 | -1.7 | -0.6 |

For the test compound solutions at all concentrations, a reduction in intraocular pressure was observed with a maximum at 2 hours after administration.

### (Note)

It has been reported that inhibition of the activity of MYLK4, as well as inhibition of other MYLK isoforms and ROCK that exert actions similar to those of MYLK4 is effective for the diseases shown in the following table. Accordingly, each of the above compounds is also considered to be effective for these diseases.

**[Table 9]**

| Literatures | Relevant kinase /signaling pathway | Relevant diseases | Inhibitors used |
|---|---|---|---|
| Invest. Ophthalmol. Vis. Sci. (2021) 62(13), 12. | ROCK, MYLK4 | Glaucoma, ocular hypertension | ITRI-E-(S) 4046 |
| Exp. Eye Res. (2000) 71(6), 551-566. | MYLK | Impaired aqueous humor outflow | ML-7 |
| Exp. Eye Res. (2005) 80(6), 837-845. | MYLK | Glaucoma, impaired aqueous humor outflow | ML-7 |
| J. Ophthalmol. (2010) 175163. | ROCK | Diabetic retinopathy | Fasudil |
| Invest. Ophthalmol. Vis. Sci. (2015) 56(2), 1335-1348. | ROCK | Age-related macular degeneration | AMA0428 |
| Invest. Ophthalmol. Vis. Sci. (2017) 58(12), 5584-5593. | ROCK | Uveitis | Ripasudil |
| Invest. Ophthalmol. Vis. Sci. (2013) 54(4), 2705-2710. | MYLK | Dry eye | ML-7 |
| Curr. Opin. Cell Biol. (2008) 20(2),242--248. | ROCK | Various pathological conditions involving ROCK | |
| Int. J. Mol. Sci. (2023) 24(13), 11219. | ROCK | Neurodegenerative diseases (e.g., Alzheimer's disease) | Fasudil |
| Dig. Dis. Sci. (2013) 58(1), 107-114. | MYLK | Inflammatory bowel disease | ML-7 |
| J. Exp. Clin. Cancer Res. (2021) 40(1), 166. | MYLK4 | Osteosarcoma | ML-7 |
| Mol. Med. Rep. (2015) 12(3), 4109-4116. | MYLK | Arteriosclerosis | ML-7 |
| J. Proteomics. (2012) 75(17), 5386-5395. | MYLK | Ischemic heart disease | ML-7 |

## Claims

1. A compound of formula (I): 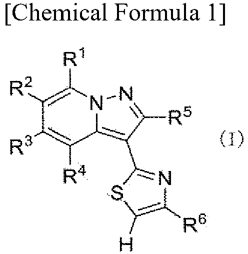
wherein R¹ to R⁵ are each independently hydrogen or a C₁₋₃ chain aliphatic hydrocarbon, and
R⁶ is an optionally substituted 5- to 10-membered aromatic hydrocarbon, an optionally substituted 5- to 10-membered heterocycle having 1 to 5 heteroatoms each independently selected from nitrogen, oxygen, and sulfur as ring-constituting atoms, or a fused ring in which either the aromatic hydrocarbon or the heterocycle is fused with an optionally substituted 4- to 10-membered heterocyclyl having 1 to 3 heteroatoms each independently selected from nitrogen, oxygen, and sulfur as ring-constituting atoms;
or a pharmaceutically acceptable derivative thereof.

2. The compound or pharmaceutically acceptable derivative thereof according to claim 1,
wherein
the 5- to 10-membered aromatic hydrocarbon, the 5- to 10-membered heterocycle, and the 4- to 10-membered heterocyclyl forming a fused ring with either the aromatic hydrocarbon and the heterocycle, in R⁶, are each independently unsubstituted, or substituted with a substituent selected from the group consisting of:
(1) halogen, nitro, cyano, hydroxy, difluoromethyl, trifluoromethyl,
(2) -NR³¹₂, -N(R³¹)(CH₂)₁₋₅NR³¹₂, -N(R³¹)S(O)₂R³², -N(R³¹)C(O)R³¹, - N(R³¹)C(O)(CH₂)₀₋₃NR³¹₂, -N(R³¹)C(O)(CH₂)₀₋₃R³²,
(3) C₁₋₆ alkyl, -(CH₂)₁₋₅OR³¹, -(CH₂)₁₋₅O(CH₂)₁₋₅OR³¹, -(CH₂)₁₋₅R³², -(CH₂)₁₋₅R³³,
(4) -(CH₂)₁₋₅NR³¹₂, -(CH₂)₁₋₅N(R³¹)R³², -(CH₂)₁₋₅N(R³¹)(CH₂)₀₋₅R³⁸, -(CH₂)₁₋₅N(R³¹)C(O)R³¹, -(CH₂)₁₋₅N(R³¹)C(O)(CH₂)₁₋₅OR³¹, -(CH₂)₁₋₅N(C(O)R³¹)(CH₂)₁₋₅C(O)OR³¹, - (CH₂)₁₋₅N(R³¹)C(O)(CH₂)₀₋₅NR³¹₂, -(CH₂)₁₋₅N(R³¹)C(O)R³²,
(5) -(CH₂)₁₋₅N(R³¹)S(O)₂R³³,
(6) -C(R³¹)₂C(O)R³²,
(7) -C(R³¹)₂C(O)NR³¹₂, -C(R³¹)₂C(O)N(R³¹)OR³¹,
(8) -C(O)R³¹, -C(O)R³²,
(9) -C(O)OR³¹,
(10) -C(O)NR³¹₂, -C(O)N(R³¹)(CH₂)₀₋₅R³²,
(11) -OC(R³¹)₂C(O)N(R³¹)(CH₂)₁₋₅OR³¹, -OC(R³¹)₂C(O)OR³¹, -OC(R³¹)₂C(O)NR³¹₂, - OC(R³¹)₂C(O)R³²,
(12) -O(CH₂)₁₋₆R³¹, -(O(CH₂)₁₋₅)₁₋₅(CH₂)₀₋₃NR³¹₂, -(O(CH₂)₁₋₅)₁₋₅(CH₂)₀₋₃NR³¹C(O)R³¹, -(O(CH₂)₁₋₅)₁₋₅NR³¹C(NH)NR³¹₂, -O((CH₂)₁₋₅O)₁₋₅R³¹,
(13) -OC(O)C(R³¹)₂(O(CH₂)₁₋₅)₁₋₅OR³¹, and
(14) 5- or 6-membered heterocyclyl containing nitrogen as a ring-constituting atom and optionally substituted with C₁₋₃ alkyl;
R³¹ is each independently hydrogen or a C₁₋₄ chain aliphatic hydrocarbon;
R³² is an optionally substituted 4- to 10-membered heterocyclyl having 1 to 3 heteroatoms selected from nitrogen, sulfur, and oxygen as ring-constituting atoms, or a fused ring in which the heterocyclyl is fused with an optionally substituted 5- or 6-membered monocyclic aromatic hydrocarbon, where the 4- to 10-membered heterocyclyl forming the fused ring is optionally substituted with a C₁₋₅ chain aliphatic hydrocarbon;
R³³ is a 3- to 10-membered cycloaliphatic hydrocarbon or a 4- to 10-membered heterocyclyl having 1 to 5 heteroatoms independently selected from nitrogen, oxygen and sulfur as ring-constituting atoms; and
R³⁸ is cyano, hydroxy, or S(O)₂R³¹.

3. The compound or pharmaceutically acceptable derivative thereof according to claim 1, wherein R⁶ is selected from the group consisting of: 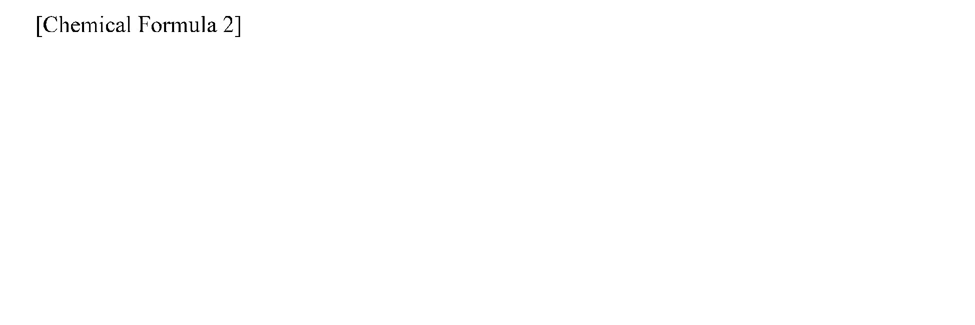 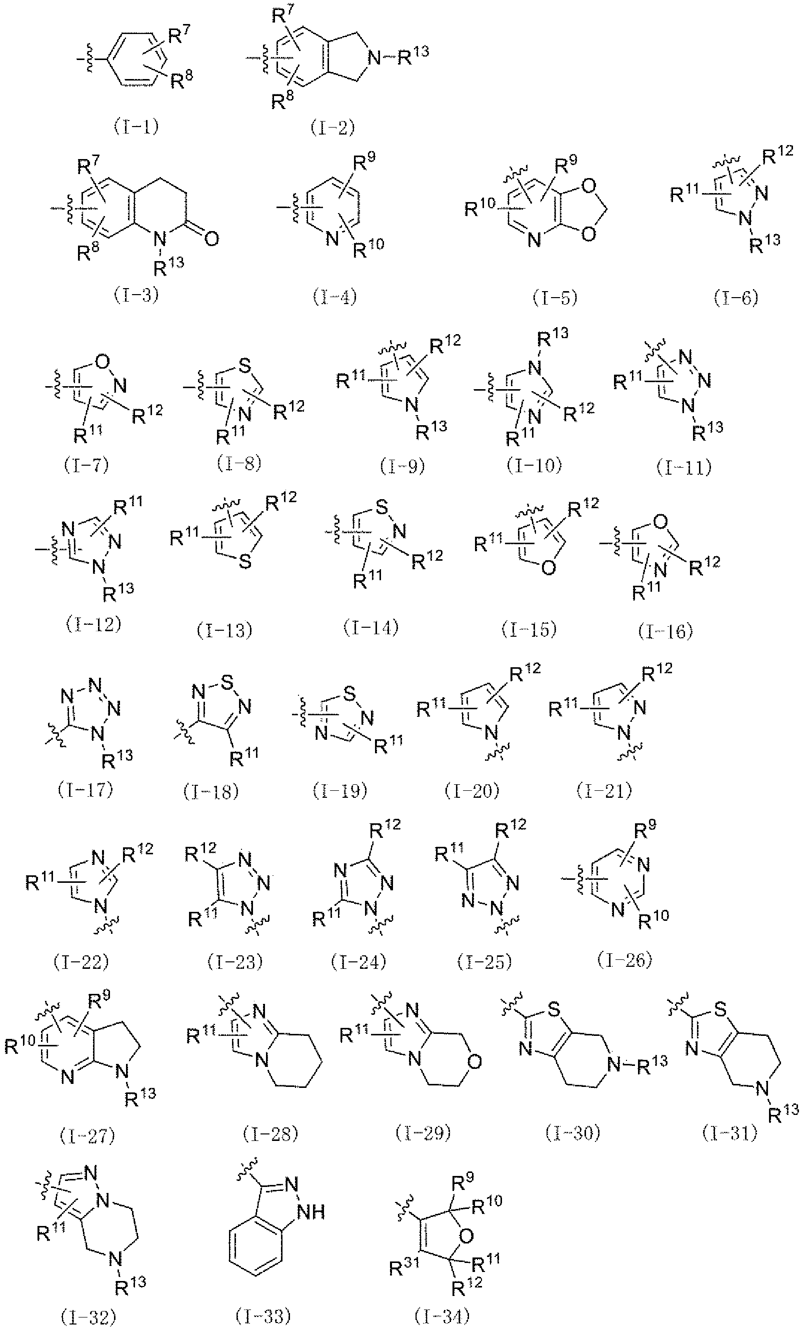 in each formula,
R⁷ is each independently hydrogen, halogen, nitro, cyano, hydroxy, C₁₋₆ alkyl, C₁₋₆ alkoxy, or -NR³¹₂;
R⁸ is each independently selected from the group consisting of:
(1) hydrogen, halogen, nitro, cyano, hydroxy,
(2) -NR³¹₂, -N(R³¹)S(O)₂R³²⁻¹, where R³²⁻¹ is an optionally substituted 4- to 10-membered heterocyclyl having 1 to 3 heteroatoms selected from nitrogen and oxygen as ring-constituting atoms, -N(R³¹)C(O)R³¹, -N(R³¹)C(O)(CH₂)₀₋₃NR³¹₂, -N(R³¹)C(O)(CH₂)₀₋₃R³²⁻², where R³²⁻² is an optionally substituted 4- to 10-membered heterocyclyl having 1 to 3 heteroatoms selected from nitrogen and oxygen as ring-constituting atoms,
(3) C₁₋₆ alkyl, -(CH₂)₁₋₅OR³¹, -(CH₂)₁₋₅R³²⁻³, where R³²⁻³ is an optionally substituted 4-to 10-membered heterocyclyl having 1 to 3 heteroatoms selected from nitrogen and oxygen as ring-constituting atoms,
(4) -(CH₂)₁₋₅NR³¹₂, -(CH₂)₁₋₅N(R³¹)R³²⁻⁴, where R³²⁻⁴ is an optionally substituted 3- to 10-membered cycloaliphatic hydrocarbon or an optionally substituted 4- to 10-membered heterocyclyl having 1 to 3 heteroatoms selected from nitrogen and oxygen as ring-constituting atoms, -(CH₂)₁₋₅N(R³¹)C(O)R³¹, -(CH₂)₁₋₅N(R³¹)C(O)(CH₂)₁₋₅OR³¹, -(CH₂)₁₋₅N(C(O)R³¹)(CH₂)₁₋₅C(O)OR³¹, -(CH₂)₁₋₅N(R³¹)C(O)(CH₂)₀₋₅NR³¹₂, -(CH₂)₁₋₅N(R³¹)C(O)R³²⁻⁵, where R³²⁻⁵ is an optionally substituted 3- to 10-membered cycloaliphatic hydrocarbon or an optionally substituted 4- to 10-membered heterocyclyl having 1 to 3 heteroatoms selected from nitrogen and oxygen as ring-constituting atoms,
(5) -(CH₂)₁₋₅N(R³¹)S(O)₂R³³⁻¹, where R³³⁻¹ is 3- to 10-membered cycloalkyl,
(6) -C(R³¹)₂C(O)R³²⁻⁶, where R³²⁻⁶ is an optionally substituted 4- to 10-membered heterocyclyl having 1 to 3 heteroatoms selected from nitrogen and oxygen as ring-constituting atoms,
(7) -C(R³¹)₂C(O)NR³¹₂, -C(R³¹)₂C(O)N(R³¹)OR³¹,
(8) -C(O)R³²⁻⁷, where R³²⁻⁷ is an optionally substituted 4- to 10-membered heterocyclyl having 1 to 3 heteroatoms selected from nitrogen and oxygen as ring-constituting atoms, or a fused ring in which the heterocyclyl is fused with an optionally substituted 5- or 6-membered monocyclic aromatic hydrocarbon,
(9) -C(O)OR³¹,
(10) -C(O)NR³¹₂, -C(O)N(R³¹)(CH₂)₀₋₅R³²⁻⁸, where R³²⁻⁸ is an optionally substituted 3-to 10-membered cycloaliphatic hydrocarbon or an optionally substituted 4- to 10-membered heterocyclyl having 1 to 3 heteroatoms selected from nitrogen and oxygen as ring-constituting atoms,
(11) -OC(R³¹)₂C(O)N(R³¹)(CH₂)₁₋₅OR³¹, -OC(R³¹)₂C(O)OR³¹, -OC(R³¹)₂C(O)NR³¹₂, - OC(R³¹)₂C(O)R³²⁻⁹, where R³²⁻⁹ is an optionally substituted 4- to 10-membered heterocyclyl having 1 to 3 heteroatoms selected from nitrogen and sulfur as ring-constituting atoms,
(12) -O(CH₂)₁₋₆R³¹, -(O(CH₂)₁₋₅)₁₋₅(CH₂)₀₋₃NR³¹₂, -(O(CH₂)₁₋₅)₁₋₅(CH₂)₀₋₃NR³¹C(O)R³¹, -(O(CH₂)₁₋₅)₁₋₅NR³¹C(NH)NR³¹₂, -O((CH₂)₁₋₅O)₁₋₅R³¹,
(13) -OC(O)C(R³¹)₂(O(CH₂)₁₋₅)₁₋₅OR³¹, and
(14) 5- or 6-membered nitrogen-containing heterocycle optionally substituted with C₁₋₃ alkyl;
R⁹, R¹⁰, R¹¹ and R¹² are each independently selected from the group consisting of: hydrogen, halogen, cyano, hydroxy, C₁₋₅ alkyl, 3- to 6-membered cycloalkyl, C₁₋₅ alkoxy, - N(R³¹)C(O)R³¹, -N(R³¹)C(O)(CH₂)₀₋₃NR³¹₂, -N(R³¹)(CH₂)₁₋₅NR³¹₂, -(CH₂)₁₋₅OR³¹, -(CH₂)₁₋₅O(CH₂)₁₋₅OR³¹, -(CH₂)₁₋₅R³⁵, -(CH₂)₁₋₅N(R³¹)C(O)R³¹, -(CH₂)₁₋₅C(O)NR³¹₂, -C(R³¹)₂NR³¹₂, - (CH₂)₁₋₅NR³¹₂, -C(O)R³⁶, -C(O)OR³¹, -C(O)NR³¹₂, -NR³¹₂, -C(H)₂R³⁵, -C(H)R³⁵₂ and -CR³⁵₃;
R³¹ is each independently hydrogen or a C₁₋₄ chain aliphatic hydrocarbon;
R³⁵ is each independently halogen, cyano, a 3- to 10-membered cycloaliphatic hydrocarbon optionally substituted with C₁₋₅ alkyl, or -OR³¹;
R³⁶ is a 4- to 10-membered heterocyclyl having 1 to 3 heteroatoms independently selected from nitrogen, oxygen and sulfur as ring-constituting atoms and optionally substituted with C₁₋₅ alkyl;
R¹³ is each independently hydrogen, C₁₋₅ alkyl, -(CH₂)₁₋₅OR³¹, -(CH₂)₁₋₅NR³¹₂, -(CH₂)₁₋₅N(R³¹)C(O)R³¹, -(CH₂)₁₋₅N(R³¹)(CH₂)₀₋₅R³⁸, -C(O)R³¹, -C(O)R³⁷ or -(CH₂)₁₋₅R³⁷;
R³⁷ is a 3- to 10-membered cycloaliphatic hydrocarbon or a 3- to 10-membered heterocyclyl having 1 to 3 heteroatoms selected from nitrogen and oxygen;
R³⁸ is cyano, hydroxy, or S(O)₂R³¹; and
the heterocycles in the structures shown in formulas (I-2), (I-3) and (I-27) to (I-32) and the benzene ring in the structure shown in formula (I-33) are each independently optionally substituted with a C₁₋₅ chain aliphatic hydrocarbon.

4. The compound or pharmaceutically acceptable derivative thereof according to claim 1, wherein R⁶ is selected from:

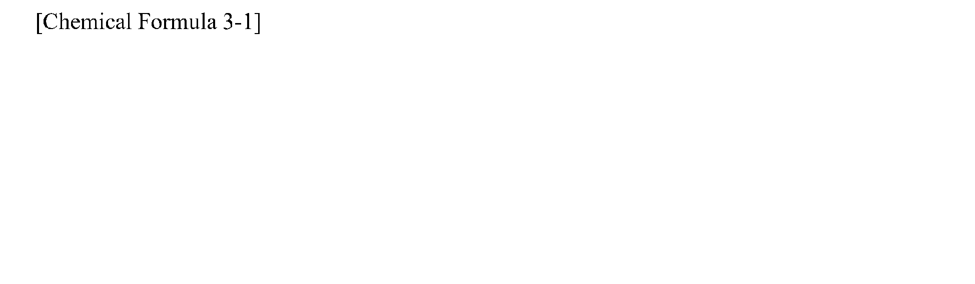

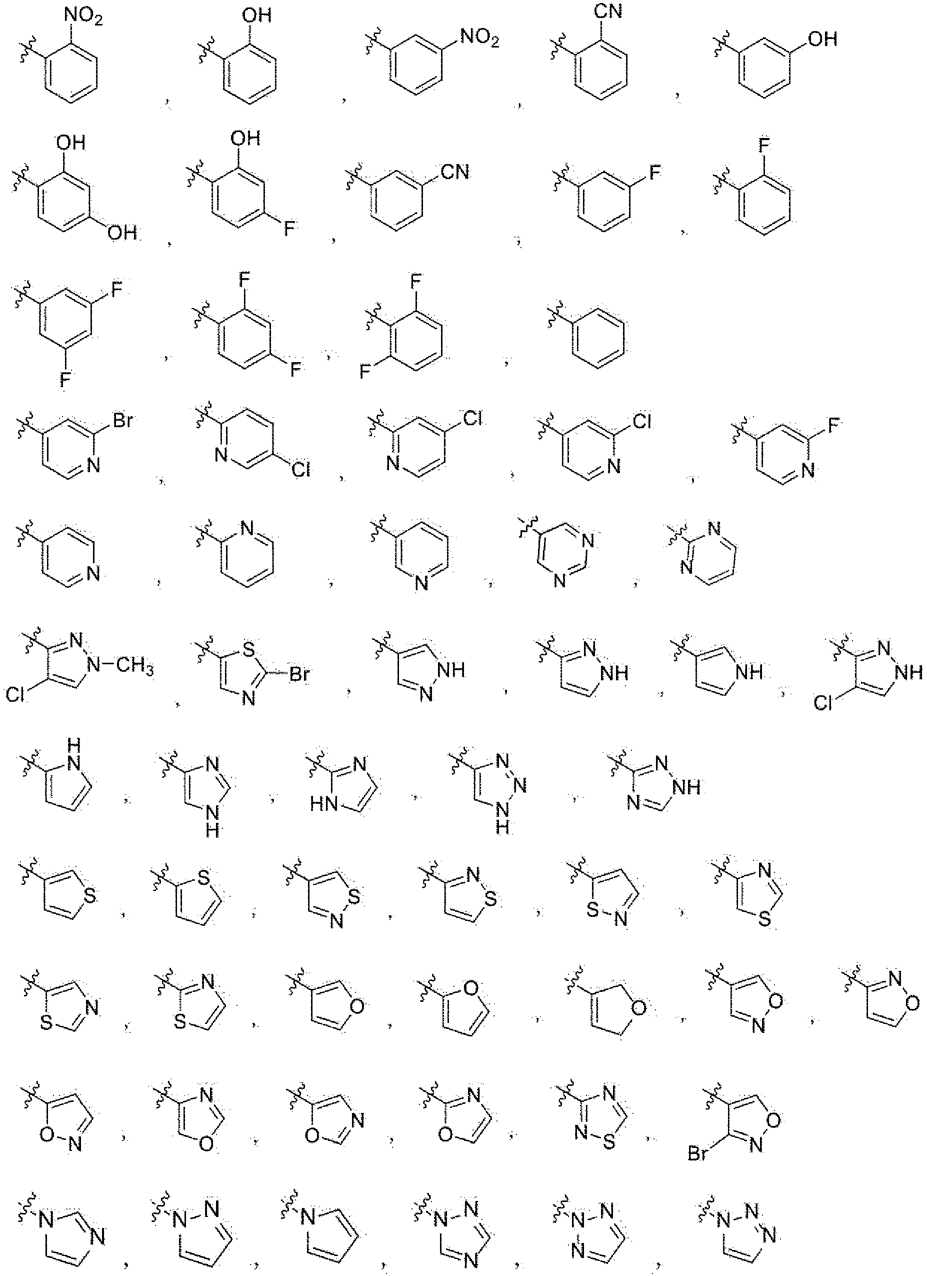

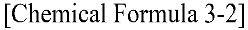

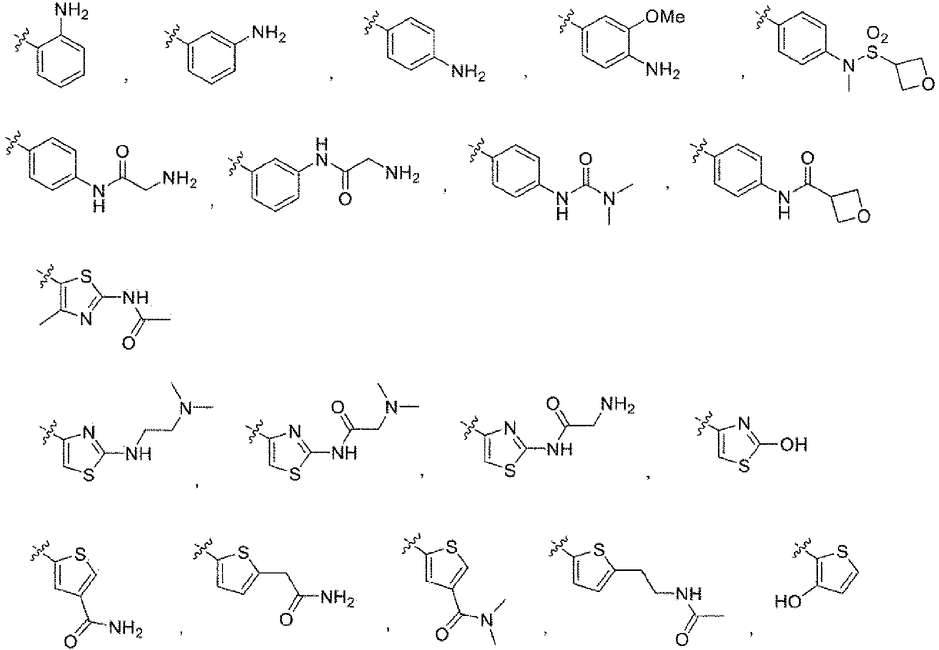

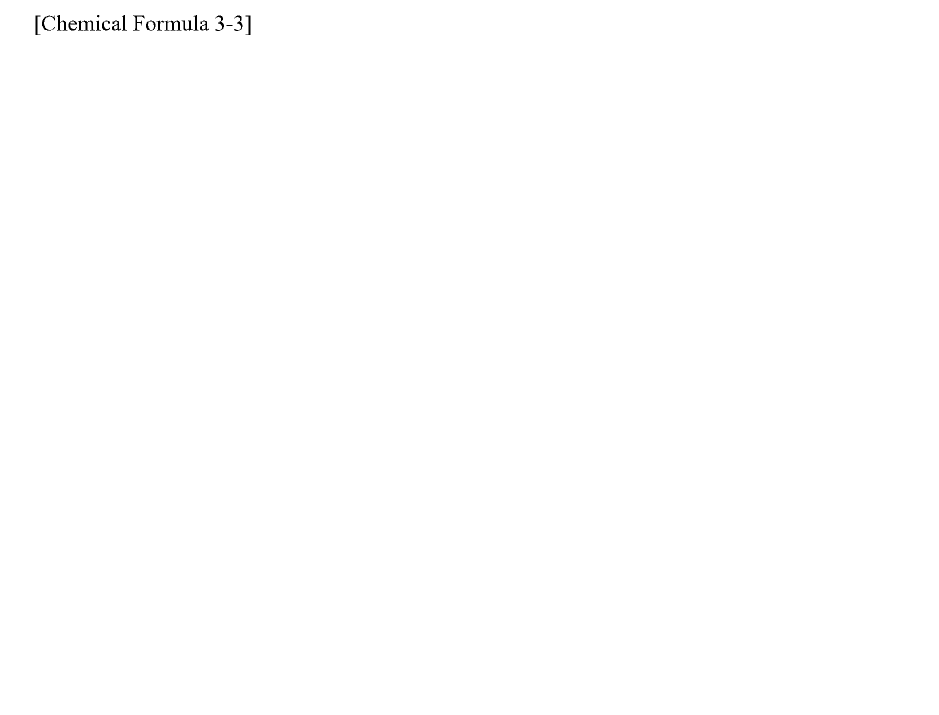

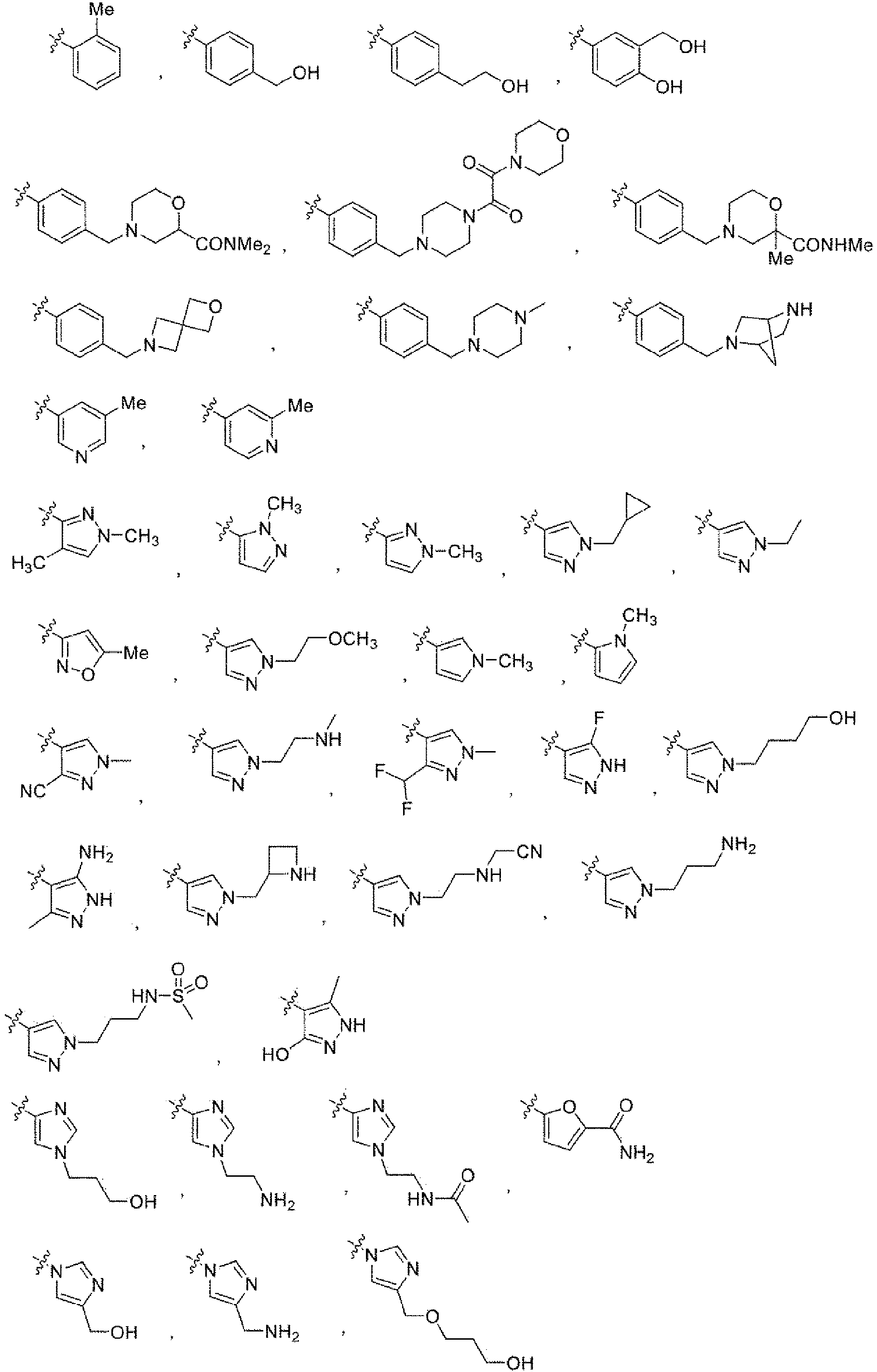

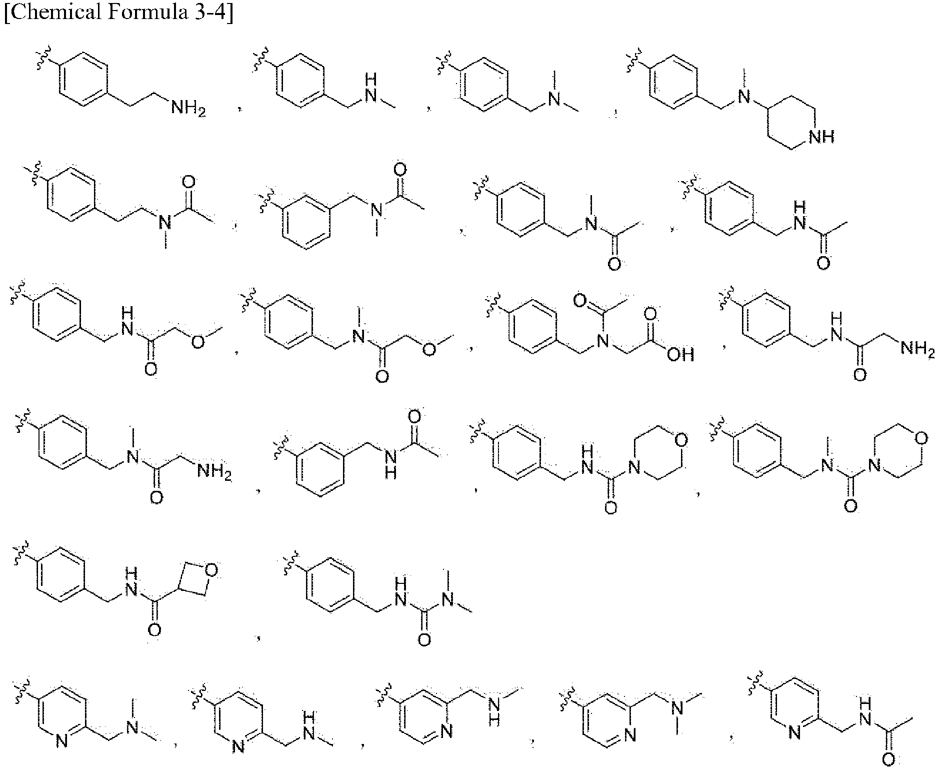

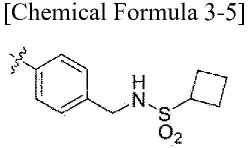

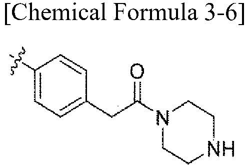

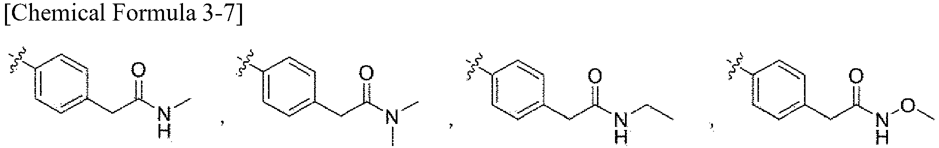

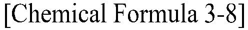

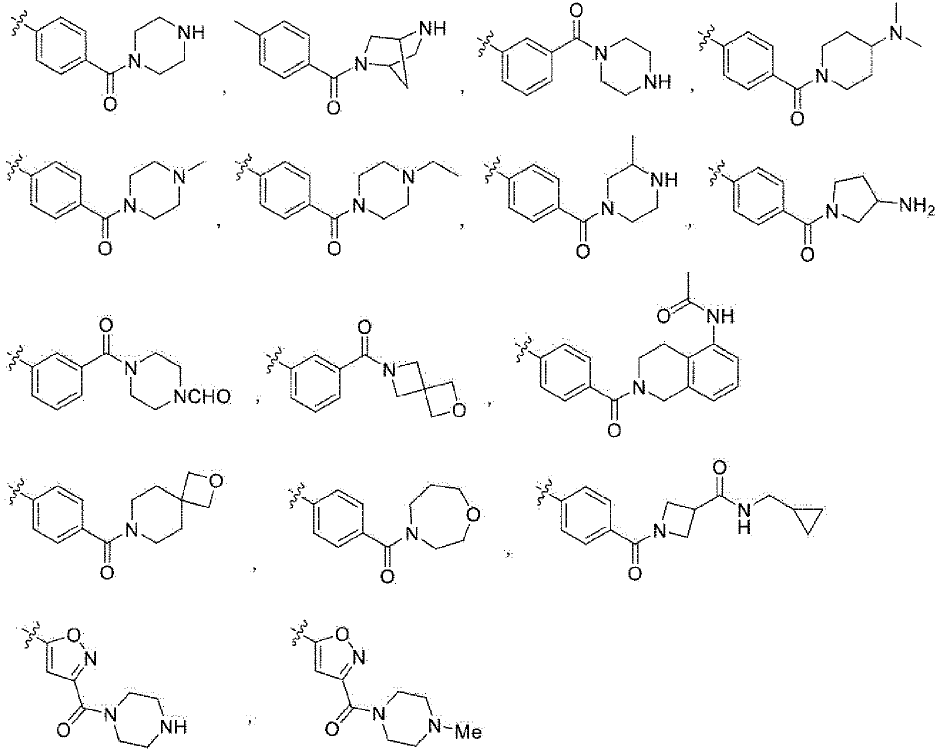

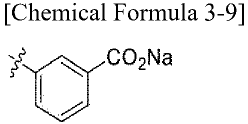

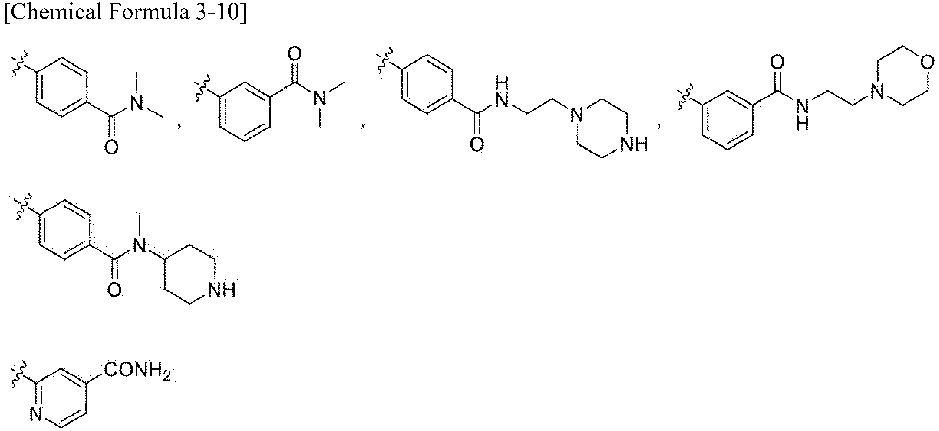

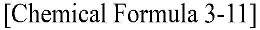

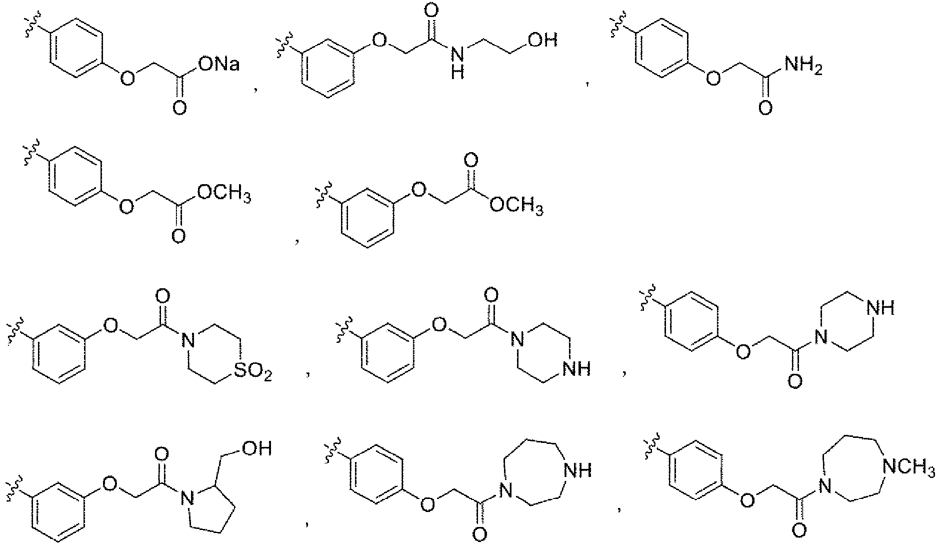

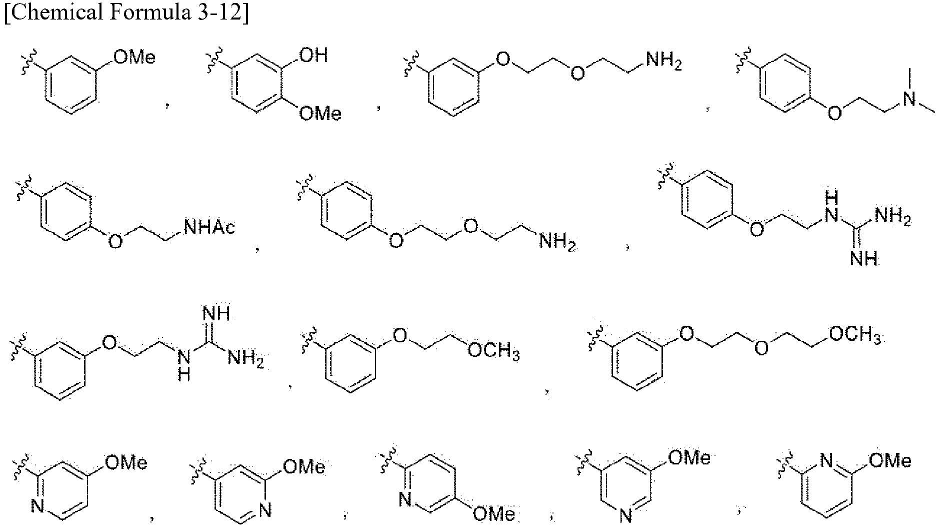

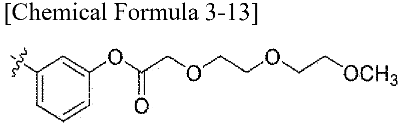

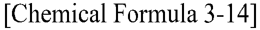

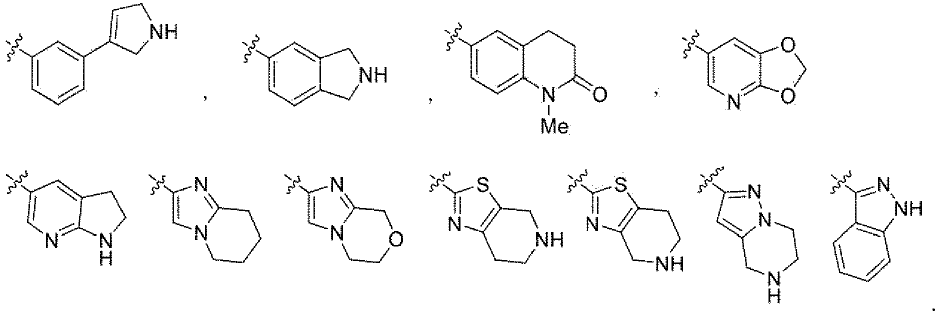

5. A compound selected from the group consisting of:
3-(2-(pyrazolo[1,5-a]pyridin-3-yl)thiazol-4-yl)phenol (compound 3),
4-phenyl-2-(pyrazolo[1,5-a]pyridin-3-yl)thiazole (compound 71),
2-methoxy-5-(2-(pyrazolo[1,5-a]pyridin-3-yl)thiazol-4-yl)phenol (compound 79),
4-(3-fluorophenyl)-2-(pyrazolo[1,5-a]pyridin-3-yl)thiazole (compound 112),
4-(2-fluorophenyl)-2-(pyrazolo[1,5-a]pyridin-3-yl)thiazole (compound 122),
4-(2-(pyrazolo[1,5-a]pyridin-3-yl)thiazol-4-yl)benzene-1,3-diol (compound 120),
4-(2,4-difluorophenyl)-2-(pyrazolo[1,5-a]pyridin-3-yl)thiazole (compound 121),
4-(3-methoxyphenyl)-2-(pyrazolo[1,5-a]pyridin-3-yl)thiazole (compound 83),
2-(pyrazolo[1,5-a]pyridin-3-yl)-4-(o-tolyl)thiazole (compound 132),
1-methyl-6-(2-(pyrazolo[1,5-a]pyridin-3-yl)thiazol-4-yl)-3,4-dihydroquinolin-2(1H)-one (compound 131),
N-(4-(2-(pyrazolo[1,5-a]pyridin-3-yl)thiazol-4-yl)benzyl)acetamide (compound 12),
4-(4-methoxypyridin-2-yl)-2-(pyrazolo[1,5-a]pyridin-3-yl)thiazole (compound 7),
2-(pyrazolo[1,5-a]pyridin-3-yl)-4-(pyridin-3-yl)thiazole (compound 15),
2-(pyrazolo[1,5-a]pyridin-3-yl)-4-(pyridin-4-yl)thiazole (compound 92),
2-(pyrazolo[1,5-a]pyridin-3-yl)-4-(pyridin-2-yl)thiazole (compound 93),
4-(2-chloropyridin-4-yl)-2-(pyrazolo[1,5-a]pyridin-3-yl)thiazole (compound 94),
4-(2-methoxypyridin-4-yl)-2-(pyrazolo[1,5-a]pyridin-3-yl)thiazole (compound 95),
4-(6-methoxypyridin-2-yl)-2-(pyrazolo[1,5-a]pyridin-3-yl)thiazole (compound 96),
4-(5-methylpyridin-3-yl)-2-(pyrazolo[1,5-a]pyridin-3-yl)thiazole (compound 97),
6-(2-(pyrazolo[1,5-a]pyridin-3-yl)thiazol-4-yl)-[1,3]dioxolo[4,5-b]pyridine (compound 98),
4-(2-methylpyridin-4-yl)-2-(pyrazolo[1,5-a]pyridin-3-yl)thiazole (compound 99),
4-(2-fluoropyridin-4-yl)-2-(pyrazolo[1,5-a]pyridin-3-yl)thiazole (compound 100),
4-(2-bromopyridin-4-yl)-2-(pyrazolo[1,5-a]pyridin-3-yl)thiazole (compound 101),
4-(5-chloropyridin-2-yl)-2-(pyrazolo[1,5-a]pyridin-3-yl)thiazole (compound 125),
4-(4-chloropyridin-2-yl)-2-(pyrazolo[1,5-a]pyridin-3-yl)thiazole (compound 135),
2'-bromo-2-(pyrazolo[1,5-a]pyridin-3-yl)-4,5'-bithiazole (compound 37),
4-(2-(pyrazolo[1,5-a]pyridin-3-yl)thiazol-4-yl)aniline hydrochloride (compound 1),
4-(3-nitrophenyl)-2-(pyrazolo[1,5-a]pyridin-3-yl)thiazole (compound 4),
3-(2-(pyrazolo[1,5-a]pyridin-3-yl)thiazol-4-yl)aniline hydrochloride (compound 10),
4-(2-nitrophenyl)-2-(pyrazolo[1,5-a]pyridin-3-yl)thiazole (compound 133),
2-(2-(pyrazolo[1,5-a]pyridin-3-yl)thiazol-4-yl)aniline hydrochloride (compound 134),
2-methoxy-4-(2-(pyrazolo[1,5-a]pyridin-3-yl)thiazol-4-yl)aniline (compound 90),
2-(2-(pyrazolo[1,5-a]pyridin-3-yl)thiazol-4-yl)phenol (compound 118),
3-(2-(pyrazolo[1,5-a]pyridin-3-yl)thiazol-4-yl)benzonitrile (compound 115),
2-(2-(pyrazolo[1,5-a]pyridin-3-yl)thiazol-4-yl)benzonitrile (compound 116),
4-(3,5-difluorophenyl)-2-(pyrazolo[1,5-a]pyridin-3-yl)thiazole (compound 119),
4-(2,6-difluorophenyl)-2-(pyrazolo[1,5-a]pyridin-3-yl)thiazole (compound 123),
5-fluoro-2-(2-(pyrazolo[1,5-a]pyridin-3-yl)thiazol-4-yl)phenol (compound 114),
2-(2-(pyrazolo[1,5-a]pyridin-3-yl)thiazol-4-yl)isonicotinamide (compound 126),
4-(5-methoxypyridin-3-yl)-2-(pyrazolo[1,5-a]pyridin-3-yl)thiazole (compound 124),
4-(5-methoxypyridin-2-yl)-2-(pyrazolo[1,5-a]pyridin-3-yl)thiazole (compound 127),
4-(1H-pyrazol-4-yl)-2-(pyrazolo[1,5-a]pyridin-3-yl)thiazole (compound 107),
4-(4-chloro-1-methyl-1H-pyrazol-3-yl)-2-(pyrazolo[1,5-a]pyridin-3-yl)thiazole (compound 53),
4-(1,4-dimethyl-1H-pyrazol-3-yl)-2-(pyrazolo[1,5-a]pyridin-3-yl)thiazole hydrobromide (compound 54),
4-(1-methyl-1H-pyrazol-5-yl)-2-(pyrazolo[1,5-a]pyridin-3-yl)thiazole hydrobromide (compound 106),
4-(1H-pyrazol-3-yl)-2-(pyrazolo[1,5-a]pyridin-3-yl)thiazole (compound 109),
5-methyl-3-(2-(pyrazolo[1,5-a]pyridin-3-yl)thiazol-4-yl)isoxazole hydrobromide (compound 105),
N-methyl-N-(4-(2-(pyrazolo[1,5-a]pyridin-3-yl)thiazol-4-yl)phenyl)oxetane-3-sulfonamide (compound 69),
N-methyl-N-(4-(2-(pyrazolo[1,5-a]pyridin-3-yl)thiazol-4-yl)benzyl)acetamide (compound 16),
N-(4-(2-(pyrazolo[1,5-a]pyridin-3-yl)thiazol-4-yl)benzyl)morpholine-4-carboxamide (compound 72),
N-methyl-1-(4-(2-(pyrazolo[1,5-a]pyridin-3-yl)thiazol-4-yl)phenyl)methanamine hydrochloride (compound 74),
N-methyl-N-(4-(2-(pyrazolo[1,5-a]pyridin-3-yl)thiazol-4-yl)benzyl)morpholine-4-carboxamide (compound 76),
2-methoxy-N-methyl-N-(4-(2-(pyrazolo[1,5-a]pyridin-3-yl)thiazol-4-yl)benzyl)acetamide (compound 78),
1,1-dimethyl-3-(4-(2-(pyrazolo[1,5-a]pyridin-3-yl)thiazol-4-yl)phenyl)urea (compound 59),
1,1-dimethyl-3-(4-(2-(pyrazolo[1,5-a]pyridin-3-yl)thiazol-4-yl)benzyl)urea (compound 75),
2-methoxy-N-(4-(2-(pyrazolo[1,5-a]pyridin-3-yl)thiazol-4-yl)benzyl)acetamide (compound 77),
N-(4-(2-(pyrazolo[1,5-a]pyridin-3-yl)thiazol-4-yl)benzyl)cyclobutanesulfonamide (compound 82),
N-(4-(2-(pyrazolo[1,5-a]pyridin-3-yl)thiazol-4-yl)benzyl)oxetane-3-carboxamide (compound 85),
2-amino-N-(4-(2-(pyrazolo[1,5-a]pyridin-3-yl)thiazol-4-yl)phenyl)acetamide hydrochloride (compound 64),
N-(4-(2-(pyrazolo[1,5-a]pyridin-3-yl)thiazol-4-yl)phenyl)oxetane-3-carboxamide (compound 66),
2-amino-N-(3-(2-(pyrazolo[1,5-a]pyridin-3-yl)thiazol-4-yl)phenyl)acetamide hydrochloride (compound 65),
2-amino-N-methyl-N-(4-(2-(pyrazolo[1,5-a]pyridin-3-yl)thiazol-4-yl)benzyl)acetamide hydrochloride (compound 81),
2-amino-N-(4-(2-(pyrazolo[1,5-a]pyridin-3-yl)thiazol-4-yl)benzyl)acetamide hydrochloride (compound 80),
sodium 2-(4-(2-(pyrazolo[1,5-a]pyridin-3-yl)thiazol-4-yl)phenoxy)acetate (compound 5),
N-acetyl-N-(4-(2-(pyrazolo[1,5-a]pyridin-3-yl)thiazol-4-yl)benzyl)glycine (compound 73),
2-(4-(2-(pyrazolo[1,5-a]pyridin-3-yl)thiazol-4-yl)phenoxy)acetamide (compound 26),
3-(2-(pyrazolo[1,5-a]pyridin-3-yl)thiazol-4-yl)phenyl-2-(2-(2-methoxyethoxy)ethoxy)acetate (compound 13),
4-(3-(2-methoxyethoxy)phenyl)-2-(pyrazolo[1,5-a]pyridin-3-yl)thiazole (compound 14),
4-(3-(2-(2-methoxyethoxy)ethoxy)phenyl)-2-(pyrazolo[1,5-a]pyridin-3-yl)thiazole (compound 22),
4-(1-(cyclopropylmethyl)-1H-pyrazol-4-yl)-2-(pyrazolo[1,5-a]pyridin-3-yl)thiazole (compound 108),
4-(1-ethyl-1H-pyrazol-4-yl)-2-(pyrazolo[1,5-a]pyridin-3-yl)thiazole (compound 110),
4-(1-(2-methoxyethyl)-1H-pyrazol-4-yl)-2-(pyrazolo[1,5-a]pyridin-3-yl)thiazole hydrochloride (compound 111),
2-(2-(4-(2-(pyrazolo[1,5-a]pyridin-3-yl)thiazol-4-yl)phenoxy)ethoxy)ethan-1-amine hydrochloride (compound 32),
2-(2-(3-(2-(pyrazolo[1,5-a]pyridin-3-yl)thiazol-4-yl)phenoxy)ethoxy)ethan-1-amine hydrochloride (compound 20),
N,N-dimethyl-2-(4-(2-(pyrazolo[1,5-a]pyridin-3-yl)thiazol-4-yl)phenoxy)ethan-1-amine hydrochloride (compound 47),
1-(2-(3-(2-(pyrazolo[1,5-a]pyridin-3-yl)thiazol-4-yl)phenoxy)ethyl)guanidine hydrochloride (compound 52),
1-(2-(4-(2-(pyrazolo[1,5-a]pyridin-3-yl)thiazol-4-yl)phenoxy)ethyl)guanidine hydrochloride (compound 51),
N-(2-(4-(2-(pyrazolo[1,5-a]pyridin-3-yl)thiazol-4-yl)phenoxy)ethyl)acetamide (compound 45),
N-(2-hydroxyethyl)-2-(3-(2-(pyrazolo[1,5-a]pyridin-3-yl)thiazol-4-yl)phenoxy)acetamide (compound 19),
methyl 2-(3-(2-(pyrazolo[1,5-a]pyridin-3-yl)thiazol-4-yl)phenoxy)acetate (compound 18),
methyl 2-(4-(2-(pyrazolo[1,5-a]pyridin-3-yl)thiazol-4-yl)phenoxy)acetate (compound 25),
1-(piperazin-1-yl)-2-(4-(2-(pyrazolo[1,5-a]pyridin-3-yl)thiazol-4-yl)phenoxy)ethan-1-one (compound 31),
1-(1,4-diazepan-1-yl)-2-(4-(2-(pyrazolo[1,5-a]pyridin-3-yl)thiazol-4-yl)phenoxy)ethan-1-one hydrochloride (compound 55),
1-(4-methyl-1,4-diazepan-1-yl)-2-(4-(2-(pyrazolo[1,5-a]pyridin-3-yl)thiazol-4-yl)phenoxy)ethan-1-one hydrochloride (compound 102),
1-(2-(hydroxymethyl)pyrrolidin-1-yl)-2-(3-(2-(pyrazolo[1,5-a]pyridin-3-yl)thiazol-4-yl)phenoxy)ethan-1-one (compound 21),
1-(piperazin-1-yl)-2-(3-(2-(pyrazolo[1,5-a]pyridin-3-yl)thiazol-4-yl)phenoxy)ethan-1-one (compound 23),
1-(1,1-dioxidothiomorpholino)-2-(3-(2-(pyrazolo[1,5-a]pyridin-3-yl)thiazol-4-yl)phenoxy)ethan-1-one (compound 24),
(4-methylpiperazin-1-yl)(5-(2-(pyrazolo[1,5-a]pyridin-3-yl)thiazol-4-yl)isoxazol-3-yl)methanone hydrochloride (compound 103),
piperazin-1-yl(5-(2-(pyrazolo[1,5-a]pyridin-3-yl)thiazol-4-yl)isoxazol-3-yl)methanone hydrochloride (compound 104),
N-(4'-methyl-2-(pyrazolo[1,5-a]pyridin-3-yl)-[4,5'-bithiazole]-2'-yl)acetamide (compound 46),
N,N-dimethyl-4-(2-(pyrazolo[1,5-a]pyridin-3-yl)thiazol-4-yl)benzamide (compound 49),
sodium 3-(2-(pyrazolo[1,5-a]pyridin-3-yl)thiazol-4-yl)benzoate (compound 6),
(3-(2-(pyrazolo[1,5-a]pyridin-3-yl)thiazol-4-yl)phenyl)(2-oxa-6-azaspiro[3.3]heptan-6-yl)methanone (compound 33),
N,N-dimethyl-2-(4-(2-(pyrazolo[1,5-a]pyridin-3-yl)thiazol-4-yl)phenyl)acetamide (compound 87),
N-(cyclopropylmethyl)-1-(4-(2-(pyrazolo[1,5-a]pyridin-3-yl)thiazol-4-yl)benzoyl)azetidine-3-carboxamide (compound 9),
(4-(2-(pyrazolo[1,5-a]pyridin-3-yl)thiazol-4-yl)phenyl)(2-oxa-7-azaspiro[3.5]nonan-7-yl)methanone (compound 40),
(1,4-oxazepan-4-yl)(4-(2-(pyrazolo[1,5-a]pyridin-3-yl)thiazol-4-yl)phenyl)methanone (compound 41),
(4-(dimethylamino)piperidin-1-yl)(4-(2-(pyrazolo[1,5-a]pyridin-3-yl)thiazol-4-yl)phenyl)methanone (compound 48),
(4-methylpiperazin-1-yl)(4-(2-(pyrazolo[1,5-a]pyridin-3-yl)thiazol-4-yl)phenyl)methanone (compound 50),
(4-ethylpiperazin-1-yl)(4-(2-(pyrazolo[1,5-a]pyridin-3-yl)thiazol-4-yl)phenyl)methanone (compound 56),
(3-methylpiperazin-1-yl)(4-(2-(pyrazolo[1,5-a]pyridin-3-yl)thiazol-4-yl)phenyl)methanone (compound 58),
N,N-dimethyl-3-(2-(pyrazolo[1,5-a]pyridin-3-yl)thiazol-4-yl)benzamide (compound 61),
4-(3-(2-(pyrazolo[1,5-a]pyridin-3-yl)thiazol-4-yl)benzoyl)piperazine-1-carbaldehyde (compound 68),
N-(2-morpholinoethyl)-3-(2-(pyrazolo[1,5-a]pyridin-3-yl)thiazol-4-yl)benzamide (compound 30),
N-methyl-2-(4-(2-(pyrazolo[1,5-a]pyridin-3-yl)thiazol-4-yl)phenyl)acetamide (compound 88),
N-ethyl-2-(4-(2-(pyrazolo[1,5-a]pyridin-3-yl)thiazol-4-yl)phenyl)acetamide (compound 89),
N-methoxy-2-(4-(2-(pyrazolo[1,5-a]pyridin-3-yl)thiazol-4-yl)phenyl)acetamide (compound 91),
(2,5-diazabicyclo[2.2.1]heptan-2-yl)(4-(2-(pyrazolo[1,5-a]pyridin-3-yl)thiazol-4-yl)phenyl)methanone hydrochloride (compound 43),
N-(2-(piperazin-1-yl)ethyl)-4-(2-(pyrazolo[1,5-a]pyridin-3-yl)thiazol-4-yl)benzamide hydrochloride (compound 11),
(3-aminopyrrolidin-1-yl)(4-(2-(pyrazolo[1,5-a]pyridin-3-yl)thiazol-4-yl)phenyl)methanone hydrochloride (compound 42),
N-methyl-N-(piperidin-4-yl)-4-(2-(pyrazolo[1,5-a]pyridin-3-yl)thiazol-4-yl)benzamide hydrochloride (compound 44),
piperazin-1-yl(4-(2-(pyrazolo[1,5-a]pyridin-3-yl)thiazol-4-yl)phenyl)methanone hydrochloride (compound 57),
piperazin-1-yl(3-(2-(pyrazolo[1,5-a]pyridin-3-yl)thiazol-4-yl)phenyl)methanone hydrochloride (compound 63),
1-(piperazin-1-yl)-2-(4-(2-(pyrazolo[1,5-a]pyridin-3-yl)thiazol-4-yl)phenyl)ethan-1-one hydrochloride (compound 86),
N-(2-(4-(2-(pyrazolo[1,5-a]pyridin-3-yl)thiazol-4-yl)benzoyl)-1,2,3,4-tetrahydroisoquinolin-5-yl)acetamide (compound 60),
2-(hydroxymethyl)-4-(2-(pyrazolo[1,5-a]pyridin-3-yl)thiazol-4-yl)phenol (compound 8),
(4-(2-(pyrazolo[1,5-a]pyridin-3-yl)thiazol-4-yl)phenyl)methanol (compound 2),
2-(4-(2-(pyrazolo[1,5-a]pyridin-3-yl)thiazol-4-yl)phenyl)ethan-1-ol (compound 84),
4-(3-(2,5-dihydro-1H-pyrrol-3-yl)phenyl)-2-(pyrazolo[1,5-a]pyridin-3-yl)thiazole hydrochloride (compound 17),
6-(4-(2-(pyrazolo[1,5-a]pyridin-3-yl)thiazol-4-yl)benzyl)-2-oxa-6-azaspiro[3.3]heptane (compound 29),
N,N-dimethyl-1-(4-(2-(pyrazolo[1,5-a]pyridin-3-yl)thiazol-4-yl)phenyl)methanamine (compound 27),
4-(4-((4-methylpiperazin-1-yl)methyl)phenyl)-2-(pyrazolo[1,5-a]pyridin-3-yl)thiazole (compound 28),
N,N-dimethyl-4-(4-(2-(pyrazolo[1,5-a]pyridin-3-yl)thiazol-4-yl)benzyl)morpholine-2-carboxamide (compound 38),
N,2-dimethyl-4-(4-(2-(pyrazolo[1,5-a]pyridin-3-yl)thiazol-4-yl)benzyl)morpholine-2-carboxamide (compound 39),
1-morpholino-2-(4-(4-(2-(pyrazolo[1,5-a]pyridin-3-yl)thiazol-4-yl)benzyl)piperazin-1-yl)ethane-1,2-dione hydrochloride (compound 36),
4-(4-((2,5-diazabicyclo[2.2.1]heptan-2-yl)methyl)phenyl)-2-(pyrazolo[1,5-a]pyridin-3-yl)thiazole hydrochloride (compound 34),
N-methyl-N-(4-(2-(pyrazolo[1,5-a]pyridin-3-yl)thiazol-4-yl)benzyl)piperidin-4-amine hydrochloride (compound 35),
4-(isoindolin-5-yl)-2-(pyrazolo[1,5-a]pyridin-3-yl)thiazole hydrochloride (compound 117),
N-methyl-N-(4-(2-(pyrazolo[1,5-a]pyridin-3-yl)thiazol-4-yl)phenethyl)acetamide (compound 62),
2-(4-(2-(pyrazolo[1,5-a]pyridin-3-yl)thiazol-4-yl)phenyl)ethan-1-amine hydrochloride (compound 113),
N-(3-(2-(pyrazolo[1,5-a]pyridin-3-yl)thiazol-4-yl)benzyl)acetamide (compound 67), N-methyl-N-(3-(2-(pyrazolo[1,5-a]pyridin-3-yl)thiazol-4-yl)benzyl)acetamide (compound 70),
N,N-dimethyl-1-(4-(2-(pyrazolo[1,5-a]pyridin-3-yl)thiazol-4-yl)pyridin-2-yl)methanamine (compound 137),
N,N-dimethyl-1-(5-(2-(pyrazolo[1,5-a]pyridin-3-yl)thiazol-4-yl)pyridin-2-yl)methanamine (compound 128),
N-methyl-1-(4-(2-(pyrazolo[1,5-a]pyridin-3-yl)thiazol-4-yl)pyridin-2-yl)methanamine (compound 136),
N-((5-(2-(pyrazolo[1,5-a]pyridin-3-yl)thiazol-4-yl)pyridin-2-yl)methyl)acetamide (compound 129),
N-methyl-1-(5-(2-(pyrazolo[1,5-a]pyridin-3-yl)thiazol-4-yl)pyridin-2-yl)methanamine (compound 130),
2-(pyrazolo[1,5-a]pyridin-3-yl)-4-(1H-pyrrol-3-yl)thiazole (compound 138),
2-(pyrazolo[1,5-a]pyridin-3-yl)-4-(1H-pyrrol-2-yl)thiazole (compound 139),
4-(1H-imidazol-4-yl)-2-(pyrazolo[1,5-a]pyridin-3-yl)thiazole (compound 140),
4-(1H-imidazol-2-yl)-2-(pyrazolo[1,5-a]pyridin-3-yl)thiazole (compound 141),
2-(pyrazolo[1,5-a]pyridin-3-yl)-4-(1H-1,2,3-triazol-4-yl)thiazole (compound 142),
2-(pyrazolo[1,5-a]pyridin-3-yl)-4-(1H-1,2,4-triazol-3-yl)thiazole (compound 143),
2-(pyrazolo[1,5-a]pyridin-3-yl)-4-(thiophen-3-yl)thiazole (compound 144),
2-(pyrazolo[1,5-a]pyridin-3-yl)-4-(thiophen-2-yl)thiazole (compound 145),
3-(2-(pyrazolo[1,5-a]pyridin-3-yl)thiazol-4-yl)isothiazole (compound 146),
4-(2-(pyrazolo[1,5-a]pyridin-3-yl)thiazol-4-yl)isothiazole (compound 147),
5-(2-(pyrazolo[1,5-a]pyridin-3-yl)thiazol-4-yl)isothiazole (compound 148),
2-(pyrazolo[1,5-a]pyridin-3-yl)-4,4'-bithiazole (compound 149),
2-(pyrazolo[1,5-a]pyridin-3-yl)-4,5'-bithiazole (compound 150),
2'-(pyrazolo[1,5-a]pyridin-3-yl)-2,4'-bithiazole (compound 151),
4-(furan-3-yl)-2-(pyrazolo[1,5-a]pyridin-3-yl)thiazole (compound 152),
4-(furan-2-yl)-2-(pyrazolo[1,5-a]pyridin-3-yl)thiazole (compound 153),
4-(2-(pyrazolo[1,5-a]pyridin-3-yl)thiazol-4-yl)isoxazole (compound 154),
3-(2-(pyrazolo[1,5-a]pyridin-3-yl)thiazol-4-yl)isoxazole (compound 155),
5-(2-(pyrazolo[1,5-a]pyridin-3-yl)thiazol-4-yl)isoxazole (compound 156),
4-(2-(pyrazolo[1,5-a]pyridin-3-yl)thiazol-4-yl)oxazole (compound 157),
5-(2-(pyrazolo[1,5-a]pyridin-3-yl)thiazol-4-yl)oxazole (compound 158),
2-(2-(pyrazolo[1,5-a]pyridin-3-yl)thiazol-4-yl)oxazole (compound 159),
4-(1-methyl-1H-pyrazol-3-yl)-2-(pyrazolo[1,5-a]pyridin-3-yl)thiazole (compound 160),
4-(1-methyl-1H-pyrrol-3-yl)-2-(pyrazolo[1,5-a]pyridin-3-yl)thiazole (compound 161),
3-(2-(pyrazolo[1,5-a]pyridin-3-yl)thiazol-4-yl)-1,2,4-thiadiazole (compound 162),
4-(1-methyl-1H-pyrrol-2-yl)-2-(pyrazolo[1,5-a]pyridin-3-yl)thiazole (compound 163),
3-bromo-4-(2-(pyrazolo[1,5-a]pyridin-3-yl)thiazol-4-yl)isoxazole (compound 164),
4-(1H-imidazol-1-yl)-2-(pyrazolo[1,5-a]pyridin-3-yl)thiazole (compound 165),
4-(1H-pyrazol-1-yl)-2-(pyrazolo[1,5-a]pyridin-3-yl)thiazole (compound 166),
2-(pyrazolo[1,5-a]pyridin-3-yl)-4-(1H-pyrrol-1-yl)thiazole (compound 167),
2-(pyrazolo[1,5-a]pyridin-3-yl)-4-(1H-1,2,4-triazol-1-yl)thiazole (compound 168),
2-(pyrazolo[1,5-a]pyridin-3-yl)-4-(2H-1,2,3-triazol-2-yl)thiazole (compound 169),
2-(pyrazolo[1,5-a]pyridin-3-yl)-4-(1H-1,2,3-triazol-1-yl)thiazole (compound 170),
5-(2-(pyrazolo[1,5-a]pyridin-3-yl)thiazol-4-yl)thiophene-3-carboxamide (compound 171),
2-(5-(2-(pyrazolo[1,5-a]pyridin-3-yl)thiazol-4-yl)thiophen-2-yl)acetamide (compound 172),
N,N-dimethyl-5-(2-(pyrazolo[1,5-a]pyridin-3-yl)thiazol-4-yl)thiophene-3-carboxamide (compound 173),
N-(2-(5-(2-(pyrazolo[1,5-a]pyridin-3-yl)thiazol-4-yl)thiophen-2-yl)ethyl)acetamide (compound 174),
4-(3-(difluoromethyl)-1-methyl-1H-pyrazol-4-yl)-2-(pyrazolo[1,5-a]pyridin-3-yl)thiazole (compound 178),
2-(2-(pyrazolo[1,5-a]pyridin-3-yl)thiazol-4-yl)thiophen-3-ol (compound 175),
1-methyl-4-(2-(pyrazolo[1,5-a]pyridin-3-yl)thiazol-4-yl)-1H-pyrazole-3-carbonitrile (compound 176),
N-methyl-2-(4-(2-(pyrazolo[1,5-a]pyridin-3-yl)thiazol-4-yl)-1H-pyrazol-1-yl)ethan-1-amine (compound 177),
4-(5-fluoro-1H-pyrazol-4-yl)-2-(pyrazolo[1,5-a]pyridin-3-yl)thiazole (compound 179),
3-methyl-4-(2-(pyrazolo[1,5-a]pyridin-3-yl)thiazol-4-yl)-1H-pyrazol-5-amine (compound 181),
4-(4-(2-(pyrazolo[1,5-a]pyridin-3-yl)thiazol-4-yl)-1H-pyrazol-1-yl)butan-1-ol (compound 180),
4-(1-(azetidin-2-ylmethyl)-1H-pyrazol-4-yl)-2-(pyrazolo[1,5-a]pyridin-3-yl)thiazole (compound 182),
2-((2-(4-(2-(pyrazolo[1,5-a]pyridin-3-yl)thiazol-4-yl)-1H-pyrazol-1-yl)ethyl)amino)acetonitrile (compound 183),
3-(4-(2-(pyrazolo[1,5-a]pyridin-3-yl)thiazol-4-yl)-1H-pyrazol-1-yl)propan-1-amine (compound 184),
N-(3-(4-(2-(pyrazolo[1,5-a]pyridin-3-yl)thiazol-4-yl)-1H-pyrazol-1-yl)propyl)methanesulfonamide (compound 185),
5-methyl-4-(2-(pyrazolo[1,5-a]pyridin-3-yl)thiazol-4-yl)-1H-pyrazol-3-ol (compound 186),
N¹,N¹-dimethyl-N²-(2'-(pyrazolo[1,5-a]pyridin-3-yl)-[4,4'-bithiazole]-2-yl)ethane-1,2-diamine (compound 187),
2-(dimethylamino)-N-(2'-(pyrazolo[1,5-a]pyridin-3-yl)-[4,4'-bithiazole]-2-yl)acetamide (compound 188),
2-amino-N-(2'-(pyrazolo[1,5-a]pyridin-3-yl)-[4,4'-bithiazole]-2-yl)acetamide hydrochloride (compound 189),
2'-(pyrazolo[1,5-a]pyridin-3-yl)-[4,4'-bithiazole]-2-ol (compound 190),
5-(2-(pyrazolo[1,5-a]pyridin-3-yl)thiazol-4-yl)furan-2-carboxamide (compound 191),
4-(2,5-dihydrofuran-3-yl)-2-(pyrazolo[1,5-a]pyridin-3-yl)thiazole (compound 192),
(1-(2-(pyrazolo[1,5-a]pyridin-3-yl)thiazol-4-yl)-1H-imidazol-4-yl)methanol (compound 193),
(1-(2-(pyrazolo[1,5-a]pyridin-3-yl)thiazol-4-yl)-1H-imidazol-4-yl)methanamine hydrochloride (compound 194),
3-((1-(2-(pyrazolo[1,5-a]pyridin-3-yl)thiazol-4-yl)-1H-imidazol-4-yl)methoxy)propan-1-ol (compound 195),
3-(4-(2-(pyrazolo[1,5-a]pyridin-3-yl)thiazol-4-yl)-1H-imidazol-1-yl)propan-1-ol (compound 196),
2-(4-(2-(pyrazolo[1,5-a]pyridin-3-yl)thiazol-4-yl)-1H-imidazol-1-yl)ethan-1-amine (compound 197),
N-(2-(4-(2-(pyrazolo[1,5-a]pyridin-3-yl)thiazol-4-yl)-1H-imidazol-1-yl)ethyl)acetamide (compound 198),
2-(pyrazolo[1,5-a]pyridin-3-yl)-4-(pyrimidin-5-yl)thiazole (compound 199),
2-(pyrazolo[1,5-a]pyridin-3-yl)-4-(pyrimidin-2-yl)thiazole (compound 200),
4-(2,3-dihydro-1H-pyrrolo[2,3-b]pyridin-5-yl)-2-(pyrazolo[1,5-a]pyridin-3-yl)thiazole (compound 201),
2-(pyrazolo[1,5-a]pyridin-3-yl)-4-(5,6,7,8-tetrahydroimidazo[1,2-a]pyridin-2-yl)thiazole (compound 202),
2-(2-(pyrazolo[1,5-a]pyridin-3-yl)thiazol-4-yl)-5,6-dihydro-8H-imidazo[2,1-c][1,4]oxazine (compound 203),
2-(2-(pyrazolo[1,5-a]pyridin-3-yl)thiazol-4-yl)-4,5,6,7-tetrahydrothiazolo[5,4-c]pyridine (compound 204),
2-(2-(pyrazolo[1,5-a]pyridin-3-yl)thiazol-4-yl)-4,5,6,7-tetrahydrothiazolo[4,5-c]pyridine (compound 205),
2-(pyrazolo[1,5-a]pyridin-3-yl)-4-(4,5,6,7-tetrahydropyrazolo[1,5-a]pyrazin-2-yl)thiazole (compound 206),
4-(1H-indazol-3-yl)-2-(pyrazolo[1,5-a]pyridin-3-yl)thiazole (compound 207),
4-(4-chloro-1H-pyrazol-3-yl)-2-(pyrazolo[1,5-a]pyridin-3-yl)thiazole (compound 208),
2-(6-methylpyrazolo[1,5-a]pyridin-3-yl)-4-phenylthiazole (compound 209),
2-(5-methylpyrazolo[1,5-a]pyridin-3-yl)-4-phenylthiazole (compound 210),
2-(4-methylpyrazolo[1,5-a]pyridin-3-yl)-4-phenylthiazole (compound 211),
4-(4-chloro-1-methyl-1H-pyrazol-3-yl)-2-(5-methylpyrazolo[1,5-a]pyridin-3-yl)thiazole (compound 212), and
N-((5-(2-(5-methylpyrazolo[1,5-a]pyridin-3-yl)thiazol-4-yl)pyridin-2-yl)methyl)acetamide (compound 213).

6. A composition comprising the compound or pharmaceutically acceptable derivative thereof according to any one of claims 1 to 5, and a pharmaceutically acceptable carrier.

7. A pharmaceutical composition comprising the compound or pharmaceutically acceptable derivative thereof according to any one of claims 1 to 5.

8. The pharmaceutical composition according to claim 7 for use in inhibiting myosin light chain kinase 4 (MYLK4) activity.

9. The pharmaceutical composition according to claim 7 for use in alleviating or treating a disease, disorder or condition involving myosin light chain kinase 4 (MYLK4).

10. The pharmaceutical composition according to claim 9, wherein the disease, disorder or condition involving myosin light chain kinase 4 (MYLK4) is arteriosclerosis, inflammatory bowel disease, osteosarcoma, ischemic heart disease, glaucoma, ocular hypertension, impaired aqueous humor outflow, dry eye, uveitis, age-related macular degeneration, or diabetic retinopathy.

11. The pharmaceutical composition according to claim 7 for use in alleviating or treating dry eye, uveitis, age-related macular degeneration, or diabetic retinopathy.

12. The pharmaceutical composition according to claim 7 for use in alleviating or treating glaucoma, ocular hypertension, or impaired aqueous humor outflow.
